(19) Europäisches Patentamt
European Patent Office
Office européen des brevets

(11) Publication number: **0 084 224**
**B1**

(12) # EUROPEAN PATENT SPECIFICATION

(45) Date of publication of patent specification: **08.10.86**

(21) Application number: **82306492.8**

(22) Date of filing: **06.12.82**

(51) Int. Cl.$^4$: **C 07 D 239/42,**
C 07 D 239/47,
C 07 D 251/16,
C 07 D 251/22,
C 07 D 405/04,
C 07 D 405/12,
C 07 D 409/12, A 01 N 47/36

(54) **Herbicidal sulfonamides.**

(30) Priority: **07.12.81 US 328018**
**20.10.82 US 434038**

(43) Date of publication of application:
**27.07.83 Bulletin 83/30**

(45) Publication of the grant of the patent:
**08.10.86 Bulletin 86/41**

(84) Designated Contracting States:
**AT BE CH DE FR IT LI LU NL SE**

(56) References cited:
**EP-A-0 009 419**
**EP-A-0 023 140**
**EP-A-0 044 808**

**JOURNAL OF THE AMERICAN CHEMICAL SOCIETY, vol. 69, 1947, Easton, W. BRAKER et al. "Substituted sulfanilamidopyrimidines", pages 3072-3078**

**The file contains technical information submitted after the application was filed and not included in this specification**

(73) Proprietor: **E.I. DU PONT DE NEMOURS AND COMPANY**
**1007 Market Street**
**Wilmington Delaware 19898 (US)**

(72) Inventor: **Shapiro, Rafael**
**2221-F Prior Road**
**Wilmington Delaware 19809 (US)**

(74) Representative: **Hildyard, Edward Martin et al**
**Frank B. Dehn & Co. Imperial House 15-19 Kingsway**
**London WC2B 6UZ (GB)**

**EP 0 084 224 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Background of the Invention

This invention relates to herbicidal sulfonamides, and, more particularly, to substituted triazinyl or pyrimidinyl sulfonylurea compounds which are selective pre-emergence and/or post-emergence herbicides.

Description of the Prior Art

U.S. Patents 4,127,405 and 4,169,719 disclose herbicidal methoxymethylpyrimidine sulfonylurea compounds of the type which contain a —$CH_2OCH_3$ heterocyclic substituent.

European Patent No. 7687 discloses herbicidal sulfonylurea compounds such as, among others,

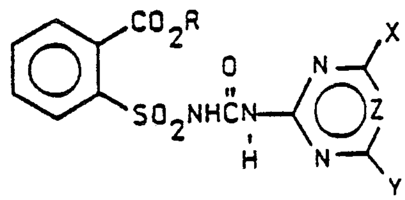

where

X is $CH_3$ or $OCH_3$;

Z is CH or N; and

Y is $C_1$—$C_4$ alkyl substituted with $OCH_3$, $OC_2H_5$, CN, C(O)L, or 1—3 atoms of F, Cl, or Br, where L is $NH_2$, OH, $N(OCH_3)CH_3$, $NH(C_1$—$C_4$ alkyl), $N(C_1$—$C_4$ alkyl$)_2$ or $C_1$—$C_6$ alkoxy.

*J. Am. Chem. Soc., 69,* 3072 (1947) teaches compounds of the formula:

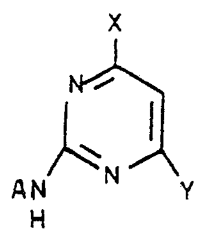

where

A is H or

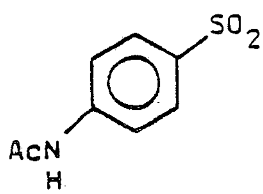
;

X is Cl, $CH_3$, $CH_3O$, $CH_2OCH_3$, or H; and

Y is $CH(OCH_3)_2$ or $CH(OC_2H_5)_2$;

and their use as antibacterial agents.

Sulfonylurea herbicides related in structure to the compounds of the present invention are also disclosed in our EP—A—9,419 and EP—A—23,140. The present compounds in general show a higher herbicidal activity than the compounds of the two aforesaid specifications.

Summary of the Invention

This invention relates to novel compounds of Formula I, suitable agricultural compositions containing them, and their method of use as general and/or selective pre-emergence and/or post-emergence herbicides or plant growth regulants.

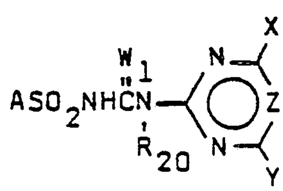

I

wherein

X is $CH_3$, $OCH_3$, Cl, $C_2H_5$ or $OC_2H_5$;

Z is CH or N;

Y is $CH_2OR$, $CH_2SR_1$, $CH(OR_1)_2$,

$OCH_2OR_1$, $OCH(OCH_3)—CO_2R_3$ or 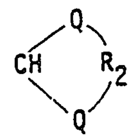

R is H, $C(O)R_3$, $CO_2R_4$, $C(O)NR_5R_6$ or 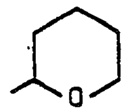 ;

$R_1$ is $C_1—C_2$ alkyl;

$R_2$ is $—CH_2CH_2—$, $—CH(CH_3)CH_2—$ or $—CH_2CH_2CH_2—$;

$R_3$ is $C_1—C_3$ alkyl;

$R_4$ is $C_1—C_3$ alkyl;

$R_5$ is H or $CH_3$;

$R_6$ is $C_1—C_3$ alkyl;

Q is O or S;

A is

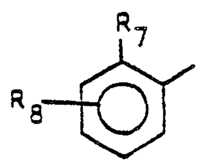 , 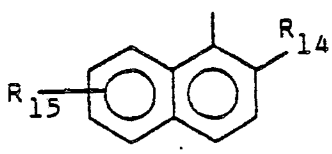 , 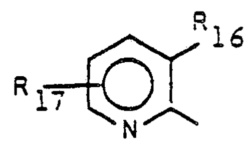 ,

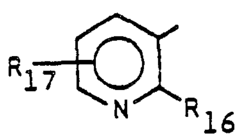 , 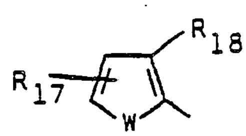 , 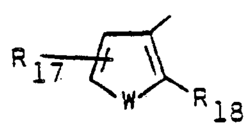 ,

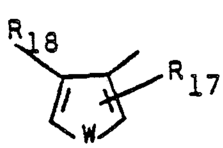 , 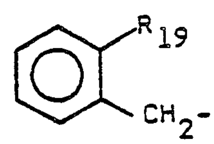 or 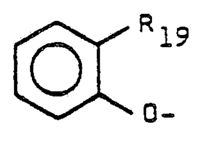 ;

$R_7$ is H, $C_1—C_4$ alkyl, $C_1—C_4$ alkoxy, F, Cl, Br, $NO_2$, $CF_3$, $CO_2R_9$, $SO_2NR_{10}R_{11}$, $SO2N(OCH_3)CH_3$, $OSO_2R_{12}$, $S(O)_nR_{13}$, $LCF_3$, $LCHF_2$, $LCF_2CF_2H$, $C_3—C_4$ alkenyloxy or $C_1—C_2$ alkyl substituted with $OCH_3$ or $OC_2H_5$;

$R_8$ is H, F, Cl, Br, $CF_3$, $NO_2$, $C_1—C_3$ alkyl or $C_1—C_3$ alkoxy;

$R_9$ is $C_1—C_4$ alkyl, $CH_2CH_2OCH_3$, $CH_2CH_2Cl$ or $CH_2CH{=}CH_2$;

$R_{10}$ and $R_{11}$ are independently $C_1—C_3$ alkyl;

$R_{12}$ is $C_1—C_4$ alkyl, $CH_2CH_2OCH_3$, $CH_2CH_2CH_2OCH_3$ or $C_1—C_4$ alkyl substituted with 1—3 atoms of F, Cl or Br;

$R_{13}$ is $C_1—C_4$ alkyl or $CH_2CH{=}CH_2$;

n is 0 or 2;

L is O, S or $SO_2$;

$R_{14}$ is H, $CH_3$, $OCH_3$, F, Cl, Br, $NO_2$, $SO_2NR_{10}R_{11}$, $OSO_2R_{12}$ or $S(O)_nR_{13}$;

$R_{15}$ is H, Cl, Br, $CH_3$, $OCH_3$ or $NO_2$;

$R_{16}$ is H, $C_1—C_4$ alkyl, $C_1—C_4$ alkoxy, F, Cl, Br, $CF_3$, $CO_2R_9$, $SO_2NR_{10}R_{11}$, $SO_2N(OCH_3)CH_3$ or $S(O)_nR_{13}$;

$R_{17}$ is H, F, Cl, Br, $CH_3$ or $OCH_3$;

W is O or S;

$W_1$ is O or S;

$R_{18}$ is H, $C_1—C_4$ alkyl, $C_1—C_4$ alkoxy, F, Cl, Br, $NO_2$, $CO_2R_9$, $SO_2NR_{10}R_{11}$, $SO_2N(OCH_3)CH_3$ or $S(O)_nR_{13}$;

$R_{19}$ is Cl, $NO_2$, $CF_3$, $CO_2R_9$, $SO_2N(OCH_3)CH_3$, $SO_2NR_{10}R_{11}$, $OSO_2R_{12}$, $S(O)_nR_{13}$ or $C_1—C_3$ alkoxy optionally substituted with 1—5 atoms of Cl or F; and

$R_{20}$ is H or $CH_3$;

and their agriculturally suitable salts; provided that

(1) the total number of carbon atoms of $R_{10}$ and $R_{11}$ is less than or equal to 4;

(2) when X is Cl, then Z is CH;

(3) when W is O, then $R_{18}$ is H, Cl, Br, $CH_3$ or $CO_2R_9$;

(4) when W is O and $R_{18}$ is H, Cl, Br, or $CH_3$, then A is

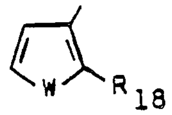 ;

(5) when $W_1$ is S, then $R_{20}$ is H and A is

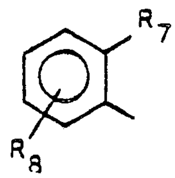 ;

(6) when Y is $OCH_2OR_1$ or $OCH(OCH_3)—CO_2R_3$,

then Z is CH;

(7) when $R_7$ is $CH_2CH_2OCH_3$ or $CH_2CH_2OCH_2CH_3$ and $R_8$ is H, F, Cl, $CH_3$, $OCH_3$ or $CF_3$ then Y is other than $CH(OCH_3)$ or

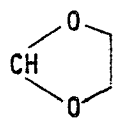

and

(8) when $R_7$ is $C_3$ alkenyloxy and $R_8$ is H, F, Cl, Br, $CH_3$, $OCH_3$ or $CF_3$, then Y is other than $CH(OCH_3)_2$ or

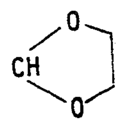

Preferred for their higher herbicidal activity and/or more favorable ease of synthesis are:

(1) Compounds of *Formula I* where $W_1$ is O, X is $CH_3$ or $OCH_3$ and $R_{20}$ is H;

(2) Compounds of *Preferred I* where Y is $CH(OR_1)_2$ or

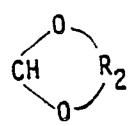

(3) Compounds of *Preferred (2)* wherein A is

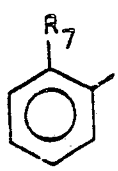 , 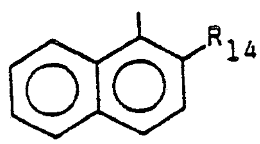 , 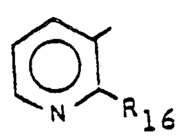 ,

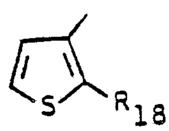 or 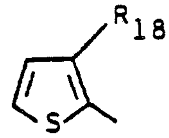 ;

$R_7$ is $C_1$—$C_3$ alkyl, $C_1$—$C_3$ alkoxy, Cl, $NO_2$, $CO_2R_9$, $SO_2(C_1$—$C_3$ alkyl), $SO_2NR_{10}R_{11}$, $SO_2N(OCH_3)CH_3$, $OSO_2R_{13}$, $CH_2OCH_3$ or $LCF_2H$;

$R_{14}$ is $CH_3$, $OCH_3$, Cl, $OSO_2CH_3$ or $SO_2(C_1$—$C_3$ alkyl);

$R_{16}$ is $CH_3$, $OCH_3$, Cl, $SO_2CH_3$ or $SO_2N(CH_3)_2$;

$R_{18}$ is $CH_3$, Cl, Br, $CO_2R_9$ or $SO_2CH_3$; and

(4) Compounds of *Preferred (3)* where A is

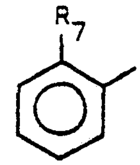

Specifically preferred are:

3-[[(4-dimethoxymethyl-6-methylpyrimidin-2-yl)aminocarbonyl]aminosulfonyl]-2-thiophenecarboxylic acid, methyl ester:

2-chloro-N-[(4-dimethoxymethyl-6-methylpyrimidin-2-yl)aminocarbonyl]benzenesulfonamide;

2-[[(4-dimethoxymethyl-6-methylpyrimidin-2-yl)aminocarbonyl]aminosulfonyl]benzoic acid, methyl ester;

N-[(4-dimethoxymethyl-6-methylpyrimidin-2-yl)aminocarbonyl]-2-methylsulfonylbenzenesulfonamide;

N-[(4-dimethoxymethyl-6-methylpyrimidin-2-yl)aminocarbonyl]-2-nitrobenzenesulfonamide;

2-[[(4-dimethoxymethyl-6-methylpyrimidin-2-yl)aminocarbonyl]aminosulfonyl]benzoic acid, 2-propyl ester;

2-[[[4-methyl-6-(tetrahydropyran-2-yloxymethyl)pyrimidin-2-yl]aminocarbonyl]aminosulfonyl]benzoic acid, methyl ester;

2-[[[4-(hydroxymethyl)-6-methylpyrimidin-2-yl]aminocarbonyl]aminosulfonyl]benzoic acid, methyl ester; and

2-[[[4-(acetyloxymethyl)-6-methylpyrimidin-2-yl]aminocarbonyl]aminosulfonyl]benzoic acid, methyl ester.

Detailed Description of the Invention

*Synthesis*

The compounds of Formula I may be prepared by the reaction of an appropriately substituted sulfonyl isocyanate of Formula II with an appropriate 2-amino-heterocycle of Formula III according to Equation 1:

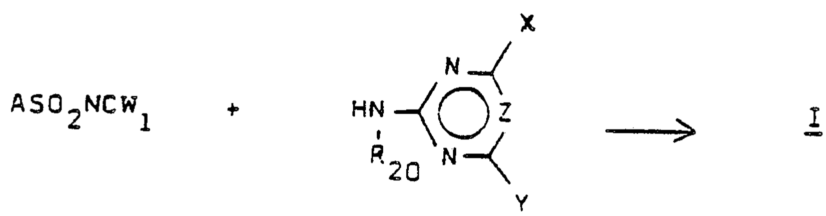

(II) (III)

wherein

A, X, $R_{20}$, W and Z are as previously defined;

Y is $CH_2OR$, $CH_2SR_1$, $CH(OR_1)_2$ or $CH(QR_2Q)$;

R is

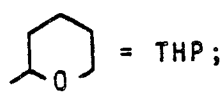

and

$R_1$ and $R_2$ are as previously defined.

The reaction of Equation 1 is best carried out in an inert aprotic solvent such as methylene chloride, tetrahydofuran, acetonitrile at a temperature between 0° and reflux, preferably between 20 and 80°. A catalytic amount of DABCO (1,4-diazabicyclo[2.2.2]octane) may be used to accelerate the reaction. In the cases in which the products are insoluble in the reaction solvent, they may be isolated by simple filtration. When the products are soluble in the reaction solvent they are isolated by evaporation of the solvent and trituration of the residue with solvents such as 1-chlorobutane, ethyl ether or methanol and filtration.

Preparation of isocyanates and isothiocyanates (II) is described in the following: *U.S.* 4,127,405, *U.S.* 4,169,719, EP—A—7,687, EP—A—13,480, EP—A—23,141, EP—A—23,422, EP—A—30,141, and GB—A—2,065,116 to which the reader is referred for further information. Furthermore, the sulfonyl isocyanates of Formula IIa, which are disclosed in our EP—A—44,212, may be prepared analogously to the teachings of U.S. 4,127,405.

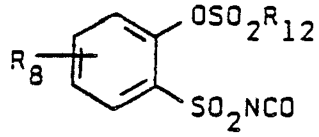

(IIa)

wherein

$R_8$ and $R_{12}$ are as previously defined.

Certain compounds of Formula III are known in the art. For example, the compounds IIIa shown below:

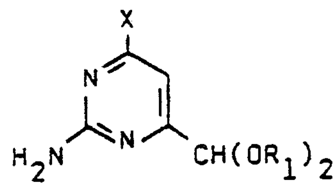

(IIIa)

wherein

X = $CH_3$, Cl or $OCH_3$; and

$R_1$ = $CH_3$ or $C_2H_5$; are described by W. Braker, et al., *J. Amer. Chem. Soc., 69,* 3072 (1947), to which the reader is referred for further information.

Triazines of Formula IIIb may be prepared according to the methods outlined in Equations 2 and 3.

Equation 2

(2a)

(2b)

(2c)

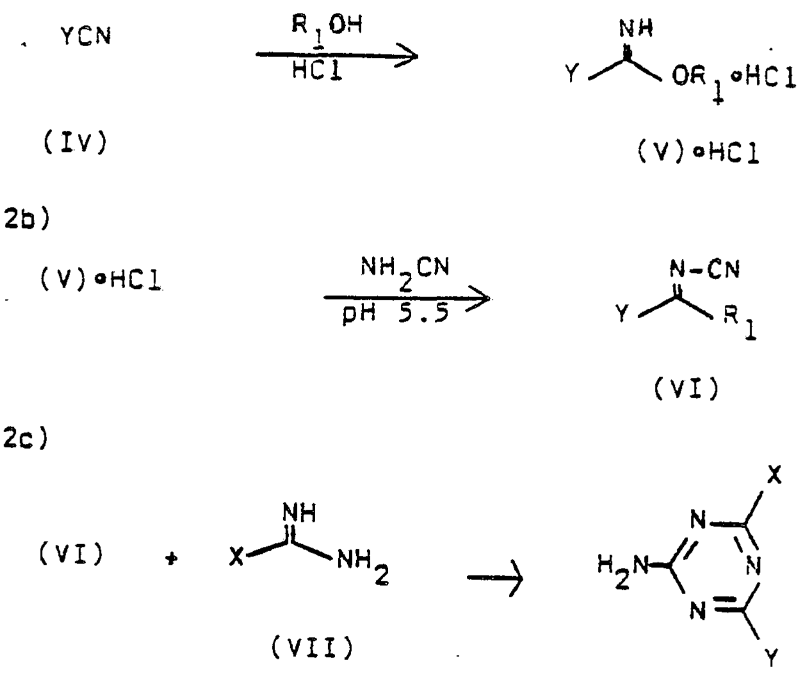


(IV) (V)·HCl (VI) (VII) (IIIb)

wherein

Y = $CH(OR_1)_2$ or $CH_2SR_1$;

$R_1$ = $CH_3$ or $C_2H_5$; and

X = $R_1$ or $OR_1$.

The reaction of Equation 2a is carried out according to the teachings of J. M. McElvain and R. L. Clarke, *J. Amer. Chem. Soc., 69,* 2657 (1947), in which the preparation of ethyl diethoxyiminoacetate is described. The intermediate N-cyanoimidates of Formula VI may be prepared according to the teaching of D. Lwowski in *Synthesis, 1971,* 263, by reacting V with cyanamide at pH 5.5, and these may be condensed according to reaction 2c with the appropriate compound of Formula VII in an alcoholic solvent at 25 to 80°C to provide the appropriate triazines. Alternatively, the reaction of Equation 3a, described for substituted acetonitriles by F. C. Schaefer and G. A. Peters in *J. Org. Chem., 26,* 412 (1961), may be used to convert nitriles of Formula IV to the corresponding iminoesters. The free base may be carried on through reactions 2b and 2c,

or, alternatively, converted to the amidinium hydrochloride salts (VIII) as described in the aforementioned reference. The compounds of Formula VIII may be converted to the compounds IIIb by the reaction of Equation 3c, in which VIII is contacted with a compound of Formula IX at 0 to 30°C in the presence of 3-4 equivalents of the appropriate sodium alkoxide in the appropriate alcohol. The product may be isolated by evaporation of solvent, neutralization with aqueous acetic acid, and filtration.

Equation 3

(3a)

(IV)

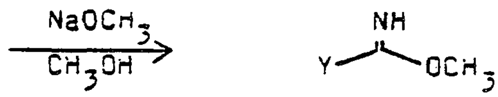


(V)

(3b)

(V)

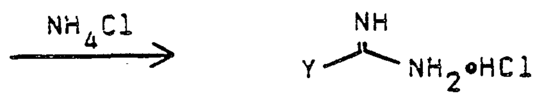


(VIII)

(3c)

(VIII) +

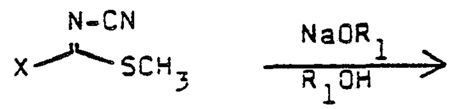


(IIIb)

(IX)

wherein
Y is $CH(OR_1)_2$, $CH_2SR_1$, $CH_2OTHP$ or $CH(OR_2O)$;
$R_1$ is $CH_3$ or $C_2H_5$; and
X is $R_1$ or $SCH_3$.
The compounds of Formula IIIc may be prepared by the sequence of reactions outlined in Equation 4.

Equation 4

(4a)

$YCO_2CH_3$

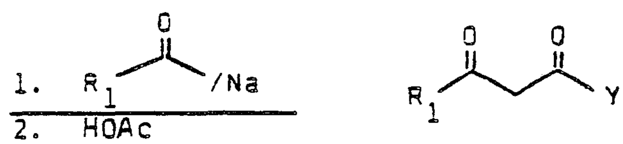


(X)

(XI)

(4b)

(XI)

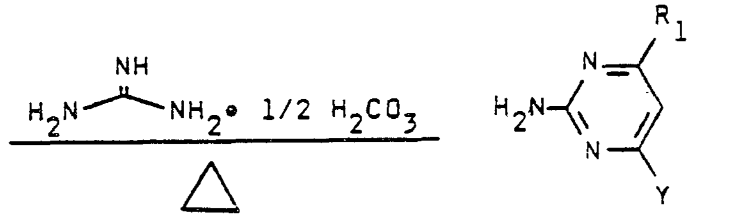


(IIIc)

wherein
Y is $CH(OR_1)_2$, $CH(OR_2O)$, $CH_2SR_1$ or $CH_2OTHP$; and
$R_1$ and $R_2$ are as previously defined.
The reactions of Equations 4a and 4b are carried out as described by W. Braker, *et al., loc. cit.*

The compounds of Formula IIId may be prepared by similar methods shown in Equation 5.

Equation 5

(5a)

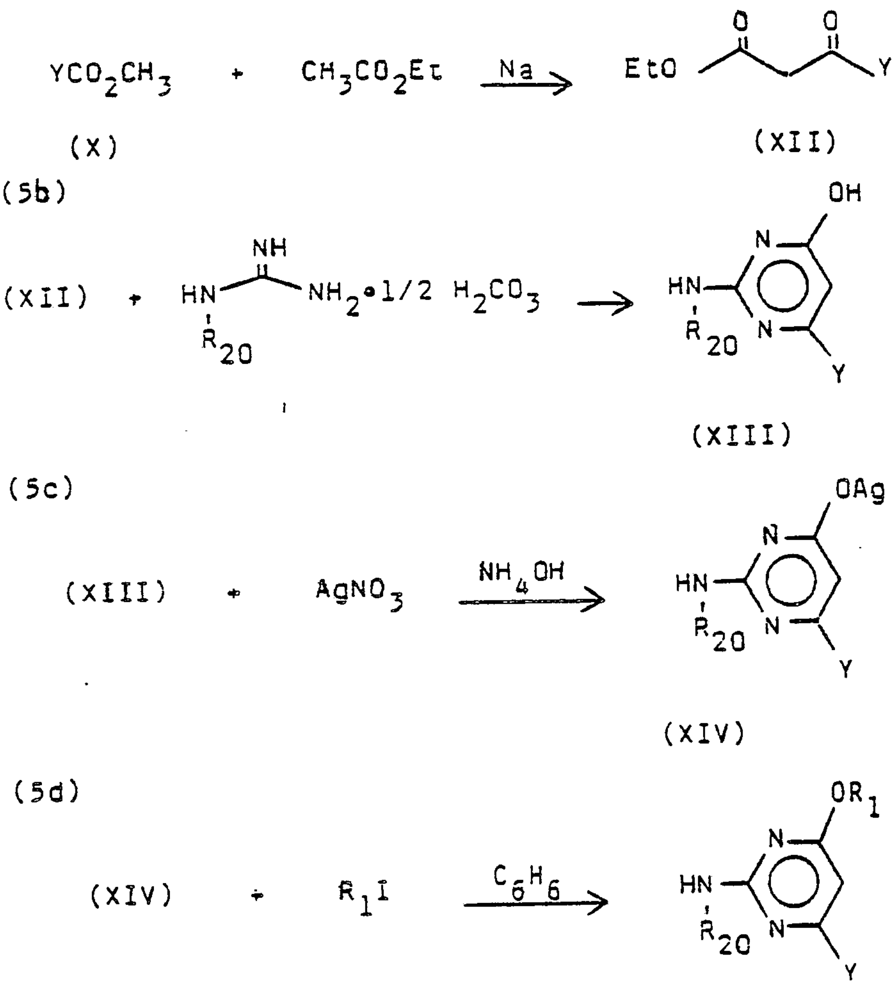


(X) (XII)

(5b) (XIII)

(5c) (XIV)

(5d) (IIId)

wherein

$R_{20}$ is H or $CH_3$; and

$R_1$ and Y are as defined in Equation 4.

The reactions of Equations 5a and 5b are conducted as described for 2-amino-4-hydroxy-6-dimethoxymethylpyrimidine (J. I. De Graw and V. H. Brown, *J. Het. Chem.*, *13*, 439 (1976)). The silver salt of XIII may be prepared by addition of ammonia to an aqueous solution of XIII and an equivalent amount of silver nitrate. The dried silver salt may be contacted with one equivalent of methyl or ethyl iodide in a nonpolar solvent such as benzene at 25 to 80°C to provide pyrimidines of Formula IIId after purification by column chromatography or recrystallization.

Alternatively, the compounds of Formula IIId may be prepared *via* the corresponding chloropyrimidines (IIIe). These may be obtained by the reaction of Equation 6a, in which a compound of Formula XIII is contacted with 2—5 equivalents of phosphorous oxychloride at 20—40°C for 3—30 hours. The product is isolated by removal of excess $POCl_3$ *in vacuo,* treatment of the residue with ice-water, extraction into an organic solvent concentration, crystallization or column chromatography. The chloropyrimidines may then be converted to the compounds of Formula IIId by contacting with one equivalent of the appropriate sodium alkoxide in alcohol at 0—30°C followed by concentration and precipitation with ice-water.

Equation 6

(6a)

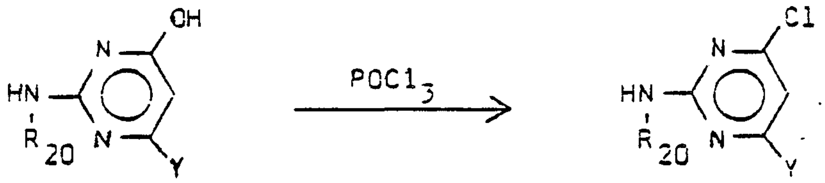


(6b)

(IIIe)

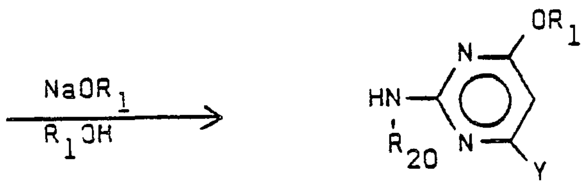


(IIId)

wherein

Y is $CH(OR_1)_2$, $CH_2SR_1$ or $CH(QR_2Q)$;

$R_{20}$ is H or $CH_3$; and

$R_1$ is $CH_3$ or $C_2H_5$.

Cyclic acetals of Formula IIIe may be prepared from compounds of Formula IIIa according to Equation 7 by acetal exchange.

Equation 7

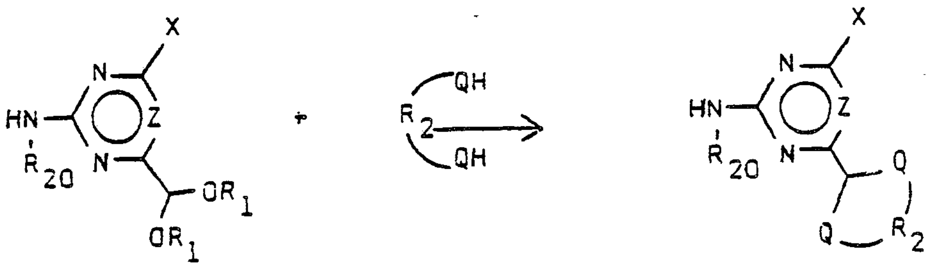

(IIIa) (XV) (IIIe)

wherein

X, Q, Z, $R_1$ and $R_2$ are as previously defined.

The reaction of Equation 7 is carried out by heating the acyclic acetal in an inert solvent in the presence of one equivalent of a compound of Formula XV and slightly more than one equivalent of an acidic catalyst, such as *p*-toluenesulfonic acid or boron trifluoride etherate with distillation of the by-product alcohol. The compound of Formula IIIe may be isolated by extraction with an organic solvent, and purified by crystallization or column chromatography.

The synthesis of N-methyl compounds of Formula IIIg may be accomplished by the procedure shown in Equation 8.

Equation 8

(8a)

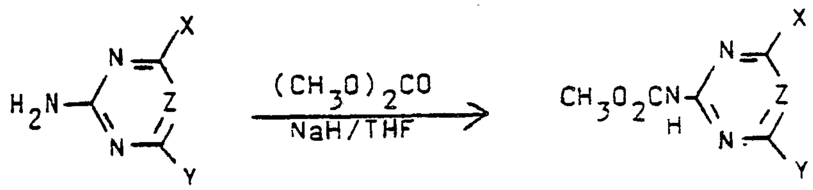


(IIIf) (XVI)

(8b)

(XVI)

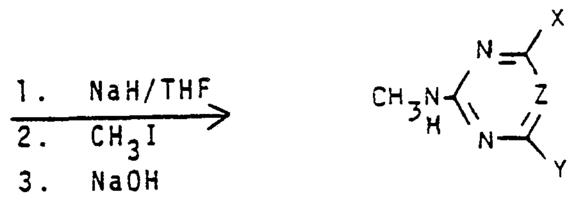


(IIIg)

wherein

X is $R_1$ or $OR_1$;

Y is $CH(OR_1)_2$, $CH(QR_2Q)$, $CH_2OR_1$ or $CH_2SR_1$; and

$R_1$, Z and Q are as previously defined.

The reaction of Equation 8a may be carried out by stirring a mixture of IIIf with one equivalent each of sodium hydride and dimethyl carbonate for 1—3 days in an inert solvent such as tetrahydrofuran or dimethylformamide. The product of Formula XVI may be isolated by evaporation of solvent, neutralization with aqueous acetic acid, and filtration of the product. The reaction of Equation 8b is performed by adding XVI to a slurry of one equivalent of sodium hydride in tetrahydrofuran at 0—30°, stirring for 1/2 hour, and adding methyl iodide, heating to reflux for 1-20 hours, and stirring with water and aqueous sodium hydroxide for 1—20 hours at 20—30°. The product is isolated by evaporating the solvent, adding ice-water, and filtering.

The compounds of Formulae IIIh and IIIi may be prepared by the reactions of Equation 9, which are carried out by analogy with the procedures described for the reactions of Equations 5c and 5d.

Equation 9

(9a)

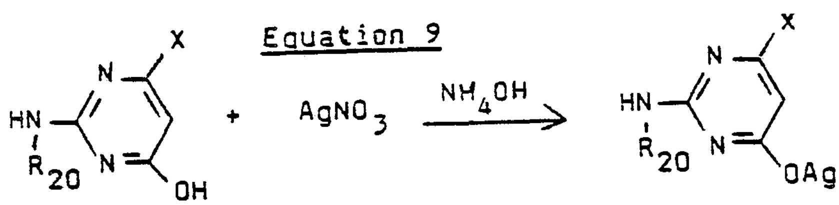


(XVII) (XVIII)

(9b) XVIII

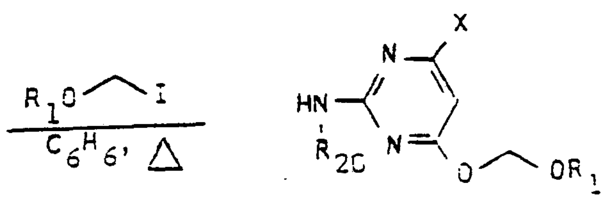


(IIIh)

(9c) XVIII +

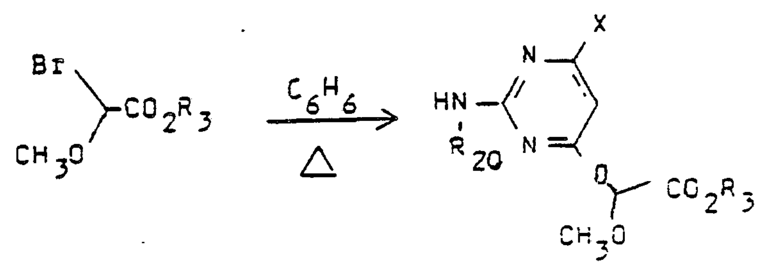


wherein X, $R_1$, $R_3$, and $R_{20}$ are as previously defined. (IIIi)

Compounds of Formula Ib may be prepared by acidic hydrolysis of compounds of Formula Ia according to Equation 10:

Equation 10

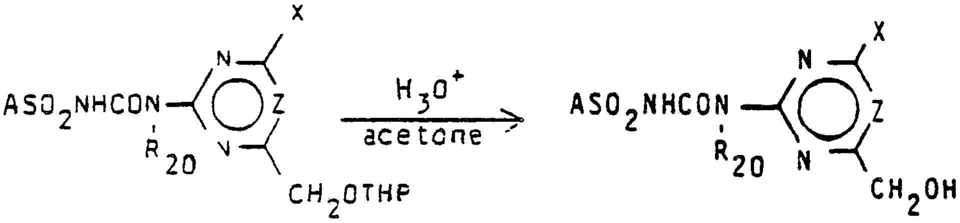


(Ia) (Ib)

wherein

A, X, $R_{20}$ and Z are as previously defined.

The reaction of Equation 10 is preferably carried out by stirring a solution or suspension of Ia in a polar protic solvent, e.g. aqueous acetone, containing a catalytic amount of a strong mineral acid, such as hydrochloric or sulfuric acid, at 0 to 30°C, followed by concentration and filtration of the product. The reaction will generally be complete in from 1 to 168 hours.

The compounds of Formula Ib may be in turn converted to compounds of Formulae Ic, Id, or Ie according to Equation 11;

(11a) Equation 11

(Ib)

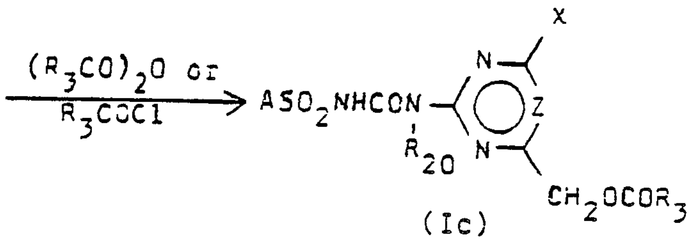


(Ic)

(11b)

(Ib)

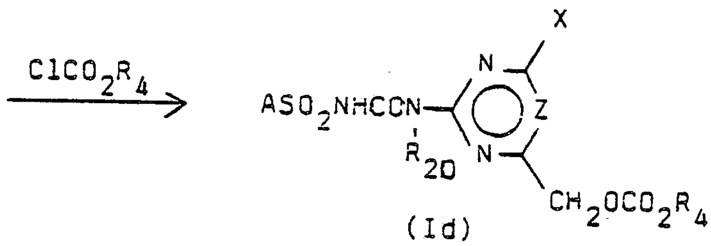


(Id)

(11c)

(Ib)

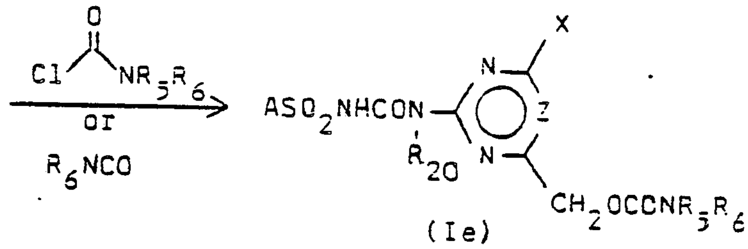


(Ie)

wherein

A, X, Z, $R_3$, $R_4$, $R_5$, $R_6$ and $R_{20}$ are as previously defined.

The reaction's of Equation 11 are carried out by contacting a compound of Formula Ib with at least one equivalent of an alkyl carboxylic anhydride, carbonyl chloride, chloroformate or carbamoyl chloride in an inert solvent in the presence of an excess of an acid acceptor such as pyridine, triethylamine, or aqueous sodium carbonate, followed by isolation of the product by acidification and crystallization or extraction into an organic solvent. Alternatively, compounds of Formula Ie wherein $R_5$ = H may be prepared by contacting the appropriate compound of Formula Ib with one equivalent of an appropriate alkyl isocyanate in an inert

solvent and filtration of the product. All these reactions may e.g. be performed at a temperature from 0° to reflux.

Certain compounds of Formula I may also be prepared by the reactions of Equation 12.

(12a) Equation 12

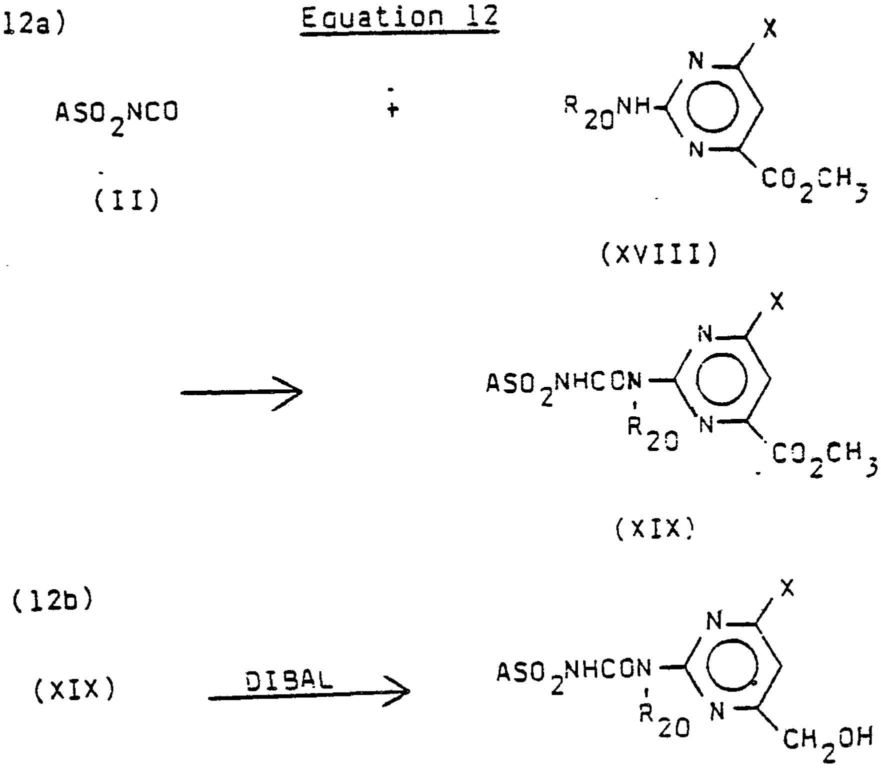


(II) (XVIII) (XIX)

(12b) (XIX)

(If)

wherein

$R_{20}$ X and A are as previously defined, provided that $R_7$, $R_{16}$, $R_{18}$ and $R_{19}$ are not $CO_2R_9$.

The reaction of Equation 12a is conducted in the same manner as described for Equation 1. The resulting compounds of Formula XIX may be converted to compounds of Formula If by contacting them with 3—5 equivalents of diisobutyl aluminum hydride in an ethereal solvent at −20 to 20°, after which the reaction mixture is quenched with aqueous mineral acid and extracted with an organic solvent. The product may be isolated by evaporation of solvent and crystallization.

Equation 13 outlines the preparation of the compounds of Formula XVIII.

Equation 13

(13a)

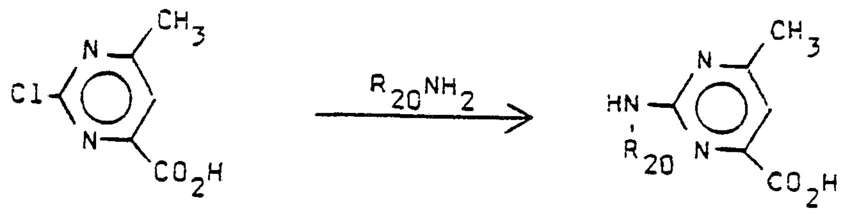


(XX) (XXI)

(13b)

(XXI)

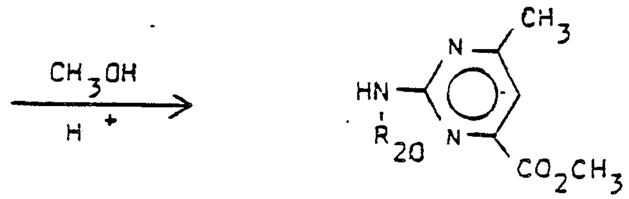


(XVIIIa)

(13c)

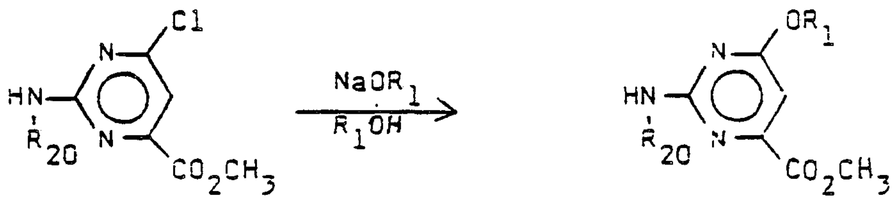


(XVIIIb) (XVIIIc)

wherein

$R_1$ and $R_{20}$ are as previously defined.

The pyrimidine of Formula XX is described by Z. Buděšinsky and F. Roubínek, in *Coll. Czech. Chem. Comm., 26,* 2871 (1961). This may be heated in an autoclave at 80 to 140° with ammonia or methyl amine to afford the amino acids of Formula XXI. These may be esterified in the usual manner with methanol and acid, followed by evaporation of solvent, neutralization and filtration to afford the compounds of Formula XVIIIa.

The preparation of XVIIIb ($R_{20}$=H) is reported in *J. Org. Chem., 26,* 2755, by G. Davies, *et al.,* and the N-methyl compounds ($R_{20}$=$CH_3$) may be prepared in an analogous fashion. The reaction of Equation 12b is conducted according to the procedure described for Equation 6b.

Example 1

2-Amino-4-(dimethoxymethyl)-6-methylpyrimidine

To 6.9 g of sodium pellets in 120 ml of toluene containing 50 g of methyl dimethoxyacetate was added 20 g of acetone dropwise over 30 minutes at 0—20°. The mixture was stirred overnight at room temperature, concentrated at reduced pressure, and the product was precipitated with ether and filtered. Ten ml of acetic acid in ice-water was added, and the β-diketone was extracted with methylene chloride. The residue from concentration of the organic extract was heated with 25 g of guanidine carbonate at 80—90° for 30 minutes, cooled, digested with methylene chloride, filtered, dried with sodium sulfate, filtered, concentrated at reduced pressure and triturated with petroleum ether to provide 11 g of 2-amino-4-dimethoxymethyl-6-methylpyrimidine, m.p. 69—71° (lit, m.p. 71—74°).

NMR ($CDCl_3$)δ: 2.3 (s, 3), 3.3 (s, 6), 5.0 (s, 1), 5.9 (br s, 2), 6.7 (s, 1).

Example 2

2-Chloro-N-[[4-dimethoxymethyl)-6-methylpyrimidin-2-yl]aminocarbonyl]benzenesulfonamide

A solution of 0.3 g of 2-amino-4-dimethoxymethyl-6-methylpyrimidine in 50 ml of ether was stirred overnight at room temperature with 0.46 g of *o*-chlorobenzenesulfonyl isocyanate. The product was filtered and washed with hexane to provide 0.5 g of the title compound, m.p. 150—153°.

NMR ($CDCl_3$)δ: 2.5 (s, 3), 3.4 (s, 6), 5.2 (s, 1), 7.1 (s, 1), 7.4 (m, 3), 7.9 (br s, 1), 8.3 (m, 1).

Example 3

2-Amino-4-methyl-6-(tetrahydropyran-2-yloxymethyl)-pyrimidine

A solution containing 30 g of methyl glycolate, 33 g of dihydropyran, and 0.2 g of *p*-toluenesulfonic acid in 100 ml of toluene was stirred at room temperature overnight. After cooling to −5°, 6.8 g of sodium pellets was added, followed by 24 ml of acetone at −5 to 8°. Stirring was continued for 20 hours at room temperature, after which time hexane was added, and the solution was poured into ice-water and partitioned. The aqueous phase was washed with ether and acidified with 20 ml of acetic acid, extracted with 600 ml of methylene chloride, the organic layer was dried ($MgSO_4$) and concentrated, and the residue was heated at 80—90° with 28 g of guanidine carbonate for 1 hour. Addition of ice-water to the cooled reaction mixture precipitated the product, which was filtered and dried to afford the title compound, m.p. 109—110.5°.

NMR ($CDCl_3$)δ: 1.7 (br s, 6), 2.3 (s, 3), 3.6 (br m, 2), 4.5 (d, 2), 4.7 (m, l), 5.4 (br s, 2), 6.6 (br s, 1).

Example 4

Methyl 2-[[[4-methyl-6-(tetrahydropyran-2-yloxymethyl)-pyrimidin-2-yl]aminocarbonyl]aminosulfonyl]benzoate

To a solution of 3.25 g of the product from Example 3 in 50 ml of ether was added 3.75 g of 2-methoxycarbonylbenzenesulfonyl isocyanate and the mixture was stirred for 3 hours under nitrogen. The precipitated product was filtered and washed with ether to afford 6.0 g of the title compound, m.p. 126—128°(d).

Example 5

Methyl [[[4-(hydroxymethyl)-6-methylpyrimidin-2-yl]-aminocarbonyl]aminosulfonyl]benzoate

A solution of 6 g of the product prepared in Example 4 in 50 ml of acetone was stirred with 3 ml of 1N aqueous HCl for 3 days at room temperature, concentrated at reduced pressure, and the residue was thoroughly washed with water to provide 4 g of the title compound, m.p. 140—141°.

NMR ($CDCl_3$ + DMSO-$d_6$)δ: 2.5 (s, 3), 3.9 (s, 3), 4.6 (br, s, 2), 7.0 (br s, 1), 7.6 (m, 3), 8.3 (m, 1), 9.6 (b r s, 1).

Example 6

Methyl [[[4-(acetoxymethyl)-6-methylpyrimidin-2-yl]-aminocarbonyl]aminosulfonyl]ben zoate

To 0.5 g of the compound from Example 5 was added 1 ml of acetic anhydride and 1 ml of pyridine. After 1 hour at room temperature, ice-water was added, the precipitated oil was triturated, filtered, and washed with water to afford 0.4 g of the title compound, m.p. 145—150°.

NMR ($CDCl_3$)δ: 2.2 (s, 3), 2.6 (s, 3), 3.9 (s, 3), 5.2 (s, 2), 6.9 (s, 1), 7.7 (m, 3), 8.3 (br s, 1), 8.4 (m, 1), 13.1 (br s, 1).

TABLE 1a

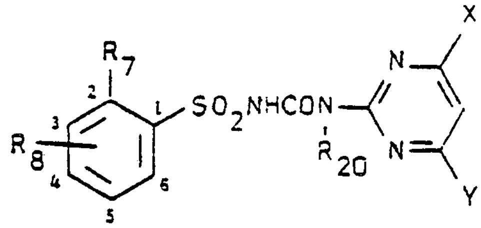

| $R_7$ | $R_8$ | $R_{20}$ | X | Y | m.p.(°C) |
|---|---|---|---|---|---|
| Cl | H | H | $CH_3$ | $CH(OCH_3)_2$ | 150—153°C |
| $CO_2CH_3$ | H | H | $CH_3$ | $CH(OCH_3)_2$ | 121—125° |
| $SO_2CH_3$ | H | H | $CH_3$ | $CH(OCH_3)_2$ | 152—155° |
| $NO_2$ | H | H | $CH_3$ | $CH(OCH_3)_2$ | 144—152° |
| $CO_2CH(CH_3)_2$ | H | H | $CH_3$ | $CH(OCH_3)_2$ | 73—79° |
| $CO_2C_2H_5$ | H | H | $CH_3$ | $CH(OCH_3)_2$ | |
| $CF_3$ | H | H | $CH_3$ | $CH(OCH_3)_2$ | |
| Br | H | H | $CH_3$ | $CH(OCH_3)_2$ | |
| $CH_3$ | H | H | $CH_3$ | $CH(OCH_3)_2$ | |
| $C_2H_5$ | H | H | $CH_3$ | $CH(OCH_3)_2$ | |
| $SO_2$-*n*-$C_3H_7$ | H | H | $CH_3$ | $CH(OCH_3)_2$ | 135—145°d |
| $SO_2N(CH_3)_2$ | H | H | $CH_2CH_3$ | $CH(OCH_3)_2$ | 146—157° |
| $OCH_3$ | H | H | $CH_3$ | $CH(OCH_3)_2$ | |
| $CO_2CH_2CH{=}CH_2$ | H | H | $CH_3$ | $CH(OCH_3)_2$ | |
| $OCF_2CF_2H$ | H | H | $CH_3$ | $CH(OCH_3)_2$ | |
| $CH_2OCH_3$ | H | H | $CH_2CH_3$ | $CH(OCH_3)_2$ | |
| $OSO_2CHCl_2$ | H | H | $CH_3$ | $CH(OCH_3)_2$ | |
| $SC_2H_5$ | H | H | $CH_2CH_3$ | $CH(OCH_3)_2$ | |
| $OSO_2CH_3$ | H | H | $CH_3$ | $CH(OCH_3)_2$ | |
| $OSO_2C_2H_5$ | H | H | $CH_3$ | $CH(OCH_3)_2$ | |
| $CO_2CH_2CH_2OCH_3$ | H | H | $CH_3$ | $CH(OCH_3)_2$ | |
| $SO_2C_2H_5$ | H | H | $CH_3$ | $CH(OCH_3)_2$ | |
| $NO_2$ | 5-$CF_3$ | H | $CH_2CH_3$ | $CH(OCH_3)_2$ | |
| $SO_2$-*n*-$C_3H_7$ | 3-Cl | H | $CH_3$ | $CH(OCH_3)_2$ | |
| $OC_2H_5$ | 5-$NO_2$ | H | $CH_3$ | $CH(OCH_3)_2$ | |
| $SO_2N(CH_3)C_2H_5$ | 6-$NO_2$ | H | $CH_3$ | $CH(OCH_3)_2$ | |
| Cl | 5-Cl | H | $CH_3$ | $CH(OCH_3)_2$ | |

TABLE 1a (continued)

| $R_7$ | $R_8$ | $R_{20}$ | X | Y | m.p. (°C) |
|---|---|---|---|---|---|
| $SO_2CH_3$ | 5-Cl | H | $CH_3$ | $CH(OCH_3)_2$ | |
| $CO_2CH_3$ | 4-F | H | $CH_3$ | $CH(OCH_3)_2$ | |
| $CH(OCH_3)CH_3$ | H | H | $CH_3$ | $CH(OCH_3)_2$ | |
| $SO_2CF_3$ | H | H | $CH_3$ | $CH(OCH_3)_2$ | |
| $SCHF_2$ | H | H | $CH_3$ | $CH(OCH_3)_2$ | |
| $OCH_2CH{=}CHCH_3$ | H | H | $CH_3$ | $CH(OCH_3)_2$ | |
| Cl | H | $CH_3$ | $CH_3$ | $CH(OCH_3)_2$ | |
| $CO_2CH_3$ | H | $CH_3$ | $CH_3$ | $CH(OCH_3)_2$ | |
| $CO_2CH_2CH_3$ | H | $CH_3$ | $CH_3$ | $CH(OCH_3)_2$ | |
| $SO_2CH_3$ | H | $CH_3$ | $CH_3$ | $CH(OCH_3)_2$ | |
| $SO_2N(CH_3)_2$ | H | $CH_3$ | $CH_3$ | $CH(OCH_3)_2$ | |
| $CH(CH_3)_2$ | H | $CH_3$ | $CH_3$ | $CH(OCH_3)_2$ | |
| $OCH_3$ | H | $CH_3$ | $CH_3$ | $CH(OCH_3)_2$ | |
| $SCH_3$ | H | $CH_3$ | $CH_3$ | $CH(OCH_3)_2$ | |
| $SO_2$-*n*-$C_3H_7$ | H | $CH_3$ | $CH_3$ | $CH(OCH_3)_2$ | |
| *n*-$C_4H_9$ | H | $CH_3$ | $CH_3$ | $CH(OCH_3)_2$ | |
| O-*n*-$C_4H_9$ | H | $CH_3$ | $CH_3$ | $CH(OCH_3)_2$ | |
| $NO_2$ | H | $CH_3$ | $CH_3$ | $CH(OCH_3)_2$ | |
| $CO_2$-*n*-$C_4H_9$ | H | $CH_3$ | $CH_3$ | $CH(OCH_3)_2$ | |

TABLE 1b

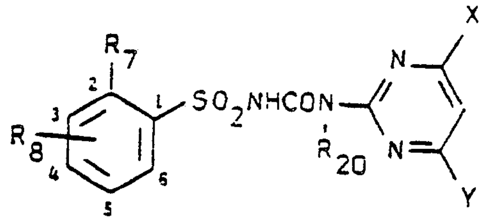

| $R_7$ | $R_8$ | $R_{20}$ | X | Y | m.p.(°C) |
|---|---|---|---|---|---|
| $NO_2$ | H | H | $CH_3$ | $CH_2OTHP$ | 129—132° |
| Br | H | H | $CH_3$ | $CH_2OTHP$ | |
| Cl | H | H | $CH_3$ | $CH_2OTHP$ | 148—150° |
| $SO_2CH_3$ | H | H | $CH_3$ | $CH_2OTHP$ | 161—165° |
| F | H | H | $CH_3$ | $CH_2OTHP$ | |
| H | H | H | $CH_3$ | $CH_2OTHP$ | |
| $CO_2CH_3$ | H | H | $CH_3$ | $CH_2OTHP$ | 126—128° |
| $CH_3$ | H | H | $CH_3$ | $CH_2OTHP$ | |
| $SO_2N(CH_3)_2$ | H | H | $CH_3$ | $CH_2OTHP$ | |
| $SO_2$-*n*-$C_3H_7$ | H | H | $CH_3$ | $CH_2OTHP$ | |
| $OCH_3$ | H | H | $CH_2CH_3$ | $CH_2OTHP$ | |
| O-*n*-$C_3H_7$ | H | H | $CH_3$ | $CH_2OTHP$ | |
| $CH_2OCH_3$ | H | H | $CH_2CH_3$ | $CH_2OTHP$ | |
| $CO_2CH_2CH{=}CH_2$ | H | H | $CH_3$ | $CH_2OTHP$ | |
| $SO_2N(CH_3)_2$ | H | $CH_3$ | $CH_3$ | $CH_2OTHP$ | |
| $OC_2H_5$ | H | $CH_3$ | $CH_3$ | $CH_2OTHP$ | |
| $CO_2Et$ | H | $CH_3$ | $CH_3$ | $CH_2OTHP$ | |
| $OCF_2CF_2H$ | H | $CH_3$ | $CH_3$ | $CH_2OTHP$ | |
| $CH_2OCH_3$ | H | $CH_3$ | $CH_3$ | $CH_2OTHP$ | |
| Cl | H | $CH_3$ | $CH_3$ | $CH_2OTHP$ | |
| $SC_2H_5$ | H | $CH_3$ | $CH_3$ | $CH_2OTHP$ | |
| $SO_2CH_2CH{=}CH_2$ | H | H | $CH_3$ | $CH_2OTHP$ | |
| $SCF_3$ | H | H | $CH_3$ | $CH_2OTHP$ | |
| $CH(OCH_3)CH_3$ | H | H | $CH_3$ | $CH_2OTHP$ | |

TABLE 1c

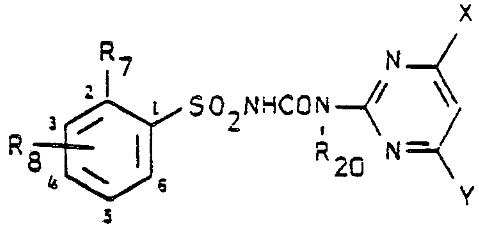

| $R_7$ | $R_8$ | $R_{20}$ | X | Y | m.p. (°C) |
|---|---|---|---|---|---|
| Cl | H | H | $CH_3$ | $CH_2OH$ | 130—132° |
| $CO_2CH_3$ | H | H | $CH_3$ | $CH_2OH$ | 140—141° |
| $SO_2CH_3$ | H | H | $CH_3$ | $CH_2OH$ | 155°d |
| $NO_2$ | H | H | $CH_3$ | $CH_2OH$ | 112—116° |
| $CO_2CH(CH_3)_2$ | H | H | $CH_3$ | $CH_2OH$ | 154—157° |
| $CO_2C_2H_5$ | H | H | $CH_3$ | $CH_2OH$ | |
| $CF_3$ | H | H | $CH_2CH_3$ | $CH_2OH$ | |
| Br | H | H | $CH_3$ | $CH_2OH$ | |
| $CH_3$ | H | H | $CH_3$ | $CH_2OH$ | |
| $C_2H_5$ | H | H | $CH_3$ | $CH_2OH$ | |
| $SO_2$-*n*-$C_3H_7$ | H | H | $CH_3$ | $CH_2OH$ | 128° |
| $SO_2N(CH_3)_2$ | H | H | $CH_3$ | $CH_2OH$ | 163—169° d |
| $OCH_3$ | H | H | $CH_3$ | $CH_2OH$ | |
| $CO_2CH_2CH{=}CH_2$ | H | H | $CH_3$ | $CH_2OH$ | |
| $OCF_2CF_2H$ | H | H | $CH_3$ | $CH_2OH$ | |
| $CH_2OCH_3$ | H | H | $CH_3$ | $CH_2OH$ | |
| $OSO_2CHCl_2$ | H | H | $CH_3$ | $CH_2OH$ | |
| $SC_2H_5$ | H | H | $CH_3$ | $CH_2OH$ | |
| $OSO_2CH_3$ | H | H | $CH_3$ | $CH_2OH$ | |
| $OSO_2C_2H_5$ | H | H | $CH_3$ | $CH_2OH$ | |
| $CO_2CH_2CH_2OCH_3$ | H | H | $CH_3$ | $CH_2OH$ | |
| $SO_2C_2H_5$ | H | H | $CH_2CH_3$ | $CH_2OH$ | |
| $NO_2$ | 5-$CF_3$ | H | $CH_3$ | $CH_2OH$ | |
| $SO_2$-*n*-$C_3H_7$ | 3-Cl | H | $CH_3$ | $CH_2OH$ | |
| $OC_2H_5$ | 5-$NO_2$ | H | $CH_3$ | $CH_2OH$ | |
| $SO_2N(CH_3)C_2H_5$ | 6-$NO_2$ | H | $CH_3$ | $CH_2OH$ | |
| Cl | 5-Cl | H | $CH_3$ | $CH_2OH$ | |

TABLE 1c (continued)

| $R_7$ | $R_8$ | $R_{20}$ | X | Y | m.p.(°C) |
|---|---|---|---|---|---|
| $SO_2CH_3$ | 5-Cl | H | $CH_2CH_3$ | $CH_2OH$ | |
| $CO_2CH_3$ | 4-F | H | $CH_3$ | $CH_2OH$ | |
| $NO_2$ | H | $CH_3$ | $CH_3$ | $CH_2OH$ | |
| Br | H | $CH_3$ | $CH_3$ | $CH_2OH$ | |
| Cl | H | $CH_3$ | $CH_3$ | $CH_2OH$ | |
| $SO_2CH_3$ | H | $CH_3$ | $CH_3$ | $CH_2OH$ | |
| F | H | $CH_3$ | $CH_3$ | $CH_2OH$ | |
| H | H | $CH_3$ | $CH_3$ | $CH_2OH$ | |
| $CO_2CH_3$ | H | $CH_3$ | $CH_3$ | $CH_2OH$ | |
| $CH_3$ | H | $CH_3$ | $CH_3$ | $CH_2OH$ | |
| $SO_2$-*i*-$C_3H_7$ | H | $CH_3$ | $CH_3$ | $CH_2OH$ | |
| $SCF_3$ | H | $CH_3$ | $CH_3$ | $CH_2OH$ | |
| $SO_2CH_2CH{=}CH_2$ | H | $CH_3$ | $CH_3$ | $CH_2OH$ | |
| $SCH_2CH_3$ | H | $CH_3$ | $CH_3$ | $CH_2OH$ | |
| $OCH_2CH{=}CH_2$ | H | $CH_3$ | $CH_3$ | $CH_2OH$ | |
| $OCH_2C(CH_3){=}CH_2$ | H | $CH_3$ | $CH_3$ | $CH_2OH$ | |
| $CH_2OEt$ | H | $CH_3$ | $CH_3$ | $CH_2OH$ | |
| $CH(OCH_3)CH_3$ | H | $CH_3$ | $CH_3$ | $CH_2OH$ | |
| $CO_2CH_2CH_2Cl$ | H | $CH_3$ | $CH_3$ | $CH_2OH$ | |
| O-*i*-$C_3H_7$ | H | $CH_3$ | $CH_3$ | $CH_2OH$ | |
| *i*-$C_4H_9$ | H | $CH_3$ | $CH_3$ | $CH_2OH$ | |
| $SO_2Et$ | H | H | $CH_3$ | $CH_2OH$ | |
| *n*-$C_4H_9$ | H | H | $CH_3$ | $CH_2OH$ | |
| O-*n*-$C_4H_9$ | H | H | $CH_3$ | $CH_2OH$ | |
| O-*t*-$C_4H_9$ | H | H | $CH_3$ | $CH_2OH$ | |
| $SO_2N(CH_3)C_2H_5$ | H | H | $CH_3$ | $CH_2OH$ | |

TABLE 1d

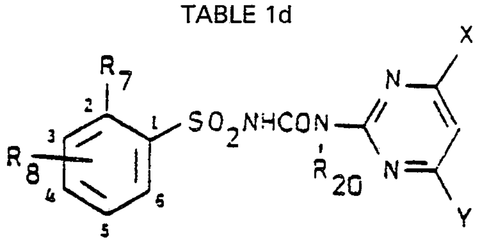

| $R_7$ | $R_8$ | $R_{20}$ | X | Y | m.p.(°C) |
|---|---|---|---|---|---|
| H | H | H | $CH_3$ | $CH(OCH_2CH_2O)$ | |
| H | H | H | $CH_3$ | $CH(OEt)_2$ | |
| H | H | H | $C_2H_5$ | $CH_2OCOCH_3$ | |
| H | H | H | $CH_3$ | $CH_2SCH_3$ | |
| H | H | H | $CH_3$ | $CH(OCH_2CH_2CH_2O)$ | |
| Cl | H | H | $CH_3$ | $CH(OCH_2CH_2O)$ | 158—163° |
| Cl | H | $CH_3$ | $CH_3$ | $CH(OCH_2CH_2O)$ | |
| Cl | H | H | $C_2H_5$ | $CH(OCH_2CH_2O)$ | |
| Cl | H | H | $CH_3$ | $CH(OCH_2CH_2CH_2O)$ | |
| Cl | 6-Cl | H | $CH_3$ | $CH(OCH_2CH_2CH_2O)$ | |
| Cl | H | $CH_3$ | $CH_3$ | $CH(OCH_2CH_2CH_2O)$ | |
| Cl | 4-F | H | $CH_3$ | $CH(OEt)_2$ | |
| Cl | H | H | $CH_3$ | $CH(OEt)_2$ | |
| $CH_3$ | H | H | $CH_3$ | $CH(OEt)_2$ | |
| $CH_3$ | H | H | $C_2H_5$ | $CH(OEt)_2$ | |
| $CH_3$ | H | H | $CH_3$ | $CH_2SCH_3$ | |
| $C_2H_5$ | H | H | $CH_3$ | $CH_2OCOCH_3$ | |
| $C_2H_5$ | H | $CH_3$ | $CH_3$ | $CH_2OCOCH_3$ | |
| $C_2H_5$ | 5-Cl | H | $CH_3$ | $CH_2OCOCH_3$ | |
| *n*-$C_4H_9$ | H | H | $CH_3$ | $CH(OCH(CH_3)CH_2O)$ | |
| $OCH_3$ | H | H | $CH_3$ | $CH(OCH(CH_3)CH_2O)$ | |
| $OCH_3$ | 3-$NO_2$ | H | $CH_3$ | $CH(OCH(CH_3)CH_2O)$ | |
| $OCH_3$ | H | H | $CH_3$ | $CH(OCH_2CH_2O)$ | |
| $OCH_3$ | H | H | $CH_3$ | $CH(OEt)_2$ | |
| $CO_2CH_3$ | H | H | $CH_3$ | $CH_2COCH_3$ | 145—150°C |
| $CO_2CH_3$ | H | H | $CH_3$ | $CH(SCH_2CH_2CH_2S)$ | |
| $CO_2CH_3$ | H | H | $C_2H_5$ | $CH(OEt)_2$ | |

TABLE 1d (continued)

| $R_7$ | $R_8$ | $R_{20}$ | X | Y | m.p.(°C) |
|---|---|---|---|---|---|
| $CO_2CH_3$ | H | H | $CH_3$ | $CH_2SEt$ | |
| $CO_2CH_3$ | H | H | $CH_3$ | $CH(OCH_2CH_2O)$ | 151—156° |
| $CO_2CH_3$ | H | $CH_3$ | $CH_3$ | $CH(OCH_2CH_2O)$ | |
| $CO_2CH_3$ | 6-$CH_3$ | H | $CH_3$ | $CH(OCH_2CH_2O)$ | |
| $CO_2CH_3$ | 5-OEt | H | $CH_3$ | $CH(OCH_2CH_2O)$ | |
| $CO_2CH_3$ | 4-$CF_3$ | H | $CH_3$ | $CH(OCH_2CH_2O)$ | |
| $SO_2CH_3$ | H | H | $CH_3$ | $CH_2OCOEt$ | |
| $SO_2CH_3$ | H | H | $CH_3$ | $CH_2OCOEt$ | |
| $SO_2CH_3$ | 3-F | H | $CH_3$ | $CH_2SCH_3$ | |
| $SO_2CH_3$ | H | H | $CH_3$ | $CH(OCH_2CH_2O)$ | 193—195° d |
| $SO_2CH_3$ | H | $CH_3$ | $CH_3$ | $CH(OCH_2CH_2O)$ | |
| $SO_2CH_3$ | 6-O-*n*-$C_3H_7$ | H | $CH_3$ | $CH(OCH_2CH_2O)$ | |
| $SO_2CH_3$ | 3-*i*-$C_3H_7$ | H | $CH_3$ | $CH(OCH_2CH_2O)$ | |
| $SO_2N(CH_3)_2$ | H | H | $CH_3$ | $CH_2OCO$-*n*-$C_3H_7$ | |
| $SO_2N(CH_3)_2$ | H | H | $CH_3$ | $CH_2OCOCH_3$ | |
| $SO_2N(CH_3)_2$ | H | H | $CH_3$ | $CH(SCH(CH_3)CH_2S)$ | |
| $SO_2N(CH_3)_2$ | H | H | $CH_3$ | $CH(OCH_2CH_2O)$ | 185—187° |
| $SO_2N(CH_3)_2$ | H | $CH_3$ | $CH_3$ | $CH(OCH_2CH_2O)$ | |
| $SO_2N(CH_3)_2$ | 6-$NO_2$ | H | $CH_3$ | $CH(OCH_2CH_2O)$ | |
| $NO_2$ | 3-Br | H | $CH_3$ | $CH(OCH_2CH_2O)$ | |
| $NO_2$ | 6-Cl | $CH_3$ | $CH_3$ | $CH(OCH_2CH_2O)$ | |
| $NO_2$ | H | H | $C_2H_5$ | $CH(OCH_2CH_2O)$ | |
| $NO_2$ | H | H | $CH_3$ | $CH(OCH_2CH_2O)$ | 179—182° d |
| $NO_2$ | 3-F | H | $CH_3$ | $CH(OCH_2CH_2O)$ | |
| $CO_2CH_2CH{=}CH_2$ | H | H | $CH_3$ | $CH(OCH_2CH_2O)$ | |
| $CO_2CH_2CH{=}CH_2$ | H | H | $CH_3$ | $CH(OCH_2CH_2CH_2O)$ | |
| Br | H | H | $CH_3$ | $CH(SCH_2CH_2S)$ | |
| Br | H | H | $CH_3$ | $CH_2OCO_2CH_3$ | |
| Br | H | $CH_3$ | $CH_3$ | $CH_2OCONHCH_3$ | |
| $CF_3$ | H | H | $CH_3$ | $CH(OCH_2CH_2O)$ | |
| $CF_3$ | H | H | $CH_3$ | $CH_2SEt$ | |

TABLE 1d (continued)

| $R_7$ | $R_8$ | $R_{20}$ | X | Y | m.p.(°C) |
|---|---|---|---|---|---|
| $SO_2N(Et)_2$ | H | H | $CH_3$ | $CH(OCH_2CH_2O)$ | |
| $OCH_2CH_3$ | H | H | $C_2H_5$ | $CH(OCH_2CH_2O)$ | |
| O-*n*-$C_4H_9$ | H | H | $CH_3$ | $CH(OCH_2CH_2O)$ | |
| O-*i*-$C_3H_7$ | H | H | $CH_3$ | $CH(OCH_2CH_2O)$ | |
| $O\text{-}CH_2CH{=}CH_2$ | H | H | $CH_3$ | $CH(OCH_2CH_2O)$ | |
| $SO_2CF_3$ | H | H | $CH_3$ | $CH(OCH_2CH_2O)$ | |
| $SO_2CF_3$ | 5-$CF_3$ | H | $CH_3$ | $CH(OEt)_2$ | |
| $SO_2CF_3$ | H | H | $CH_3$ | $CH(OEt)_2$ | |
| $SO_2$-*n*-$C_3H_7$ | H | H | $CH_3$ | $CH(OEt)_2$ | |
| $SO_2$-*n*-$C_3H_7$ | H | $CH_3$ | $CH_3$ | $CH(OEt)_2$ | |
| $SO_2$-*n*-$C_3H_7$ | H | H | $CH_3$ | $CH(OCH_2CH_2O)$ | 144° d |
| $OCF_2CF_2H$ | H | H | $CH_3$ | $CH_2OCOCH_3$ | |
| $OCF_2CF_2H$ | H | H | $C_2H_5$ | $CH_2OCOCH_3$ | |
| $OCF_2CF_2H$ | H | H | $CH_3$ | $CH_2OCOCH_3$ | |
| $OSO_2C_2H_5$ | H | H | $CH_3$ | $CH_2OCOCH_3$ | |
| $CH_2OEt$ | 6-F | H | $CH_3$ | $CH_2OCOCH_3$ | |
| $CH_2CH_2CH_2Cl$ | H | H | $CH_3$ | $CH(OCH(CH_3)CH_2O)$ | |
| $SO_2N(CH_3)Et$ | H | H | $CH_3$ | $CH_2OCO_2Et$ | |
| $SO_2N(CH_3)Et$ | H | H | $CH_3$ | $CH_2OCON(CH_3)_2$ | |
| $SO_2N(CH_3)Et$ | H | H | $CH_3$ | $CH(OCH_2CH_2CH_2O)$ | |

TABLE 1e

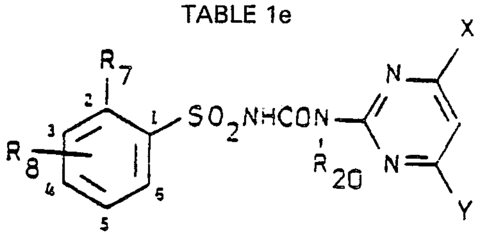

| $R_7$ | $R_8$ | $R_{20}$ | X | Y | m.p.(°C) |
|---|---|---|---|---|---|
| Cl | H | H | $OCH_3$ | $CH(OCH_3)_2$ | 179—182° |
| $CO_2CH_3$ | H | H | $OCH_3$ | $CH(OCH_3)_2$ | 120—133° |
| $SO_2CH_3$ | H | H | $OCH_3$ | $CH(OCH_3)_2$ | 162°d |
| $NO_2$ | H | H | $OCH_3$ | $CH(OCH_3)_2$ | 150°d |
| $CO_2CH(CH_3)_2$ | H | H | $OCH_3$ | $CH(OCH_3)_2$ | |
| $CO_2C_2H_5$ | H | H | $OCH_3$ | $CH(OCH_3)_2$ | |
| $CF_3$ | H | H | $OCH_3$ | $CH(OCH_3)_2$ | |
| Br | H | $CH_3$ | $OCH_3$ | $CH(OCH_3)_2$ | |
| $CH_3$ | H | H | $OCH_3$ | $CH(OCH_3)_2$ | |
| $C_2H_5$ | H | H | $OCH_2CH_3$ | $CH(OCH_3)_2$ | |
| $SO_2$-*n*-$C_3H_7$ | H | H | $OCH_3$ | $CH(OCH_3)_2$ | 161—167°d |
| $SO_2N(CH_3)_2$ | H | H | $OCH_3$ | $CH(OCH_3)_2$ | 93—102°d |
| $OCH_3$ | H | H | $OCH_3$ | $CH(OCH_3)_2$ | |
| $CO_2CH_2CH{=}CH_2$ | H | H | $OCH_3$ | $CH(OCH_3)_2$ | |
| $OCF_2CF_2H$ | H | H | $OCH_3$ | $CH(OCH_3)_2$ | |
| $CH_2OCH_3$ | H | H | $OCH_2CH_3$ | $CH(OCH_3)_2$ | |
| $OSO_2CHCl_2$ | H | H | $OCH_3$ | $CH(OCH_3)_2$ | |
| $SC_2H_5$ | H | $CH_3$ | $OCH_3$ | $CH(OCH_3)_2$ | |
| $OSO_2CH_3$ | H | H | $OCH_3$ | $CH(OCH_3)_2$ | |
| $OSO_2C_2H_4$ | H | H | $OCH_3$ | $CH(OCH_3)_2$ | |
| $CO_2CH_2CH_2OCH_3$ | H | H | $OCH_3$ | $CH(OCH_3)_2$ | |
| $SO_2C_2H_5$ | H | H | $OCH_3$ | $CH(OCH_3)_2$ | |
| $NO_2$ | 5-$CF_3$ | H | $OCH_3$ | $CH(OCH_3)_2$ | |
| $SO_2$-*n*-$C_3H_7$ | 3-Cl | H | $OCH_3$ | $CH(OCH_3)_2$ | |
| $CO_2H_5$ | 5-$NO_2$ | H | $OCH_2CH_3$ | $CH(OCH_3)_2$ | |
| $SO_2N(CH_3)C_2H_5$ | 6-$NO_2$ | H | $OCH_3$ | $CH(OCH_3)_2$ | |
| Cl | 5-Cl | H | $OCH_3$ | $CH(OCH_3)_2$ | |

TABLE 1e (continued)

| $R_7$ | $R_8$ | $R_{20}$ | X | Y | m.p.(°C) |
|---|---|---|---|---|---|
| $SO_2CH_3$ | 5-Cl | H | $OCH_3$ | $CH(OCH_3)_2$ | |
| $CO_2CH_3$ | 4-F | H | $OCH_3$ | $CH(OCH_3)_2$ | |

TABLE 1f

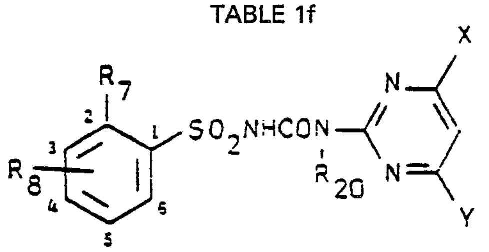

| $R_7$ | $R_8$ | $R_{20}$ | X | Y | m.p.(°C) |
|---|---|---|---|---|---|
| $NO_2$ | H | H | $OCH_3$ | $CH_2OTHP$ | |
| Br | H | H | $OCH_3$ | $CH_2OTHP$ | |
| Cl | H | H | $OCH_3$ | $CH_2OTHP$ | |
| $SO_2CH_3$ | H | H | $OCH_3$ | $CH_2OTHP$ | |
| F | H | H | $OCH_3$ | $CH_2OTHP$ | |
| H | H | H | $OCH_3$ | $CH_2OTHP$ | |
| $CO_2CH_3$ | H | H | $OCH_3$ | $CH_2OTHP$ | |
| $CH_3$ | H | H | $OCH_3$ | $CH_2OTHP$ | |
| $SO_2N(CH_3)_2$ | H | H | $OCH_3$ | $CH_2OTHP$ | |
| $SO_2$-*n*-$C_3H_7$ | H | H | $OCH_3$ | $CH_2OTHP$ | |
| $OCH_3$ | H | H | $OCH_3$ | $CH_2OTHP$ | |
| O-*n*-$C_3H_7$ | H | $CH_3$ | $OCH_3$ | $CH_2OTHP$ | |
| $CH_2OCH_3$ | H | $CH_3$ | $OCH_3$ | $CH_2OTHP$ | |
| $CO_2CH_2CH=CH_2$ | H | H | $OCH_3$ | $CH_2OTHP$ | |
| Cl | 6-Cl | H | $OCH_3$ | $CH_2OTHP$ | |

TABLE 1g

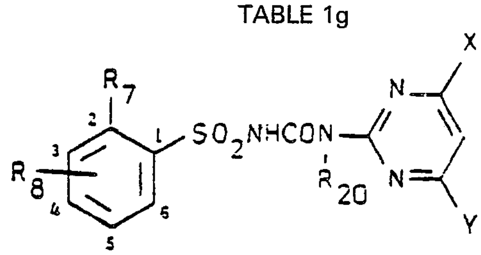

| $R_7$ | $R_8$ | $R_{20}$ | X | Y | m.p.(°C) |
|---|---|---|---|---|---|
| Cl | H | H | $OCH_3$ | $CH_2OH$ | |
| $CO_2CH_3$ | H | H | $OCH_3$ | $CH_2OH$ | |
| $SO_2CH_3$ | H | H | $OCH_3$ | $CH_2OH$ | 170°d |
| $NO_2$ | H | H | $OCH_3$ | $CH_2OH$ | |
| $CO_2CH(CH_3)_2$ | H | H | $OCH_3$ | $CH_2OH$ | |
| $CO_2C_2H_5$ | H | H | $OCH_3$ | $CH_2OH$ | |
| $CF_3$ | H | H | $OCH_3$ | $CH_2OH$ | |
| Br | H | H | $OCH_3$ | $CH_2OH$ | |
| $CH_3$ | H | $CH_3$ | $OCH_3$ | $CH_2OH$ | |
| $C_2H_5$ | H | H | $OCH_3$ | $CH_2OH$ | |
| $SO_2$-*n*-$C_3H_7$ | H | H | $OCH_3$ | $CH_2OH$ | |
| $SO_2N(CH_3)_2$ | H | H | $OCH_3$ | $CH_2OH$ | |
| $OCH_3$ | H | H | $OCH_3$ | $CH_2OH$ | |
| $CO_2CH_2CH=CH_2$ | H | H | $OCH_2CH_3$ | $CH_2OH$ | |
| $OCF_2CF_2H$ | H | H | $OCH_3$ | $CH_2OH$ | |
| $CH_2OCH_3$ | H | H | $OCH_3$ | $CH_2OH$ | |
| $OSO_2CHCl_2$ | H | H | $OCH_3$ | $CH_2OH$ | |
| $SC_2H_5$ | H | $CH_3$ | $OCH_3$ | $CH_2OH$ | |
| $OSO_2CH_3$ | H | H | $OCH_2CH_3$ | $CH_2OH$ | |
| $OSO_2C_2H_5$ | H | H | $OCH_3$ | $CH_2OH$ | |
| $CO_2CH_2CH_2OCH_3$ | H | H | $OCH_3$ | $CH_2OH$ | |
| $SO_2C_2H_5$ | H | H | $OCH_3$ | $CH_2OH$ | |
| $NO_2$ | 5-$CF_3$ | H | $OCH_3$ | $CH_2OH$ | |
| $SO_2$-*n*-$C_3H_7$ | 3-Cl | H | $OCH_3$ | $CH_2OH$ | |
| $OC_2H_5$ | 5-$NO_2$ | H | $OCH_3$ | $CH_2OH$ | |

TABLE 1g (continued)

| R$_7$ | R$_8$ | R$_{20}$ | X | Y | m.p.(°C) |
|---|---|---|---|---|---|
| $SO_2N(CH_3)C_2H_5$ | 6-$NO_2$ | H | $OCH_3$ | $CH_2OH$ | |
| Cl | 5-Cl | H | $OCH_3$ | $CH_2OH$ | |
| $SO_2CH_3$ | 5-Cl | H | $OCH_3$ | $CH_2OH$ | |
| $CO_2CH_3$ | 4-F | H | $OCH_3$ | $CH_2OH$ | |

TABLE 1h

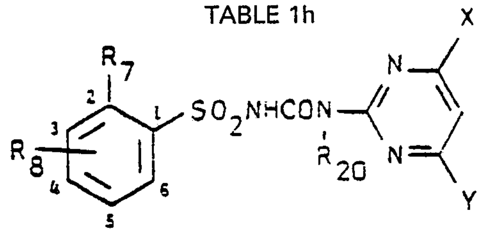

| R$_7$ | R$_8$ | R$_{20}$ | X | Y | m.p.(°C) |
|---|---|---|---|---|---|
| H | H | H | $OCH_3$ | $CH(OCH_2CH_2O)$ | |
| H | H | H | $OCH_3$ | $CH(OEt)_2$ | |
| H | H | H | $OC_2H_5$ | $CH_2OCOCH_3$ | |
| H | H | H | $OCH_3$ | $CH_2SCH_3$ | |
| H | H | H | $OCH_3$ | $CH(OCH_2CH_2CH_2O)$ | |
| Cl | H | H | $OCH_3$ | $CH(OCH_2CH_2O)$ | 105—112°d |
| Cl | H | $CH_3$ | $OCH_3$ | $CH(OCH_2CH_2O)$ | |
| Cl | H | H | $OC_2H_5$ | $CH(OCH_2CH_2O)$ | |
| Cl | H | H | $OCH_3$ | $CH(OCH_2CH_2CH_2O)$ | |
| Cl | 6-Cl | H | $OCH_3$ | $OCH(OCH_2CH_2CH_2O)$ | |
| Cl | H | $CH_3$ | $OCH_3$ | $CH(OCH_2CH_2CH_2O)$ | |
| Cl | 4-F | H | $OCH_3$ | $CH(OEt)_2$ | |
| Cl | H | H | $OCH_3$ | $CH(OEt)_2$ | 143—145° |
| $CH_3$ | H | H | $OCH_3$ | $CH(OEt)_2$ | |
| $CH_3$ | H | H | $OC_2H_5$ | $CH(OEt)_2$ | |
| $CH_3$ | H | H | $OCH_3$ | $CH_2SCH_3$ | |
| $C_2H_5$ | H | H | $OCH_3$ | $CH_2OCOCH_3$ | |
| $C_2H_5$ | H | $CH_3$ | $OCH_3$ | $CH_2OCOCH_3$ | |
| $C_2H_5$ | 5-Cl | H | $OCH_3$ | $CH_2OCOCH_3$ | |
| *n*-$C_4H_9$ | H | H | $OCH_3$ | $CH(OCH(CH_3)CH_2O)$ | |
| $OCH_3$ | H | H | $OCH_3$ | $CH(OCH(CH_3)CH_2O)$ | |

TABLE 1h (continued)

| $R_7$ | $R_8$ | $R_{20}$ | X | Y | m.p.(°C) |
|---|---|---|---|---|---|
| $OCH_3$ | 3-$NO_2$ | H | $OCH_3$ | $CH(OCH(CH_3)CH_2O)$ | |
| $OCH_3$ | H | H | $OCH_3$ | $CH(OCH_2CH_2O)$ | |
| $OCH_3$ | H | H | $OCH_3$ | $CH(OEt)_2$ | |
| $CO_2CH_3$ | H | H | $OCH_3$ | $CH_2OCOCH_3$ | |
| $CO_2CH_3$ | H | H | $OCH_3$ | $CH(SCH_2CH_2CH_2S)$ | |
| $CO_2CH_3$ | H | H | $OCH_3$ | $CH(OEt)_2$ | 127—144° |
| $CO_2CH_3$ | H | H | $OCH_3$ | $CH_2SEt$ | |
| $CO_2CH_3$ | H | H | $OCH_3$ | $CH(OCH_2CH_2O)$ | 161—163° |
| $CO_2CH_3$ | H | $CH_3$ | $OCH_3$ | $CH(OCH_2CH_2O)$ | |
| $CO_2CH_3$ | 6-$CH_3$ | H | $OCH_3$ | $CH(OCH_2CH_2O)$ | |
| $CO_2CH_3$ | 5-OEt | H | $OCH_3$ | $CH(OCH_2CH_2O)$ | |
| $CO_2CH_3$ | 4-$CF_3$ | H | $OCH_3$ | $CH(OCH_2CH_2O)$ | |
| $SO_2CH_3$ | H | H | $OCH_3$ | $CH_2OCOEt$ | |
| $SO_2CH_3$ | H | H | $OCH_3$ | $CH(OEt)_2$ | 210—213° |
| $SO_2CH_3$ | 3-F | H | $OCH_3$ | $CH_2SCH_3$ | |
| $SO_2CH_3$ | H | H | $OCH_3$ | $CH(OCH_2CH_2O)$ | |
| $So_2CH_3$ | H | $CH_3$ | $OCH_3$ | $CH(OCH_2CH_2O)$ | |
| $SO_2CH_3$ | 6-O-*n*-$C_3H_7$ | H | $OCH_3$ | $CH(OCH_2CH_2O)$ | |
| $SO_2CH_3$ | 3-*i*-$C_3H_7$ | H | $OCH_3$ | $CH(OCH_2CH_2O)$ | |
| $SO_2N(CH_3)_2$ | H | H | $OCH_3$ | $CH_2OCO$-*n*-$C_3H_7$ | |
| $SO_2N(CH_3)_2$ | H | H | $OCH_3$ | $CH(OEt)_2$ | 138—141° |
| $SO_2N(CH_3)_2$ | H | H | $OCH_3$ | $CH(SCH(CH_3)CH_2S)$ | |
| $SO_2N(CH_3)_2$ | H | H | $OCH_3$ | $CH(OCH_2CH_2O)$ | |
| $SO_2N(CH_3)_2$ | H | $CH_3$ | $OCH_3$ | $CH(OCH_2CH_2O)$ | |
| $SO_2N(CH_3)_2$ | 6-$NO_2$ | H | $OCH_3$ | $CH(OCH_2CH_2O)$ | |
| $NO_2$ | 3-Br | H | $OCH_3$ | $CH(OCH_2CH_2O)$ | |
| $NO_2$ | 6-Cl | $CH_3$ | $OCH_3$ | $CH(OCH_2CH_2O)$ | |
| $NO_2$ | H | H | $OC_2H_5$ | $CH(OCH_2CH_2O)$ | |
| $NO_2$ | H | H | $OCH_3$ | $CH(OCH_2CH_2O)$ | |
| $NO_2$ | 3-F | H | $OCH_3$ | $CH(OCH_2CH_2O)$ | |
| $CO_2CH_2CH{=}CH_2$ | H | H | $OCH_3$ | $CH(OCH_2CH_2O)$ | |

TABLE 1h (continued)

| $R_7$ | $R_8$ | $R_{20}$ | X | Y | m.p.(°C) |
|---|---|---|---|---|---|
| $CO_2CH_2CH{=}CH_2$ | H | H | $OCH_3$ | $CH(OCH_2CH_2CH_2O)$ | |
| Br | H | H | $OCH_3$ | $CH(SCH_2CH_2S)$ | |
| Br | H | H | $OCH_3$ | $CH_2OCO_2CH_3$ | |
| Br | H | $CH_3$ | $OCH_3$ | $CH_2OCONHCH_3$ | |
| $CF_3$ | H | H | $OCH_3$ | $CH(OCH_2CH_2O)$ | |
| $CF_3$ | H | H | $OCH_3$ | $CH_2SEt$ | |
| $SO_2N(Et)_2$ | H | H | $OCH_3$ | $CH(OCH_2CH_2O)$ | |
| $OCH_2CH_3$ | H | H | $OC_2H_5$ | $CH(OCH_2CH_2O)$ | |
| O-*n*-$C_4H_9$ | H | H | $OCH_3$ | $CH(OCH_2CH_2O)$ | |
| O-*i*-$C_3H_7$ | H | H | $OCH_3$ | $CH(OCH_2CH_2O)$ | |
| O-$CH_2CH{=}CH_2$ | H | H | $OCH_3$ | $CH(OCH_2CH_2O)$ | |
| $SO_2CF_3$ | H | H | $OCH_3$ | $CH(OCH_2CH_2O)$ | |
| $SO_2CF_3$ | 5-$CF_3$ | H | $OCH_3$ | $CH(OEt)_2$ | |
| $SO_2CF_3$ | H | H | $OCH_3$ | $CH(OEt)_2$ | |
| $SO_2$-*n*-$C_3H_7$ | H | H | $OCH_3$ | $CH(OEt)_2$ | |
| $SO_2$-*n*-$C_3H_7$ | H | $CH_3$ | $OCH_3$ | $CH(OEt)_2$ | |
| $SO_2$-*n*-$C_3H_7$ | H | H | $OCH_3$ | $CH(OCH_2CH_2O)$ | |
| $OCF_2CF_2H$ | H | H | $OCH_3$ | $CH_2OCOCH_3$ | |
| $OCF_2CF_2H$ | H | H | $OC_2H_5$ | $CH_2OCOCH_3$ | |
| $OCF_2CF_2H$ | H | H | $OCH_3$ | $CH_2OCOCH_3$ | |
| $OSO_2C_2H_5$ | H | H | $OCH_3$ | $CH_2OCOCH_3$ | |
| $CH_2OEt$ | 6-F | H | $OCH_3$ | $CH_2OCOCH_3$ | |
| $CH_2CH_2CH_2Cl$ | H | H | $OCH_3$ | $CH(OCH(CH_3)CH_2O)$ | |
| $SO_2N(CH_3)Et$ | H | H | $OCH_3$ | $CH_2OCO_2Et$ | |
| $SO_2N(CH_3)Et$ | H | H | $OCH_3$ | $CH_2OCON(CH_3)_2$ | |
| $SO_2N(CH_3)Et$ | H | H | $OCH_3$ | $CH(OCH_2CH_2CH_2O)$ | |

TABLE 1i

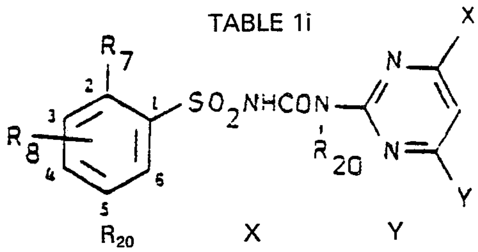

| $R_7$ | $R_8$ | $R_{20}$ | X | Y | m.p.(°C) |
|---|---|---|---|---|---|
| Cl | H | H | Cl | $CH(OCH_3)_2$ | 164—166° |
| $CO_2CH_3$ | H | H | Cl | $CH(OCH_3)_2$ | 149—152° |
| $SO_2CH_3$ | H | H | Cl | $CH(OCH_3)_2$ | |
| $NO_2$ | H | H | Cl | $CH(OC_2H_5)_2$ | |
| $CO_2CH(CH_3)_2$ | H | H | Cl | $CH_2SCH_3$ | |
| $CO_2C_2H_5$ | H | $CH_3$ | Cl | $CH_2SC_2H_5$ | |
| $CF_3$ | H | H | Cl | $CH(OCH_2CH_2CH_2O)$ | |
| Br | H | H | Cl | $CH(OCH_3)_2$ | |
| $CH_3$ | H | H | Cl | $CH(OCH_3)_2$ | |
| $C_2H_5$ | H | H | Cl | $CH(OCH_3)_2$ | |
| $SO_2$-*n*-$C_3H_7$ | H | H | Cl | $CH(OCH_3)_2$ | |
| $SO_2N(CH_3)_2$ | H | H | Cl | $CH(OCH_3)_2$ | |
| $OCH_3$ | H | $CH_3$ | Cl | $CH(OCH_3)_2$ | |
| $CO_2CH_2CH{=}CH_2$ | H | H | Cl | $CH(OCH_3)_2$ | |
| $OCF_2CF_2H$ | H | H | Cl | $CH(OCH_3)_2$ | |
| $CH_2OCH_3$ | H | H | Cl | $CH(OCH_3)_2$ | |
| $OSO_2CHCl_2$ | H | H | Cl | $CH(OCH_3)_2$ | |
| $SC_2H_5$ | H | H | Cl | $CH(OCH_3)_2$ | |
| $OSO_2CH_3$ | H | H | Cl | $CH(OCH_3)_2$ | |
| $OSO_2C_2H_5$ | H | H | Cl | $CH(OCH_3)_2$ | |
| $CO_2CH_2CH_2OCH_3$ | H | H | Cl | $CH(OCH_3)_2$ | |
| $SO_2C_2H_5$ | H | H | Cl | $CH(OCH_3)_2$ | |
| $NO_2$ | 5-$CF_3$ | H | Cl | $CH(OCh_3)_2$ | |
| $SO_2$-*n*-$C_3$-$_7$ | 3-Cl | H | Cl | $CH(OCH_3)_2$ | |
| $OC_2H_5$ | 5-$NO_2$ | H | Cl | $CH(OCH_3)_2$ | |
| $SO_2N(CH_3)C_2H_5$ | 6-$NO_2$ | H | Cl | $CH(OCH_3)_2$ | |
| Cl | 5-Cl | H | Cl | $CH(OCH_3)_2$ | |
| $SO_2CH_3$ | 5-Cl | H | Cl | $CH(OCH_3)_2$ | |
| $CO_2CH_3$ | 4-F | H | Cl | $CH(OCH_3)_2$ | |

TABLE 1j

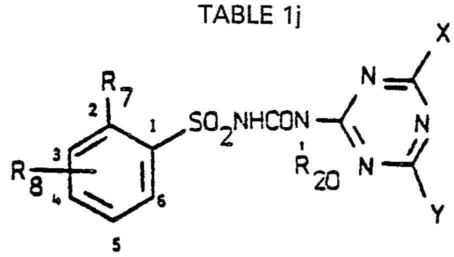

| $R_7$ | $R_8$ | $R_{20}$ | X | Y | m.p.(°C) |
|---|---|---|---|---|---|
| Cl | H | H | $CH_3$ | $CH(OCH_3)_2$ | |
| $CO_2CH_3$ | H | H | $CH_3$ | $CH(OCH_3)_2$ | 113—120° |
| $SO_2CH_3$ | H | H | $CH_2CH_3$ | $CH(OCH_3)_2$ | |
| $NO_2$ | H | H | $CH_3$ | $CH(OCH_3)_2$ | |
| $CO_2CH(CH_3)_2$ | H | H | $CH_3$ | $CH(OCH_3)_2$ | |
| $CO_2C_2H_5$ | H | H | $CH_3$ | $CH(OCH_3)_2$ | |
| $CF_3$ | H | H | $CH_3$ | $CH(OCH_3)_2$ | |
| Br | H | H | $CH_2CH_3$ | $CH(OCH_3)_2$ | |
| $CH(CH_3)_2$ | H | H | $CH_3$ | $CH(OCH_3)_2$ | |
| $C_2H_5$ | H | H | $CH_3$ | $CH(OCH_3)_2$ | |
| $SO_2$-*n*-$C_3H_7$ | H | H | $CH_3$ | $CH(OCH_3)_2$ | |
| $SO_2N(CH_3)_2$ | H | H | $CH_3$ | $CH(OCH_3)_2$ | |
| $OCH_2CH{=}CHCH_3$ | H | H | $CH_3$ | $CH(OCH_3)_2$ | |
| $CO_2CH_2CH{=}CH_2$ | H | $CH_3$ | $CH_3$ | $CH(OCH_3)_2$ | |
| $OCF_2CF_2H$ | H | H | $CH_3$ | $CH(OCH_3)_2$ | |
| $CH_2OCH_3$ | H | H | $CH_2CH_3$ | $CH(OCH_3)_2$ | |
| $OSO_2CHCl_2$ | H | H | $CH_3$ | $CH(OCH_3)_2$ | |
| $SC_2H_5$ | H | H | $CH_3$ | $CH(OCH_3)_2$ | |
| $OSO_2CH_3$ | H | H | $CH_3$ | $CH(OCH_3)_2$ | |
| $OSO_2C_2H_5$ | H | H | $CH_3$ | $CH(OCH_3)_2$ | |
| $CO_2CH_2CH_2OCH_3$ | H | H | $CH_3$ | $CH(OCH_3)_2$ | |
| $SO_2C_2H_5$ | H | H | $CH_3$ | $CH(OCH_3)_2$ | |
| $NO_2$ | 5-$CF_3$ | H | $CH_3$ | $CH(OCH_3)_2$ | |
| $SO_2$-*n*-$C_3H_7$ | 3-Cl | H | $CH_3$ | $CH(OCH_3)_2$ | |
| $OC_2H_5$ | 5-$NO_2$ | H | $CH_3$ | $CH(OCH_3)_2$ | |
| $SO_2N(CH_3)C_2H_5$ | 6-$NO_2$ | H | $CH_3$ | $CH(OCH_3)_2$ | |
| Cl | 5-Cl | H | $CH_3$ | $CH(OCH_3)_2$ | |

TABLE 1j (continued)

| $R_7$ | $R_8$ | $R_{20}$ | X | Y | m.p.(°C) |
|---|---|---|---|---|---|
| $SO_2CH_3$ | 5-Cl | H | $CH_3$ | $CH(OCH_3)_2$ | |
| $CO_2CH_3$ | 4-F | H | $CH_3$ | $CH(OCH_3)_2$ | |

TABLE 1k

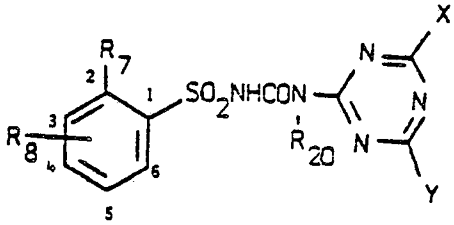

| $R_7$ | $R_8$ | $R_{20}$ | X | Y | m.p.(°C) |
|---|---|---|---|---|---|
| $NO_2$ | H | H | $CH_3$ | $CH_2OTHP$ | |
| Br | H | H | $CH_3$ | $CH_2OTHP$ | |
| Cl | H | H | $CH_3$ | $CH_2OTHP$ | |
| F | H | H | $CH_3$ | $CH_2OTHP$ | |
| $SO_2CH_3$ | H | $CH_3$ | $CH_3$ | $CH_2OTHP$ | |
| H | H | H | $CH_3$ | $CH_2OTHP$ | |
| $CO_2CH_3$ | H | H | $CH_3$ | $CH_2OTHP$ | |
| $CH_3$ | H | H | $CH_3$ | $CH_2OTHP$ | |
| $SO_2N(CH_3)_2$ | H | H | $CH_3$ | $CH_2OTHP$ | |
| $SO_2$-*n*-$C_3H_7$ | H | H | $CH_3$ | $CH_2OTHP$ | |
| $OCH_3$ | H | H | $CH_2CH_3$ | $CH_2OTHP$ | |
| O-*n*-$C_3H_7$ | H | H | $CH_3$ | $CH_2OTHP$ | |
| $CH_2OCH_3$ | H | H | $CH_3$ | $CH_2OTHP$ | |
| $CO_2CH_2CH{=}CH_2$ | H | H | $CH_3$ | $CH_2OTHP$ | |

TABLE 1I

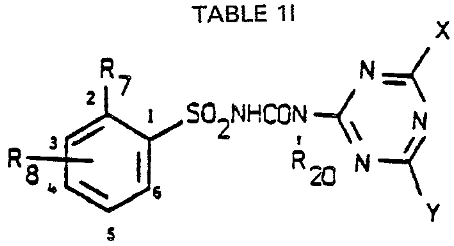

| $R_7$ | $R_8$ | $R_{20}$ | X | Y | m.p.(°C) |
|---|---|---|---|---|---|
| Cl | H | H | $CH_3$ | $CH_2OH$ | |
| $CO_2CH_3$ | H | $CH_3$ | $CH_3$ | $CH_2OH$ | |
| $SO_2CH_3$ | H | H | $CH_3$ | $CH_2OH$ | |
| $NO_2$ | H | H | $CH_3$ | $CH_2OH$ | |
| $CO_2CH(CH_3)_2$ | H | H | $CH_3$ | $CH_2OH$ | |
| $CO_2C_2H_5$ | H | H | $CH_2CH_3$ | $CH_2OH$ | |
| $CF_3$ | H | H | $CH_3$ | $CH_2OH$ | |
| Br | H | H | $CH_3$ | $CH_2OH$ | |
| $CH_3$ | H | H | $CH_3$ | $CH_2OH$ | |
| $C_2H_5$ | H | H | $CH_3$ | $CH_2OH$ | |
| $SO_2$-*n*-$C_3H_7$ | H | H | $CH_3$ | $CH_2OH$ | |
| $SO_2N(CH_3)_2$ | H | H | $CH_3$ | $CH_2OH$ | |
| $OCH_3$ | H | H | $CH_3$ | $CH_2OH$ | |
| $CO_2CH_2CH{=}CH_2$ | H | $CH_3$ | $CH_3$ | $CH_2CH$ | |
| $OCF_2CF_2H$ | H | H | $CH_3$ | $CH_2OH$ | |
| $CH_2OCH_3$ | H | H | $CH_3$ | $CH_2OH$ | |
| $OSO_2CHCl_2$ | H | H | $CH_3$ | $CH_2OH$ | |
| $SC_2H_5$ | H | H | $CH_3$ | $CH_2OH$ | |
| $OSO_2CH_3$ | H | H | $CH_2CH_3$ | $CH_2OH$ | |
| $OSO_2C_2H_5$ | H | H | $CH_3$ | $CH_2OH$ | |
| $CH_2CH_2CH_2OCH_3$ | H | H | $CH_3$ | $CH_2OH$ | |
| $SO_2C_2H_5$ | H | H | $CH_3$ | $CH_2OH$ | |
| $NO_2$ | 5-$CF_3$ | H | $CH_3$ | $CH_2OH$ | |
| $SO_2$-*n*-$C_3H_7$ | 3-Cl | H | $CH_3$ | $CH_2OH$ | |
| $OC_2H_5$ | 5-$NO_3$ | H | $CH_3$ | $CH_2OH$ | |
| $SO_2N(CH_3)C_2H_5$ | 6-$NO_2$ | H | $CH_3$ | $CH_2OH$ | |
| Cl | 5-Cl | H | $CH_3$ | $CH_2OH$ | |

TABLE 1l (contined)

| $R_7$ | $R_8$ | $R_{20}$ | X | Y | m.p.(°C) |
|---|---|---|---|---|---|
| $SO_2CH_3$ | 5-Cl | H | $CO_3$ | $CH_2OH$ | |
| $CH_2CH_3$ | 4-FH | H | $CH_3$ | $CH_2OH$ | |

TABLE 1m

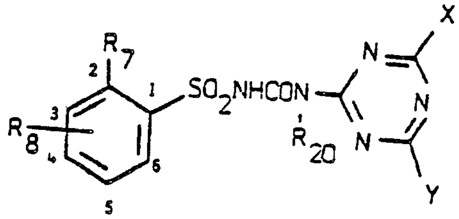

| $R_7$ | $R_8$ | $R_{20}$ | X | Y | m.p.(°C) |
|---|---|---|---|---|---|
| $NO_2$ | H | H | $CH_3$ | $CH(OC_2H_5)_2$ | |
| $NO_2$ | H | $CH_3$ | $CH_3$ | $CH_2OCOCH_3$ | |
| $NO_2$ | H | H | $CH_3$ | $CH_2SCH_3$ | |
| Br | H | H | $CH_3$ | $CH_2OCONHCH_3$ | |
| Cl | H | H | $CH_3$ | $CH_2OCO_2CH_3$ | |
| F | H | H | $CH_3$ | $CH_2SC_2H_5$ | |
| F | H | H | $CH_3$ | $CH(OCH_2CH_2O)$ | |
| $SO_2CH_3$ | H | H | $CH_2CH_3$ | $CH_2OCOC_2H_5$ | |
| $SO_2CH_3$ | H | H | $CH_3$ | $CH(OCH(CH_3)CH_2O)$ | |
| $SO_2CH_3$ | H | H | $CH_3$ | $CH(OCH_2CH_2CH_2O)$ | |
| $SO_2CH_3$ | H | H | $CH_3$ | $CH_2SCH_2CH_3$ | |
| H | H | H | $CH_3$ | $CH_2OCOCH_3$ | |
| $CO_2CH_3$ | H | H | $CH_3$ | $CH_2OCON(CH_3)_2$ | |
| $CO_2CH_3$ | H | H | $CH_3$ | $CH(OCH_2CH_2O)$ | |
| $CO_2CH_3$ | H | $CH_3$ | $CH_3$ | $CH_2OCOCH_3$ | |
| $CO_2CH_3$ | H | H | $CH_3$ | $CH_2SC_2H_5$ | |
| $CH_3$ | H | H | $CH_2CH_3$ | $CH(OC_2H_5)_2$ | |
| $CH_3$ | H | H | $CH_3$ | $CH_2OCO_2C_2H_5$ | |
| $SO_2N(CH_3)_2$ | H | H | $CH_3$ | $CH_2OCON(C_2H_5)_2$ | |
| $SO_2N(CH_3)_2$ | H | H | $CH_3$ | $CH_2OCO\text{-}i\text{-}C_3H_7$ | |
| $SO_2\text{-}n\text{-}C_3H_7$ | H | H | $CH_3$ | $CH_2OCONH\text{-}n\text{-}C_3H_7$ | |
| $SO_2\text{-}n\text{-}C_3H_7$ | H | H | $CH_3$ | $CH(OC_2H_5)_2$ | |

TABLE 1m (continued)

| $R_7$ | $R_8$ | $R_{20}$ | X | Y | m.p.(°C) |
|---|---|---|---|---|---|
| $OCH_3$ | H | H | $CH_3$ | $CH(SCH_2CH_2S)$ | |
| $OCH_3$ | H | H | $CH_3$ | $CH_2SCH_3$ | |
| O-*n*-$C_3H_7$ | H | H | $CH_3$ | $CH_2OCO_2CH_3$ | |
| O-*n*-$C_3H_7$ | H | H | $CH_3$ | $CH_2OCON(C_2H_5)_2$ | |
| $CH_2OCH_3$ | H | H | $CH_3$ | $CH_2OCOCH_3$ | |
| $CH_2OCH_3$ | H | H | $CH_3$ | $CH_2OCO_2CH_3$ | |
| $CO_2CH_2CH{=}CH_2$ | H | H | $CH_3$ | $CH(OC_2H_5)_2$ | |

TABLE 1n

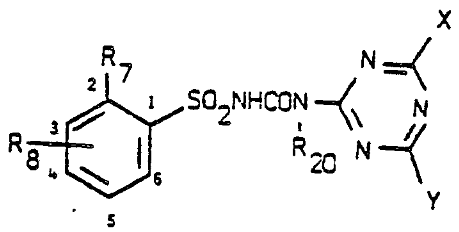

| $R_7$ | $R_8$ | $R_{20}$ | X | Y | m.p.(°C) |
|---|---|---|---|---|---|
| Cl | H | H | $OCH_3$ | $CH(OCH_3)_2$ | 117—121° |
| $CO_2CH_3$ | H | H | $OCH_3$ | $CH(OCH_3)_2$ | 135—158° |
| $SO_2CH_3$ | H | H | $OCH_3$ | $CH(OCH_3)_2$ | |
| $NO_2$ | H | H | $OCH_3$ | $CH(OCH_3)_2$ | |
| $CO_2CH(CH_3)_2$ | H | H | $OCH_3$ | $CH(OCH_3)_2$ | |
| $CO_2C_2H_5$ | H | H | $OCH_3$ | $CH(OCH_3)_2$ | |
| $CF_3$ | H | H | $OCH_3CH_3$ | $CH(OCH_3)_2$ | |
| Br | H | H | $OCH_3$ | $CH(OCH_3)_2$ | |
| $CH_3$ | H | H | $OCH_3$ | $CH(OCH_3)_2$ | |
| $C_2H_5$ | H | H | $OCH_3$ | $CH(OCH_3)_2$ | |
| $SO_2$*n*-$C_3H_7$ | H | H | $OCH_3$ | $CH(OCH_3)_2$ | |
| $SO_2N(CH_3)_2$ | H | H | $OCH_3$ | $CH(OCH_3)_2$ | |
| $OCH_3$ | H | H | $OCH_2CH_3$ | $CH(OCH_3)_2$ | |
| $CO_2CH_2CH{=}CH_2$ | H | $CH_3$ | $OCH_3$ | $CH(OCH_3)_2$ | |
| $OCF_2CF_2H$ | H | H | $OCH_3$ | $CH(OCH_3)_2$ | |
| $CH_2OCH_3$ | H | H | $OCH_3$ | $CH(OCH_3)_2$ | |

TABLE 1n (continued)

| $R_7$ | $R_8$ | $R_{20}$ | X | Y | m.p.(°C) |
|---|---|---|---|---|---|
| $OSO_2CHCl_2$ | H | H | $OCH_3$ | $CH(OCH_3)_2$ | |
| $SC_2H_5$ | H | H | $OCH_3$ | $CH(OCH_3)_2$ | |
| $OSO_2CH_3$ | H | H | $OCH_3$ | $CH(OCH_3)_2$ | |
| $OSO_2C_2H_5$ | H | H | $OCH_3$ | $CH(OCH_3)_2$ | |
| $CO_2CH_2CH_2OCH_3$ | H | H | $OCH_3$ | $CH(OCH_3)_2$ | |
| $SO_2C_2H_5$ | H | H | $OCH_3$ | $CH(OCH_3)_2$ | |
| $NO_2$ | 5-$CF_3$ | H | $OCH_3$ | $CH(OCH_3)_2$ | |
| $SO_2$-*n*-$C_3H_7$ | 3-Cl | H | $OCH_3$ | $CH(OCH_3)_2$ | |
| $OC_2H_5$ | 5-$NO_2$ | H | $OCH_3$ | $CH(OCH_3)_2$ | |
| $SO_2N(CH_3(C_2H_5$ | 6-$NO_2$ | H | $OCH_3$ | $CH(OCH_3)_2$ | |
| Cl | 5-Cl | H | $OCH_3$ | $CH(OCH_3)_2$ | |
| $SO_2CH_3$ | 5-Cl | H | $OCH_3$ | $CH(OCH_3)_2$ | |
| $CO_2CH_3$ | 4-F | H | $OCH_3$ | $CH(OCH_3)_2$ | |

TABLE 1o

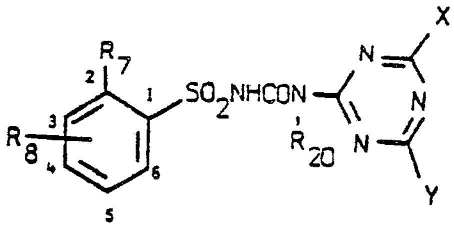

| $R_7$ | $R_8$ | $R_{20}$ | X | Y | m.p.(°C) |
|---|---|---|---|---|---|
| $NO_2$ | H | H | $OCH_3$ | $CH_2OTHP$ | |
| Br | H | H | $OCH_3$ | $CH_2OTHP$ | |
| Cl | H | H | $OCH_3$ | $CH_2OTHP$ | 136—139° |
| $SO_2CH_3$ | H | H | $OCH_3$ | $CH_2OTHP$ | |
| F | H | H | $OCH_3$ | $CH_2OTHP$ | |
| H | H | H | $OCH_3$ | $CH_2OTHP$ | |
| $CO_2CH_3$ | H | H | $OCH_3$ | $CH_2OTHP$ | |
| $CH_3$ | H | H | $OCH_3$ | $CH_2OTHP$ | |
| $SO_2N(CH_3)_2$ | H | H | $OCH_3$ | $CH_2OTHP$ | |
| $SO_2\textit{n}C_3H_7$ | H | H | $OCH_3$ | $CH_2OTHP$ | |
| $OCH_3$ | H | $CH_3$ | $OCH_3$ | $CH_2OTHP$ | |
| O-*n*-$C_3H_7$ | H | H | $OCH_2CH_3$ | $CH_2OTHP$ | |
| $CH_2OCH_3$ | H | H | $OCH_3$ | $CH_2OTHP$ | |
| $CO_2CH_2CH{=}CH_2$ | H | H | $OCH_3$ | $CH_2OTHP$ | |

TABLE 1p

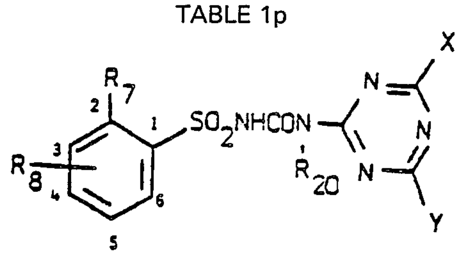

| $R_7$ | $R_8$ | $R_{20}$ | X | Y | m.p.(°C) |
|---|---|---|---|---|---|
| Cl | H | H | $OCH_3$ | $CH_2OH$ | 133—135° |
| $CO_2CH_3$ | H | H | $OCH_3$ | $CH_2OH$ | 115°d |
| $SO_2CH_3$ | H | H | $OCH_3$ | $CH_2OH$ | |
| $NO_2$ | H | H | $OCH_3$ | $CH_2OH$ | |
| $CO_2CH(CH_3)_2$ | H | H | $OCH_3$ | $CH_2OH$ | |
| $CO_2C_2H_5$ | H | H | $OCH_3$ | $CH_2OH$ | |
| $CF_3$ | H | H | $OCH_3$ | $CH_2OH$ | |
| Br | H | H | $OCH_2CH_3$ | $CH_2OH$ | |
| $CH_3$ | H | H | $OCH_3$ | $CH_2OH$ | |
| $C_2H_5$ | H | H | $OCH_3$ | $CH_2OH$ | |
| $SO_2$-*n*-$C_3H_7$ | H | H | $OCH_3$ | $CH_2OH$ | 152°d |
| $SO_2N(CH_3)_2$ | H | H | $OCH_3$ | $CH_2OH$ | |
| $OCH_3$ | H | H | $OCH_3$ | $CH_2OH$ | |
| $CO_2CH_2CH{=}CH_2$ | H | H | $OCH_3$ | $CH_2OH$ | |
| $OCF_2CF_2H$ | H | H | $OCH_3$ | $CH_2OH$ | |
| $CH_2OCH_3$ | H | H | $OCH_3$ | $CH_2OH$ | |
| $OSO_2CHCl_2$ | H | H | $OCH_3$ | $CH_2OH$ | |
| $SC_2H_5$ | H | $CH_3$ | $OCH_3$ | $CH_2OH$ | |
| $OSO_2CH_3$ | H | H | $OCH_3$ | $CH_2OH$ | |
| $OSO_2C_2H_5$ | H | H | $OCH_3$ | $CH_2OH$ | |
| $CO_2CH_2CH_2OCH_3$ | H | H | $OCH_3$ | $CH_2OH$ | |
| $SO_2C_2H_5$ | H | H | $OCH_3$ | $CH_2OH$ | |
| $NO_2$ | 5-$CF_3$ | H | $OCH_2CH_3$ | $CH_2OH$ | |
| $SO_2$-*n*-$C_3H_7$ | 3-Cl | H | $OCH_3$ | $CH_2OH$ | |
| $OC_2H_5$ | 5-$NO_2$ | H | $OCH_3$ | $CH_2OH$ | |
| $SO_2N(CH_3)C_2H_5$ | 6-$NO_2$ | H | $OCH_3$ | $CH_2OH$ | |

TABLE 1p (continued)

| $R_7$ | $R_8$ | $R_{20}$ | X | Y | m.p.(°C) |
|---|---|---|---|---|---|
| Cl | 5-Cl | H | $OCH_3$ | $CH_2OH$ | |
| $SO_2CH_3$ | 5-Cl | H | $OCH_3$ | $CH_2OH$ | |
| $CO_2CH_3$ | 4-F | H | $OCH_3$ | $CH_2OH$ | |

Table 1q

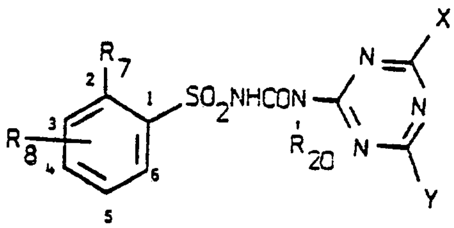

| $R_7$ | $R_8$ | $R_{20}$ | X | Y | m.p.(°C) |
|---|---|---|---|---|---|
| $NO_2$ | H | H | $OCH_3$ | $CH(OC_2H_5)_2$ | |
| $NO_2$ | H | H | $OCH_3$ | $CH_2OCOCH_3$ | |
| $NO_2$ | H | H | $OCH_3$ | $CH_2SCH_3$ | |
| Br | H | H | $OCH_3$ | $CH_2OCONHCH_3$ | |
| Cl | H | H | $OCH_3$ | $CH_2OCO_2H_3$ | |
| F | H | H | $OCH_3$ | $CH_2SC_2H_5$ | |
| F | H | H | $OCH_3$ | $CH(OCH_2CH_2O)$ | |
| $SO_2CH_3$ | H | H | $OCH_3$ | $CH_2OCOC_2H_5$ | |
| $SO_2CH_3$ | H | H | $OCH_3$ | $CH(OCH(CH_3)CH_2O)$ | |
| $SO_2CH_3$ | H | H | $OCH_3$ | $CH(OCH_2CH_2CH_2O)$ | |
| $SO_2CH_3$ | H | H | $OCH_2CH_3$ | $CH_2SCH_3$ | |
| H | H | H | $OCH_3$ | $CH_2OCOCH_3$ | |
| $CO_2CH_3$ | H | H | $OCH_3$ | $CH_2OCON(CH_3)_2$ | |
| $CO_2CH_3$ | H | H | $OCH_3$ | $CH(OCH_2CH_2O)$ | |
| $CO_2CH_3$ | H | H | $OCH_3$ | $CH_2OCOCH_3$ | |
| $CO_2CH_3$ | H | $CH_3$ | $OCH_3$ | $CH_2SC_2H_5$ | |
| $CH_3$ | H | H | $OCH_3$ | $CH(OC_2H_5)_2$ | |
| $CH_3$ | H | H | $OCH_3$ | $CH_2OCO_2C_2H_5$ | |
| $SO_2N(CH_3)_2$ | H | H | $OCH_3$ | $CH_2OCON(C_2H_5)_2$ | |
| $SO_2N(CH_3)_2$ | H | H | $OCH_3$ | $CH_2OCO\text{-}i\text{-}C_3H_7$ | |

TABLE 1q (continued)

| $R_7$ | $R_8$ | $R_{20}$ | X | Y | m.p.(°C) |
|---|---|---|---|---|---|
| $SO_2$-*n*-$C_3H_7$ | H | H | $OCH_3$ | $CH_2OCONH$-*n*-$C_3H_7$ | |
| $SO_2$-*n*-$C_3H_7$ | H | H | $OCH_3$ | $CH(OC_2H_5)_2$ | |
| $OCH_3$ | H | H | $OCH_2CH_3$ | $CH(OCH_2CH_3O)$ | |
| $OCH_3$ | H | H | $OCH_3$ | $CH_2SCH_3$ | |
| O-*n*-$C_3H_7$ | H | H | $OCH_3$ | $CH_2OCO_2OCH_3$ | |
| O-*n*-$C_3H_7$ | H | H | $OCH_3$ | $CH_2OCON(C_2H_5)_2$ | |
| $CH_2OCH_3$ | H | H | $OCH_3$ | $CH_2OCOCH_3$ | |
| $CH_2OCH_3$ | H | H | $OCH_3$ | $CH_2OCO_2CH_3$ | |
| $CO_2CH_2CH{=}CH_2$ | H | H | $OCH_3$ | $CH(OC_2H_5)_2$ | |

TABLE 1r

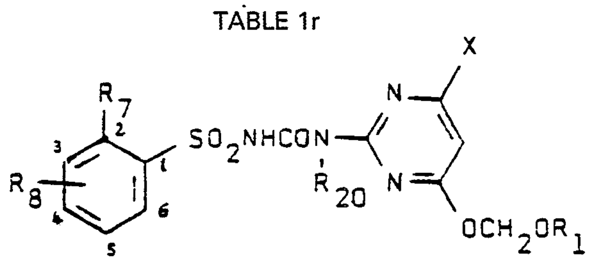

| $R_7$ | $R_8$ | $R_1$ | $R_{20}$ | X | m.p.(°C) |
|---|---|---|---|---|---|
| $NO_2$ | H | $CH_3$ | H | $CH_3$ | |
| $NO_2$ | 5-$CF_3$ | $CH_3$ | H | $OCH_3$ | |
| $CF_3$ | H | $CH_3$ | H | $CH_3$ | |
| Br | H | $C_2H_5$ | H | $OCH_3$ | |
| Cl | H | $CH_3$ | H | $CH_3$ | |
| Cl | 5-Cl | $CH_3$ | H | $OCH_3$ | |
| $SCH_2CH_3$ | H | $CH_3$ | H | $CH_3$ | |
| F | H | $CH_3$ | $CH_3$ | $OCH_3$ | |
| $SO_2CH_3$ | H | $CH_3$ | H | Cl | |
| $SO_2CH_3$ | H | $C_2H_5$ | H | $OCH_3$ | |
| $SO_2$-*n*-$C_3H_7$ | H | $CH_3$ | H | $OC_2H_5$ | |
| $CO_2CH_3$ | H | $CH_3$ | H | $C_2H_5$ | |
| $CO_2CH_3$ | H | $CH_3$ | $CH_3$ | $CH_3$ | |
| $OSO_2CHCl_2$ | H | $CH_3$ | H | $OCH_3$ | |
| $SO_2N(CH_3)_2$ | H | $CH_3$ | H | $C_2H_5$ | |
| $SO_2N(CH_3)_2$ | 4-F | $CH_3$ | H | $OCH_3$ | |
| $OCH_3$ | H | $CH_3$ | H | $OC_2H_5$ | |
| $OCF_2CF_2H$ | H | $C_2H_5$ | H | $OCH_3$ | |
| $CH_3$ | H | $CH_3$ | H | $CH_3$ | |
| $CO_2C_2H_5$ | H | $CH_3$ | H | $OCH_3$ | |
| $CO_2CH_2CH{=}CH_2$ | H | $C_2H_5$ | H | $CH_3$ | |
| $CH_2OCH_3$ | H | $CH_3$ | H | $OCH_3$ | |
| $SO_2N(CH_3)C_2H_5$ | H | $CH_3$ | H | $OC_2H_5$ | |
| O-*n*-$C_4H_9$ | H | $CH_3$ | H | $OCH_3$ | |

TABLE 1s

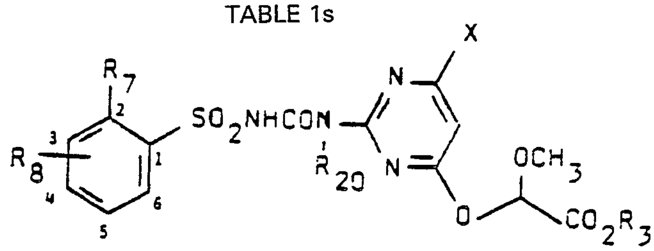

| $R_7$ | $R_8$ | $R_{20}$ | $R_3$ | X | m.p.(°C) |
|---|---|---|---|---|---|
| $NO_2$ | H | H | $CH_3$ | $CH_3$ | |
| $NO_2$ | 5-$CF_3$ | H | $CH_3$ | $OCH_3$ | |
| $CF_3$ | H | H | $CH_3$ | $CH_3$ | |
| Br | H | H | $CH_3$ | Cl | |
| Cl | H | H | $CH_3$ | $C_2H_5$ | |
| Cl | 5-Cl | H | $CH_3$ | $OCH_3$ | |
| $SCH_2CH_3$ | H | H | $C_2H_5$ | $OC_2H_5$ | |
| F | H | $CH_3$ | $CH_3$ | $OCH_3$ | |
| $SO_2CH_3$ | H | H | $CH_3$ | $CH_3$ | |
| $SO_2CH_3$ | H | H | *i*-$C_3H_7$ | $OCH_3$ | |
| $SO_2$-*n*-$C_3H_7$ | H | H | $CH_3$ | $CH_3$ | |
| $CO_2CH_3$ | H | H | $CH_3$ | $C_2H_3$ | |
| $CO_2CH_3$ | H | $CH_3$ | $CH_3$ | $CH_3$ | |
| $OSO_2CHCl_2$ | H | H | $CH_3$ | $OCH_3$ | |
| $SO_2N(CH_3)_2$ | H | H | $CH_3$ | $CH_3$ | |
| $SO_2N(CH_3)_2$ | 4-F | H | $CH_3$ | OH | |
| $OCH_3$ | H | H | *n*-$C_3H_7$ | $CH_3$ | |
| $OCF_2CF_2H$ | H | H | $CH_3$ | $OCH_3$ | |
| $CH_3$ | H | H | $CH_3$ | $CH_3$ | |
| $CO_2CH_2CH_3$ | H | H | $CH_3$ | $OCH_3$ | |
| $CO_2CH_2CH{=}CH_2$ | H | H | $CH_3$ | $CH_3$ | |
| $CH_2OCH_3$ | H | H | $C_2H_5$ | $OCH_3$ | |
| $SO_2N(CH_3)C_2H_5$ | H | H | $CH_3$ | $CH_3$ | |
| O-*n*-$C_4H_9$ | H | H | $CH_3$ | $OCH_3$ | |

TABLE 2a

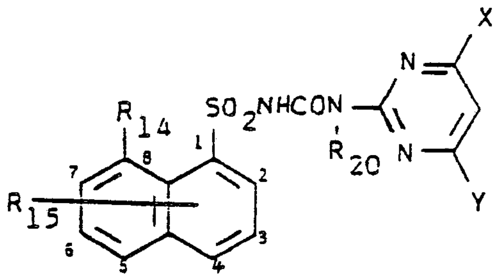

| $R_{14}$ | $R_{15}$ | $R_{20}$ | X | Y | m.p.(°C) |
|---|---|---|---|---|---|
| Cl | H | H | $CH_3$ | $CH(OCH_3)_2$ | |
| Br | H | H | $CH_3$ | $CH(OCH_3)_2$ | |
| F | H | H | $OCH_3$ | $CH(OCH_3)_2$ | |
| H | H | H | $OCH_3$ | $CH(OCH_3)_2$ | |
| Cl | H | H | Cl | $CH(OCH_3)_2$ | |
| Cl | 3-Cl | H | $CH_3$ | $CH(OCH_3)_2$ | |
| Cl | H | H | $OCH_3$ | $CH(OCH_3)_2$ | |
| $OCH_3$ | H | H | $CH_3$ | $CH(OCH_3)_2$ | |
| $CH_3$ | H | H | $OCH_2CH_3$ | $CH(OCH_3)_2$ | |
| Br | H | H | Cl | $CH(OCH_3)_2$ | |
| $CH_3$ | H | H | $CH_3$ | $CH(OCH_3)_2$ | |
| $NO_2$ | H | $CH_3$ | $OCH_3$ | $CH(OCH_3)_2$ | |
| $CH_3$ | 4-F | H | $OCH_3$ | $CH(OCH_3)_2$ | |
| Cl | 6-$NO_2$ | H | $OCH_3$ | $CH(OCH_3)_2$ | |
| $NO_2$ | H | H | $CH_3$ | $CH(OCH_3)_2$ | |
| $NO_2$ | H | H | Cl | $CH(OCH_3)_2$ | |
| Cl | 6-F | H | $CH_2CH_3$ | $CH(OCH_3)_2$ | |
| $NO_2$ | 4-Cl | H | $CH_3$ | $CH(OCH_3)_2$ | |
| Cl | 5-$OCH_3$ | H | $OCH_3$ | $CH(OCH_3)_2$ | |
| F | H | H | $CH_3$ | $CH(OCH_3)_2$ | |
| H | H | H | $CH_3$ | $CH(OCH_3)_2$ | |
| $SO_2N(CH_3)_2$ | H | H | $OCH_3$ | $CH(OCH_3)_2$ | |
| $SO_2N(CH_3)C_2H_5$ | H | H | $OCH_2CH_3$ | $CH(OCH_3)_2$ | |
| $OCH_3$ | H | $CH_3$ | $CH_3$ | $CH(OCH_3)_2$ | |
| $OSO_2CH_3$ | H | H | $CH_3$ | $CH(OCH_3)_2$ | |

TABLE 2b

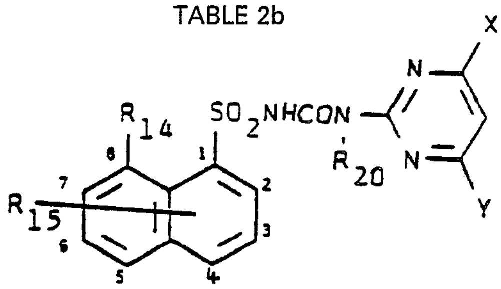

| $R_{14}$ | $R_{15}$ | $R_{20}$ | X | Y | m.p.(°C) |
|---|---|---|---|---|---|
| Cl | H | H | $CH_3$ | $CH_2OTHP$ | |
| Br | H | H | $CH_3$ | $CH_2OTHP$ | |
| F | H | H | $OCH_3$ | $CH_2OTHP$ | |
| H | H | H | $OCH_3$ | $CH_2OTHP$ | |
| Cl | 3-Cl | H | $CH_3$ | $CH_2OTHP$ | |
| Cl | H | H | $OCH_2CH_3$ | $CH_2OTHP$ | |
| $OCH_3$ | H | H | $CH_3$ | $CH_2OTHP$ | |
| $CH_3$ | H | $CH_3$ | $OCH_3$ | $CH_2OTHP$ | |
| $CH_3$ | H | H | $CH_3$ | $CH_2OTHP$ | |
| $NO_2$ | H | H | $OCH_3$ | $CH_2OTHP$ | |
| $CH_3$ | 4-F | H | $OCH_3$ | $CH_2OTHP$ | |
| Cl | 6-$NO_2$ | H | $OCH_3$ | $CH_2OTHP$ | |
| $NO_2$ | H | H | $CH_2CH_3$ | $CH_2OTHP$ | |
| Cl | 6-F | H | $CH_3$ | $CH_2OTHP$ | |
| $NO_2$ | 4-Cl | H | $CH_3$ | $CH_2OTHP$ | |
| Cl | 5-$OCH_3$ | H | $OCH_3$ | $CH_2OTHP$ | |
| F | H | H | $CH_3$ | $CH_2OTHP$ | |
| H | H | H | $CH_3$ | $CH_2OTHP$ | |
| $SO_2N(CH_3)_2$ | H | H | $OCH_3$ | $CH_2OTHP$ | |
| $SO_2N(CH_3)C_2H_5$ | H | H | $OCH_3$ | $CH_2OTHP$ | |
| $OCH_3$ | H | H | $CH_3$ | $CH_2OTHP$ | |
| $OSO_2CH_3$ | H | H | $CH_3$ | $CH_2OTHP$ | |

TABLE 2c

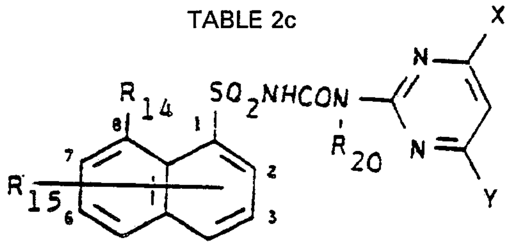

| $R_{14}$ | $R_{15}$ | $R_{20}$ | X | Y | m.p.(°C) |
|---|---|---|---|---|---|
| Cl | H | H | $CH_3$ | $CH_2OH$ | |
| Br | H | H | $CH_3$ | $CH_2OH$ | |
| F | H | H | $OCH_3$ | $CH_2OH$ | |
| H | H | H | $OCH_3$ | $CH_2OH$ | |
| Cl | 3-Cl | H | $CH_3$ | $CH_2OH$ | |
| Cl | H | H | $OCH_3$ | $CH_2OH$ | |
| $OCH_3$ | H | H | $CH_2CH_3$ | $CH_2OH$ | |
| $CH_3$ | H | H | $OCH_3$ | $CH_2OH$ | |
| $CH_3$ | H | H | $CH_3$ | $CH_2OH$ | |
| $NO_2$ | H | $CH_3$ | $OCH_3$ | $CH_2OH$ | |
| $CH_3$ | 4-F | H | $OCH_3$ | $CH_2OH$ | |
| Cl | 6-$NO_2$ | H | $OCH_2CH_3$ | $CH_2OH$ | |
| $NO_2$ | H | H | $CH_3$ | $CH_2OH$ | |
| Cl | 6-F | H | $CH_3$ | $CH_2OH$ | |
| $NO_2$ | 4-Cl | H | $CH_3$ | $CH_2OH$ | |
| Cl | 5-$OCH_3$ | H | $OCH_3$ | $CH_2OH$ | |
| F | H | H | $CH_3$ | $CH_2OH$ | |
| H | H | H | $CH_3$ | $CH_2OH$ | |
| $SO_2N(CH_3)_2$ | H | H | $OCH_3$ | $CH_2OH$ | |
| $SO_2N(CH_3)C_2H_5$ | H | H | $OCH_3$ | $CH_2OH$ | |
| $OCH_3$ | H | H | $CH_3$ | $CH_2OH$ | |
| $OSO_2CH_3$ | H | $CH_3$ | $CH_3$ | $CH_2OH$ | |
| $SO_2$-$n$-$O_3H_7$ | H | H | $OCH_2CH_3$ | $CH_2OH$ | |

TABLE 2d

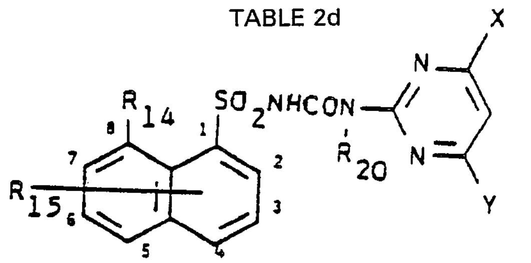

| $R_{14}$ | $R_{15}$ | $R_{20}$ | X | Y | m.p.(°C) |
|---|---|---|---|---|---|
| Cl | H | H | $CH_3$ | $CH(OCH_2CH_2O)$ | |
| Br | H | H | $CH_3$ | $CH_2OCOCH_3$ | |
| F | H | H | $OCH_3$ | $CH_3SC_3H_5$ | |
| H | H | H | $OCH_3$ | $CH_2OCO_2CH_3$ | |
| Cl | 3-Cl | H | $CH_3$ | $CH_2SCH_3$ | |
| Cl | H | H | $OCH_3$ | $CH(OC_2H_5)_2$ | |
| $OCH_3$ | H | H | $CH_3$ | $CH(OCH_2CH_2CH_2O)$ | |
| $CH_3$ | H | H | $OCH_3$ | $CH_2OCO$-*i*-$C_2H_7$ | |
| $CH_3$ | H | H | $CH_3$ | $CH_2OCO_2C_2H_5$ | |
| $NO_2$ | H | H | $OCH_3$ | $CH(OC_2H_5)_2$ | |
| $CH_3$ | 4-F | $CH_3$ | $OCH_3$ | $CH(OCH_2CH_2O)$ | |
| Cl | 6-$NO_2$ | H | $OCH_3$ | $CH_2OCOCH_3$ | |
| $NO_2$ | H | H | $CH_3$ | $CH_2OCOC_2H_5$ | |
| Cl | 6-F | H | $CH_3$ | $CH_2OCO_2$-*n*-$C_3H_7$ | |
| $NO_2$ | 4-Cl | H | $CH_3$ | $CH_2SCH_3$ | |
| Cl | 5-$OCH_3$ | H | $OCH_3$ | $CH_2SC_2H_5$ | |
| F | H | H | $CH_3$ | $CH(SCH(CH_3)CH_2S)$ | |
| H | H | H | $CH_3$ | $CH(OC_2H_5)_2$ | |
| $SO_2N(CH_3)_2$ | H | H | $OCH_3$ | $CH(OCH_2CH_2O)$ | |
| $SO_2N(CH_3)C_2H_5$ | H | H | $OCH_3$ | $CH_2OCONHCH_3$ | |
| $OCH_3$ | H | H | $CH_3$ | $CH_2OCONH$-*n*-$C_3H_7$ | |
| $OSO_2CH_3$ | H | H | $CH_3$ | $CH_2OCOCH_3$ | |
| $SO_2$-*n*-$C_3H_7$ | H | H | $OCH_3$ | $CH(OC_2H_5)_2$ | |

TABLE 2e

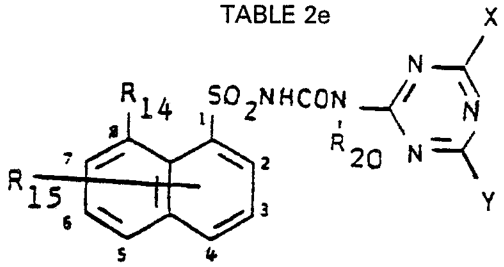

| $R_{14}$ | $R_{15}$ | $R_{20}$ | X | Y | m.p.(°C) |
|---|---|---|---|---|---|
| Cl | H | H | $CH_3$ | $CH(OCH_3)_2$ | |
| Br | H | H | $CH_2CH_3$ | $CH(OCH_3)_2$ | |
| F | H | H | $OCH_3$ | $CH(OCH_3)_2$ | |
| H | H | H | $OCH_3$ | $CH(OCH_3)_2$ | |
| Cl | 3-Cl | H | $CH_3$ | $CH(OCH_3)_2$ | |
| Cl | H | H | $OCH_2CH_3$ | $CH(OCH_3)_2$ | |
| $OCH_3$ | H | H | $CH_3$ | $CH(OCH_3)_2$ | |
| $CH_3$ | H | H | $OCH_3$ | $CH(OCH_3)_2$ | |
| $CH_3$ | H | $CH_3$ | $CH_3$ | $CH(OCH_3)_2$ | |
| $NO_2$ | H | H | $OCH_3$ | $CH(OCH_3)_2$ | |
| $CH_3$ | 4-F | H | $OCH_3$ | $CH(OCH_3)_2$ | |
| Cl | 6-$NO_2$ | H | $OCH_3$ | $CH(OCH_3)_2$ | |
| $NO_2$ | H | H | $CH_2$ | $CH(OCH_3)_2$ | |
| Cl | 6-F | H | $CH_3$ | $CH(OCH_3)_2$ | |
| $NO_2$ | 4-Cl | H | $CH_2CH_3$ | $CH(OCH_3)_2$ | |
| Cl | 5-$OCH_3$ | H | $OCH_3$ | $CH(OCH_3)_2$ | |
| F | H | H | $CH_3$ | $CH(OCH_3)_2$ | |
| H | H | H | $CH_3$ | $CH(OCH_3)_2$ | |
| $SO_2N(CH_3)_2$ | H | H | $OCH_3$ | $CH(OCH_3)_2$ | |
| $SO_2N(CH_3)C_2H_5$ | H | H | $OCH_2CH_3$ | $CH(OCH_3)_2$ | |
| $OCH_3$ | H | H | $CH_3$ | $CH(OCH_3)_2$ | |
| $OSO_2CH_3$ | H | H | $CH_3$ | $CH(OCH_3)_2$ | |
| $SO_2$-*n*-$C_3H_7$ | H | H | $OCH_3$ | $CH(OCH_3)_2$ | |

TABLE 2f

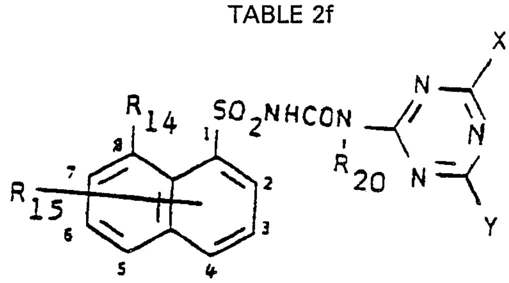

| R14 | R15 | R20 | X | Y | m.p.(°C) |
|---|---|---|---|---|---|
| Cl | H | $CH_3$ | $CH_2CH_3$ | $CH_2OTHP$ | |
| Br | H | H | $CH_3$ | $CH_2OTHP$ | |
| F | H | H | $OCH_3$ | $CH_2OTHP$ | |
| H | H | H | $OCH_3$ | $CH_2OTHP$ | |
| Cl | 3-Cl | H | $CH_3$ | $CH_2OTHP$ | |
| Cl | H | H | $OCH_2CH_3$ | $CH_2OTHP$ | |
| $OCH_3$ | H | H | $CH_3$ | $CH_2OTHP$ | |
| $CH_3$ | H | H | $OCH_3$ | $CH_2OTHP$ | |
| $CH_3$ | H | H | $CH_3$ | $CH_2OTHP$ | |
| $NO_2$ | H | H | $OCH_3$ | $CH_2OTHP$ | |
| $CH_3$ | 4-F | H | $OCH_3$ | $CH_2OTHP$ | |
| Cl | 6-$NO_2$ | H | $OCH_3$ | $CH_2OTHP$ | |
| $NO_2$ | H | H | $CH_3$ | $CH_2OTHP$ | |
| Cl | 6—F | H | $CH_3$ | $CH_2OTHP$ | |
| $NO_2$ | 4-Cl | H | $CH_3$ | $CH_2OTHP$ | |
| Cl | 5-$OCH_3$ | H | $OCH_3$ | $CH_2OTHP$ | |
| F | H | H | $CH_3$ | $CH_2OTHP$ | |
| H | H | H | $CH_3$ | $CH_2OTHP$ | |
| $SO_2N(CH_3)_2$ | H | $CH_3$ | $OCH_3$ | $CH_2OTHP$ | |
| $SO_2N(CH_3)C_2H_5$ | H | H | $OCH_3$ | $CH_2OTHP$ | |
| $OCH_3$ | H | H | $CH_2CH_3$ | $CH_2OTHP$ | |
| $OSO_2CH_3$ | H | H | $CH_3$ | $CH_2OTHP$ | |
| $SO_2$-*n*-$C_3H_7$ | H | H | $OCH_3$ | $CH_2OTHP$ | |

TABLE 2g

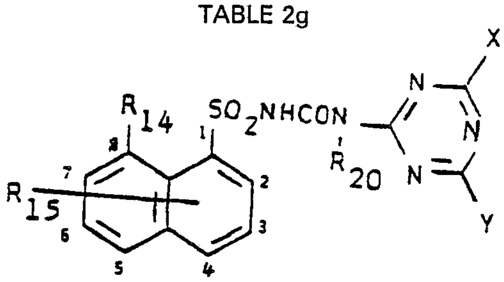

| $R_{14}$ | $R_{15}$ | $R_{20}$ | X | Y | m.p.(°C) |
|---|---|---|---|---|---|
| Cl | H | H | $CH_3$ | $CH_2OH$ | |
| Br | H | H | $CH_3$ | $CH_2OH$ | |
| F | H | H | $OCH_2CH_3$ | $CH_2OH$ | |
| H | H | H | $OCH_3$ | $CH_2OH$ | |
| Cl | 3-Cl | H | $CH_3$ | $CH_2OH$ | |
| Cl | H | H | $OCH_3$ | $CH_2OH$ | |
| $OCH_3$ | H | H | $CH_3$ | $CH_2OH$ | |
| $CH_3$ | H | H | $OCH_3$ | $CH_2OH$ | |
| $CH_3$ | H | H | $CH_3$ | $CH_2OH$ | |
| $NO_2$ | H | H | $OCH_3$ | $CH_2OH$ | |
| $CH_3$ | 4-F | H | $OCH_3$ | $CH_2OH$ | |
| Cl | 6-$NO_2$ | H | $OCH_3$ | $CH_2OH$ | |
| $NO_2$ | H | H | $CH_3$ | $CH_2OH$ | |
| Cl | 6-F | H | $CH_3$ | $CH_2OH$ | |
| $NO_2$ | 4-Cl | H | $CH_3$ | $CH_2OH$ | |
| Cl | 5-$OCH_3$ | H | $OCH_3$ | $CH_2OH$ | |
| F | H | H | $CH_3$ | $CH_2OH$ | |
| H | H | H | $CH_3$ | $CH_2OH$ | |
| $SO_2N(CH_3)_2$ | H | $CH_3$ | $OCH_3$ | $CH_2OH$ | |
| $SO_2N(CH_3)C_2H_5$ | H | H | $OCH_3$ | $CH_2OH$ | |
| $OCH_3$ | H | H | $CH_3$ | $CH_2OH$ | |
| $OSO_2CH_3$ | H | H | $CH_2CH_3$ | $CH_2OH$ | |
| $SO_2$-*n*-$C_3H_7$ | H | H | $OCH_3$ | $CH_2OH$ | |

Table 2h

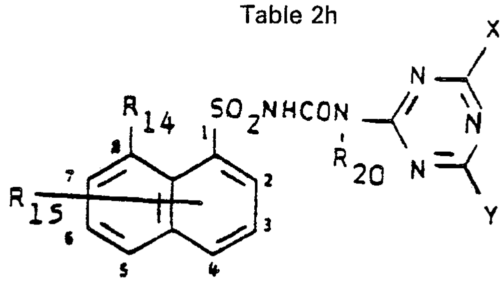

| $R_{14}$ | $R_{15}$ | $R_{20}$ | X | Y | m.p.(°C) |
|---|---|---|---|---|---|
| Cl | H | H | $CH_3$ | $CH(OCH_2CH_2O)$ | |
| Br | H | H | $CH_3$ | $CH_2OCOCH_3$ | |
| F | H | H | $OCH_3$ | $CH_2SC_2H_5$ | |
| H | H | H | $OCH_3$ | $CH_2OCO_2CH_3$ | |
| Cl | 3-Cl | H | $CH_3$ | $CH_2SCH_3$ | |
| Cl | H | H | $OCH_3$ | $CH(OC_2H_5)_2$ | |
| $OCH_3$ | H | H | $CH_3$ | $CH(OCH_2CH_2CH_2O)$ | |
| $CH_3$ | H | H | $OCH_3$ | $CH_2OCO$-*i*-$C_3H_7$ | |
| $CH_3$ | H | H | $CH_3$ | $CH_2OCO_2C_2H_5$ | |
| $NO_2$ | H | H | $OCH_3$ | $CH(OC_2H_5)_2$ | |
| $CH_3$ | 4-F | H | $OCH_3$ | $CH(OCH_2CH_2O)$ | |
| Cl | 6-$NO_2$ | H | $OCH_3$ | $CH_2OCOCH_3$ | |
| $NO_2$ | H | $CH_3$ | $CH_3$ | $CH_2OCOC_2H_5$ | |
| Cl | 6-F | H | $CH_3$ | $CH_2OCO_2$-*n*-$C_3H_7$ | |
| $NO_2$ | 4-Cl | $CH_3$ | $CH_3$ | $CH_2SCH_3$ | |
| Cl | 5-$OCH_3$ | H | $OCH_3$ | $CH_2SC_2H_5$ | |
| F | H | H | $CH_3$ | $CH(SCH(CH_3)CH_2S)$ | |
| H | H | H | $CH_3$ | $CH(OC_2H_5)_2$ | |
| $SO_2N(CH_3)_2$ | H | H | $OCH_3$ | $CH(OCH_2CH_2O)$ | |
| $SO_2N(CH_3)C_2H_5$ | H | H | $OCH_3$ | $CH_2OCONHCH_3$ | |
| $OCH_3$ | H | H | $CH_3$ | $CH_2OCONH$-*n*-$C_2H_7$ | |
| $OSO_2CH_3$ | H | H | $CH_3$ | $CH_2OCOCH_3$ | |
| $SO_2$-*n*-$C_3H_7$ | H | H | $OCH_3$ | $CH(OC_2H_5)_2$ | |

TABLE 3a

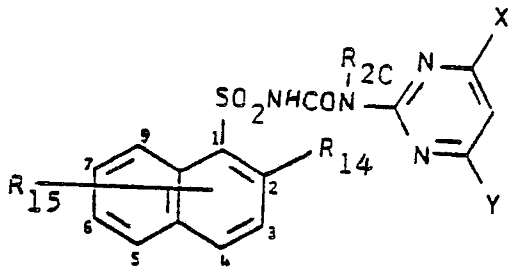

| $R_{14}$ | $R_{15}$ | $R_{20}$ | X | Y | m.p.(°C) |
|---|---|---|---|---|---|
| Cl | H | H | $CH_3$ | $CH(OCH_3)_2$ | |
| Br | H | H | $CH_3$ | $CH(OCH_3)_2$ | |
| F | H | H | $OCH_3$ | $CH(OCH_3)_2$ | |
| H | H | H | $OCH_3$ | $CH(OCH_3)_2$ | |
| Cl | H | H | Cl | $CH(OCH_3)_2$ | |
| Cl | 3-Cl | H | $CH_3$ | $CH(OCH_3)_2$ | |
| Cl | H | H | $OCH_3$ | $CH(OCH_3)_2$ | |
| $OCH_3$ | H | H | $CH_2CH_3$ | $CH(OCH_3)_2$ | |
| $CH_3$ | H | H | $OCH_3$ | $CH(OCH_3)_2$ | |
| Br | H | H | Cl | $CH(OCH_3)_2$ | |
| $CH_3$ | H | H | $CH_3$ | $CH(OCH_3)_2$ | |
| $NO_2$ | H | H | $OCH_3$ | $CH(OCH_3)_2$ | |
| $CH_3$ | 4-F | H | $OCH_3$ | $CH(OCH_3)_2$ | |
| Cl | 6-$NO_2$ | H | $OCH_3$ | $CH(OCH_3)_2$ | |
| $NO_2$ | H | H | $CH_3$ | $CH(OCH_3)_2$ | |
| $NO_2$ | H | H | Cl | $CH(OCH_3)_2$ | |
| Cl | 6-F | $CH_3$ | $CH_3$ | $CH(OCH_3)_2$ | |
| $NO_2$ | 4-Cl | H | $CH_3$ | $CH(OCH_3)_2$ | |
| Cl | 5-$OCH_3$ | H | $OCH_3$ | $CH(OCH_3)_2$ | |
| F | H | H | $CH_3$ | $CH(OCH_3)_2$ | |
| H | H | H | $CH_3$ | $CH(OCH_3)_2$ | |
| $SO_2N(CH_3)_2$ | H | H | $OCH_3$ | $CH(OCH_3)_2$ | |
| $SO_2N(CH_3)C_2H_5$ | H | H | $OCH_2CH_3$ | $CH(OCH_3)_2$ | |
| $OCH_3$ | H | H | $CH_3$ | $CH(OCH_3)_2$ | |
| $OSO_2CH_3$ | H | H | $CH_3$ | $CH(OCH_3)_2$ | |

TABLE 3b

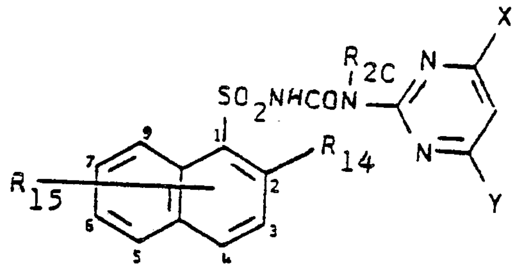

| $R_{14}$ | $R_{15}$ | $R_{20}$ | X | Y | m.p.(°C) |
|---|---|---|---|---|---|
| Cl | H | H | $CH_2CH_3$ | $CH_2OTHP$ | |
| Br | H | H | $CH_3$ | $CH_2OTHP$ | |
| F | H | H | $OCH_3$ | $CH_2OTHP$ | |
| H | H | H | $OCH_3$ | $CH_2OTHP$ | |
| Cl | 3-Cl | H | $CH_3$ | $CH_2OTHP$ | |
| Cl | H | H | $OCH_2CH_3$ | $CH_2OTHP$ | |
| $OCH_3$ | H | H | $CH_3$ | $CH_2OTHP$ | |
| $CH_3$ | H | H | $OCH_3$ | $CH_2OTHP$ | |
| $CH_3$ | H | H | $CH_3$ | $CH_2OTHP$ | |
| $NO_2$ | H | H | $OCH_3$ | $CH_2OTHP$ | |
| $CH_3$ | 4-F | H | $OCH_3$ | $CH_2OTHP$ | |
| Cl | 6-$NO_2$ | H | $OCH_3$ | $CH_2OTHP$ | |
| $NO_2$ | H | H | $CH_3$ | $CH_2OTHP$ | |
| Cl | 6-F | H | $CH_3$ | $CH_2OTHP$ | |
| $NO_2$ | 4-Cl | H | $CH_3$ | $CH_2OTHP$ | |
| Cl | 5-$OCH_3$ | H | $OCH_3$ | $CH_2OTHP$ | |
| F | H | $CH_3$ | $CH_3$ | $CH_2OTHP$ | |
| H | H | $CH_3$ | $CH_3$ | $CH_2OTHP$ | |
| $SO_2N(CH_3)_2$ | H | H | $OCH_2CH_3$ | $CH_2OTHP$ | |
| $SO_2N(CH_3)C_2H_5$ | H | H | $OCH_3$ | $CH_2OTHP$ | |
| $OCH_3$ | H | H | $CH_3$ | $CH_2OTHP$ | |
| $OSO_2CH_3$ | H | H | $CH_3$ | $CH_2OTHP$ | |
| $SO_2$-$n$-$C_3H_7$ | H | H | $OCH_3$ | $CH_2OTHP$ | |
| $SO_2N(OCH_3)CH_3$ | H | H | $CH_3$ | $CH_2OTHP$ | |
| O-$n$-$C_3H_7$ | H | H | $CH_3$ | $CH_2OTHP$ | |

TABLE 3c

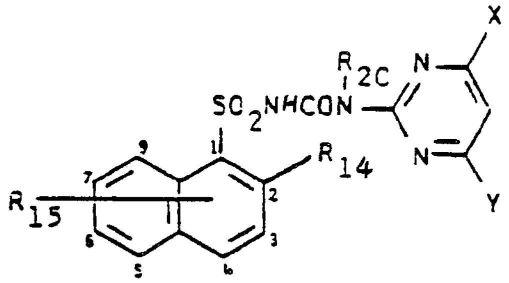

| $R_{14}$ | $R_{15}$ | $R_{20}$ | X | Y | m.p.(°C) |
|---|---|---|---|---|---|
| Cl | H | $CH_3$ | $CH_3$ | $CH_2OH$ | |
| Br | H | H | $CH_3$ | $CH_2OH$ | |
| F | H | H | $OCH_3$ | $CH_2OH$ | |
| H | H | H | $OCH_3$ | $CH_2OH$ | |
| Cl | 3-Cl | H | $CH_3$ | $CH_2OH$ | |
| Cl | H | H | $OCH_3$ | $CH_2OH$ | |
| $OCH_3$ | H | H | $CH_3$ | $CH_2OH$ | |
| $CH_3$ | H | H | $OCH_3$ | $CH_2OH$ | |
| $CH_3$ | H | H | $CH_2CH_3$ | $CH_2OH$ | |
| $NO_2$ | H | H | $OCH_3$ | $CH_2OH$ | |
| $CH_3$ | 4-F | H | $OCH_3$ | $CH_2OH$ | |
| Cl | 6-$NO_2$ | H | $OCH_3$ | $CH_2OH$ | |
| $NO_2$ | H | H | $CH_3$ | $CH_2OH$ | |
| Cl | 6-F | H | $CH_3$ | $CH_2OH$ | |
| $NO_2$ | 4-Cl | H | $CH_3$ | $CH_2OH$ | |
| Cl | 5-$OCH_3$ | H | $OCH_2CH_3$ | $CH_2OH$ | |
| F | H | H | $CH_3$ | $CH_2OH$ | |
| H | H | H | $CH_3$ | $CH_2OH$ | |
| $SO_2N(CH_3)_2$ | H | $CH_3$ | $OCH_3$ | $CH_2OH$ | |
| $SO_2N(CH_3)C_2H_5$ | H | H | $OCH_3$ | $CH_2OH$ | |
| $OCH_3$ | H | H | $CH_3$ | $CH_2OH$ | |
| $OSO_2CH_3$ | H | H | $CH_2CH_3$ | $CH_2OH$ | |
| $SO_2$-*n*-$C_3H_7$ | H | H | $OCH_3$ | $CH_2OH$ | |

TABLE 3d

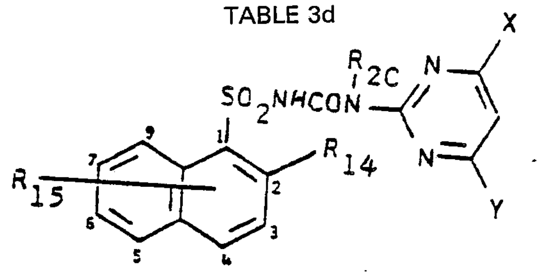

| $R_{14}$ | $R_{15}$ | $R_{20}$ | X | Y | m.p.(°C) |
|---|---|---|---|---|---|
| Cl | H | H | $CH_3$ | $CH(OCH_2CH_2O)$ | |
| Br | H | H | $CH_3$ | $CH_2OCOCH_3$ | |
| F | H | H | $OCH_3$ | $CH_2SC_2H_5$ | |
| H | H | $CH_3$ | $OCH_3$ | $CH_2OCO_2CH_3$ | |
| Cl | 3-Cl | H | $CH_3$ | $CH_2SCH_3$ | |
| Cl | H | H | $OCH_3$ | $CH(OC_2H_5)_2$ | |
| $OCH_3$ | H | H | $CH_2CH_3$ | $CH(SCH_2CH_2CH_2S)$ | |
| $CH_3$ | H | H | $OCH_3$ | $CH_2OCO$-*i*-$C_3H_7$ | |
| $CH_3$ | H | H | $CH_3$ | $CH_2OCO_2C_2H_5$ | |
| $NO_2$ | H | H | $OCH_3$ | $CH(OC_2H_5)_2$ | |
| $CH_3$ | 4-F | H | $OCH_3$ | $CH(OCH_2CH_2O)$ | |
| Cl | 6-$NO_2$ | H | $OCH_3$ | $CH_2OCOCH_3$ | |
| $NO_2$ | H | H | $CH_3$ | $CH_2OCOC_2H_5$ | |
| Cl | 6-F | H | $CH_3$ | $CH_2OCO_2$-*n*-$C_3H_7$ | |
| $NO_2$ | 4-Cl | H | $CH_3$ | $CH_2SCH_3$ | |
| Cl | 5-$OCH_3$ | H | $OCH_3$ | $CH_2SC_2H_5$ | |
| F | H | $CH_3$ | $CH_3$ | $CH(OCHCH_3(CH_2O)$ | |
| H | H | H | $CH_3$ | $CH(OC_2H_5)_2$ | |
| $SO_2N(CH_3)_2$ | H | H | $OCH_3$ | $CH(OCH_2CH_2O)$ | |
| $SO_2N(CH_3)C_3H_5$ | H | H | $OCH_2CH_3$ | $CH_2OCONHCH_3$ | |
| $OCH_3$ | H | H | $CH_3$ | $CH_2OCONH$-*n*-$C_3H_7$ | |
| $OSO_2CH_3$ | H | H | $CH_3$ | $CH_2OCOCH_3$ | |
| $SO_2$-*n*-$C_3H_7$ | H | H | $OCH_3$ | $CH(OC_2H_5)_2$ | |

TABLE 3e

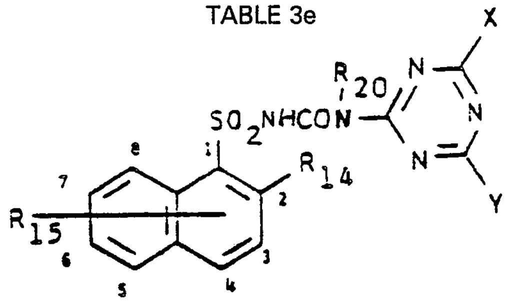

| R14 | R15 | R20 | X | Y | m.p.(°C) |
|---|---|---|---|---|---|
| Cl | H | H | $CH_3$ | $CH(OCH_3)_2$ | |
| Br | H | H | $CH_3$ | $CH(OCH_3)_2$ | |
| F | H | H | $OCH_3$ | $CH(OCH_3)_2$ | |
| H | H | H | $OCH_3$ | $CH(OCH_3)_2$ | |
| Cl | 3-Cl | H | $CH_3$ | $CH(OCH_3)_2$ | |
| Cl | H | H | $OCH_2CH_3$ | $CH(OCH_3)_2$ | |
| $OCH_3$ | H | H | $CH_3$ | $CH(OCH_3)_2$ | |
| $CH_3$ | H | H | $OCH_3$ | $CH(OCH_3)_2$ | |
| $CH_3$ | H | H | $CH_3$ | $CH(OCH_3)_2$ | |
| $NO_2$ | H | H | $OCH_2CH_3$ | $CH(OCH_3)_2$ | |
| $CH_3$ | 4-F | $CH_3$ | $OCH_3$ | $CH(OCH_3)_2$ | |
| Cl | 6-$NO_2$ | H | $OCH_3$ | $CH(OCH_3)_2$ | |
| $NO_2$ | H | H | $CH_3$ | $CH(OCH_3)_2$ | |
| Cl | 6-F | H | $CH_3$ | $CH(OCH_3)_2$ | |
| $NO_2$ | 4-Cl | H | $CH_3$ | $CH(OCH_3)_2$ | |
| Cl | 5-$OCH_3$ | H | $OCH_3$ | $CH(OCH_3)_2$ | |
| F | H | H | $CH_3$ | $CH(OCH_3)_2$ | |
| H | H | H | $CH_3$ | $CH(OCH_3)_2$ | |
| $SO_2N(CH_3)_2$ | H | H | $OCH_3$ | $CH(OCH_3)_2$ | |
| $SO_2N(CH_3)C_2H_5$ | H | H | $OCH_3$ | $CH(OCH_3)_2$ | |
| $OCH_3$ | H | H | $CH_2CH_3$ | $CH(OCH_3)_2$ | |
| $OSO_2CH_3$ | H | H | $CH_3$ | $CH(OCH_3)_2$ | |
| $SO_2$-*n*-$C_3H_7$ | H | H | $OCH_3$ | $CH(OCH_3)_2$ | |

TABLE 3f

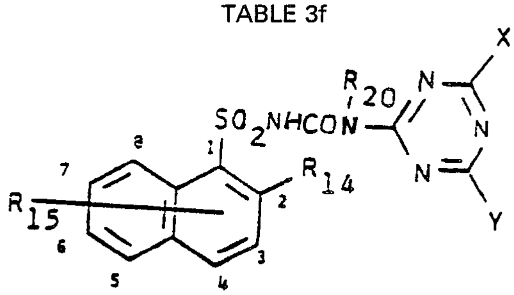

| $R_{14}$ | $R_{15}$ | $R_{20}$ | X | Y | m.p.(°C) |
|---|---|---|---|---|---|
| Cl | H | $CH_3$ | $CH_2CH_3$ | $CH_2OTHP$ | |
| Br | H | H | $CH_3$ | $CH_2OTHP$ | |
| F | H | H | $OCH_3$ | $CH_2OTHP$ | |
| H | H | H | $OCH_3$ | $CH_2OTHP$ | |
| Cl | 3-Cl | H | $CH_3$ | $CH_2OTHP$ | |
| Cl | H | H | $OCH_3$ | $CH_2OTHP$ | |
| $OCH_3$ | H | H | $CH_3$ | $CH_2OTHP$ | |
| $CH_3$ | H | H | $OCH_3$ | $CH_2OTHP$ | |
| $CH_3$ | H | H | $CH_3$ | $CH_2OTHP$ | |
| $NO_2$ | H | H | $OCH_2CH_3$ | $CH_2OTHP$ | |
| $CH_3$ | 4-F | H | $OCH_3$ | $CH_2OTHP$ | |
| Cl | 6-$NO_2$ | H | $OCH_3$ | $CH_2OTHP$ | |
| $NO_2$ | H | H | $CH_3$ | $CH_2OTHP$ | |
| Cl | 6-F | H | $CH_3$ | $CH_2OTHP$ | |
| $NO_2$ | 4-Cl | H | $CH_3$ | $CH_2OTHP$ | |
| Cl | 5-$OCH_3$ | H | $OCH_3$ | $CH_2OTHP$ | |
| F | H | H | $CH_3$ | $CH_2OTHP$ | |
| H | H | H | $CH_3$ | $CH_2OTHP$ | |
| $SO_2N(CH_3)_2$ | H | $CH_3$ | $OCH_3$ | $CH_2OTHP$ | |
| $SO_2N(CH_3)C_2H_5$ | H | H | $OCH_3$ | $CH_2OTHP$ | |
| $OCH_3$ | H | H | $CH_3$ | $CH_2OTHP$ | |
| $OSO_2CH_3$ | H | H | $CH_3$ | $CH_2OTHP$ | |
| $SO_2$-*n*-$C_3H_7$ | H | H | $OCH_2CH_3$ | $CH_2OTHP$ | |

TABLE 3g

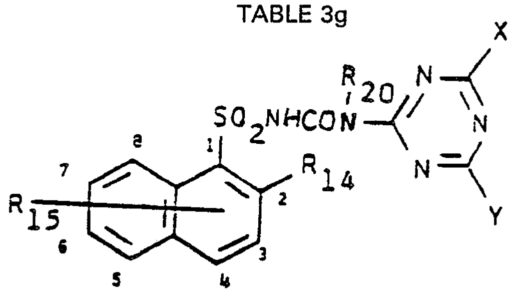

| $R_{14}$ | $R_{15}$ | $R_{20}$ | X | Y | m.p.(°C) |
|---|---|---|---|---|---|
| Cl | H | H | $CH_3$ | $CH_2OH$ | |
| Br | H | H | $CH_3$ | $CH_2OH$ | |
| F | H | H | $OCH_3$ | $CH_2OH$ | |
| H | H | H | $OCH_2CH_3$ | $CH_2OH$ | |
| Cl | 3-Cl | H | $CH_3$ | $CH_2OH$ | |
| Cl | H | H | $OCH_3$ | $CH_2OH$ | |
| $OCH_3$ | H | H | $CH_3$ | $CH_2OH$ | |
| $CH_3$ | H | H | $OCH_3$ | $CH_2OH$ | |
| $CH_3$ | H | H | $CH_3$ | $CH_2OH$ | |
| $NO_2$ | H | H | $OCH_3$ | $CH_2OH$ | |
| $CH_3$ | 4-F | H | $OCH_3$ | $CH_2OH$ | |
| Cl | 6-$NO_2$ | H | $OCH_3$ | $CH_2OH$ | |
| $NO_2$ | H | H | $CH_3$ | $CH_2OH$ | |
| Cl | 6-F | H | $CH_3$ | $CH_2OH$ | |
| $NO_2$ | 4-Cl | H | $CH_3$ | $CH_2OH$ | |
| Cl | 5-$OCH_3$ | H | $OCH_3$ | $CH_2OH$ | |
| F | H | H | $CH_3$ | $CH_2OH$ | |
| H | H | H | $CH_3$ | $CH_2OH$ | |
| $SO_2N(CH_3)_2$ | H | H | $OCH_3$ | $CH_2OH$ | |
| $SO_2N(CH_3)C_2H_5$ | H | $CH_3$ | $OCH_3$ | $CH_2OH$ | |
| $OCH_3$ | H | H | $CH_2CH_3$ | $CH_2OH$ | |
| $OSO_2CH_3$ | H | H | $CH_3$ | $CH_2OH$ | |
| $SO_2$-*n*-$C_3H_7$ | H | H | $OCH_3$ | $CH_2OH$ | |

TABLE 3n

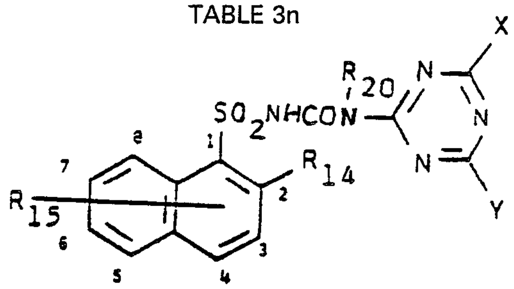

| $R_{14}$ | $R_{15}$ | $R_{20}$ | X | Y | m.p.(°C) |
|---|---|---|---|---|---|
| Cl | H | $CH_3$ | $CH_3$ | $CH(OCH_2CH_2O)$ | |
| Br | H | H | $CH_3$ | $CH_2OCOCH_3$ | |
| F | H | H | $OCH_2CH_3$ | $CH_2SC_2H_5$ | |
| H | H | H | $OCH_3$ | $CH_2OCO_2CH_3$ | |
| Cl | 3-Cl | H | $CH_3$ | $CH_2SCH_3$ | |
| Cl | H | H | $OCH_3$ | $CH(OC_2H_5)_2$ | |
| $OCH_3$ | H | H | $CH_3$ | $CH(OCH_2CH_2CH_2O)$ | |
| $CH_3$ | H | H | $OCH_3$ | $CH_2OCO$-*i*-$C_3H_7$ | |
| $CH_3$ | H | H | $CH_3$ | $CH_2OCO_2C_2H_5$ | |
| $NO_3$ | H | H | $OCH_3$ | $CH(OC_2H_5)_2$ | |
| $CH_3$ | 4-F | H | $OCH_3$ | $CH(OCH_2CH_2O)$ | |
| Cl | 6-$NO_2$ | H | $OCH_3$ | $CH_2OCOCH_3$ | |
| $NO_2$ | H | H | $CH_2CH_3$ | $CH_2OCOC_2H_5$ | |
| Cl | 6-F | H | $CH_3$ | $CH_2OCO_2$-*n*-$C_3H_7$ | |
| $NO_2$ | 4-Cl | H | $CH_3$ | $CH_2SCH_3$ | |
| Cl | 5-$OCH_3$ | H | $OCH_3$ | $CH_2SC_2H_5$ | |
| F | H | H | $CH_3$ | $CH(SCH(CH_3)CH_2S)$ | |
| H | H | H | $CH_3$ | $CH(OC_2H_5)_2$ | |
| $SO_2N(CH_3)_2$ | H | $CH_3$ | $OCH_3$ | $CH(OCH_2CH_2O)$ | |
| $SO_2N(CH_3)C_2H_5$ | H | H | $OCH_3$ | $CH_2OCONHCH_3$ | |
| $OCH_3$ | H | H | $CH_3$ | $CH_2OCONH$-*n*-$C_3H_7$ | |
| $OSO_2CH_3$ | H | H | $CH_3$ | $CH_2OCOCH_3$ | |
| $SO_2$-*n*-$C_3H_7$ | H | H | $OCH_3$ | $CH(OC_2H_5)_2$ | |

TABLE 4a

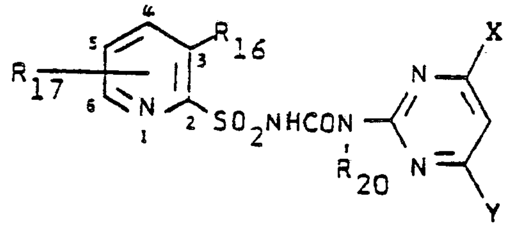

| $R_{16}$ | $R_{17}$ | $R_{20}$ | X | Y | m.p.(°C) |
|---|---|---|---|---|---|
| $CO_2CH_3$ | H | H | $CH_3$ | $CH(OCH_3)_2$ | |
| Cl | H | H | $CH_2CH_3$ | $CH(OCH_3)_2$ | |
| $CO_2CH_3$ | H | H | Cl | $CH(OCH_3)_2$ | |
| Cl | H | $CH_3$ | $OCH_3$ | $CH(OCH_3)_2$ | |
| H | H | H | $OCH_3$ | $CH(OCH_3)_2$ | |
| $CO_2CH(CH_3)_2$ | 5-F | H | $CH_3$ | $CH(OCH_3)_2$ | |
| $CO_2CH(CH_3)_2$ | H | H | $CH_3$ | $CH(OCH_3)_2$ | |
| $NO_2$ | H | H | $CH_3$ | $CH(OCH_3)_2$ | |
| $NO_2$ | 6-Br | H | $CH_3$ | $CH(OCH_3)_2$ | |
| Cl | 4-Cl | H | Cl | $CH(OCH_3)_2$ | |
| $CF_3$ | H | H | $OCH_3$ | $CH(OCH_3)_2$ | |
| Cl | 6-$CH_3$ | $CH_3$ | $OCH_3$ | $CH(OCH_3)_2$ | |
| $C_2H_5$ | H | H | $CH_3$ | $CH(OCH_3)_2$ | |
| $CH(CH_3)_2$ | H | H | $CH_3$ | $CH(OCH_3)_2$ | |
| $SO_2CH_3$ | H | H | $CH_3$ | $CH(OCH_3)_2$ | |
| $SO_2CH_3$ | 4-$OCH_3$ | H | $OCH_3$ | $CH(OCH_3)_2$ | |
| $SO_2CH_3$ | 6-F | H | $OCH_3$ | $CH(OCH_3)_2$ | |
| $SO_2$-*n*-$C_4H_9$ | H | H | $CH_3$ | $CH(OCH_3)_2$ | |
| $SO_2N(CH_3)_2$ | H | H | $OCH_2CH_3$ | $CH(OCH_3)_2$ | |
| $SO_2N(C_2H_5)_2$ | H | H | $CH_3$ | $CH(OCH_3)_2$ | |
| $SO_2N(OCH_3)CH_3$ | H | H | $CH_3$ | $CH(OCH_3)_2$ | |
| $SO_2$-*n*-$C_3H_7$ | H | H | $CH_3$ | $CH(OCH_3)_2$ | |
| $SO_2$-*i*-$C_3H_7$ | H | H | $OCH_3$ | $CH(OCH_3)_2$ | |
| $SCH_3$ | H | H | $CH_3$ | $CH(OCH_3)_2$ | |
| $S(O)CH_3$ | H | H | $CH_3$ | $CH(OCH_3)_2$ | |
| $SCH_2CH_3$ | H | H | $OCH_3$ | $CH(OCH_3)_2$ | |
| $S(O)(CH_2)_3CH_3$ | H | H | $OCH_3$ | $CH(OCH_3)_2$ | |

TABLE 4b

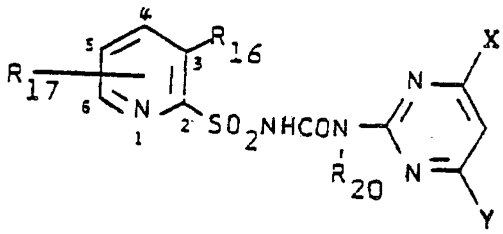

| $R_{16}$ | $R_{17}$ | $R_{20}$ | X | Y | m.p.(°C) |
|---|---|---|---|---|---|
| $CO_2CH_3$ | H | H | $CH_3$ | $CH_2OTHP$ | |
| Cl | H | H | $CH_2CH_3$ | $CH_2OTHP$ | |
| Cl | H | H | $OCH_3$ | $CH_2OTHP$ | |
| H | H | H | $OCH_3$ | $CH_2OTHP$ | |
| $CO_2CH(CH_3)_2$ | 5-F | H | $CH_3$ | $CH_2OTHP$ | |
| $CO_2CH(CH_3)_2$ | H | H | $CH_3$ | $CH_2OTHP$ | |
| $NO_2$ | H | H | $CH_3$ | $CH_2OTHP$ | |
| $NO_2$ | 6-Br | H | $CH_3$ | $CH_2OTHP$ | |
| Cl | 4-Cl | H | $OCH_3$ | $CH_2OTHP$ | |
| $CF_3$ | H | H | $OCH_3$ | $CH_2OTHP$ | |
| Cl | 6-$CH_3$ | H | $OCH_3$ | $CH_2OTHP$ | |
| $C_2H_5$ | H | H | $CH_3$ | $CH_2OTHP$ | |
| $CH(CH_3)_2$ | H | H | $CH_3$ | $CH_2OTHP$ | |
| $SO_2CH_3$ | H | H | $CH_3$ | $CH_2OTHP$ | |
| $SO_2CH_3$ | 4-$OCH_3$ | H | $OCH_3$ | $CH_2OTHP$ | |
| $SO_2CH_3$ | 6-F | H | $OCH_3$ | $CH_2OTHP$ | |
| $SO_2$-*n*-$C_4H_9$ | H | H | $CH_3$ | $CH_2OTHP$ | |
| $SO_2N(CH_3)_2$ | H | $CH_3$ | $OCH_3$ | $CH_2OTHP$ | |
| $SO_2N(C_2H_5)_2$ | H | H | $CH_3$ | $CH_2OTHP$ | |
| $SO_2N(OCH_3)CH_3$ | H | H | $CH_3$ | $CH_2OTHP$ | |
| $SO_2$-*n*-$C_3H_7$ | H | H | $CH_3$ | $CH_2OTHP$ | |
| $SO_2$-*n*-$C_3H_7$ | H | H | $OCH_2CH_3$ | $CH_2OTHP$ | |
| $SCH_3$ | H | H | $CH_3$ | $CH_2OTHP$ | |
| $S(O)CH_3$ | H | H | $CH_3$ | $CH_2OTHP$ | |
| $SCH_2CH_3$ | H | H | $OCH_3$ | $CH_2OTHP$ | |
| $S(O)$-*n*-$C_4H_9$ | H | H | $OCH_3$ | $CH_2OTHP$ | |

TABLE 4c

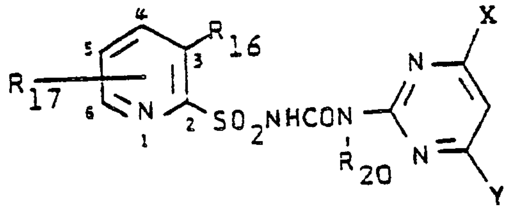

| $R_{16}$ | $R_{17}$ | $R_{20}$ | X | Y | m.p.(°C) |
|---|---|---|---|---|---|
| $CO_2CH_3$ | H | H | $CH_3$ | $CH_2OH$ | |
| Cl | H | H | $CH_3$ | $CH_2OH$ | |
| Cl | H | $CH_3$ | $OCH_3$ | $CH_2OH$ | |
| H | H | H | $OCH_3$ | $CH_2OH$ | |
| $CO_2CH(CH_3)_2$ | 5-F | H | $CH_3$ | $CH_2OH$ | |
| $CO_2CH(CH_3)_2$ | H | H | $CH_2CH_3$ | $CH_2OH$ | |
| $NO_2$ | H | H | $CH_3$ | $CH_2OH$ | |
| $NO_2$ | 6-Br | H | $CH_3$ | $CH_2OH$ | |
| Cl | 4-Cl | H | $OCH_3$ | $CH_2OH$ | |
| $CF_3$ | H | H | $OCH_3$ | $CH_2OH$ | |
| Cl | 6-$CH_3$ | $CH_3$ | $OCH_3$ | $CH_2OH$ | |
| $C_2H_5$ | H | H | $CH_3$ | $CH_2OH$ | |
| $CH(CH_3)_2$ | H | H | $CH_3$ | $CH_2OH$ | |
| $SO_2CH_3$ | H | H | $CH_3$ | $CH_2OH$ | |
| $SO_2CH_3$ | 4-$OCH_3$ | H | $OCH_2CH_3$ | $CH_2OH$ | |
| $SO_2CH_3$ | 6-F | H | $OCH_3$ | $CH_2OH$ | |
| $SO_2$-*n*-$C_4H_9$ | H | H | $CH_3$ | $CH_2OH$ | |
| $SO_2N(CH_3)_2$ | H | H | $OCH_3$ | $CH_2OH$ | |
| $SO_2N(C_2H_5)_2$ | H | H | $CH_3$ | $CH_2OH$ | |
| $SO_2N(OCH_3)CH_3$ | H | H | $CH_3$ | $CH_2OH$ | |
| $SO_2$-*n*-$C_3H_7$ | H | H | $CH_3$ | $CH_2OH$ | |
| $SO_2$-*i*-$C_3H_7$ | H | H | $OCH_3$ | $CH_2OH$ | |
| $SCH_3$ | H | H | $CH_2CH_3$ | $CH_2OH$ | |
| $S(O)CH_3$ | H | H | $CH_3$ | $CH_2OH$ | |
| $SCH_2CH_3$ | H | H | $OCH_3$ | $CH_2OH$ | |
| $S(O)$-*n*-$C_4H_9$ | H | H | $OCH_3$ | $CH_2OH$ | |

TABLE 4d

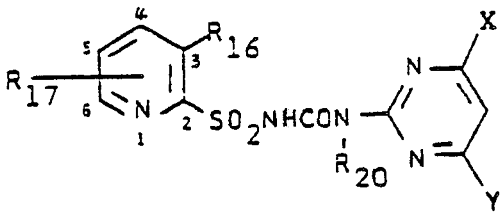

| $R_{16}$ | $R_{17}$ | $R_{20}$ | X | Y | m.p.(°C) |
|---|---|---|---|---|---|
| $CO_2CH_3$ | H | $CH_3$ | $CH_3$ | $CH_2OCOCH_3$ | |
| Cl | H | H | $CH_2CH_3$ | $CH(OC_2H_5)_2$ | |
| Cl | H | H | $OCH_3$ | $CH_2SCH_3$ | |
| H | H | H | $OCH_3$ | $CH_2OCONHCH_3$ | |
| $CO_2CH(CH_3)_2$ | 5-F | H | $CH_3$ | $CH_2OCO_2CH_3$ | |
| $CO_2CH(CH_3)_2$ | H | H | $CH_3$ | $CH_2SC_2H_5$ | |
| $NO_2$ | H | H | $CH_3$ | $CH(SCH_2CH_2S)$ | |
| $NO_2$ | 6-Br | H | $CH_3$ | $CH_2OCOC_2H_5$ | |
| Cl | 4-Cl | H | $OCH_3$ | $CH(OCH_2CH_2CH_2O)$ | |
| $CF_3$ | H | $CH_3$ | $OCH_3$ | $CH(OCH(CH_3)CH_2)$ | |
| Cl | 6-$CH_3$ | H | $OCH_3$ | $CH_2SCH_3$ | |
| $C_2H_5$ | H | H | $CH_3$ | $CH_2OCOCH_3$ | |
| $CH(CH_3)_2$ | H | H | $CH_3$ | $CH_2OCON(CH_3)_2$ | |
| $SO_2CH_3$ | H | H | $CH_3$ | $CH(OCH_2CH_2O)$ | |
| $SO_2CH_3$ | 4-$OCH_3$ | H | $OCH_3$ | $CH_2OCOCH_3$ | |
| $SO_2CH_3$ | 6-F | H | $OCH_3$ | $CH_2SC_2H_5$ | |
| $SO_2$-*n*-$C_4H_9$ | H | H | $CH_3$ | $CH(OC_2H_5)_2$ | |
| $SO_2N(CH_3)_2$ | H | H | $OCH_2CH_3$ | $CH_2OCO_2$-*n*-$C_3H_7$ | |
| $SO_2N(C_2H_5)_2$ | H | H | $CH_3$ | $CH_2OCON(C_2H_5)_2$ | |
| $SO_2N(OCH_3)CH_3$ | H | H | $CH_3$ | $CH_2OCON(CH_3)n—C_3H_7$ | |
| $SO_2$-*n*-$C_3H_7$ | H | H | $CH_3$ | $CH_2OCO$-*i*-$C_3H_7$ | |
| $SO_2$-*i*-$C_3H_7$ | H | H | $OCH_3$ | $CH_2OCONH$(*n*-$C_3H_7$) | |
| $SCH_3$ | H | H | $CH_3$ | $CH(OC_2H_5)_2$ | |
| $S(O)CH_3$ | H | H | $CH_3$ | $CH(OC_2H_5)_2$ | |
| $SCH_2CH_3$ | H | H | $OCH_3$ | $CH_2OCO_2$-*i*-$C_3H_7$ | |
| $S(O)$-*n*-$C_4H_9$ | H | $CH_3$ | $OCH_3$ | $CH_2SCH_3$ | |

TABLE 4e

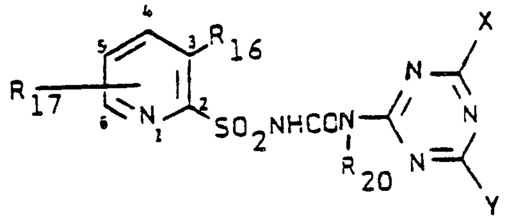

| $R_{16}$ | $R_{17}$ | $R_{20}$ | X | Y | m.p.(°C) |
|---|---|---|---|---|---|
| $CO_2CH_3$ | H | H | $CH_3$ | $CH(OCH_3)_2$ | |
| Cl | H | H | $CH_2CH_3$ | $CH(OCH_3)_2$ | |
| Cl | H | $CH_3$ | $OCH_3$ | $CH(OCH_3)_2$ | |
| H | H | H | $OCH_3$ | $CH(OCH_3)_2$ | |
| $CO_2CH(CH_3)_2$ | 5-F | H | $CH_3$ | $CH(OCH_3)_2$ | |
| $CO_2CH(CH_3)_2$ | H | H | $CH_3$ | $CH(OCH_3)_2$ | |
| $NO_2$ | H | H | $CH_3$ | $CH(OCH_3)_2$ | |
| $NO_2$ | 6-Br | H | $CH_3$ | $CH(OCH_3)_2$ | |
| $CF_3$ | H | H | $OCH_3$ | $CH(OCH_3)_2$ | |
| Cl | 6-$CH_3$ | H | $OCH_3$ | $CH(OCH_3)_2$ | |
| $C_2H_5$ | H | H | $CH_3$ | $CH(OCH_3)_2$ | |
| $CH(CH_3)_2$ | H | H | $CH_3$ | $CH(OCH_3)_2$ | |
| $SO_2CH_3$ | H | H | $CH_3$ | $CH(OCH_3)_2$ | |
| $SO_2CH_3$ | 4-$OCH_3$ | H | $OCH_2CH_3$ | $CH(OCH_3)_2$ | |
| $SO_2CH_3$ | 6-F | H | $OCH_3$ | $CH(OCH_3)_2$ | |
| $SO_2$-*n*-$C_4H_9$ | H | H | $CH_3$ | $CH(OCH_3)_2$ | |
| $SO_2N(CH_3)_2$ | H | H | $OCH_3$ | $CH(OCH_3)_2$ | |
| $SO_2N(C_2H_5)_2$ | H | H | $CH_3$ | $CH(OCH_3)_2$ | |
| $SO_2N(OCH_3)CH_3$ | H | H | $CH_3$ | $CH(OCH_3)_2$ | |
| $SO_2$-*n*-$C_3H_7$ | H | H | $CH_3$ | $CH(OCH_3)_2$ | |
| $SO_2$-*i*-$C_3H_7$ | H | H | $OCH_2CH_3$ | $CH(OCH_3)_2$ | |
| $SCH_3$ | H | $CH_3$ | $CH_3$ | $CH(OCH_3)_2$ | |
| $S(O)CH_3$ | H | H | $CH_3$ | $CH(OCH_3)_2$ | |
| $SCH_2CH_3$ | H | H | $OCH_3$ | $CH(OCH_3)_2$ | |
| $S(O)(CH_2)_3CH_3$ | H | H | $OCH_3$ | $CH(OCH_3)_2$ | |

TABLE 4f

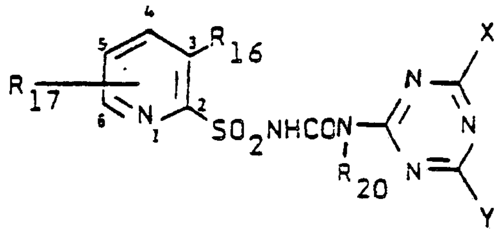

| $R_{16}$ | $R_{17}$ | $R_{20}$ | X | Y | m.p.(°C) |
|---|---|---|---|---|---|
| $CO_2CH_3$ | H | H | $CH_3$ | $CH_2OTHP$ | |
| Cl | H | H | $CH_3$ | $CH_2OTHP$ | |
| Cl | H | H | $OCH_3$ | $CH_2OTHP$ | |
| H | H | H | $OCH_2CH_3$ | $CH_2OTHP$ | |
| $CO_2CH(CH_3)_2$ | 5-F | H | $CH_3$ | $CH_2OTHP$ | |
| $CO_2CH(CH_3)_2$ | H | H | $CH_3$ | $CH_2OTHP$ | |
| $NO_2$ | H | H | $CH_3$ | $CH_2OTHP$ | |
| $NO_2$ | 6-Br | H | $CH_3$ | $CH_2OTHP$ | |
| Cl | 4-Cl | H | $OCH_3$ | $CH_2OTHP$ | |
| $CF_3$ | H | $CH_3$ | $OCH_3$ | $CH_2OTHP$ | |
| Cl | 6-$CH_3$ | H | $OCH_2CH_3$ | $CH_2OTHP$ | |
| $C_2H_5$ | H | H | $CH_3$ | $CH_2OTHP$ | |
| $CH(CH_3)_2$ | H | H | $CH_3$ | $CH_2OTHP$ | |
| $SO_2CH_3$ | H | H | $CH_3$ | $CH_2OTHP$ | |
| $SO_2CH_3$ | 4-$OCH_3$ | H | $OCH_3$ | $CH_2OTHP$ | |
| $SO_2CH_3$ | 6-F | H | $OCH_3$ | $CH_2OTHP$ | |
| $SO_2$-*n*-$C_4H_9$ | H | H | $CH_3$ | $CH_2OTHP$ | |
| $SO_2N(CH_3)_2$ | H | H | $OCH_3$ | $CH_2OTHP$ | |
| $SO_2N(O_2H_5)_2$ | H | H | $CH_3$ | $CH_2OTHP$ | |
| $SO_2N(OCH_3)CH_3$ | H | H | $CH_3$ | $CH_2OTHP$ | |
| $SO_2$-*n*-$C_3H_7$ | H | H | $CH_3$ | $CH_2OTHP$ | |
| $SO_2$-*i*-$C_3H_7$ | H | H | $OCH_3$ | $CH_2OTHP$ | |
| $SCH_3$ | H | H | $CH_3$ | $CH_2OTHP$ | |
| $S(O)CH_3$ | H | H | $CH_2CH_3$ | $CH_2OTHP$ | |
| $SCH_2CH_3$ | H | H | $OCH_3$ | $CH_2OTHP$ | |
| S(O)(-*n*-$C_4H_9$ | H | H | $OCH_3$ | $CH_2OTHP$ | |

TABLE 4g

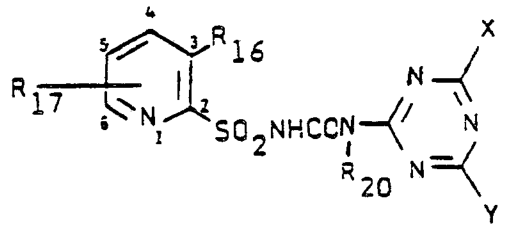

| $R_{16}$ | $R_{17}$ | $R_{20}$ | X | Y | m.p.(°C) |
|---|---|---|---|---|---|
| $CO_2CH_3$ | H | $CH_3$ | $CH_3$ | $CH_2OH$ | |
| Cl | H | H | $CH_3$ | $CH_2OH$ | |
| Cl | H | H | $OCH_3$ | $CH_2OH$ | |
| H | H | H | $OCH_3$ | $CH_2OH$ | |
| $CO_2CH(CH_3)_2$ | 5-F | H | $CH_2CH_3$ | $CH_2OH$ | |
| $CO_2CH(CH_3)_2$ | H | H | $CH_3$ | $CH_2OH$ | |
| $NO_2$ | H | H | $CH_3$ | $CH_2OH$ | |
| $NO_2$ | 6-Br | H | $CH_3$ | $CH_2OH$ | |
| Cl | 4-Cl | H | $OCH_3$ | $CH_2OH$ | |
| $CF_3$ | H | H | $OCH_3$ | $CH_2OH$ | |
| Cl | 6-$CH_3$ | H | $OCH_3$ | $CH_2OH$ | |
| $C_2H_5$ | H | H | $CH_3$ | $CH_2OH$ | |
| $CH(CH_3)_2$ | H | H | $CH_3$ | $CH_2OH$ | |
| $SO_2CH_3$ | H | H | $CH_3$ | $CH_2OH$ | |
| $SO_2CH_3$ | 4-$OCH_3$ | H | $OCH_3$ | $CH_2OH$ | |
| $SO_2CH_3$ | 6-F | H | $OCH_3$ | $CH_2OH$ | |
| $SO_3$-*n*-$C_4H_9$ | H | H | $CH_3$ | $CH_2OH$ | |
| $SO_2N(CH_3)_2$ | H | H | $OCH_3$ | $CH_2OH$ | |
| $SO_2N(C_2H_5)_2$ | H | $CH_3$ | $CH_3$ | $CH_2OH$ | |
| $SO_2N(OCH_3)CH_3$ | H | H | $CH_3$ | $CH_2OH$ | |
| $SO_2$-*n*-$C_3H_7$ | H | H | $CH_3$ | $CH_2OH$ | |
| $SO_2$-*i*-$C_3H_7$ | H | H | $OCH_3$ | $CH_2OH$ | |
| $SCH_3$ | H | H | $CH_3$ | $CH_2OH$ | |
| $S(O)CH_3$ | H | H | $CH_3$ | $CH_2OH$ | |
| $SCH_2CH_3$ | H | H | $OCH_2CH_3$ | $CH_2OH$ | |
| S(O)-*n*-$C_4H_9$ | H | H | $OCH_3$ | $CH_2OH$ | |

TABLE 4h

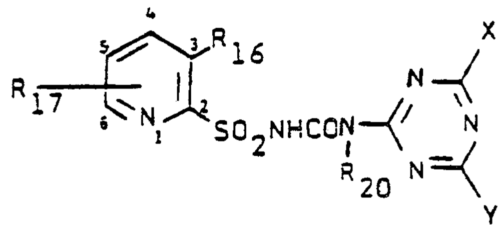

| $R_{16}$ | $R_{17}$ | $R_{20}$ | X | Y | m.p.(°C) |
|---|---|---|---|---|---|
| $CO_2CH_3$ | H | H | $CH_2CH_3$ | $CH_2OCOCH_3$ | |
| Cl | H | H | $CH_3$ | $CH(OC_2H_5)_2$ | |
| Cl | H | H | $OCH_3$ | $CH_2SCH_3$ | |
| H | H | H | $OCH_3$ | $CH_2OCONHCH_3$ | |
| $CO_2CH(CH_3)_2$ | 5-F | H | $CH_3$ | $CH_2OCO_2CH_3$ | |
| $CO_2CH(CH_3)_2$ | H | H | $CH_3$ | $CH_2SC_2H_5$ | |
| $NO_2$ | H | H | $CH_3$ | $CH(OCH_2CH_2O)$ | |
| $NO_2$ | 6-Br | $CH_3$ | $CH_3$ | $CH_2OCOC_2H_5$ | |
| Cl | 4-Cl | H | $OCH_3$ | $CH(CH_2CH_2CH_2O)$ | |
| $CF_3$ | H | H | $OCH_2CH_3$ | $CH(OCH(CH_3)CH_2)$ | |
| Cl | 6-$CH_3$ | H | $OCH_3$ | $CH_2SCH_3$ | |
| $C_2H_5$ | H | H | $CH_3$ | $CH_2OCOCH_3$ | |
| $CH(CH_3)_2$ | H | H | $CH_3$ | $CH_2OCON(CH_3)_2$ | |
| $SO_2CH_3$ | H | H | $CH_3$ | $CH(OCH_2CH_2O)$ | |
| $SO_2CH_3$ | 4-$OCH_3$ | H | $OCH_3$ | $CH_2OCOCH_3$ | |
| $SO_2CH_3$ | 6-F | H | $OCH_3$ | $CH_2SC_2H_5$ | |
| $SO_2$-*n*-$C_4H_9$ | H | H | $CH_3$ | $CH(OC_2H_5)_2$ | |
| $SO_2N(CH_3)_2$ | H | H | $OCH_3$ | $CH_2OCO_2$-*n*-$C_3H_7$ | |
| $SO_2N(C_2H_5)_2$ | H | H | $CH_3$ | $CH_2OCON(C_2H_5)_2$ | |
| $SO_2N(OCH_3)CH_3$ | H | $CH_3$ | $CH_3$ | $CH_2OCON(CH_3)$(*n*-$C_3H_7$) | |
| $SO_2$-*n*-$C_3H_7$ | H | H | $CH_3$ | $CH_2OCO$-*i*-$C_3H_7$ | |
| $SO_1$-*i*-$C_3H_7$ | H | H | $OCH_3$ | $CH_2OCONH$(*n*-$C_3H_7$) | |
| $SCH_3$ | H | H | $CH_3$ | $CH(OC_2H_5)_2$ | |
| $S(O)CH_3$ | H | H | $CH_3$ | $CH(OC_2H_5)_2$ | |
| $SCH_2CH_3$ | H | H | $OCH_3$ | $CH_2OCO_2$-*i*-$C_3H_7$ | |
| S(O)-*n*-$C_4H_9$ | H | H | $OCH_3$ | $CH_2SCH_3$ | |

TABLE 5a

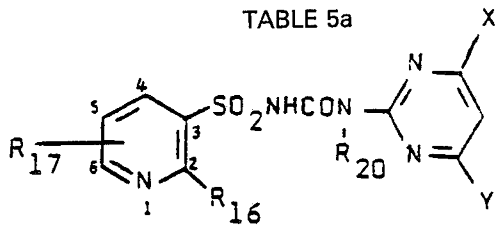

| $R_{16}$ | $R_{17}$ | $R_{20}$ | X | Y | m.p. (°C) |
|---|---|---|---|---|---|
| $CO_2CH_3$ | H | H | $CH_3$ | $CH(OCH_3)_2$ | |
| Cl | H | H | $CH_3$ | $CH(OCH_3)_2$ | |
| $CO_2CH_3$ | H | $CH_3$ | Cl | $CH(OCH_3)_2$ | |
| Cl | H | H | $OCH_2CH_3$ | $CH(OCH_3)_2$ | |
| H | H | H | $OCH_3$ | $CH(OCH_3)_2$ | |
| $CO_2CH(CH_3)_2$ | 5-F | H | $CH_3$ | $CH(OCH_3)_2$ | |
| $CO_2CH(CH_3)_2$ | H | H | $CH_3$ | $CH(OCH_3)_2$ | |
| $NO_2$ | H | H | $CH_2CH_3$ | $CH(OCH_3)_2$ | |
| $NO_2$ | 6-Br | H | $CH_3$ | $CH(OCH_3)_2$ | |
| Cl | 4-Cl | H | Cl | $CH(OCH_3)_2$ | |
| $CF_3$ | H | H | $OCH_3$ | $CH(OCH_3)_2$ | |
| Cl | 6-$CH_3$ | H | $OCH_3$ | $CH(OCH_3)_2$ | |
| $C_2H_5$ | H | H | $CH_3$ | $CH(OCH_3)_2$ | |
| $CH(CH_3)_2$ | H | H | $CH_2CH_3$ | $CH(OCH_3)_2$ | |
| $SO_2CH_3$ | H | H | $CH_3$ | $CH(OCH_3)_2$ | |
| $SO_2CH_3$ | 4-$OCH_3$ | H | $OCH_3$ | $CH(OCH_3)_2$ | |
| $SO_2CH_3$ | 6-F | H | $OCH_3$ | $CH(OCH_3)_2$ | |
| $SO_2$-*n*-$C_4H_9$ | H | $CH_3$ | $CH_3$ | $CH(OCH_3)_2$ | |
| $SO_2N(CH_3)_2$ | H | H | $OCH_3$ | $CH(OCH_3)_2$ | |
| $SO_2N(C_2H_5)_2$ | H | H | $CH_3$ | $CH(OCH_3)_2$ | |
| $SO_2N(OCH_3)CH_3$ | H | H | $CH_3$ | $CH(OCH_3)_2$ | |
| $SO_2$-*n*-$C_3H_7$ | H | H | $CH_3$ | $CH(OCH_3)_2$ | |
| $SO_2$-*i*-$C_3H_7$ | H | H | $OCH_3$ | $CH(OCH_3)_2$ | |
| $SCH_3$ | H | H | $CH_3$ | $CH(OCH_3)_2$ | |
| $S(O)CH_3$ | H | H | $CH_3$ | $CH(OCH_3)_2$ | |
| $SCH_2CH_3$ | H | H | $OCH_3$ | $CH(OCH_3)_2$ | |
| $S(O)(CH_2)_3CH_3$ | H | H | $OCH_3$ | $CH(OCH_3)_2$ | |

TABLE 5b

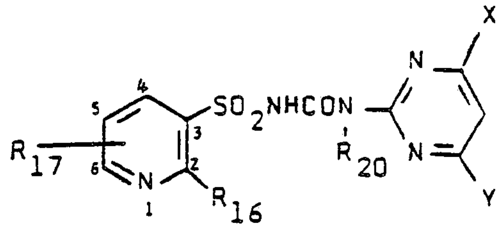

| $R_{16}$ | $R_{17}$ | $R_{20}$ | X | Y | m.p. (°C) |
|---|---|---|---|---|---|
| $CO_2CH_3$ | H | H | $CH_3$ | $CH_2OTHP$ | |
| Cl | H | H | $CH_3$ | $CH_2OTHP$ | |
| Cl | H | H | $OCH_3$ | $CH_2OTHP$ | |
| H | H | H | $OCH_3$ | $CH_2OTHP$ | |
| $CO_2CH(CH_3)_2$ | 5-F | H | $CH_3$ | $CH_2OTHP$ | |
| $CO_2CH(CH_3)_2$ | H | H | $CH_2CH_3$ | $CH_2OTHP$ | |
| $NO_2$ | H | H | $CH_3$ | $CH_2OTHP$ | |
| $NO_2$ | 6-Br | H | $CH_3$ | $CH_2OTHP$ | |
| Cl | 4-Cl | H | $OCH_3$ | $CH_2OTHP$ | |
| $CF_3$ | H | H | $OCH_3$ | $CH_2OTHP$ | |
| Cl | 6-$CH_3$ | H | $OCH_3$ | $CH_2OTHP$ | |
| $C_2H_5$ | H | H | $CH_3$ | $CH_2OTHP$ | |
| $CH(CH_3)_2$ | H | $CH_3$ | $CH_3$ | $CH_2OTHP$ | |
| $SO_2CH_3$ | H | H | $CH_3$ | $CH_2OTHP$ | |
| $SO_2CH_3$ | 4-$OCH_3$ | H | $OCH_2CH_3$ | $CH_2OTHP$ | |
| $SO_2CH_3$ | 6-F | H | $OCH_3$ | $CH_2OTHP$ | |
| $SO_2$-*n*-$C_4H_9$ | H | H | $CH_3$ | $CH_2OTHP$ | |
| $SO_2N(CH_3)_2$ | H | H | $OCH_3$ | $CH_2OTHP$ | |
| $SO_2N(C_2H_5)_2$ | H | H | $CH_3$ | $CH_2OTHP$ | |
| $SO_2N(OCH_3)CH_3$ | H | H | $CH_3$ | $CH_2OTHP$ | |
| $SO_2$-*n*-$C_3H_7$ | H | H | $CH_3$ | $CH_2OTHP$ | |
| $SO_2$-*i*-$C_3H_7$ | H | H | $OCH_3$ | $CH_2OTHP$ | |
| $SCH_3$ | H | H | $CH_3$ | $CH_2OTHP$ | |
| $S(O)CH_3$ | H | H | $CH_3$ | $CH_2OTHP$ | |
| $SCH_2CH_3$ | H | H | $OCH_3$ | $CH_2OTHP$ | |
| $S(O)$-*n*-$C_4H_9$ | H | H | $OCH_3$ | $CH_2OTHP$ | |

TABLE 5c

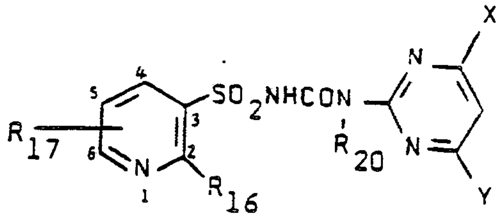

| $R_{16}$ | $R_{17}$ | $R_{20}$ | X | Y | m.p. (°C) |
|---|---|---|---|---|---|
| $CO_2CH_3$ | H | H | $CH_3$ | $CH_2OH$ | |
| Cl | H | H | $CH_3$ | $CH_2OH$ | |
| Cl | H | H | $OCH_2CH_3$ | $CH_2OH$ | |
| H | H | H | $OCH_3$ | $CH_2OH$ | |
| $CO_2CH(CH_3)_2$ | 5-F | H | $CH_3$ | $CH_2OH$ | |
| $CO_2CH(CH_3)_2$ | H | $CH_3$ | $CH_3$ | $CH_2OH$ | |
| $NO_2$ | H | H | $CH_3$ | $CH_2OH$ | |
| $NO_2$ | 6-Br | H | $CH_3$ | $CH_2OH$ | |
| Cl | 4-Cl | H | $OCH_3$ | $CH_2OH$ | |
| $CF_3$ | H | H | $OCH_3$ | $CH_2OH$ | |
| Cl | 6-$CH_3$ | H | $OCH_2CH_3$ | $CH_2OH$ | |
| $C_2H_5$ | H | $CH_3$ | $CH_3$ | $CH_2OH$ | |
| $CH(CH_3)_2$ | H | H | $CH_3$ | $CH_2OH$ | |
| $SO_2CH_3$ | H | H | $CH_3$ | $CH_2OH$ | |
| $SO_2CH_3$ | 4-$OCH_3$ | H | $OCH_3$ | $CH_2OH$ | |
| $SO_2CH_3$ | 6-F | H | $OCH_3$ | $CH_2OH$ | |
| $SO_2$-$n$-$C_4H_9$ | H | H | $CH_3$ | $CH_2OH$ | |
| $SO_2N(CH_3)_2$ | H | H | $OCH_3$ | $CH_2OH$ | |
| $SO_2N(C_2H_5)_2$ | H | H | $CH_3$ | $CH_2OH$ | |
| $SO_2N(OCH_3)CH_3$ | H | H | $CH_3$ | $CH_2OH$ | |
| $SO_2$-$n$-$C_3H_7$ | H | H | $CH_3$ | $CH_2OH$ | |
| $SO_2$-$i$-$C_3H_7$ | H | H | $OCH_3$ | $CH_2OH$ | |
| $SCH_3$ | H | H | $CH_3$ | $CH_2OH$ | |
| $S(O)CH_3$ | H | H | $CH_3$ | $CH_2OH$ | |
| $SCH_2CH_3$ | H | H | $OCH_3$ | $CH_2OH$ | |
| $S(O)$-$n$-$C_4H_9$ | H | H | $OCH_3$ | $CH_2OH$ | |

TABLE 5d

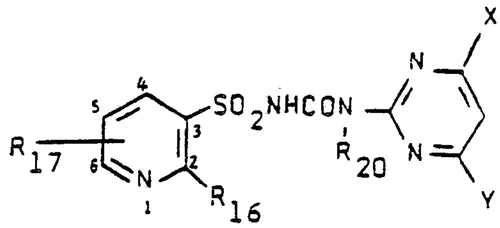

| $R_{16}$ | $R_{17}$ | $R_{20}$ | X | Y | m.p. (°C) |
|---|---|---|---|---|---|
| $CO_2CH_3$ | H | H | $CH_3$ | $CH_2OCOCH_3$ | |
| Cl | H | $CH_3$ | $CH_3$ | $CH(OC_2H_5)_2$ | |
| Cl | H | H | $OCH_2CH_3$ | $CH_2SCH_3$ | |
| H | H | H | $OCH_3$ | $CH_2OCONHCH_3$ | |
| $CO_2CH(CH_3)_2$ | 5-F | H | $CH_3$ | $CH_2OCO_2CH_3$ | |
| $CO_2CH(CH_3)_2$ | H | H | $CH_3$ | $CH_2SC_2H_5$ | |
| $NO_2$ | H | H | $CH_3$ | $CH(OCH_2CH_2O)$ | |
| $NO_2$ | 6-Br | H | $CH_3$ | $CH_2OCOC_2H_5$ | |
| Cl | 4-Cl | H | $OCH_3$ | $CH(OCH_2CH_2CH_2O)$ | |
| $CF_3$ | H | H | $OCH_3$ | $CH(OCH(CH_3)CH_2)$ | |
| Cl | 6-$CH_3$ | H | $OCH_3$ | $CH_2SCH_3$ | |
| $C_2H_5$ | H | H | $CH_2CH_3$ | $CH_2OCOCH_3$ | |
| $CH(CH_3)_2$ | H | H | $CH_3$ | $CH_2OCON(CH_3)_2$ | |
| $SO_2CH_3$ | H | H | $CH_3$ | $CH(OCH_2CH_2O)$ | |
| $SO_2CH_3$ | 4-$OCH_3$ | H | $OCH_3$ | $CH_2OCOCH_3$ | |
| $SO_2CH_3$ | 6-F | H | $OCH_3$ | $CH_2SC_2H_5$ | |
| $SO_2$-*n*-$C_4H_9$ | H | H | $CH_3$ | $CH(OC_2H_5)_2$ | |
| $SO_2N(CH_3)_2$ | H | H | $OCH_3$ | $CH_2OCO_2$-*n*-$C_3H_7$ | |
| $SO_2N(C_2H_5)_2$ | H | H | $CH_3$ | $CH_2OCON(C_2H_5)_2$ | |
| $SO_2N(OCH_3)CH_3$ | H | H | $CH_3$ | $CH_2OCON(CH_3)$(*n*-$C_3H_7$) | |
| $SO_2$-*n*-$C_3H_7$ | H | H | $CH_3$ | $CH_2OCO$-*i*-$C_3H_7$ | |
| $SO_2$-*i*-$C_3H_7$ | H | H | $OCH_3$ | $CH_2OCONH$(*n*-$C_3H_7$) | |
| $SCH_3$ | H | $CH_3$ | $CH_3$ | $CH(OC_2H_5)_2$ | |
| $S(O)CH_3$ | H | H | $CH_3$ | $CH(OC_2H_5)_2$ | |
| $SCH_2CH_3$ | H | H | $OCH_3$ | $CH_2OCO_2$-*i*-$C_3H_7$ | |
| S(O)-*n*-$C_4H_9$ | H | H | $OCH_3$ | $CH_2SCH_3$ | |

TABLE 5e

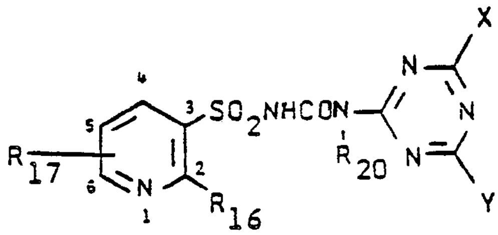

| $R_{16}$ | $R_{17}$ | $R_{20}$ | X | Y | m.p. (°C) |
|---|---|---|---|---|---|
| $CO_2CH_3$ | H | H | $CH_3$ | $CH(OCH_3)_2$ | |
| Cl | H | H | $CH_3$ | $CH(OCH_3)_2$ | |
| Cl | H | H | $OCH_3$ | $CH(OCH_3)_2$ | |
| H | H | $CH_3$ | $OCH_3$ | $CH(OCH_3)_2$ | |
| $CO_2CH(CH_3)_2$ | 5-F | H | $CH_3$ | $CH(OCH_3)_2$ | |
| $CO_2CH(CH_3)_2$ | H | H | $CH_3$ | $CH(OCH_3)_2$ | |
| $NO_2$ | H | H | $CH_3$ | $CH(OCH_3)_2$ | |
| $NO_2$ | 6-Br | H | $CH_3$ | $CH(OCH_3)_2$ | |
| $CF_3$ | H | H | $OCH_3$ | $CH(OCH_3)_2$ | |
| Cl | 6-$CH_3$ | H | $OCH_3$ | $CH(OCH_3)_2$ | |
| $C_2H_5$ | H | H | $CH_3$ | $CH(OCH_3)_2$ | |
| $CH(CH_3)_2$ | H | H | $CH_3$ | $CH(OCH_3)_2$ | |
| $SO_2CH_3$ | H | H | $CH_3$ | $CH(OCH_3)_2$ | |
| $SO_2CH_3$ | 4-$OCH_3$ | H | $OCH_3$ | $CH(OCH_3)_2$ | |
| $SO_2CH_3$ | 6-F | H | $OCH_3$ | $CH(OCH_3)_2$ | |
| $SO_2$-*n*-$C_4H_9$ | H | H | $CH_3$ | $CH(OCH_3)_2$ | |
| $SO_2N(CH_3)_2$ | H | H | $OCH_3$ | $CH(OCH_3)_2$ | |
| $SO_2N(C_2H_5)_2$ | H | H | $CH_3$ | $CH(OCH_3)_2$ | |
| $SO_2N(OCH_3)CH_3$ | H | H | $CH_2CH_3$ | $CH(OCH_3)_2$ | |
| $SO_2$-*n*-$C_3H_7$ | H | H | $CH_3$ | $CH(OCH_3)_2$ | |
| $SO_2$-*i*-$C_3H_7$ | H | H | $OCH_3$ | $CH(OCH_3)_2$ | |
| $SCH_3$ | H | H | $CH_3$ | $CH(OCH_3)_2$ | |
| $S(O)CH_3$ | H | H | $CH_3$ | $CH(OCH_3)_2$ | |
| $SCH_2CH_3$ | H | H | $OCH_2CH_3$ | $CH(OCH_3)_2$ | |
| $S(O)(CH_2)_3CH_3$ | H | $CH_3$ | $OCH_3$ | $CH(OCH_3)_2$ | |

TABLE 5f

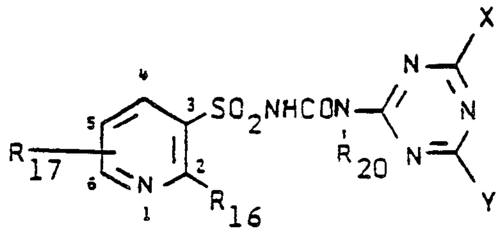

| $R_{16}$ | $R_{17}$ | $R_{20}$ | X | Y | m.p. (°C) |
|---|---|---|---|---|---|
| $CO_2CH_3$ | H | H | $CH_3$ | $CH_2OTHP$ | |
| Cl | H | H | $CH_3$ | $CH_2OTHP$ | |
| Cl | H | H | $OCH_2CH_3$ | $CH_2OTHP$ | |
| H | H | H | $OCH_3$ | $CH_2OTHP$ | |
| $CO_2CH(CH_3)_2$ | 5-F | H | $CH_3$ | $CH_2OTHP$ | |
| $CO_2CH(CH_3)_2$ | H | H | $CH_3$ | $CH_2OTHP$ | |
| $NO_2$ | H | H | $CH_3$ | $CH_2OTHP$ | |
| $NO_2$ | 6-Br | H | $CH_3$ | $CH_2OTHP$ | |
| Cl | 4-Cl | H | $OCH_3$ | $CH_2OTHP$ | |
| $CF_3$ | H | $CH_3$ | $OCH_3$ | $CH_2OTHP$ | |
| Cl | 6-$CH_3$ | H | $OCH_3$ | $CH_2OTHP$ | |
| $C_2H_5$ | H | H | $CH_3$ | $CH_2OTHP$ | |
| $CH(CH_3)_2$ | H | H | $CH_3$ | $CH_2OTHP$ | |
| $SO_2CH_3$ | H | H | $CH_3$ | $CH_2OTHP$ | |
| $SO_2CH_3$ | 4-$OCH_3$ | H | $OCH_3$ | $CH_2OTHP$ | |
| $SO_2CH_3$ | 6-F | H | $OCH_3$ | $CH_2OTHP$ | |
| $SO_2$-*n*-$C_4H_9$ | H | H | $CH_2CH_3$ | $CH_2OTHP$ | |
| $SO_2N(CH_3)_2$ | H | H | $OCH_3$ | $CH_2OTHP$ | |
| $SO_2N(C_2H_5)_2$ | H | H | $CH_3$ | $CH_2OTHP$ | |
| $SO_2N(OCH_3)CH_3$ | H | H | $CH_3$ | $CH_2OTHP$ | |
| $SO_2$-*n*-$C_3H_7$ | H | H | $CH_3$ | $CH_2OTHP$ | |
| $SO_2$-*i*-$C_3H_7$ | H | H | $OCH_3$ | $CH_2OTHP$ | |
| $SCH_3$ | H | H | $CH_3$ | $CH_2OTHP$ | |
| $S(O)CH_3$ | H | H | $CH_3$ | $CH_2OTHP$ | |
| $SCH_2CH_3$ | H | H | $OCH_3$ | $CH_2OTHP$ | |
| $S(O)$-*n*-$C_4H_9$ | H | H | $OCH_3$ | $CH_2OTHP$ | |

TABLE 5g

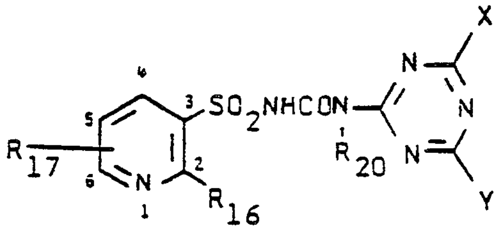

| $R_{16}$ | $R_{17}$ | $R_{20}$ | X | Y | m.p. (°C) |
|---|---|---|---|---|---|
| $CO_2CH_3$ | H | H | $CH_3$ | $CH_2OH$ | |
| Cl | H | $CH_3$ | $CH_3$ | $CH_2OH$ | |
| Cl | H | H | $OCH_3$ | $CH_2OH$ | |
| H | H | H | $OCH_3$ | $CH_2OH$ | |
| $CO_2CH(CH_3)_2$ | 5-F | H | $CH_3$ | $CH_2OH$ | |
| $CO_2CH(CH_3)_2$ | H | H | $CH_2CH_3$ | $CH_2OH$ | |
| $NO_2$ | H | H | $CH_3$ | $CH_2OH$ | |
| $NO_2$ | 6-Br | H | $CH_3$ | $CH_2OH$ | |
| Cl | 4-Cl | H | $OCH_3$ | $CH_2OH$ | |
| $CF_3$ | H | H | $OCH_3$ | $CH_2OH$ | |
| Cl | 6-$CH_3$ | H | $OCH_3$ | $CH_2OH$ | |
| $C_2H_5$ | H | H | $CH_3$ | $CH_2OH$ | |
| $CH(CH_3)_2$ | H | H | $CH_3$ | $CH_2OH$ | |
| $SO_2CH_3$ | H | H | $CH_3$ | $CH_2OH$ | |
| $SO_2CH_3$ | 4-$OCH_3$ | H | $OCH_3$ | $CH_2OH$ | |
| $SO_2CH_3$ | 6-F | H | $OCH_3$ | $CH_2OH$ | |
| $SO_2$-*n*-$C_4H_9$ | H | H | $CH_3$ | $CH_2OH$ | |
| $SO_2N(CH_3)_2$ | H | H | $OCH_3$ | $CH_2OH$ | |
| $SO_2N(C_2H_5)_2$ | H | H | $CH_3$ | $CH_2OH$ | |
| $SO_2N(OCH_3)CH_3$ | H | H | $CH_3$ | $CH_2OH$ | |
| $SO_2$-*n*-$C_3H_7$ | H | H | $CH_3$ | $CH_2OH$ | |
| $SO_2$-*i*-$C_3H_7$ | H | $CH_3$ | $OCH_3$ | $CH_2OH$ | |
| $SCH_3$ | H | H | $CH_3$ | $CH_2OH$ | |
| $S(O)CH_3$ | H | H | $CH_3$ | $CH_2OH$ | |
| $SCH_2CH_3$ | H | H | $OCH_3$ | $CH_2OH$ | |
| $S(O)$-*n*-$C_4H_9$ | H | H | $OCH_2CH_3$ | $CH_2OH$ | |

TABLE 5h

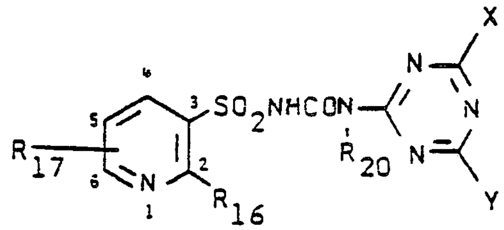

| $R_{16}$ | $R_{17}$ | $R_{20}$ | X | Y | m.p. (°C) |
|---|---|---|---|---|---|
| $CO_2CH_3$ | H | H | $CH_3$ | $CH_2OCOCH_3$ | |
| Cl | H | H | $CH_3$ | $CH(OC_2H_5)_2$ | |
| Cl | H | H | $OCH_3$ | $CH_2SCH_3$ | |
| H | H | H | $OCH_3$ | $CH_2OCONHCH_3$ | |
| $CO_2CH(CH_3)_2$ | 5-F | H | $CH_3$ | $CH_2OCO_2CH_3$ | |
| $CO_2CH(CH_3)_2$ | H | $CH_3$ | $CH_3$ | $CH_2SC_2H_5$ | |
| $NO_2$ | H | H | $CH_3$ | $CH(OCH_2CH_2O)$ | |
| $NO_2$ | 6-Br | H | $CH_3$ | $CH_2OCOC_2H_5$ | |
| Cl | 4-Cl | H | $OCH_3$ | $CH(OCH_2CH_2CH_2O)$ | |
| $CF_3$ | H | H | $OCH_3$ | $CH(OCH(CH_3)CH_2)$ | |
| Cl | 6-$CH_3$ | H | $OCH_3$ | $CH_2SCH_3$ | |
| $C_2H_5$ | H | H | $CH_2CH_3$ | $CH_2OCOCH_3$ | |
| $CH(CH_3)_2$ | H | H | $CH_3$ | $CH_2OCON(CH_3)_2$ | |
| $SO_2CH_3$ | H | H | $CH_3$ | $CH(OCH_2CH_2O)$ | |
| $SO_2CH_3$ | 4-$OCH_3$ | H | $OCH_3$ | $CH_2OCOCH_3$ | |
| $SO_2CH_3$ | 6-F | H | $OCH_3$ | $CH_2SC_2H_5$ | |
| $SO_2$-$n$-$C_4H_9$ | H | H | $CH_3$ | $CH(OC_2H_5)_2$ | |
| $SO_2N(CH_3)_2$ | H | H | $OCH_2CH_3$ | $CH_2OCO_2$-$n$-$C_3H_7$ | |
| $SO_2N(C_2H_5)_2$ | H | H | $CH_3$ | $CH_2OCON(C_2H_5)_2$ | |
| $SO_2N(OCH_3)CH_3$ | H | H | $CH_3$ | $CH_2OCON(CH_3)(n$-$C_3H_7)$ | |
| $SO_2$-$n$-$C_3H_7$ | H | H | $CH_3$ | $CH_2OCO$-$i$-$C_3H_7$ | |
| $SO_2$-$i$-$C_3H_7$ | H | H | $OCH_3$ | $CH_2OCONH(n$-$C_3H_7)$ | |
| $SCH_3$ | H | H | $CH_3$ | $CH(OC_2H_5)_2$ | |
| $S(O)CH_3$ | H | H | $CH_3$ | $CH(OC_2H_5)_2$ | |
| $SCH_2CH_3$ | H | $CH_3$ | $OCH_3$ | $CH_2OCO_2$-$i$-$C_3H_7$ | |
| $S(O)$-$n$-$C_4H_9$ | H | H | $OCH_3$ | $CH_2SCH_3$ | |

TABLE 6a

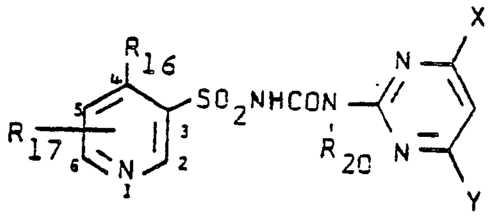

| $R_{16}$ | $R_{17}$ | $R_{20}$ | X | Y | m.p. (°C) |
|---|---|---|---|---|---|
| $CO_2CH_3$ | H | H | $CH_3$ | $CH(OCH_3)_2$ | |
| Cl | H | H | $CH_3$ | $CH(OCH_3)_2$ | |
| $CO_2CH_3$ | H | H | Cl | $CH(OCH_3)_2$ | |
| Cl | H | H | $OCH_3$ | $CH(OCH_3)_2$ | |
| H | H | H | $OCH_3$ | $CH(OCH_3)_2$ | |
| $CO_2CH(CH_3)_2$ | 5-F | H | $CH_3$ | $CH(OCH_3)_2$ | |
| $CO_2CH(CH_3)_2$ | H | H | $CH_3$ | $CH(OCH_3)_2$ | |
| $NO_2$ | H | H | $CH_3$ | $CH(OCH_3)_2$ | |
| $NO_2$ | 6-Br | H | $CH_3$ | $CH(OCH_3)_2$ | |
| $CF_3$ | H | H | $OCH_2CH_3$ | $CH(OCH_3)_2$ | |
| Cl | 6-$CH_3$ | H | $OCH_3$ | $CH(OCH_3)_2$ | |
| $C_2H_5$ | H | H | $CH_3$ | $CH(OCH_3)_2$ | |
| $CH(CH_3)_2$ | H | H | $CH_3$ | $CH(OCH_3)_2$ | |
| $SO_2CH_3$ | H | H | $CH_3$ | $CH(OCH_3)_2$ | |
| $SO_2CH_3$ | 6-F | H | $OCH_3$ | $CH(OCH_3)_2$ | |
| $SO_2$-*n*-$C_4H_9$ | H | $CH_3$ | $CH_3$ | $CH(OCH_3)_2$ | |
| $SO_2N(CH_3)_2$ | H | H | $OCH_3$ | $CH(OCH_3)_2$ | |
| $SO_2N(C_2H_5)_2$ | H | H | $CH_3$ | $CH(OCH_3)_2$ | |
| $SO_2N(OCH_3)CH_3$ | H | H | $CH_3$ | $CH(OCH_3)_2$ | |
| $SO_2$-*n*-$C_3H_7$ | H | H | $CH_3$ | $CH(OCH_3)_2$ | |
| $SO_2$-*i*-$C_3H_7$ | H | H | $OCH_3$ | $CH(OCH_3)_2$ | |
| $SCH_3$ | H | H | $CH_3$ | $CH(OCH_3)_2$ | |
| $S(O)CH_3$ | H | H | $CH_2CH_3$ | $CH(OCH_3)_2$ | |
| $SCH_2CH_3$ | H | H | $OCH_3$ | $CH(OCH_3)_2$ | |
| $S(O)(CH_2)_3CH_3$ | H | H | $OCH_3$ | $CH(OCH_3)_2$ | |

TABLE 6b

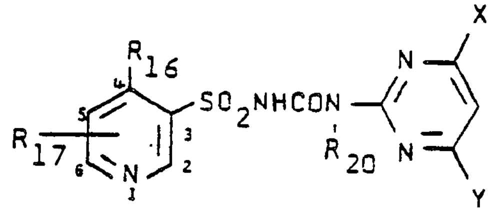

| $R_{16}$ | $R_{17}$ | $R_{20}$ | X | Y | m.p. (°C) |
|---|---|---|---|---|---|
| $CO_2CH_3$ | H | H | $CH_3$ | $CH_2OTHP$ | |
| Cl | H | $CH_3$ | $CH_3$ | $CH_2OTHP$ | |
| Cl | H | H | $OCH_3$ | $CH_2OTHP$ | |
| H | H | H | $OCH_2CH_3$ | $CH_2OTHP$ | |
| $CO_2CH(CH_3)_2$ | 5-F | H | $CH_3$ | $CH_2OTHP$ | |
| $CO_2CH(CH_3)_2$ | H | H | $CH_3$ | $CH_2OTHP$ | |
| $NO_2$ | H | H | $CH_3$ | $CH_2OTHP$ | |
| $NO_2$ | 6-Br | H | $CH_3$ | $CH_2OTHP$ | |
| Cl | 2-Cl | H | $OCH_3$ | $CH_2OTHP$ | |
| $CF_3$ | H | H | $OCH_3$ | $CH_2OTHP$ | |
| Cl | 6-$CH_3$ | H | $OCH_3$ | $CH_2OTHP$ | |
| $C_2H_5$ | H | H | $CH_2CH_3$ | $CH_2OTHP$ | |
| $CH(CH_3)_2$ | H | H | $CH_3$ | $CH_2OTHP$ | |
| $SO_2CH_3$ | H | H | $CH_3$ | $CH_2OTHP$ | |
| $SO_2CH_3$ | 5-$OCH_3$ | H | $OCH_3$ | $CH_2OTHP$ | |
| $SO_2CH_3$ | 6-F | H | $OCH_3$ | $CH_2OTHP$ | |
| $SO_2$-*n*-$C_4H_9$ | H | H | $CH_3$ | $CH_2OTHP$ | |
| $SO_2N(CH_3)_2$ | H | $CH_3$ | $OCH_3$ | $CH_2OTHP$ | |
| $SO_2N(C_2H_5)_2$ | H | $CH_3$ | $CH_3$ | $CH_2OTHP$ | |
| $SO_2N(OCH_3)CH_3$ | H | H | $CH_3$ | $CH_2OTHP$ | |
| $SO_2$-*n*-$C_3H_7$ | H | H | $CH_3$ | $CH_2OTHP$ | |
| $SO_2$-*i*-$C_3H_7$ | H | H | $OCH_3$ | $CH_2OTHP$ | |
| $SCH_3$ | H | H | $CH_3$ | $CH_2OTHP$ | |
| $S(O)CH_3$ | H | H | $CH_3$ | $CH_2OTHP$ | |
| $SCH_2CH_3$ | H | H | $OCH_3$ | $CH_2OTHP$ | |
| $S(O)$-*n*-$C_4H_9$ | H | H | $OCH_3$ | $CH_2OTHP$ | |

TABLE 6c

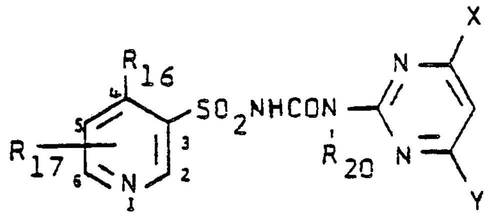

| $R_{16}$ | $R_{17}$ | $R_{20}$ | X | Y | m.p. (°C) |
|---|---|---|---|---|---|
| $CO_2CH_3$ | H | H | $CH_3$ | $CH_2OH$ | |
| Cl | H | H | $CH_2CH_3$ | $CH_2OH$ | |
| Cl | H | H | $OCH_3$ | $CH_2OH$ | |
| H | H | H | $OCH_3$ | $CH_2OH$ | |
| $CO_2CH(CH_3)_2$ | 5-F | H | $CH_3$ | $CH_2OH$ | |
| $CO_2CH(CH_3)_2$ | H | H | $CH_3$ | $CH_2OH$ | |
| $NO_2$ | H | H | $CH_3$ | $CH_2OH$ | |
| $NO_2$ | 6-Br | H | $CH_3$ | $CH_2OH$ | |
| Cl | 2-Cl | H | $OCH_2CH_3$ | $CH_2OH$ | |
| $CF_3$ | H | H | $OCH_3$ | $CH_2OH$ | |
| Cl | 6-$CH_3$ | H | $OCH_3$ | $CH_2OH$ | |
| $C_2H_5$ | H | H | $CH_3$ | $CH_2OH$ | |
| $CH(CH_3)_2$ | H | H | $CH_3$ | $CH_2OH$ | |
| $SO_2CH_3$ | H | H | $CH_3$ | $CH_2OH$ | |
| $SO_2CH_3$ | 5-$OCH_3$ | H | $OCH_3$ | $CH_2OH$ | |
| $SO_2CH_3$ | 6-F | H | $OCH_3$ | $CH_2OH$ | |
| $SO_2$-*n*-$C_4H_9$ | H | H | $CH_3$ | $CH_2OH$ | |
| $SO_2N(CH_3)_2$ | H | H | $OCH_3$ | $CH_2OH$ | |
| $SO_2N(C_2H_5)_2$ | H | H | $CH_3$ | $CH_2OH$ | |
| $SO_2N(OCH_3)CH_3$ | H | H | $CH_3$ | $CH_2OH$ | |
| $SO_2$-*n*-$C_3H_7$ | H | H | $CH_3$ | $CH_2OH$ | |
| $SO_2$-*i*-$C_3H_7$ | H | $CH_3$ | $OCH_3$ | $CH_2OH$ | |
| $SCH_3$ | H | $CH_3$ | $CH_3$ | $CH_2OH$ | |
| $S(O)CH_3$ | H | H | $CH_3$ | $CH_2OH$ | |
| $SCH_2CH_3$ | H | H | $OCH_3$ | $CH_2OH$ | |
| $S(O)$-*n*-$C_4H_9$ | H | H | $OCH_3$ | $CH_2OH$ | |

TABLE 6d

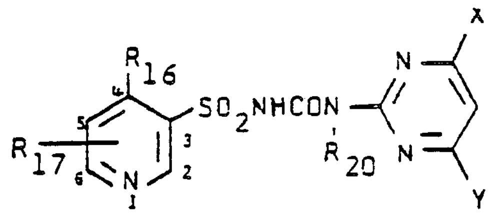

| $R_{16}$ | $R_{17}$ | $R_{20}$ | X | Y | m.p. (°C) |
|---|---|---|---|---|---|
| $CO_2CH_3$ | H | H | $CH_3$ | $CH_2OCOCH_3$ | |
| Cl | H | H | $CH_3$ | $CH(OC_2H_5)_2$ | |
| Cl | H | H | $OCH_3$ | $CH_2SCH_3$ | |
| H | H | H | $OCH_3$ | $CH_2OCONHCH_3$ | |
| $CO_2CH(CH_3)_2$ . | 5-F | H | $CH_3$ | $CH_2OCO_2CH_3$ | |
| $CO_2CH(CH_3)_2$ | H | H | $CH_3$ | $CH_2SC_2H_5$ | |
| $NO_2$ | H | H | $CH_3$ | $CH(OCH_2CH_2O)$ | |
| $NO_2$ | 6-Br | H | $CH_3$ | $CH_2OCOC_2H_5$ | |
| Cl | 2-Cl | H | $OCH_3$ | $CH(OCH_2CH_2CH_2O)$ | |
| $CF_3$ | H | H | $OCH_3$ | $CH(OCH(CH_3)CH_2)$ | |
| Cl | 6-$CH_3$ | $CH_3$ | $OCH_3$ | $CH_2SCH_3$ | |
| $C_2H_5$ | H | $CH_3$ | $CH_3$ | $CH_2OCOCH_3$ | |
| $CH(CH_3)_2$ | H | H | $CH_2CH_3$ | $CH_2OCON(CH_3)_2$ | |
| $SO_2CH_3$ | H | H | $CH_3$ | $CH(OCH_2CH_2O)$ | |
| $SO_2CH_3$ | 5-$OCH_3$ | H | $OCH_3$ | $CH_2OCOCH_3$ | |
| $SO_2CH_3$ | 6-F | H | $OCH_3$ | $CH_2SC_2H_5$ | |
| $SO_2$-*n*-$C_4H_9$ | H | H | $CH_3$ | $CH(OC_2H_5)_2$ | |
| $SO_2N(CH_3)_2$ | H | H | $OCH_3$ | $CH_2OCO_2$-*n*-$C_3H_7$ | |
| $SO_2N(C_2H_5)_2$ | H | H | $CH_3$ | $CH_2OCON(C_2H_5)_2$ | |
| $SO_2N(OCH_3)CH_3$ | H | H | $CH_3$ | $CH_2OCON(CH_3)$(*n*-$C_3H_7$) | |
| $SO_2$-*n*-$C_3H_7$ | H | H | $CH_3$ | $CH_2OCO$-*i*-$C_3H_7$ | |
| $SO_2$-*i*-$C_3H_7$ | H | H | $OCH_3$ | $CH_2OCONH$(*n*-$C_3H_7$) | |
| $SCH_3$ | H | H | $CH_3$ | $CH(OC_2H_5)_2$ | |
| $S(O)CH_3$ | H | H | $CH_3$ | $CH(OC_2H_5)_2$ | |
| $SCH_2CH_3$ | H | H | $OCH_3$ | $CH_2OCO_2$-*i*-$C_3H_7$ | |
| S(O)-*n*-$C_4H_9$ | H | H | $OCH_2CH_3$ | $CH_2SCH_3$ | |

TABLE 6e

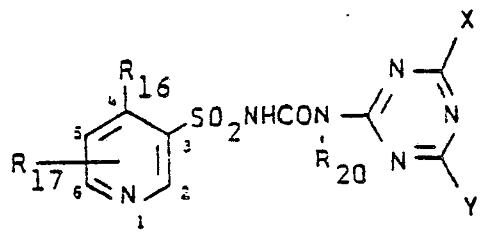

| $R_{16}$ | $R_{17}$ | $R_{20}$ | X | Y | m.p. (°C) |
|---|---|---|---|---|---|
| $CO_2CH_3$ | H | $CH_3$ | $CH_3$ | $CH(OCH_3)_2$ | |
| Cl | H | H | $CH_3$ | $CH(OCH_3)_2$ | |
| Cl | H | H | $OCH_3$ | $CH(OCH_3)_2$ | |
| H | H | H | $OCH_3$ | $CH(OCH_3)_2$ | |
| $CO_2CH(CH_3)_2$ | 5-F | H | $CH_3$ | $CH(OCH_3)_2$ | |
| $CO_2CH(CH_3)_2$ | H | H | $CH_3$ | $CH(OCH_3)_2$ | |
| $NO_2$ | H | H | $CH_3$ | $CH(OCH_3)_2$ | |
| $NO_2$ | 6-Bar | H | $CH_3$ | $CH(OCH_3)_2$ | |
| $CF_3$ | H | H | $OCH_3$ | $CH(OCH_3)_2$ | |
| Cl | 6-$CH_3$ | H | $OCH_3$ | $CH(OCH_3)_2$ | |
| $C_2H_5$ | H | H | $CH_3$ | $CH(OCH_3)_2$ | |
| $CH(CH_3)_2$ | H | H | $CH_3$ | $CH(OCH_3)_2$ | |
| $SO_2CH_3$ | H | H | $CH_3$ | $CH(OCH_3)_2$ | |
| $SO_2CH_3$ | 5-$OCH_3$ | H | $OCH_3$ | $CH(OCH_3)_2$ | |
| $SO_2CH_3$ | 6-F | H | $OCH_3$ | $CH(OCH_3)_2$ | |
| $SO_2$-*n*-$C_4H_9$ | H | H | $CH_3$ | $CH(OCH_3)_2$ | |
| $SO_2N(CH_3)_2$ | H | H | $OCH_3$ | $CH(OCH_3)_2$ | |
| $SO_2N(C_2H_5)_2$ | H | H | $CH_3$ | $CH(OCH_3)_2$ | |
| $SO_2N(OCH_3)CH_3$ | H | $CH_3$ | $CH_2CH_3$ | $CH(OCH_3)_2$ | |
| $SO_2$-*n*-$C_3H_7$ | H | H | $CH_3$ | $CH(OCH_3)_2$ | |
| $SO_2$-*i*-$C_3H_7$ | H | H | $OCH_2CH_3$ | $CH(OCH_3)_2$ | |
| $SCH_3$ | H | H | $CH_3$ | $CH(OCH_3)_2$ | |
| $S(C)CH_3$ | H | H | $CH_3$ | $CH(OCH_3)_2$ | |
| $SCH_2CH_3$ | H | H | $OCH_3$ | $CH(OCH_3)_2$ | |
| $S(C)(CH_2)_3CH_3$ | H | H | $OCH_3$ | $CH(OCH_3)_2$ | |

TABLE 6f

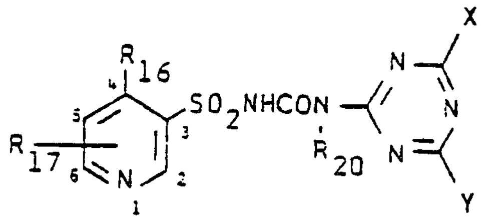

| $R_{16}$ | $R_{17}$ | $R_{20}$ | X | Y | m.p. (°C) |
|---|---|---|---|---|---|
| $CO_2CH_3$ | H | H | $CH_3$ | $CH_2OTHP$ | |
| Cl | H | H | $CH_3$ | $CH_2OTHP$ | |
| Cl | H | H | $OCH_3$ | $CH_2OTHP$ | |
| H | H | H | $OCH_3$ | $CH_2OTHP$ | |
| $CO_2CH(CH_3)_2$ | 5-F | $CH_3$ | $CH_3$ | $CH_2OTHP$ | |
| $CO_2CH(CH_3)_2$ | H | $CH_3$ | $CH_3$ | $CH_2OTHP$ | |
| $NO_2$ | H | H | $CH_3$ | $CH_2OTHP$ | |
| $NO_2$ | 6-Br | H | $CH_3$ | $CH_2OTHP$ | |
| Cl | 2-Cl | H | $OCH_2CH_3$ | $CH_2OTHP$ | |
| $CF_3$ | H | H | $OCH_3$ | $CH_2OTHP$ | |
| Cl | 6-$CH_3$ | H | $OCH_3$ | $CH_2OTHP$ | |
| $C_2H_5$ | H | H | $CH_3$ | $CH_2OTHP$ | |
| $CH(CH_3)_2$ | H | H | $CH_3$ | $CH_2OTHP$ | |
| $SO_2CH_3$ | H | H | $CH_3$ | $CH_2OTHP$ | |
| $SO_2CH_3$ | 5-$OCH_3$ | H | $OCH_3$ | $CH_2OTHP$ | |
| $SO_2CH_3$ | 6-F | H | $OCH_3$ | $CH_2OTHP$ | |
| $SO_2$-*n*-$C_4H_9$ | H | H | $CH_3$ | $CH_2OTHP$ | |
| $SO_2N(CH_3)_2$ | H | H | $OCH_3$ | $CH_2OTHP$ | |
| $SO_2N(C_2H_5)_2$ | H | H | $CH_2CH_3$ | $CH_2OTHP$ | |
| $SO_2N(OCH_3)CH_3$ | H | H | $CH_3$ | $CH_2OTHP$ | |
| $SO_2$-*n*-$C_3H_7$ | H | H | $CH_3$ | $CH_2OTHP$ | |
| $SO_2$-*i*-$C_3H_7$ | H | H | $OCH_3$ | $CH_2OTHP$ | |
| $SCH_3$ | H | H | $CH_3$ | $CH_2OTHP$ | |
| $S(O)CH_3$ | H | H | $CH_3$ | $CH_2OTHP$ | |
| $SCH_2CH_3$ | H | H | $OCH_3$ | $CH_2OTHP$ | |
| $S(O)$-*n*-$C_4H_9$ | H | H | $OCH_3$ | $CH_2OTHP$ | |

TABLE 6g

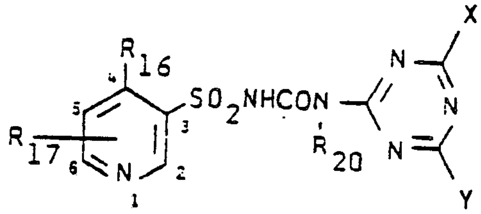

| $R_{16}$ | $R_{17}$ | $R_{20}$ | X | Y | m.p. (°C) |
|---|---|---|---|---|---|
| $CO_2CH_3$ | H | H | $CH_3$ | $CH_2OH$ | |
| Cl | H | H | $CH_3$ | $CH_2OH$ | |
| Cl | H | H | $OCH_3$ | $CH_2OH$ | |
| H | H | H | $OCH_3$ | $CH_2OH$ | |
| $CO_2CH(CH_3)_2$ | 5-F | H | $CH_2CH_3$ | $CH_2OH$ | |
| $CO_2CH(CH_3)_2$ | H | H | $CH_3$ | $CH_2OH$ | |
| $NO_2$ | H | H | $CH_3$ | $CH_2OH$ | |
| $NO_2$ | 6-Br | H | $CH_3$ | $CH_2OH$ | |
| Cl | 2-Cl | H | $OCH_3$ | $CH_2OH$ | |
| $CF_3$ | H | H | $OCH_3$ | $CH_2OH$ | |
| Cl | 6-$CH_3$ | H | $OCH_3$ | $CH_2OH$ | |
| $C_2H_5$ | H | H | $CH_3$ | $CH_2OH$ | |
| $CH(CH_3)_2$ | H | H | $CH_3$ | $CH_2OH$ | |
| $SO_2CH_3$ | H | H | $CH_3$ | $CH_2OH$ | |
| $SO_2CH_3$ | 5-$OCH_3$ | H | $OCH_3$ | $CH_2OH$ | |
| $SO_2CH_3$ | 6-F | H | $OCH_3$ | $CH_2OH$ | |
| $SO_2$-*n*-$C_4H_9$ | H | $CH_3$ | $CH_3$ | $CH_2OH$ | |
| $SO_2N(CH_3)_2$ | H | H | $OCH_3$ | $CH_2OH$ | |
| $SO_2N(C_2H_5)_2$ | H | H | $CH_3$ | $CH_2OH$ | |
| $SO_2N(OCH_3)CH_3$ | H | H | $CH_3$ | $CH_2OH$ | |
| $SO_2$-*n*-$C_3H_7$ | H | H | $CH_3$ | $CH_2OH$ | |
| $SO_2$-*i*-$C_3H_7$ | H | H | $OCH_3$ | $CH_2OH$ | |
| $SCH_3$ | H | H | $CH_3$ | $CH_2OH$ | |
| $S(O)CH_3$ | H | H | $CH_3$ | $CH_2OH$ | |
| $SCH_2CH_3$ | H | H | $OCH_2CH_3$ | $CH_2OH$ | |
| $S(O)$-*n*-$C_4H_9$ | H | H | $OCH_3$ | $CH_2OH$ | |

TABLE 6h

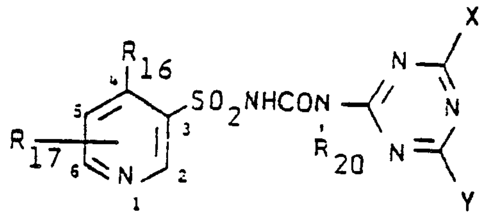

| $R_{16}$ | $R_{17}$ | $R_{20}$ | X | Y | m.p. (°C) |
|---|---|---|---|---|---|
| $CO_2CH_3$ | H | H | $CH_3$ | $CH_2OCOCH_3$ | |
| Cl | H | H | $CH_3$ | $CH(OC_2H_5)_2$ | |
| Cl | H | H | $OCH_3$ | $CH_2SCH_3$ | |
| H | H | H | $OCH_3$ | $CH_2OCONHCH_3$ | |
| $CO_2CH(CH_3)_2$ | 5-F | H | $CH_3$ | $CH_2OCO_2CH_3$ | |
| $CO_2CH(CH_3)_2$ | H | H | $CH_3$ | $CH_2SC_2H_5$ | |
| $NO_2$ | H | H | $CH_3$ | $CH(OCH_2CH_2O)$ | |
| $NO_2$ | 6-Br | H | $CH_3$ | $CH_2OCOC_2H_5$ | |
| Cl | 2-Cl | H | $OCH_3$ | $CH(OCH_2CH_2CH_2O)$ | |
| $CF_3$ | H | H | $OCH_3$ | $CH(OCH(CH_3)CH_2)$ | |
| Cl | 6-$CH_3$ | H | $OCH_3$ | $CH_2SCH_3$ | |
| $C_2H_5$ | H | H | $CH_3$ | $CH_2OCOCH_3$ | |
| $CH(CH_3)_2$ | H | $CH_3$ | $CH_3$ | $CH_2OCON(CH_3)_2$ | |
| $SO_2CH_3$ | H | H | $CH_3$ | $CH(OCH_2CH_2O)$ | |
| $SO_2CH_3$ | 5-$OCH_3$ | H | $OCH_3$ | $CH_2OCOCH_3$ | |
| $SO_2CH_3$ | 6-F | H | $OCH_3$ | $CH_2SC_2H_5$ | |
| $SO_2$-*n*-$C_4H_9$ | H | H | $CH_3$ | $CH(OC_2H_5)_2$ | |
| $SO_2N(CH_3)_2$ | H | H | $OCH_2CH_3$ | $CH_2OCO_2$-*n*-$C_3H_7$ | |
| $SO_2N(C_2H_5)_2$ | H | H | $CH_3$ | $CH_2OCON(C_2H_5)_2$ | |
| $SO_2N(OCH_3)CH_3$ | H | H | $CH_3$ | $CH_2OCON(CH_3)$(*n*-$C_3H_7$) | |
| $SO_2$-*n*-$C_3H_7$ | H | H | $CH_3$ | $CH_2OCO$-*i*-$C_3H_7$ | |
| $SO_2$-*i*-$C_3H_7$ | H | H | $OCH_3$ | $CH_2OCONH$(*n*-$C_3H_7$) | |
| $SCH_3$ | H | H | $CH_3$ | $CH(OC_2H_5)_2$ | |
| $S(O)CH_3$ | H | H | $CH_3$ | $CH(OC_2H_5)_2$ | |
| $SCH_2CH_3$ | H | H | $OCH_2CH_3$ | $CH_2OCO_2$-*i*-$C_3H_7$ | |
| S(O)-*n*-$C_4H_9$ | H | $CH_3$ | $OCH_3$ | $CH_2SCH_3$ | |

TABLE 7a

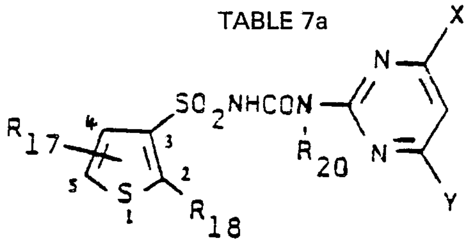

| R17 | R18 | R20 | X | Y | m.p. (°C) |
|---|---|---|---|---|---|
| H | $CO_2CH_3$ | H | $CH_3$ | $CH(OCH_3)_2$ | |
| 4-$CH_3$ | $CO_2CH_3$ | H | $CH_3$ | $CH(OCH_3)_2$ | |
| H | $CO_2CH_3$ | H | $OCH_3$ | $CH(OCH_3)_2$ | |
| H | $CO_2CH_2CH{=}CH_2$ | H | $CH_3$ | $CH(OCH_3)_2$ | |
| H | H | H | $CH_3$ | $CH(OCH_3)_2$ | |
| H | $SCH_2CH_2CH_3$ | H | $OCH_3$ | $CH(OCH_3)_2$ | |
| H | $SCH_3$ | H | $CH_3$ | $CH(OCH_3)_2$ | |
| H | $CH(CH_3)_2$ | H | $OCH_2CH_3$ | $CH(OCH_3)_2$ | |
| H | $CH_2CH_3$ | H | $CH_3$ | $CH(OCH_3)_2$ | |
| H | F | H | $OCH_3$ | $CH(OCH_3)_2$ | |
| H | Cl | H | $OCH_3$ | $CH(OCH_3)_2$ | |
| 5-Cl | Cl | H | $CH_3$ | $CH(OCH_3)_2$ | |
| H | Br | $CH_3$ | $OCH_3$ | $CH(OCH_3)_2$ | |
| H | $NO_2$ | Br | $CH_3$ | $CH(OCH_3)_2$ | |
| H | $SO_2N(CH_3)CH_2CH_3$ | H | $CH_3$ | $CH(OCH_3)_2$ | |
| H | $SO_2N(CH_3)_2$ | H | $OCH_3$ | $CH(OCH_3)_2$ | |
| H | $SO_2N(OCH_3)CH_3$ | H | $OCH_3$ | $CH(OCH_3)_2$ | |
| H | $OCH_3$ | $CH_3$ | $CH_3$ | $CH(OCH_3)_2$ | |
| 5-$OCH_3$ | $OCH_3$ | H | $CH_2CH_3$ | $CH(OCH_3)_2$ | |
| 5-F | $O(CH_2)_3CH_3$ | H | $OCH_3$ | $CH(OCH_3)_2$ | |
| H | $SO_2CH_2CH_2CH_3$ | H | $OCH_3$ | $CH(OCH_3)_2$ | |
| H | $SO_2CH_3$ | H | $CH_3$ | $CH(OCH_3)_2$ | |
| 4-Br | $SO_2CH_3$ | H | $CH_3$ | $CH(OCH_3)_2$ | |
| H | $CO_2CH_3$ | H | Cl | $CH(OCH_3)_2$ | |
| H | $NO_2$ | H | Cl | $CH(OCH_3)_2$ | |
| H | $SO_2CH_2CH_2CH_3$ | H | Cl | $CH(OCH_3)_2$ | |
| H | $SO_2CH_3$ | H | Cl | $CH(OCH_3)_2$ | |
| H | $SCH_3$ | H | Cl | $CH(OCH_3)_2$ | |

TABLE 7b

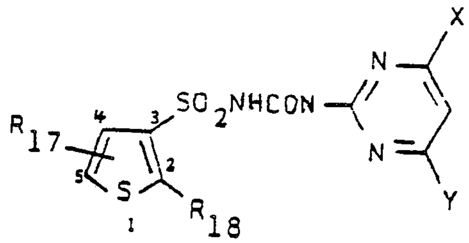

| $R_{17}$ | $R_{18}$ | $R_{20}$ | X | Y | m.p. (°C) |
|---|---|---|---|---|---|
| H | $CO_2CH_3$ | H | $CH_3$ | $CH_2OTHP$ | |
| 4-$CH_3$ | $CO_2CH_3$ | H | $CH_2CH_3$ | $CH_2OTHP$ | |
| H | $CO_2CH_2CH_3$ | H | $OCH_3$ | $CH_2OTHP$ | |
| H | $CO_2CH_2CH{=}CH_2$ | H | $CH_3$ | $CH_2OTHP$ | |
| H | H | H | $CH_3$ | $CH_2OTHP$ | |
| H | $SCH_2CH_2CH_3$ | H | $OCH_3$ | $CH_2OTHP$ | |
| H | $SCH_3$ | H | $CH_3$ | $CH_2OTHP$ | |
| H | $CH(CH_3)_2$ | H | $OCH_3$ | $CH_2OTHP$ | |
| H | $CH_2CH_3$ | H | $CH_3$ | $CH_2OTHP$ | |
| H | F | H | $OCH_3$ | $CH_2OTHP$ | |
| H | Cl | H | $OCH_3$ | $CH_2OTHP$ | |
| 5-Cl | Cl | H | $CH_3$ | $CH_2OTHP$ | |
| H | Br | $CH_3$ | $OCH_3$ | $CH_2OTHP$ | |
| H | $NO_2$ | $CH_3$ | $CH_3$ | $CH_2OTHP$ | |
| H | $SO_2N(CH_3)CH_2CH_3$ | H | $CH_3$ | $CH_2OTHP$ | |
| H | $SO_2N(CH_3)_2$ | H | $OCH_3$ | $CH_2OTHP$ | |
| H | $SO_2N(OCH_3)CH_3$ | H | $OCH_2CH_3$ | $CH_2OTHP$ | |
| H | $OCH_3$ | H | $CH_3$ | $CH_2OTHP$ | |
| 5-$OCH_3$ | $OCH_3$ | H | $CH_3$ | $CH_2OTHP$ | |
| 5-F | $O(CH_2)_3CH_3$ | H | $OCH_3$ | $CH_2OTHP$ | |
| H | $SO_2CH_2CH_2CH_3$ | H | $OCH_3$ | $CH_2OTHP$ | |
| H | $SO_2CH_3$ | H | $CH_3$ | $CH_2OTHP$ | |
| 4-Br | $SO_2CH_3$ | H | $CH_3$ | $CH_2OTHP$ | |

TABLE 7c

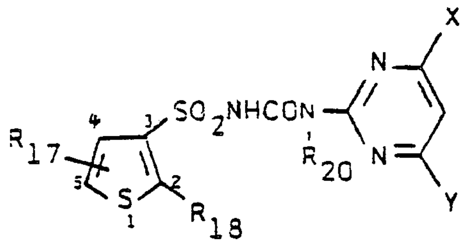

| $R_{17}$ | $R_{18}$ | $R_{20}$ | X | Y | m.p. (°C) |
|---|---|---|---|---|---|
| H | $CO_2CH_3$ | H | $CH_3$ | $CH_2OH$ | 143—147° |
| 4-$CH_3$ | $CO_2CH_3$ | H | $CH_3$ | $CH_2OH$ | |
| H | $CO_2CH_2CH_3$ | H | $OCH_3$ | $CH_2OH$ | |
| H | $CO_2CH_2CH{=}CH_2$ | H | $CH_3$ | $CH_2OH$ | |
| H | H | H | $CH_3$ | $CH_2OH$ | |
| H | $SCH_2CH_2CH_3$ | H | $OCH_3$ | $CH_2OH$ | |
| H | $SCH_3$ | H | $CH_3$ | $CH_2OH$ | |
| H | $CH(CH_3)_2$ | H | $OCH_3$ | $CH_2OH$ | |
| H | $CH_2CH_3$ | $CH_3$ | $CH_3$ | $CH_2OH$ | |
| H | F | H | $OCH_2CH_3$ | $CH_2OH$ | |
| H | Cl | $CH_3$ | $OCH_3$ | $CH_2OH$ | |
| 5-Cl | Cl | H | $CH_2CH_3$ | $CH_2OH$ | |
| H | Br | H | $OCH_3$ | $CH_2OH$ | |
| H | $NO_2$ | H | $CH_3$ | $CH_2OH$ | |
| H | $SO_2N(CH_3)CH_2CH_3$ | H | $CH_3$ | $CH_2OH$ | |
| H | $SO_2N(CH_3)_2$ | H | $OCH_3$ | $CH_2OH$ | |
| H | $SO_2N(OCH_3)CH_3$ | H | $OCH_3$ | $CH_2OH$ | |
| H | $OCH_3$ | H | $CH_3$ | $CH_2OH$ | |
| 5-$OCH_3$ | $OCH_3$ | H | $CH_3$ | $CH_2OH$ | |
| 5-F | $O(CH_2)_3CH_3$ | H | $OCH_3$ | $CH_2OH$ | |
| H | $SO_2CH_2CH_2CH_3$ | H | $OCH_3$ | $CH_2OH$ | |
| H | $SO_2CH_3$ | H | $CH_3$ | $CH_2OH$ | |
| 4-Br | $SO_2CH_3$ | H | $CH_3$ | $CH_2OH$ | |

TABLE 7d

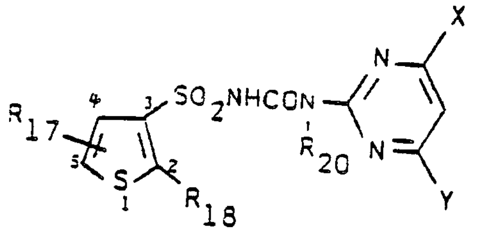

| $R_{17}$ | $R_{18}$ | $R_{20}$ | X | Y | m.p. (°C) |
|---|---|---|---|---|---|
| H | $CO_2CH_3$ | H | $CH_3$ | $CH(OC_2H_5)_2$ | |
| 4-$CH_3$ | $CO_2CH_3$ | H | $CH_3$ | $CH_2OCOCH_3$ | |
| H | $CO_2CH_3$ | H | $OCH_3$ | $CH_2SCH_3$ | |
| H | $CO_2CH_2CH=CH_2$ | H | $CH_3$ | $CH_2OCONHCH_3$ | |
| H | H | H | $CH_3$ | $CH_2OCO_2CH_3$ | |
| H | $SCH_2CH_2CH_3$ | H | $OCH_3$ | $CH_2SC_2H_5$ | |
| H | $SCH_3$ | H | $CH_3$ | $CH(OCH_2CH_2O)$ | |
| H | $CH(CH_3)_2$ | H | $OCH_3$ | $CH_2OCOC_2H_5$ | |
| H | $CH_2CH_3$ | H | $CH_3$ | $CH(OCH(CH_3)CH_2O)$ | |
| H | F | H | $OCH_3$ | $CH(OCH_2CH_2CH_2O)$ | |
| H | Cl | H | $OCH_3$ | $CH_2SCH_3$ | |
| 5-Cl | Cl | H | $CH_3$ | $CH_2OCOCH_3$ | |
| H | Br | $CH_3$ | $OCH_3$ | $CH_2OCON(CH_3)_2$ | |
| H | $NO_2$ | $CH_3$ | $CH_3$ | $CH(OCH_2CH_2O)$ | |
| H | $SO_2N(CH_3)C_2H_5$ | H | $CH_3$ | $CH_2OCOCH_3$ | |
| H | $SO_2N(CH_3)_2$ | H | $OCH_2CH_3$ | $CH_2SCH_2CH_3$ | |
| H | $SO_2N(OCH_3)CH_3$ | H | $CH_3$ | $CH(OC_2H_5)_2$ | |
| H | $OCH_3$ | H | $CH_3$ | $CH_2OCO_2C_2H_5$ | |
| 5-$OCH_3$ | $OCH_3$ | H | $CH_3$ | $CH_2OCON(C_2H_5)_2$ | |
| 5-F | $O(CH_2)_3CH_3$ | H | $OCH_3$ | $CH_2OCON(C_2H_5)_2$ | |
| H | $SO_2CH_2CH_2CH_3$ | H | $CH_3$ | $CH_2OCO(i\text{-}C_3H_7)$ | |
| H | $SO_2CH_3$ | H | $CH_2CH_3$ | $CH_2OCONH(n\text{-}C_3H_7)$ | |

TABLE 7e

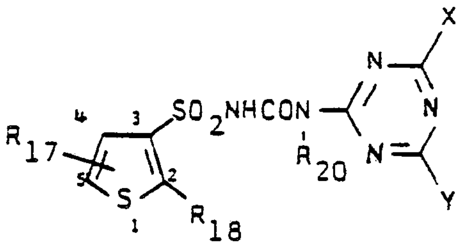

| $R_{17}$ | $R_{18}$ | $R_{20}$ | X | Y | m.p. (°C) |
|---|---|---|---|---|---|
| H | $CO_2CH_3$ | H | $CH_3$ | $CH(OCH_3)_2$ | |
| 4-$CH_3$ | $CO_2CH_3$ | H | $CH_3$ | $CH(OCH_3)_2$ | |
| H | $CO_2CH_3$ | H | $OCH_2CH_3$ | $CH(OCH_3)_2$ | |
| H | $CO_2CH_2CH=CH_2$ | H | $CH_3$ | $CH(OCH_3)_2$ | |
| H | H | $CH_3$ | $CH_3$ | $CH(OCH_3)_2$ | |
| H | $SCH_2CH_2CH_3$ | H | $OCH_3$ | $CH(OCH_3)_2$ | |
| H | $SCH_3$ | H | $CH_3$ | $CH(OCH_3)_2$ | |
| H | $CH(CH_3)_2$ | H | $OCH_3$ | $CH(OCH_3)_2$ | |
| H | $CH_2CH_3$ | H | $CH_3$ | $CH(OCH_3)_2$ | |
| H | F | H | $OCH_3$ | $CH(OCH_3)_2$ | |
| H | Cl | H | $OCH_3$ | $CH(OCH_3)_2$ | |
| 5-Cl | Cl | H | $CH_3$ | $CH(OCH_3)_2$ | |
| H | Br | H | $OCH_3$ | $CH(OCH_3)_2$ | |
| H | $NO_2$ | H | $CH_3$ | $CH(OCH_3)_2$ | |
| H | $SO_2N(CH_3)CH_2CH_3$ | H | $CH_3$ | $CH(OCH_3)_2$ | |
| H | $SO_2N(CH_3)_2$ | $CH_3$ | $OCH_3$ | $CH(OCH_3)_2$ | |
| H | $SO_2N(OCH_3)CH_3$ | H | $OCH_3$ | $CH(OCH_3)_2$ | |
| H | $OCH_3$ | H | $CH_2CH_3$ | $CH(OCH_3)_2$ | |
| 5-$OCH_3$ | $OCH_3$ | H | $CH_3$ | $CH(OCH_3)_2$ | |
| 5-F | $O(CH_2)_3CH_3$ | H | $OCH_3$ | $CH(OCH_3)_2$ | |
| H | $SO_2CH_2CH_2CH_3$ | H | $OCH_3$ | $CH(OCH_3)_2$ | |
| H | $SO_2CH_3$ | H | $CH_3$ | $CH(OCH_3)_2$ | |
| 4-Br | $SO_2CH_3$ | H | $CH_3$ | $CH(OCH_3)_2$ | |

TABLE 7f

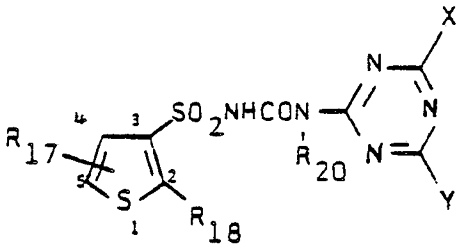

| $R_{17}$ | $R_{18}$ | $R_{20}$ | X | Y | m.p. (°C) |
|---|---|---|---|---|---|
| H | $CO_2CH_3$ | H | $CH_3$ | $CH_2OTHP$ | |
| 4-$CH_3$ | $CO_2CH_3$ | H | $CH_3$ | $CH_2OTHP$ | |
| H | $CO_2CH_2CH_3$ | H | $OCH_3$ | $CH_2OTHP$ | |
| H | $CO_2CH_2CH{=}CH_2$ | H | $CH_2CH_3$ | $CH_2OTHP$ | |
| H | H | H | $CH_3$ | $CH_2OTHP$ | |
| H | $SCH_2CH_2CH_3$ | H | $OCH_3$ | $CH_2OTHP$ | |
| H | $SCH_3$ | H | $CH_3$ | $CH_2OTHP$ | |
| H | $CH(CH_3)_2$ | H | $OCH_3$ | $CH_2OTHP$ | |
| H | $CH_2CH_3$ | H | $CH_3$ | $CH_2OTHP$ | |
| H | F | H | $OCH_2CH_3$ | $CH_2OTHP$ | |
| H | Cl | H | $OCH_3$ | $CH_2OTHP$ | |
| 5-Cl | Cl | H | $CH_3$ | $CH_2OTHP$ | |
| H | Br | $CH_3$ | $OCH_3$ | $CH_2OTHP$ | |
| H | $NO_2$ | H | $CH_3$ | $CH_2OTHP$ | |
| H | $SO_2N(CH_3)CH_2CH_3$ | $CH_3$ | $CH_3$ | $CH_2OTHP$ | |
| H | $SO_2N(CH_3)_2$ | H | $OCH_3$ | $CH_2OTHP$ | |
| H | $SO_2N(OCH_3)CH_3$ | H | $OCH_3$ | $CH_2OTHP$ | |
| H | $OCH_3$ | H | $CH_3$ | $CH_2OTHP$ | |
| 5-$OCH_3$ | $OCH_3$ | H | $CH_3$ | $CH_2OTHP$ | |
| 5-F | $O(CH_2)_3CH_3$ | H | $OCH_3$ | $CH_2OTHP$ | |
| H | $SO_2CH_2CH_2CH_3$ | H | $OCH_3$ | $CH_2OTHP$ | |
| H | $SO_2CH_3$ | H | $CH_3$ | $CH_2OTHP$ | |
| 4-Br | $SO_2CH_3$ | H | $CH_3$ | $CH_2OTHP$ | |

TABLE 7g

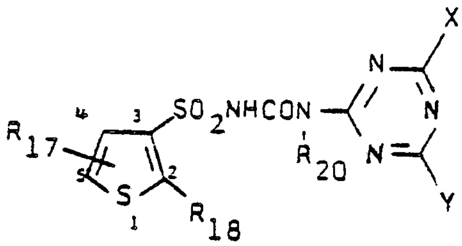

| $R_{17}$ | $R_{18}$ | $R_{20}$ | X | Y | m.p. (°C) |
|---|---|---|---|---|---|
| H | $CO_2CH_3$ | H | $CH_3$ | $CH_2OH$ | |
| 4-$CH_3$ | $CO_2CH_3$ | H | $CH_3$ | $CH_2OH$ | |
| H | $CO_2CH_2CH_3$ | $CH_3$ | $OCH_3$ | $CH_2OH$ | |
| H | $CO_2CH_2CH{=}CH_2$ | H | $CH_3$ | $CH_2OH$ | |
| H | H | H | $CH_3$ | $CH_2OH$ | |
| H | $SCH_2CH_2CH_3$ | H | $OCH_3$ | $CH_2OH$ | |
| H | $SCH_3$ | H | $CH_3$ | $CH_2OH$ | |
| H | $CH(CH_3)_2$ | H | $OCH_3$ | $CH_2OH$ | |
| H | $CH_2CH_3$ | H | $CH_3$ | $CH_2OH$ | |
| H | F | H | $OCH_3$ | $CH_2OH$ | |
| H | Cl | H | $OCH_2CH_3$ | $CH_2OH$ | |
| 5-Cl | Cl | H | $CH_2CH_3$ | $CH_2OH$ | |
| H | Br | H | $OCH_3$ | $CH_2OH$ | |
| H | $NO_2$ | H | $CH_3$ | $CH_2OH$ | |
| H | $SO_2N(CH_3)CH_2CH_3$ | H | $CH_3$ | $CH_2OH$ | |
| H | $SO_2N(CH_3)_2$ | H | $OCH_3$ | $CH_2OH$ | |
| H | $SO_2N(OCH_3)CH_3$ | H | $OCH_3$ | $CH_2OH$ | |
| H | $OCH_3$ | H | $CH_3$ | $CH_2OH$ | |
| 5-$OCH_3$ | $OCH_3$ | H | $CH_3$ | $CH_2OH$ | |
| 5-F | $O(CH_2)_3CH_3$ | H | $OCH_3$ | $CH_2OH$ | |
| H | $SO_2CH_2CH_2CH_3$ | H | $OCH_3$ | $CH_2OH$ | |
| H | $SO_2CH_3$ | H | $CH_3$ | $CH_2OH$ | |
| 4-Br | $SO_2CH_3$ | H | $CH_3$ | $CH_2OH$ | |

Table 7h

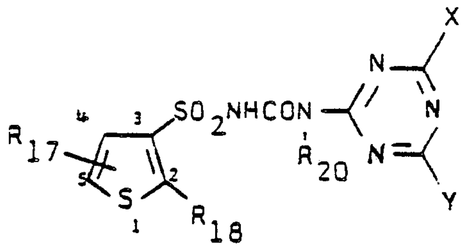

| $R_{17}$ | $R_{18}$ | $R_{20}$ | X | Y | m.p. (°C) |
|---|---|---|---|---|---|
| H | $CO_2CH_3$ | H | $CH_3$ | $CH(OC_2H_5)_2$ | |
| 4-$CH_3$ | $CO_2CH_3$ | H | $CH_3$ | $CH_2OCOCH_3$ | |
| H | $CO_2CH_3$ | H | $OCH_3$ | $CH_2SCH_3$ | |
| H | $CO_2CH_2CH{=}CH_2$ | H | $CH_3$ | $CH_2OCONHCH_3$ | |
| H | H | H | $CH_3$ | $CH_2OCO_2CH_3$ | |
| H | $SCH_2CH_2CH_3$ | H | $OCH_3$ | $CH_2SC_2H_5$ | |
| H | $SCH_3$ | H | $CH_3$ | $CH(OCH_2CH_2O)$ | |
| H | $CH(CH_3)_2$ | H | $OCH_3$ | $CH_2OCOC_2H_5$ | |
| H | $CH_2CH_3$ | $CH_3$ | $CH_3$ | $CH(OCH(CH_3)CH_2O)$ | |
| H | F | H | $OCH_3$ | $CH(OCH_2CH_2CH_2O)$ | |
| H | Cl | H | $OCH_3$ | $CH_2SCH_3$ | |
| 5-Cl | Cl | H | $CH_2CH_3$ | $CH_2OCOCH_3$ | |
| H | Br | H | $OCH_2CH_3$ | $CH_2OCON(CH_3)_2$ | |
| H | $NO_2$ | H | $CH_3$ | $CH(OCH_2CH_2O)$ | |
| H | $SO_2N(CH_3)C_2H_5$ | H | $CH_3$ | $CH_2OCOCH_3$ | |
| H | $SO_2N(CH_3)_2$ | H | $OCH_3$ | $CH_2SCH_2CH_3$ | |
| H | $SO_2N(OCH_3)CH_3$ | H | $CH_3$ | $CH(OC_2H_5)_2$ | |
| H | $OCH_3$ | H | $CH_3$ | $CH_2OCO_2C_2H_5$ | |
| 5-$OCH_3$ | $OCH_3$ | H | $CH_3$ | $CH_2OCON(C_2H_5)_2$ | |
| 5-F | $O(CH_2)_3CH_3$ | H | $OCH_3$ | $CH_2OCON(C_2H_5)_2$ | |
| H | $SO_2CH_2CH_2CH_3$ | H | $CH_3$ | $CH_2OCO(i\text{-}C_3H_7)$ | |
| H | $SO_2CH_3$ | H | $CH_3$ | $CH_2OCONH(n\text{-}C_3H_7)$ | |

TABLE 8a

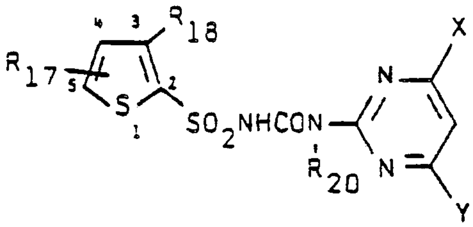

| $R_{17}$ | $R_{18}$ | $R_{20}$ | X | Y | m.p. (°C) |
|---|---|---|---|---|---|
| H | $CO_2CH_3$ | H | $CH_3$ | $CH(OCH_3)_2$ | |
| 4-$CH_3$ | $CO_2CH_3$ | H | $CH_3$ | $CH(OCH_3)_2$ | |
| H | $CO_2CH_3$ | H | $OCH_3$ | $CH(OCH_3)_2$ | |
| H | $CO_2CH_2CH{=}CH_2$ | H | $CH_3$ | $CH(OCH_3)_2$ | |
| H | H | H | $CH_2CH_3$ | $CH(OCH_3)_2$ | |
| H | $SCH_2CH_2CH_3$ | H | $OCH_2CH_3$ | $CH(OCH_3)_2$ | |
| H | $SCH_3$ | H | $CH_3$ | $CH(OCH_3)_2$ | |
| H | $CH(CH_3)_2$ | H | $OCH_3$ | $CH(OCH_3)_2$ | |
| H | $CH_2CH_3$ | H | $CH_3$ | $CH(OCH_3)_2$ | |
| H | F | H | $OCH_3$ | $CH(OCH_3)_2$ | |
| H | Cl | H | $OCH_3$ | $CH(OCH_3)_2$ | |
| 5-Cl | Cl | H | $CH_3$ | $CH(OCH_3)_2$ | |
| H | Br | H | $OCH_3$ | $CH(OCH_3)_2$ | |
| H | $NO_2$ | H | $CH_3$ | $CH(OCH_3)_2$ | |
| H | $SO_2N(CH_3)CH_2CH_3$ | H | $CH_3$ | $CH(OCH_3)_2$ | |
| H | $SO_2N(CH_3)_2$ | H | $OCH_3$ | $CH(OCH_3)_2$ | |
| H | $SO_2N(OCH_3)CH_3$ | H | $OCH_3$ | $CH(OCH_3)_2$ | |
| H | $OCH_3$ | $CH_3$ | $CH_3$ | $CH(OCH_3)_2$ | |
| 5-$OCH_3$ | $OCH_3$ | H | $CH_3$ | $CH(OCH_3)_2$ | |
| 5-F | $O(CH_2)_3CH_3$ | H | $OCH_3$ | $CH(OCH_3)_2$ | |
| H | $SO_2CH_2CH_2CH_3$ | H | $OCH_3$ | $CH(OCH_3)_2$ | |
| H | $SO_2CH_3$ | H | $CH_3$ | $CH(OCH_3)_2$ | |
| 4-Br | $SO_2CH_3$ | H | $CH_3$ | $CH(OCH_3)_2$ | |
| H | $CO_2CH_3$ | H | Cl | $CH(OCH_3)_2$ | |
| H | $NO_2$ | H | Cl | $CH(OCH_3)_2$ | |
| H | $SO_2CH_2CH_2CH_3$ | H | Cl | $CH(OCH_3)_2$ | |
| H | $SO_2CH_3$ | H | Cl | $CH(OCH_3)_2$ | |
| H | $SCH_3$ | H | Cl | $CH(OCH_3)_2$ | |
| H | $SO_2$-$n$-$C_3H_7$ | H | $CH_3$ | $CH(OCH_3)_2$ | 120—124°d |

TABLE 8b

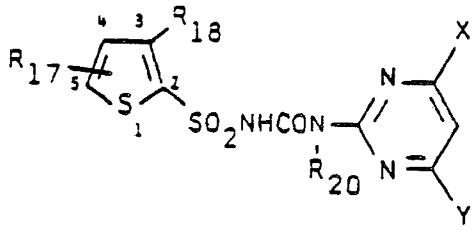

| $R_{17}$ | $R_{18}$ | $R_{20}$ | X | Y | m.p. (°C) |
|---|---|---|---|---|---|
| H | $CO_2CH_3$ | H | $CH_3$ | $CH_2OTHP$ | |
| 4-$CH_3$ | $CO_2CH_3$ | H | $CH_3$ | $CH_2OTHP$ | |
| H | $CO_2CH_2CH_3$ | H | $OCH_3$ | $CH_2OTHP$ | |
| H | $CO_2CH_2CH{=}CH_2$ | H | $CH_3$ | $CH_2OTHP$ | |
| H | H | H | $CH_3$ | $CH_2OTHP$ | |
| H | $SCH_2CH_2CH_3$ | H | $OCH_3$ | $CH_2OTHP$ | |
| H | $SCH_3$ | H | $CH_3$ | $CH_2OTHP$ | |
| H | $CH(CH_3)_2$ | H | $OCH_3$ | $CH_2OTHP$ | |
| H | $CH_2CH_3$ | H | $CH_3$ | $CH_2OTHP$ | |
| H | F | H | $OCH_3$ | $CH_2OTHP$ | |
| H | Cl | H | $OCH_3$ | $CH_2OTHP$ | |
| 5-Cl | Cl | H | $CH_3$ | $CH_2OTHP$ | |
| H | Br | H | $OCH_3$ | $CH_2OTHP$ | |
| H | $NO_2$ | $CH_3$ | $CH_3$ | $CH_2OTHP$ | |
| H | $SO_2N(CH_3)CH_2CH_3$ | H | $CH_3$ | $CH_2OTHP$ | |
| H | $SO_2N(CH_3)_2$ | H | $OCH_3$ | $CH_2OTHP$ | |
| H | $SO_2N(OCH_3)CH_3$ | H | $OCH_3$ | $CH_2OTHP$ | |
| H | $OCH_3$ | H | $CH_3$ | $CH_2OTHP$ | |
| 5-$OCH_3$ | $OCH_3$ | H | $CH_3$ | $CH_2OTHP$ | |
| 5-F | $O(CH_2)_3CH_3$ | H | $OCH_3$ | $CH_2OTHP$ | |
| H | $SO_2CH_2CH_2CH_3$ | H | $OCH_2CH_3$ | $CH_2OTHP$ | |
| H | $SO_2CH_3$ | H | $CH_2CH_3$ | $CH_2OTHP$ | |
| 4-Br | $SO_2CH_3$ | H | $CH_3$ | $CH_2OTHP$ | |

TABLE 8c

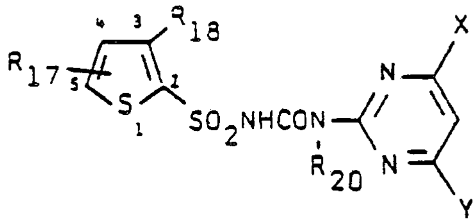

| $R_{17}$ | $R_{18}$ | $R_{20}$ | X | Y | m.p. (°C) |
|---|---|---|---|---|---|
| H | $CO_2CH_3$ | H | $CH_3$ | $CH_2OH$ | |
| 4-$CH_3$ | $CO_2CH_3$ | H | $CH_2CH_3$ | $CH_2OH$ | |
| H | $CO_2CH_2CH_3$ | H | $OCH_2CH_3$ | $CH_2OH$ | |
| H | $CO_2CH_2CH=CH_2$ | H | $CH_3$ | $CH_2OH$ | |
| H | H | H | $CH_3$ | $CH_2OH$ | |
| H | $SCH_2CH_2CH_3$ | H | $OCH_3$ | $CH_2OH$ | |
| H | $SCH_3$ | $CH_3$ | $CH_3$ | $CH_2OH$ | |
| H | $CH(CH_3)_2$ | H | $OCH_3$ | $CH_2OH$ | |
| H | $CH_2CH_3$ | H | $CH_3$ | $CH_2OH$ | |
| H | F | H | $OCH_3$ | $CH_2OH$ | |
| H | Cl | H | $OCH_3$ | $CH_2OH$ | |
| 5-Cl | Cl | H | $CH_3$ | $CH_2OH$ | |
| H | Br | H | $OCH_3$ | $CH_2OH$ | |
| H | $NO_2$ | H | $CH_3$ | $CH_2OH$ | |
| H | $SO_2N(CH_3)CH_2CH_3$ | H | $CH_3$ | $CH_2OH$ | |
| H | $SO_2N(CH_3)_2$ | H | $OCH_3$ | $CH_2OH$ | |
| H | $SO_2N(OCH_3)CH_3$ | H | $OCH_3$ | $CH_2OH$ | |
| H | $OCH_3$ | H | $CH_3$ | $CH_2OH$ | |
| 5-$OCH_3$ | $OCH_3$ | H | $CH_3$ | $CH_2OH$ | |
| 5-F | $O(CH_2)_3CH_3$ | H | $OCH_3$ | $CH_2OH$ | |
| H | $SO_2CH_2CH_2CH_3$ | H | $OCH_3$ | $CH_2OH$ | |
| H | $SO_2CH_3$ | H | $CH_3$ | $CH_2OH$ | |
| 4-Br | $SO_2CH_3$ | H | $CH_3$ | $CH_2OH$ | |

TABLE 8d

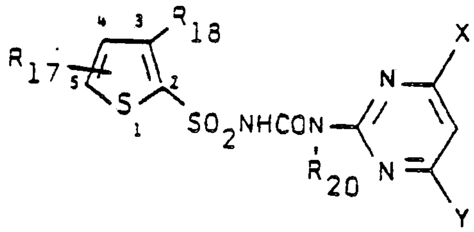

| $R_{17}$ | $R_{18}$ | $R_{20}$ | X | Y | m.p. (°C) |
|---|---|---|---|---|---|
| H | $CO_2CH_3$ | H | $CH_3$ | $CH(OC_2H_5)_2$ | |
| 4-$CH_3$ | $CO_2CH_3$ | H | $CH_3$ | $CH_2OCOCH_3$ | |
| H | $CO_2CH_3$ | H | $OCH_3$ | $CH_2SCH_3$ | |
| H | $CO_2CH_2CH{=}CH_2$ | H | $CH_3$ | $CH_2OCONHCH_3$ | |
| H | H | H | $CH_3$ | $CH_2OCO_2CH_3$ | |
| H | $SCH_2CH_2CH_3$ | H | $OCH_2CH_3$ | $CH_2SC_2H_5$ | |
| H | $SCH_3$ | H | $CH_2CH_3$ | $CH(OCH_2CH_2O)$ | |
| H | $CH(CH_3)_2$ | H | $OCH_3$ | $CH_2OCOC_2H_5$ | |
| H | $CH_2CH_3$ | H | $CH_3$ | $CH(OCH(CH_3)CH_2O)$ | |
| H | F | H | $OCH_3$ | $CH(OCH_2CH_2CH_2O)$ | |
| H | Cl | H | $OCH_3$ | $CH_2SCH_3$ | |
| 5-Cl | Cl | H | $CH_3$ | $CH_2OCOCH_3$ | |
| H | Br | H | $OCH_3$ | $CH_2OCON(CH_3)_2$ | |
| H | $NO_2$ | H | $CH_3$ | $CH(OCH_2CH_2O)$ | |
| H | $SO_2N(CH_3)C_2H_5$ | H | $CH_3$ | $CH_2OCOCH_3$ | |
| H | $SO_2N(CH_3)_2$ | H | $OCH_3$ | $CH_2SCH_2CH_3$ | |
| H | $SO_2N(OCH_3)CH_3$ | H | $CH_3$ | $CH(OC_2H_5)_2$ | |
| H | $OCH_3$ | $CH_3$ | $CH_3$ | $CH_2OCO_2C_2H_5$ | |
| 5-$OCH_3$ | $OCH_3$ | H | $CH_3$ | $CH_2OCON(C_2H_5)_2$ | |
| 5-F | $O(CH_2)_3CH_3$ | H | $OCH_3$ | $CH_2OCON(C_2H_5)_2$ | |
| H | $SO_2CH_2CH_2CH_3$ | H | $CH_3$ | $CH_2OCO(i\text{-}C_3H_7)$ | |
| H | $SO_2CH_3$ | H | $CH_3$ | $CH_2OCONH(n\text{-}C_3H_7)$ | |

TABLE 8e

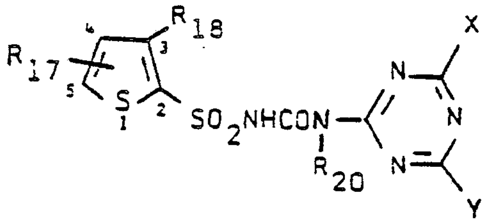

| $R_{17}$ | $R_{18}$ | $R_{20}$ | X | Y | m.p. (°C) |
|---|---|---|---|---|---|
| H | $CO_2CH_3$ | H | $CH_3$ | $CH(OCH_3)_2$ | |
| 4-$CH_3$ | $CO_2CH_3$ | H | $CH_3$ | $CH(OCH_3)_2$ | |
| H | $CO_2CH_3$ | H | $OCH_3$ | $CH(OCH_3)_2$ | |
| H | $CO_2CH_2CH{=}CH_2$ | H | $CH_3$ | $CH(OCH_3)_2$ | |
| H | H | H | $CH_3$ | $CH(OCH_3)_2$ | |
| H | $SCH_2CH_2CH_3$ | H | $OCH_3$ | $CH(OCH_3)_2$ | |
| H | $SCH_3$ | H | $CH_3$ | $CH(OCH_3)_2$ | |
| H | $CH(CH_3)_2$ | H | $OCH_3$ | $CH(OCH_3)_2$ | |
| H | $CH_2CH_3$ | H | $CH_3$ | $CH(OCH_3)_2$ | |
| H | F | H | $OCH_3$ | $CH(OCH_3)_2$ | |
| H | Cl | H | $OCH_3$ | $CH(OCH_3)_2$ | |
| 5-Cl | Cl | H | $CH_3$ | $CH(OCH_3)_2$ | |
| H | Br | H | $OCH_3$ | $CH(OCH_3)_2$ | |
| H | $NO_2$ | H | $CH_3$ | $CH(OCH_3)_2$ | |
| H | $SO_2N(CH_3)CH_2CH_3$ | H | $CH_2CH_3$ | $CH(OCH_3)_2$ | |
| H | $SO_2N(CH_3)_2$ | H | $OCH_2CH_3$ | $CH(OCH_3)_2$ | |
| H | $SO_2N(OCH_3)CH_3$ | $CH_3$ | $OCH_3$ | $CH(OCH_3)_2$ | |
| H | $OCH_3$ | H | $CH_3$ | $CH(OCH_3)_2$ | |
| 5-$OCH_3$ | $OCH_3$ | H | $CH_3$ | $CH(OCH_3)_2$ | |
| 5-F | $O(CH_2)_3CH_3$ | H | $OCH_3$ | $CH(OCH_3)_2$ | |
| H | $SO_2CH_2CH_2CH_3$ | H | $OCH_3$ | $CH(OCH_3)_2$ | |
| H | $SO_2CH_3$ | H | $CH_3$ | $CH(OCH_3)_2$ | |
| 4-Br | $SO_2CH_3$ | H | $CH_3$ | $CH(OCH_3)_2$ | |

TABLE 8f

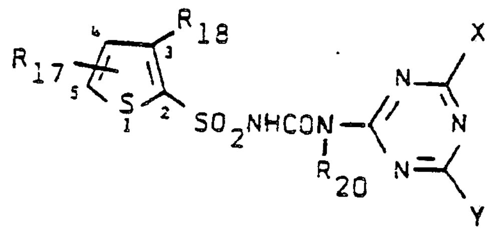

| $R_{17}$ | $R_{18}$ | $R_{20}$ | X | Y | m.p. (°C) |
|---|---|---|---|---|---|
| H | $CO_2CH_3$ | H | $CH_3$ | $CH_2OTHP$ | |
| 4-$CH_3$ | $CO_2CH_3$ | H | $CH_3$ | $CH_2OTHP$ | |
| H | $CO_2CH_2CH_3$ | H | $OCH_3$ | $CH_2OTHP$ | |
| H | $CO_2CH_2CH{=}CH_2$ | H | $CH_3$ | $CH_2OTHP$ | |
| H | H | H | $CH_3$ | $CH_2OTHP$ | |
| H | $SCH_2CH_2CH_3$ | H | $OCH_3$ | $CH_2OTHP$ | |
| H | $SCH_3$ | H | $CH_3$ | $CH_2OTHP$ | |
| H | $CH(CH_3)_2$ | H | $OCH_3$ | $CH_2OTHP$ | |
| H | $CH_2CH_3$ | H | $CH_3$ | $CH_2OTHP$ | |
| H | F | H | $OCH_3$ | $CH_2OTHP$ | |
| H | Cl | $CH_3$ | $OCH_3$ | $CH_2OTHP$ | |
| 5-Cl | Cl | H | $CH_3$ | $CH_2OTHP$ | |
| H | Br | H | $OCH_2CH_3$ | $CH_2OTHP$ | |
| H | $NO_2$ | H | $CH_2CH_3$ | $CH_2OTHP$ | |
| H | $SO_2N(CH_3)CH_2CH_3$ | H | $CH_3$ | $CH_2OTHP$ | |
| H | $SO_2N(CH_3)_2$ | H | $OCH_3$ | $CH_2OTHP$ | |
| H | $SO_2N(OCH_3)CH_3$ | H | $OCH_3$ | $CH_2OTHP$ | |
| H | $OCH_3$ | H | $CH_3$ | $CH_2OTHP$ | |
| 5-$OCH_3$ | $OCH_3$ | H | $CH_3$ | $CH_2OTHP$ | |
| 5-F | $O(CH_2)_3CH_3$ | H | $OCH_3$ | $CH_2OTHP$ | |
| H | $SO_2CH_2CH_2CH_3$ | H | $OCH_3$ | $CH_2OTHP$ | |
| H | $SO_2CH_3$ | H | $CH_3$ | $CH_2OTHP$ | |
| 4-Br | $SO_2CH_3$ | H | $CH_3$ | $CH_2OTHP$ | |

TABLE 8g

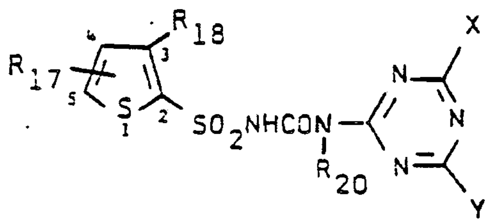

| $R_{17}$ | $R_{18}$ | $R_{20}$ | X | Y | m.p. (°C) |
|---|---|---|---|---|---|
| H | $CO_2CH_3$ | H | $CH_3$ | $CH_2OH$ | |
| 4-$CH_3$ | $CO_2CH_3$ | H | $CH_2CH_3$ | $CH_2OH$ | |
| H | $CO_2CH_2CH_3$ | H | $OCH_2CH_3$ | $CH_2OH$ | |
| H | $CO_2CH_2CH{=}CH_2$ | H | $CH_3$ | $CH_2OH$ | |
| H | H | H | $CH_3$ | $CH_2OH$ | |
| H | $SCH_2CH_2CH_3$ | H | $OCH_3$ | $CH_2OH$ | |
| H | $SCH_3$ | H | $CH_3$ | $CH_2OH$ | |
| H | $CH(CH_3)_2$ | H | $OCH_3$ | $CH_2OH$ | |
| H | $CH_2CH_3$ | H | $CH_3$ | $CH_2OH$ | |
| H | F | H | $OCH_3$ | $CH_2OH$ | |
| H | Cl | H | $OCH_3$ | $CH_2OH$ | |
| 5-Cl | Cl | H | $CH_3$ | $CH_2OH$ | |
| H | Br | H | $OCH_3$ | $CH_2OH$ | |
| H | $NO_2$ | H | $CH_3$ | $CH_2OH$ | |
| H | $SO_2N(CH_3)CH_2CH_3$ | H | $CH_3$ | $CH_2OH$ | |
| H | $SO_2N(CH_3)_2$ | H | $OCH_3$ | $CH_2OH$ | |
| H | $SO_2N(OCH_3)CH_3$ | H | $OCH_3$ | $CH_2OH$ | |
| H | $OCH_3$ | H | $CH_3$ | $CH_2OH$ | |
| 5-$OCH_3$ | $OCH_3$ | H | $CH_3$ | $CH_2OH$ | |
| 5-F | $O(CH_2)_3CH_3$ | H | $OCH_3$ | $CH_2OH$ | |
| H | $SO_2CH_2CH_2CH_3$ | $CH_3$ | $OCH_3$ | $CH_2OH$ | |
| H | $SO_2CH_3$ | $CH_3$ | $CH_3$ | $CH_2OH$ | |
| 4-Br | $SO_2CH_3$ | H | $CH_3$ | $CH_2OH$ | |

TABLE 8h

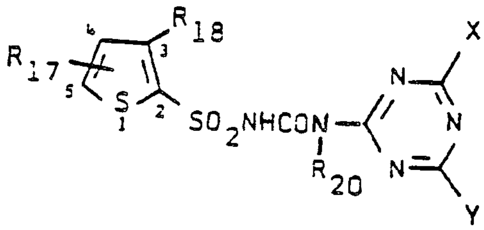

| $R_{17}$ | $R_{18}$ | $R_{20}$ | X | Y | m.p. (°C) |
|---|---|---|---|---|---|
| H | $CO_2CH_3$ | H | $CH_3$ | $CH(OC_2H_5)_2$ | |
| 4-$CH_3$ | $CO_2CH_3$ | H | $CH_3$ | $CH_2OCOCH_3$ | |
| H | $CO_2CH_3$ | $CH_3$ | $OCH_3$ | $CH_2SCH_3$ | |
| H | $CO_2CH_2CH{=}CH_2$ | $CH_3$ | $CH_3$ | $CH_2OCONHCH_3$ | |
| H | H | H | $CH_3$ | $CH_2OCO_2CH_3$ | |
| H | $SCH_2CH_2CH_3$ | H | $OCH_3$ | $CH_2SC_2H_5$ | |
| H | $SCH_3$ | H | $CH_3$ | $CH(OCH_2CH_2O)$ | |
| H | $CH(CH_3)_2$ | H | $OCH_3$ | $CH_2OCOC_2H_5$ | |
| H | $CH_2CH_3$ | H | $CH_3$ | $CH(OCH(CH_3)CH_2O)$ | |
| H | F | H | $OCH_3$ | $CH(OCH_2CH_2CH_2O)$ | |
| H | Cl | H | $OCH_2CH_3$ | $CH_2SCH_3$ | |
| 5-Cl | Cl | H | $CH_2CH_3$ | $CH_2OCOCH_3$ | |
| H | Br | H | $OCH_3$ | $CH_2OCON(CH_3)_2$ | |
| H | $NO_2$ | H | $CH_3$ | $CH(OCH_2CH_2O)$ | |
| H | $SO_2N(CH_3)C_2H_5$ | H | $CH_3$ | $CH_2OCOCH_3$ | |
| H | $SO_2N(CH_3)_2$ | H | $OCH_3$ | $CH_2SCH_2CH_3$ | |
| H | $SO_2N(OCH_3)CH_3$ | H | $CH_3$ | $CH(OC_2H_5)_2$ | |
| H | $OCH_3$ | H | $CH_3$ | $CH_2OCO_2C_2H_5$ | |
| 5-$OCH_3$ | $OCH_3$ | H | $CH_3$ | $CH_2OCON(C_2H_5)_2$ | |
| 5-F | $O(CH_2)_3CH_3$ | H | $OCH_3$ | $CH_2OCON(C_2H_5)_2$ | |
| H | $SO_2CH_2CH_2CH_3$ | H | $CH_3$ | $CH_2OCO(i\text{-}C_3H_7)$ | |
| H | $SO_2CH_3$ | H | $CH_3$ | $CH_2OCONH(n\text{-}C_3H_7)$ | |

TABLE 9a

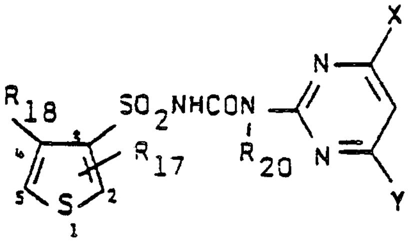

| $R_{17}$ | $R_{18}$ | $R_{20}$ | X | Y | m.p. (°C) |
|---|---|---|---|---|---|
| H | $CO_2CH_3$ | H | $CH_3$ | $CH(OCH_3)_2$ | |
| H | $CO_2CH_3$ | H | $OCH_3$ | $CH(OCH_3)_2$ | |
| H | $CO_2CH_2CH{=}CH_2$ | H | $CH_3$ | $CH(OCH_3)_2$ | |
| H | H | H | $CH_3$ | $CH(OCH_3)_2$ | |
| H | $SCH_2CH_2CH_3$ | H | $OCH_3$ | $CH(OCH_3)_2$ | |
| H | $SCH_3$ | H | $CH_3$ | $CH(OCH_3)_2$ | |
| H | $CH(CH_3)_2$ | H | $OCH_3$ | $CH(OCH_3)_2$ | |
| H | $CH_2CH_3$ | H | $CH_3$ | $CH(OCH_3)_2$ | |
| H | F | $CH_3$ | $OCH_3$ | $CH(OCH_3)_2$ | |
| H | Cl | H | $OCH_3$ | $CH(OCH_3)_2$ | |
| 5-Cl | Cl | $CH_3$ | $CH_3$ | $CH(OCH_3)_2$ | |
| H | Br | H | $OCH_2CH_3$ | $CH(OCH_3)_2$ | |
| H | $NO_2$ | H | $CH_2CH_3$ | $CH(OCH_3)_2$ | |
| H | $SO_2N(CH_3)CH_2CH_3$ | H | $CH_3$ | $CH(OCH_3)_2$ | |
| H | $SO_2N(CH_3)_2$ | H | $OCH_3$ | $CH(OCH_3)_2$ | |
| H | $SO_2N(OCH_3)CH_3$ | H | $OCH_3$ | $CH(OCH_3)_2$ | |
| H | $OCH_3$ | H | $CH_3$ | $CH(OCH_3)_2$ | |
| 5-$OCH_3$ | $OCH_3$ | H | $CH_3$ | $CH(OCH_3)_2$ | |
| 5-F | $O(CH_2)_3CH_3$ | H | $OCH_3$ | $CH(OCH_3)_2$ | |
| H | $SO_2CH_2CH_2CH_3$ | H | $OCH_3$ | $CH(OCH_3)_2$ | |
| H | $SO_2CH_3$ | H | $CH_3$ | $CH(OCH_3)_2$ | |
| H | $CO_2CH_3$ | H | Cl | $CH(OCH_3)_2$ | |
| H | $NO_2$ | H | Cl | $CH(OCH_3)_2$ | |
| H | $SO_2CH_2CH_2CH_3$ | H | Cl | $CH(OCH_3)_2$ | |
| H | $SO_2CH_3$ | H | Cl | $CH(OCH_3)_2$ | |
| H | $SCH_3$ | H | Cl | $CH(OCH_3)_2$ | |

TABLE 9b

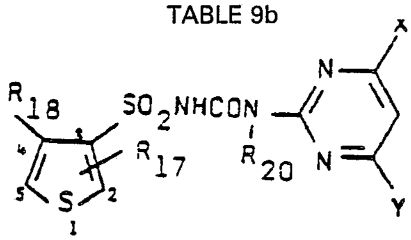

| $R_{17}$ | $R_{18}$ | $R_{20}$ | X | Y | m.p. (°C) |
|---|---|---|---|---|---|
| H | $CO_2CH_3$ | H | $CH_3$ | $CH_2OTHP$ | |
| H | $CO_2CH_2CH_3$ | H | $OCH_3$ | $CH_2OTHP$ | |
| H | $CO_2CH_2CH{=}CH_2$ | H | $CH_3$ | $CH_2OTHP$ | |
| H | H | H | $CH_3$ | $CH_2OTHP$ | |
| H | $SCH_2CH_2CH_3$ | H | $OCH_3$ | $CH_2OTHP$ | |
| H | $SCH_3$ | H | $CH_3$ | $CH_2OTHP$ | |
| H | $CH(CH_3)_2$ | H | $OCH_3$ | $CH_2OTHP$ | |
| H | $CH_2CH_3$ | H | $CH_3$ | $CH_2OTHP$ | |
| H | F | H | $OCH_3$ | $CH_2OTHP$ | |
| H | Cl | H | $OCH_2CH_3$ | $CH_2OTHP$ | |
| 5-Cl | Cl | H | $CH_2CH_3$ | $CH_2OTHP$ | |
| H | Br | H | $OCH_3$ | $CH_2OTHP$ | |
| H | $NO_2$ | H | $CH_3$ | $CH_2OTHP$ | |
| H | $SO_2N(CH_3)CH_2CH_3$ | H | $CH_3$ | $CH_2OTHP$ | |
| H | $SO_2N(CH_3)_2$ | H | $OCH_3$ | $CH_2OTHP$ | |
| H | $SO_2N(OCH_3)CH_3$ | H | $OCH_3$ | $CH_2OTHP$ | |
| H | $OCH_3$ | $CH_3$ | $CH_3$ | $CH_2OTHP$ | |
| 5-$OCH_3$ | $OCH_3$ | H | $CH_3$ | $CH_2OTHP$ | |
| 5-F | $O(CH_2)_3CH_3$ | H | $OCH_3$ | $CH_2OTHP$ | |
| H | $SO_2CH_2CH_2CH_3$ | H | $OCH_3$ | $CH_2OTHP$ | |
| H | $SO_2CH_3$ | H | $CH_3$ | $CH_2OTHP$ | |

TABLE 9c

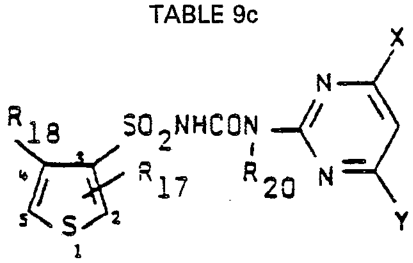

| $R_{17}$ | $R_{18}$ | $R_{20}$ | X | Y | m.p. (°C) |
|---|---|---|---|---|---|
| H | $CO_2CH_3$ | H | $CH_3$ | $CH_2OH$ | |
| H | $CO_2CH_2CH_3$ | H | $OCH_3$ | $CH_2OH$ | |
| H | $CO_2CH_2CH{=}CH_2$ | H | $CH_3$ | $CH_2OH$ | |
| H | H | H | $CH_3$ | $CH_2OH$ | |
| H | $SCH_2CH_2CH_3$ | H | $OCH_3$ | $CH_2OH$ | |
| H | $SCH_3$ | $CH_3$ | $CH_3$ | $CH_2OH$ | |
| H | $CH(CH_3)_2$ | H | $OCH_3$ | $CH_2OH$ | |
| H | $CH_2CH_3$ | H | $CH_3$ | $CH_2OH$ | |
| H | F | H | $OCH_3$ | $CH_2OH$ | |
| H | Cl | H | $OCH_2CH_3$ | $CH_2OH$ | |
| 5-Cl | Cl | H | $CH_2CH_3$ | $CH_2OH$ | |
| H | Br | H | $OCH_3$ | $CH_2OH$ | |
| H | $NO_2$ | H | $CH_3$ | $CH_2OH$ | |
| H | $SO_2N(CH_3)CH_2CH_3$ | H | $CH_3$ | $CH_2OH$ | |
| H | $SO_2N(CH_3)_2$ | H | $OCH_3$ | $CH_2OH$ | |
| H | $SO_2N(OCH_3)CH_3$ | H | $OCH_3$ | $CH_2OH$ | |
| H | $OCH_3$ | H | $CH_3$ | $CH_2OH$ | |
| 5-$OCH_3$ | $OCH_3$ | H | $CH_3$ | $CH_2OH$ | |
| 5-F | $O(CH_2)_3CH_3$ | H | $OCH_3$ | $CH_2OH$ | |
| H | $SO_2CH_2CH_2CH_3$ | H | $OCH_3$ | $CH_2OH$ | |
| H | $SO_2CH_3$ | H | $CH_3$ | $CH_2OH$ | |

TABLE 9d

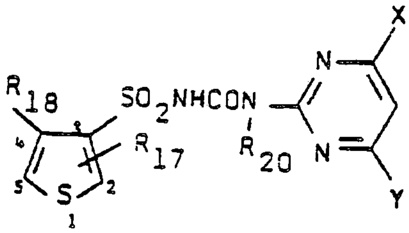

| $R_{17}$ | $R_{18}$ | $R_{20}$ | X | Y | m.p. (°C) |
|---|---|---|---|---|---|
| H | $CO_2CH_3$ | H | $CH_3$ | $CH(OC_2H_5)_2$ | |
| H | $CO_2CH_3$ | H | $OCH_3$ | $CH_2SCH_3$ | |
| H | $CO_2CH_2CH{=}CH_2$ | H | $CH_3$ | $CH_2OCONHCH_3$ | |
| H | H | H | $CH_3$ | $CH_2OCO_2CH_3$ | |
| H | $SCH_2CH_2CH_3$ | H | $OCH_3$ | $CH_2SC_2H_5$ | |
| H | $SCH_3$ | H | $CH_3$ | $CH(OCH_2CH_2O)$ | |
| H | $CH(CH_3)_2$ | H | $OCH_3$ | $CH_2OCOC_2H_5$ | |
| H | $CH_2CH_3$ | H | $CH_3$ | $CH(OCH(CH_3)CH_2O)$ | |
| H | F | H | $OCH_3$ | $CH(OCH_2CH_2CH_2O)$ | |
| H | Cl | H | $OCH_2CH_3$ | $CH_2SCH_3$ | |
| 5-Cl | Cl | H | $CH_2CH_3$ | $CH_2OCOCH_3$ | |
| H | Br | $CH_3$ | $OCH_3$ | $CH_2OCON(CH_3)_2$ | |
| H | $NO_2$ | H | $CH_3$ | $CH(OCH_2CH_2O)$ | |
| H | $SO_2N(CH_3)C_2H_5$ | H | $CH_3$ | $CH_2OCOCH_3$ | |
| H | $SO_2N(CH_3)_2$ | H | $OCH_3$ | $CH_2SCH_2CH_3$ | |
| H | $SO_2N(OCH_3)CH_3$ | H | $CH_3$ | $CH(OC_2H_5)_2$ | |
| H | $OCH_3$ | H | $CH_3$ | $CH_2OCO_2C_2H_5$ | |
| 5-$OCH_3$ | $OCH_3$ | H | $CH_3$ | $CH_2OCON(C_2H_5)_2$ | |
| 5-F | $O(CH_2)_3CH_3$ | H | $OCH_3$ | $CH_2OCON(C_2H_5)_2$ | |
| H | $SO_2CH_2CH_2CH_3$ | H | $CH_3$ | $CH_2OCO(i\text{-}C_3H_7)$ | |
| H | $SO_2CH_3$ | H | $CH_3$ | $CH_2OCONH(n\text{-}C_3H_7)$ | |

TABLE 9e

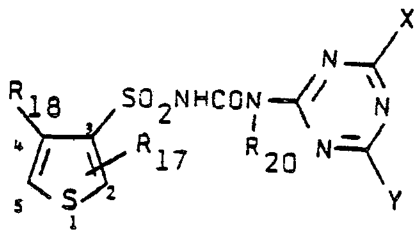

| $R_{17}$ | $R_{18}$ | $R_{20}$ | X | Y | m.p. (°C) |
|---|---|---|---|---|---|
| H | $CO_2CH_3$ | H | $CH_3$ | $CH(OCH_3)_2$ | |
| H | $CO_2CH_3$ | H | $OCH_3$ | $CH(OCH_3)_2$ | |
| H | $CO_2CH_2CH{=}CH_2$ | H | $CH_3$ | $CH(OCH_3)_2$ | |
| H | H | H | $CH_3$ | $CH(OCH_3)_2$ | |
| H | $SCH_2CH_2CH_3$ | $CH_3$ | $OCH_3$ | $CH(OCH_3)_2$ | |
| H | $SCH_3$ | H | $CH_3$ | $CH(OCH_3)_2$ | |
| H | $CH(CH_3)_2$ | H | $OCH_2CH_3$ | $CH(OCH_3)_2$ | |
| H | $CH_2CH_3$ | H | $CH_2CH_3$ | $CH(OCH_3)_2$ | |
| H | F | H | $OCH_3$ | $CH(OCH_3)_2$ | |
| H | Cl | H | $OCH_3$ | $CH(OCH_3)_2$ | |
| 5-Cl | Cl | H | $CH_3$ | $CH(OCH_3)_2$ | |
| H | Br | H | $OCH_3$ | $CH(OCH_3)_2$ | |
| H | $NO_2$ | H | $CH_3$ | $CH(OCH_3)_2$ | |
| H | $SO_2N(CH_3)CH_2CH_3$ | H | $CH_3$ | $CH(OCH_3)_2$ | |
| H | $SO_2N(CH_3)_2$ | H | $OCH_3$ | $CH(OCH_3)_2$ | |
| H | $SO_2N(OCH_3)CH_3$ | H | $OCH_3$ | $CH(OCH_3)_2$ | |
| H | $OCH_3$ | H | $CH_3$ | $CH(OCH_3)_2$ | |
| 5-$OCH_3$ | $OCH_3$ | H | $CH_3$ | $CH(OCH_3)_2$ | |
| 5-F | $O(CH_2)_3CH_3$ | H | $OCH_3$ | $CH(OCH_3)_2$ | |
| H | $SO_2CH_2CH_2CH_3$ | H | $OCH_3$ | $CH(OCH_3)_2$ | |
| H | $SO_2CH_3$ | H | $CH_3$ | $CH(OCH_3)_2$ | |

TABLE 9f

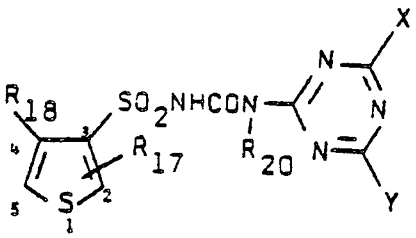

| $R_{17}$ | $R_{18}$ | $R_{20}$ | X | Y | m.p. (°C) |
|---|---|---|---|---|---|
| H | $CO_2CH_3$ | H | $CH_3$ | $CH_2OTHP$ | |
| H | $CO_2CH_2CH_3$ | H | $OCH_3$ | $CH_2OTHP$ | |
| H | $CO_2CH_2CH{=}CH_2$ | H | $CH_3$ | $CH_2OTHP$ | |
| H | H | H | $CH_3$ | $CH_2OTHP$ | |
| H | $SCH_2CH_2CH_3$ | H | $OCH_2CH_3$ | $CH_2OTHP$ | |
| H | $SCH_3$ | H | $CH_2CH_3$ | $CH_2OTHP$ | |
| H | $CH(CH_3)_2$ | H | $OCH_3$ | $CH_2OTHP$ | |
| H | $CH_2CH_3$ | H | $CH_3$ | $CH_2OTHP$ | |
| H | F | H | $OCH_3$ | $CH_2OTHP$ | |
| H | Cl | H | $OCH_3$ | $CH_2OTHP$ | |
| 5-Cl | Cl | H | $CH_3$ | $CH_2OTHP$ | |
| H | Br | H | $OCH_3$ | $CH_2OTHP$ | |
| H | $NO_2$ | $CH_3$ | $CH_3$ | $CH_2OTHP$ | |
| H | $SO_2N(CH_3)CH_2CH_3$ | H | $CH_3$ | $CH_2OTHP$ | |
| H | $SO_2N(CH_3)_2$ | H | $OCH_3$ | $CH_2OTHP$ | |
| H | $SO_2N(OCH_3)CH_3$ | H | $OCH_3$ | $CH_2OTHP$ | |
| H | $OCH_3$ | H | $CH_3$ | $CH_2OTHP$ | |
| 5-$OCH_3$ | $OCH_3$ | H | $CH_3$ | $CH_2OTHP$ | |
| 5-F | $O(CH_2)_3CH_3$ | H | $OCH_3$ | $CH_2OTHP$ | |
| H | $SO_2CH_2CH_2CH_3$ | H | $OCH_3$ | $CH_2OTHP$ | |
| H | $SO_2CH_3$ | H | $CH_3$ | $CH_2OTHP$ | |

TABLE 9g

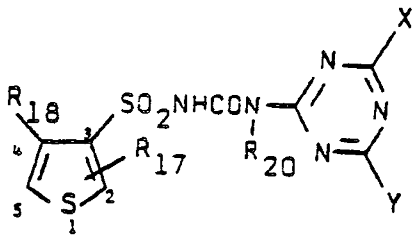

| $R_{17}$ | $R_{18}$ | $R_{20}$ | X | Y | m.p. (°C) |
|---|---|---|---|---|---|
| H | $CO_2CH_3$ | H | $CH_3$ | $CH_2OH$ | |
| H | $CO_2CH_2CH_3$ | H | $OCH_3$ | $CH_2OH$ | |
| H | $CO_2CH_2CH{=}CH_2$ | H | $CH_3$ | $CH_2OH$ | |
| H | H | H | $CH_3$ | $CH_2OH$ | |
| H | $SCH_2CH_2CH_3$ | H | $OCH_3$ | $CH_2OH$ | |
| H | $SCH_3$ | H | $CH_3$ | $CH_2OH$ | |
| H | $CH(CH_3)_2$ | H | $OCH_3$ | $CH_2OH$ | |
| H | $CH_2CH_3$ | H | $CH_3$ | $CH_2OH$ | |
| H | F | H | $OCH_3$ | $CH_2OH$ | |
| H | Cl | H | $OCH_2CH_3$ | $CH_2OH$ | |
| 5-Cl | Cl | H | $CH_2CH_3$ | $CH_2OH$ | |
| H | Br | H | $OCH_3$ | $CH_2OH$ | |
| H | $NO_2$ | H | $CH_3$ | $CH_2OH$ | |
| H | $SO_2N(CH_3)CH_2CH_3$ | $CH_3$ | $CH_3$ | $CH_2OH$ | |
| H | $SO_2N(CH_3)_2$ | H | $OCH_3$ | $CH_2OH$ | |
| H | $SO_2N(OCH_3)CH_3$ | H | $OCH_3$ | $CH_2OH$ | |
| H | $OCH_3$ | H | $CH_3$ | $CH_2OH$ | |
| 5-$OCH_3$ | $OCH_3$ | H | $CH_3$ | $CH_2OH$ | |
| 5-F | $O(CH_2)_3CH_3$ | H | $OCH_3$ | $CH_2OH$ | |
| H | $SO_2CH_2CH_2CH_3$ | H | $OCH_3$ | $CH_2OH$ | |
| H | $SO_2CH_3$ | H | $CH_3$ | $CH_2OH$ | |

TABLE 9h

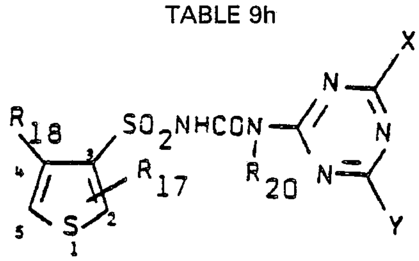

| $R_{17}$ | $R_{18}$ | $R_{20}$ | X | Y | m.p. (°C) |
|---|---|---|---|---|---|
| H | $CO_2CH_3$ | H | $CH_3$ | $CH(OC_2H_5)_2$ | |
| H | $CO_2CH_3$ | $CH_3$ | $OCH_3$ | $CH_2SCH_3$ | |
| H | $CO_2CH_2CH{=}CH_2$ | H | $CH_3$ | $CH_2OCONHCH_3$ | |
| H | H | H | $CH_3$ | $CH_2OCO_2CH_3$ | |
| H | $SCH_2CH_2CH_3$ | H | $OCH_2CH_3$ | $CH_2SC_2H_5$ | |
| H | $SCH_3$ | H | $CH_2CH_3$ | $CH(OCH_2CH_2O)$ | |
| H | $CH(CH_3)_2$ | H | $OCH_3$ | $CH_2OCOC_2H_5$ | |
| H | $CH_2CH_3$ | H | $CH_3$ | $CH(OCH(CH_3)CH_2O)$ | |
| H | F | H | $OCH_3$ | $CH(SCH_2CH_2CH_2S)$ | |
| H | Cl | H | $OCH_3$ | $CH_2SCH_3$ | |
| 5-Cl | Cl | H | $CH_3$ | $CH_2OCOCH_3$ | |
| H | Br | H | $OCH_3$ | $CH_2OCON(CH_3)_2$ | |
| H | $NO_2$ | H | $CH_3$ | $CH(OCH_3CH_2O)$ | |
| H | $SO_2N(CH_3)C_2H_5$ | H | $CH_3$ | $CH_2OCOCH_3$ | |
| H | $SO_2N(CH_3)_2$ | H | $OCH_3$ | $CH_2SCH_2CH_3$ | |
| H | $SO_2N(OCH_3)CH_3$ | H | $CH_3$ | $CH(OC_2H_5)_2$ | |
| H | $OCH_3$ | H | $CH_3$ | $CH_2OCO_2C_2H_5$ | |
| 5-$OCH_3$ | $OCH_3$ | H | $CH_3$ | $CH_2OCON(C_2H_5)_2$ | |
| 5-F | $O(CH_2)_3CH_3$ | H | $OCH_3$ | $CH_2OCON(C_2H_5)_2$ | |
| H | $SO_2CH_2CH_2CH_3$ | H | $CH_3$ | $CH_2OCO(i\text{-}C_3H_7$ | |
| H | $SO_2CH_3$ | H | $CH_3$ | $CH_2OCONH(n\text{-}C_3H_7)$ | |

TABLE 10a

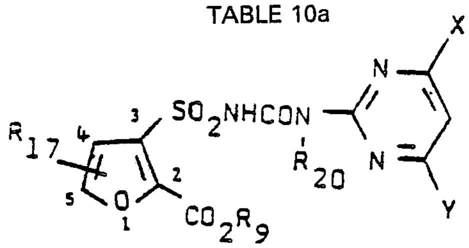

| $R_{17}$ | $R_9$ | $R_{20}$ | X | Y | m.p. (°C) |
|---|---|---|---|---|---|
| H | $CH_2CH{=}CH_2$ | H | $CH_3$ | $CH(OCH_3)_2$ | |
| H | $CH_3$ | H | $CH_3$ | $CH(OC_2H_5)_2$ | |
| H | $CH_3$ | H | $OCH_3$ | $CH_2SCH_3$ | |
| H | $CH_3$ | H | $CH_2CH_3$ | $CH_2OH$ | |
| H | $CH_3$ | H | $OCH_2CH_3$ | $CH_2OCOCH_3$ | |
| H | $CH_3$ | H | $CH_3$ | $CH_2OCONHC_2H_5$ | |
| 5-F | $C_2H_5$ | H | $OCH_3$ | $CH(OCH_3)_2$ | |
| 4-Br | $CH_3$ | H | $OCH_3$ | $CH_2OTHP$ | |
| 4-Br | $CH_3$ | H | $OCH_3$ | $CH_2OH$ | |
| 5-$OCH_3$ | $CH_3$ | H | $CH_3$ | $CH(OCH_3)_2$ | |
| 5-Cl | $CH_3$ | H | $OCH_3$ | $CH_2OH$ | |
| 5-Cl | $CH_3$ | H | $CH_3$ | $CH(OC_2H_5)_2$ | |
| 5-$CH_3$ | $CH_3$ | H | $CH_3$ | $CH(OCH_3)_2$ | |
| 5-$CH_3$ | $CH_3$ | H | $CH_3$ | $CH_2OTHP$ | |
| H | $i$-$C_3H_7$ | H | $OCH_3$ | $CH_2OH$ | |
| H | $CH_2CH_2OCH_3$ | $CH_3$ | $CH_3$ | $CH_2OH$ | |
| H | $(CH_2)_3CH_3$ | H | $CH_3$ | $CH(OCH_3)_2$ | |
| H | $CH_3$ | H | $OCH_3$ | $CH(OCH_3)_2$ | |
| H | $CH_3$ | H | $OCH_3$ | $CH_2OH$ | |
| H | $CH_2CH_2Cl$ | H | $CH_3$ | $CH_2OH$ | |
| H | $CH_3$ | H | $CH_3$ | $CH_2OCOC_2H_5$ | |
| H | $n$-$C_3H_7$ | H | $CH_3$ | $CH(OCH_3)_2$ | |
| H | $CH_3$ | H | $OCH_3$ | $CH(OCH_2CH_2O)$ | |
| H | $CH_3$ | H | $CH_3$ | $CH_2OCO_2CH_3$ | |
| H | $C_2H_5$ | H | $CH_3$ | $CH(OCH_3)_2$ | |
| H | $CH_3$ | H | $OCH_3$ | $CH(OC_2H_5)_2$ | |

TABLE 10b

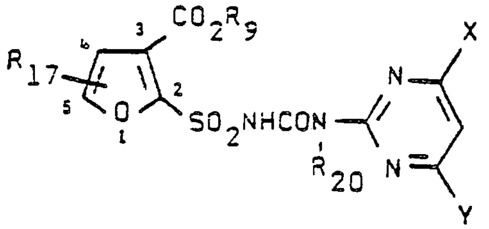

| $R_{17}$ | $R_9$ | $R_{20}$ | X | Y | m.p. (°C) |
|---|---|---|---|---|---|
| H | $CH_2CH=CH_2$ | H | $CH_3$ | $CH(OCH_3)_2$ | |
| H | $CH_3$ | $CH_3$ | $CH_3$ | $CH(OC_2H_5)_2$ | |
| H | $CH_3$ | H | $OCH_3$ | $CH_2SCH_3$ | |
| H | $CH_3$ | H | $CH_2CH_3$ | $CH_2OH$ | |
| H | $CH_3$ | H | $OCH_2CH_3$ | $CH_2OCOCH_3$ | |
| H | $CH_3$ | H | $CH_3$ | $CH_2OCONHC_2H_5$ | |
| 5-F | $C_2H_5$ | H | $OCH_3$ | $CH(OCH_3)_2$ | |
| 4-Br | $CH_3$ | H | $OCH_3$ | $CH_2OTHP$ | |
| 4-Br | $CH_3$ | H | $OCH_3$ | $CH_2OH$ | |
| 5-$OCH_3$ | $CH_3$ | H | $CH_3$ | $CH(OCH_3)_2$ | |
| 5-Cl | $CH_3$ | H | $OCH_3$ | $CH_2OH$ | |
| 5-Cl | $CH_3$ | H | $CH_3$ | $CH(OC_2H_5)_2$ | |
| 5-$CH_3$ | $CH_3$ | H | $CH_3$ | $CH(OCH_3)_2$ | |
| 5-$CH_3$ | $CH_3$ | H | $CH_3$ | $CH_2OTHP$ | |
| H | *i*-$C_3H_7$ | H | $OCH_3$ | $CH_2OH$ | |
| H | $CH_2CH_2OCH_3$ | H | $CH_3$ | $CH_2OH$ | |
| H | $(CH_2)_3CH_3$ | H | $CH_3$ | $CH(OCH_3)_2$ | |
| H | $CH_3$ | H | $OCH_3$ | $CH(OCH_3)_2$ | |
| H | $CH_3$ | H | $OCH_3$ | $CH_2OH$ | |
| H | $CH_2CH_2Cl$ | H | $CH_3$ | $CH_2OH$ | |
| H | $CH_3$ | H | $CH_3$ | $CH_2OCOC_2H_5$ | |
| H | *n*-$C_3H_7$ | H | $CH_3$ | $CH(OCH_3)_2$ | |
| H | $CH_3$ | H | $OCH_3$ | $CH(OCH_2CH_2O)$ | |
| H | $CH_3$ | H | $CH_3$ | $CH_2OCO_2CH_3$ | |
| H | $C_2H_5$ | H | $CH_3$ | $CH(OCH_3)_2$ | |
| H | $CH_3$ | H | $OCH_3$ | $CH(OC_2H_5)_2$ | |

TABLE 10c

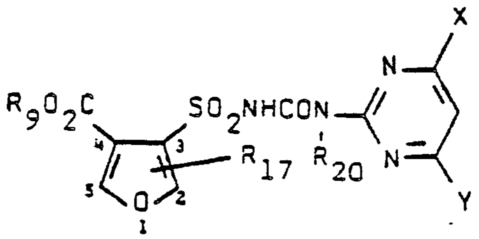

| $R_{17}$ | $R_9$ | $R_{20}$ | X | Y | m.p. (°C) |
|---|---|---|---|---|---|
| H | $CH_2CH{=}CH_2$ | H | $CH_3$ | $CH(OCH_3)_2$ | |
| H | $CH_3$ | H | $CH_3$ | $CH(OC_2H_5)_2$ | |
| H | $CH_3$ | H | $OCH_3$ | $CH_2SCH_3$ | |
| H | $CH_3$ | H | $CH_3$ | $CH_2OH$ | |
| H | $CH_3$ | H | $OCH_3$ | $CH_2OCOCH_3$ | |
| H | $CH_3$ | H | $CH_3$ | $CH_2OCONHC_2H_5$ | |
| 2-F | $C_2H_5$ | H | $OCH_3$ | $CH(OCH_3)_2$ | |
| 5-Br | $CH_3$ | H | $OCH_3$ | $CH_2OTHP$ | |
| 5-Br | $CH_3$ | H | $OCH_3$ | $CH_2OH$ | |
| 2-$OCH_3$ | $CH_3$ | H | $CH_3$ | $CH(OCH_3)_2$ | |
| 2-Cl | $CH_3$ | H | $OCH_2CH_3$ | $CH_2OH$ | |
| 2-Cl | $CH_3$ | H | $CH_2CH_3$ | $CH(OC_2H_5)_2$ | |
| 5-$CH_3$ | $CH_3$ | H | $CH_3$ | $CH(OCH_3)_2$ | |
| 5-$CH_3$ | $CH_3$ | H | $CH_3$ | $CH_2OTHP$ | |
| H | *i*-$C_3H_7$ | H | $OCH_3$ | $CH_2OH$ | |
| H | $CH_2CH_2OCH_3$ | $CH_3$ | $CH_3$ | $CH_2OH$ | |
| H | $(CH_2)_3CH_3$ | H | $CH_3$ | $CH(OCH_3)_2$ | |
| H | $CH_3$ | H | $OCH_3$ | $CH(OCH_3)_2$ | |
| H | $CH_3$ | H | $OCH_3$ | $CH_2OH$ | |
| H | $CH_2CH_2Cl$ | H | $CH_3$ | $CH_2OH$ | |
| H | $CH_3$ | H | $CH_3$ | $CH_2OCOC_2H_5$ | |
| H | *n*-$C_3H_7$ | H | $CH_3$ | $CH(OCH_3)_2$ | |
| H | $CH_3$ | H | $OCH_3$ | $CH(OCH_2CH_2O)$ | |
| H | $CH_3$ | H | $CH_3$ | $CH_2OCO_2CH_3$ | |
| H | $C_2H_5$ | H | $CH_3$ | $CH(OCH_3)_2$ | |
| H | $CH_3$ | H | $OCH_3$ | $CH(OC_2H_5)_2$ | |

TABLE 10d

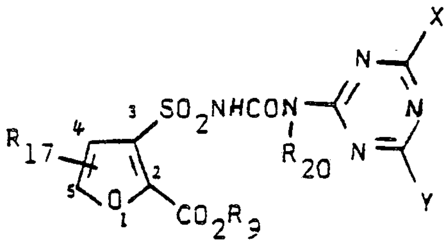

| $R_{17}$ | $R_9$ | $R_{20}$ | X | Y | m.p. (°C) |
|---|---|---|---|---|---|
| H | $CH_2CH{=}CH_2$ | H | $CH_3$ | $CH(OCH_3)_2$ | |
| H | $CH_3$ | H | $CH_3$ | $CH(OC_2H_5)_2$ | |
| H | $CH_3$ | H | $OCH_3$ | $CH_2SCH_3$ | |
| H | $CH_3$ | H | $CH_3$ | $CH_2OH$ | |
| H | $CH_3$ | H | $OCH_3$ | $CH_2OCOCH_3$ | |
| H | $CH_3$ | H | $CH_3$ | $CH_2OCONHC_2H_5$ | |
| 5-F | $C_2H_5$ | H | $OCH_3$ | $CH(OCH_3)_2$ | |
| 4-Br | $CH_3$ | H | $OCH_3$ | $CH_2OTHP$ | |
| 4-Br | $CH_3$ | H | $OCH_3$ | $CH_2OH$ | |
| 5-$OCH_3$ | $CH_3$ | H | $CH_3$ | $CH(OCH_3)_2$ | |
| 5-Cl | $CH_3$ | H | $OCH_3$ | $CH_2OH$ | |
| 5-Cl | $CH_3$ | H | $CH_3$ | $CH(OC_2H_5)_2$ | |
| 5-$CH_3$ | $CH_3$ | H | $CH_3$ | $CH(OCH_3)_2$ | |
| 5-$CH_3$ | $CH_3$ | H | $CH_3$ | $CH_2OTHP$ | |
| H | *i*-$C_3H_7$ | H | $OCH_3$ | $CH_2OH$ | |
| H | $CH_2CH_2OCH_3$ | H | $CH_2CH_3$ | $CH_2OH$ | |
| H | $(CH_2)_3CH_3$ | H | $CH_3$ | $CH(OCH_3)_2$ | |
| H | $CH_3$ | $CH_3$ | $OCH_3$ | $CH(OCH_3)_2$ | |
| H | $CH_3$ | H | $OCH_3$ | $CH_2OH$ | |
| H | $CH_2CH_2Cl$ | H | $CH_3$ | $CH_2OH$ | |
| H | $CH_3$ | H | $CH_3$ | $CH_2OCOC_2H_5$ | |
| H | *n*-$C_3H_7$ | H | $CH_3$ | $CH(OCH_3)_2$ | |
| H | $CH_3$ | H | $OCH_3$ | $CH(OCH_2CH_2O)$ | |
| H | $CH_3$ | H | $CH_3$ | $CH_2OCO_2CH_3$ | |
| H | $C_2H_5$ | H | $CH_3$ | $CH(OCH_3)_2$ | |
| H | $CH_3$ | H | $OCH_2CH_3$ | $CH(OC_2H_5)_2$ | |

TABLE 10e

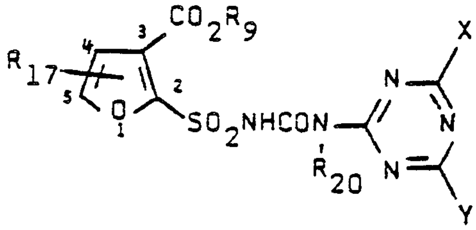

| $R_{17}$ | $R_9$ | $R_{20}$ | X | Y | m.p. (°C) |
|---|---|---|---|---|---|
| H | $CH_2CH{=}CH_2$ | H | $CH_3$ | $CH(OCH_3)_2$ | |
| H | $CH_3$ | $CH_3$ | $CH_3$ | $CH(OC_2H_5)_2$ | |
| H | $CH_3$ | $CH_3$ | $OCH_3$ | $CH_2SCH_3$ | |
| H | $CH_3$ | H | $CH_3$ | $CH_2OH$ | |
| H | $CH_3$ | H | $OCH_3$ | $CH_2OCOCH_3$ | |
| H | $CH_3$ | H | $CH_3$ | $CH_2OCONHC_2H_5$ | |
| 5-F | $C_2H_5$ | H | $OCH_3$ | $CH(OCH_3)_2$ | |
| 4-Br | $CH_3$ | H | $OCH_3$ | $CH_2OTHP$ | |
| 4-Br | $CH_3$ | H | $OCH_3$ | $CH_2OH$ | |
| 5-$OCH_3$ | $CH_3$ | H | $CH_3$ | $CH(OCH_3)_2$ | |
| 5-Cl | $CH_3$ | H | $OCH_3$ | $CH_2OH$ | |
| 5-Cl | $CH_3$ | H | $CH_3$ | $CH(OC_2H_5)_2$ | |
| 5-$CH_3$ | $CH_3$ | H | $CH_2CH_3$ | $CH(OCH_3)_2$ | |
| 5-$CH_3$ | $CH_3$ | H | $CH_3$ | $CH_2OTHP$ | |
| H | *i*-$C_3H_7$ | H | $OCH_2CH_3$ | $CH_2OH$ | |
| H | $CH_2CH_2OCH_3$ | H | $CH_3$ | $CH_2OH$ | |
| H | $(CH_2)_3CH_3$ | H | $CH_3$ | $CH(OCH_3)_2$ | |
| H | $CH_3$ | H | $OCH_3$ | $CH(OCH_3)_2$ | |
| H | $CH_3$ | H | $OCH_3$ | $CH_2OH$ | |
| H | $CH_2CH_2Cl$ | H | $CH_3$ | $CH_2OH$ | |
| H | $CH_3$ | H | $CH_3$ | $CH_2OCOC_2H_5$ | |
| H | *n*-$C_3H_7$ | H | $CH_3$ | $CH(OCH_3)_2$ | |
| H | $CH_3$ | H | $OCH_3$ | $CH(OCH_2CH_2O)$ | |
| H | $CH_3$ | H | $CH_3$ | $CH_2OCO_2CH_3$ | |
| H | $C_2H_5$ | H | $CH_3$ | $CH(OCH_3)_2$ | |
| H | $CH_3$ | H | $OCH_3$ | $CH(OC_2H_5)_2$ | |

TABLE 10f

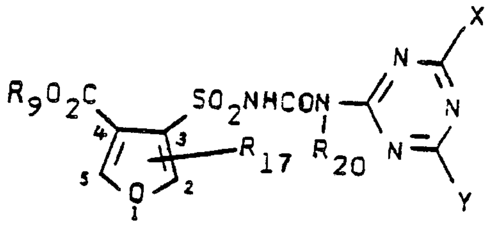

| $R_{17}$ | $R_9$ | $R_{20}$ | X | Y | m.p. (°C) |
|---|---|---|---|---|---|
| H | $CH_2CH{=}CH_2$ | H | $CH_3$ | $CH(OCH_3)_2$ | |
| H | $CH_3$ | H | $CH_2CH_3$ | $CH(OC_2H_5)_2$ | |
| H | $CH_3$ | H | $OCH_2CH_3$ | $CH_2SCH_3$ | |
| H | $CH_3$ | H | $CH_3$ | $CH_2OH$ | |
| H | $CH_3$ | H | $OCH_3$ | $CH_2OCOCH_3$ | |
| H | $CH_3$ | H | $CH_3$ | $CH_2OCONHC_2H_5$ | |
| 2-F | $C_2H_5$ | H | $OCH_3$ | $CH(OCH_3)_2$ | |
| 5-Br | $CH_3$ | H | $OCH_3$ | $CH_2OTHP$ | |
| 5-Br | $CH_3$ | H | $OCH_3$ | $CH_2OH$ | |
| 2-$OCH_3$ | $CH_3$ | H | $CH_3$ | $CH(OCH_3)_2$ | |
| 2-Cl | $CH_3$ | H | $OCH_3$ | $CH_2OH$ | |
| 2-Cl | $CH_3$ | $CH_3$ | $CH_3$ | $CH(OC_2H_5)_2$ | |
| 5-$CH_3$ | $CH_3$ | H | $CH_3$ | $CH(OCH_3)_2$ | |
| 5-$CH_3$ | $CH_3$ | H | $CH_3$ | $CH_2OTHP$ | |
| H | *i*-$C_3H_7$ | H | $OCH_3$ | $CH_2OH$ | |
| H | $CH_2CH_2OCH_3$ | H | $CH_3$ | $CH_2OH$ | |
| H | $(CH_2)_3CH_3$ | H | $CH_3$ | $CH(OCH_3)_2$ | |
| H | $CH_3$ | H | $OCH_3$ | $CH(OCH_3)_2$ | |
| H | $CH_3$ | H | $OCH_3$ | $CH_2OH$ | |
| H | $CH_2CH_2Cl$ | H | $CH_3$ | $CH_2OH$ | |
| H | $CH_3$ | H | $CH_3$ | $CH_2OCOC_2H_5$ | |
| H | *n*-$C_3H_7$ | H | $CH_3$ | $CH(OCH_3)_2$ | |
| H | $CH_3$ | H | $OCH_3$ | $CH(OCH_2CH_2O)$ | |
| H | $CH_3$ | H | $CH_3$ | $CH_2OCO_2CH_3$ | |
| H | $C_2H_5$ | H | $CH_3$ | $CH(OCH_3)_2$ | |
| H | $CH_3$ | H | $OCH_3$ | $CH(OC_2H_5)_2$ | |

TABLE 11a

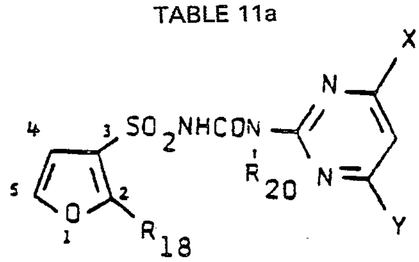

| $R_{18}$ | $R_{20}$ | X | Y | m.p. (°C) |
|---|---|---|---|---|
| H | H | $CH_3$ | $CH(OCH_3)_2$ | |
| H | H | $CH_3$ | $CH_2OTHP$ | |
| H | H | $OCH_2CH_3$ | $CH_2OH$ | |
| H | H | $OCH_3$ | $CH(OCH_2CH_2O)$ | |
| Cl | H | $CH_3$ | $CH(OCH_3)_2$ | |
| Cl | H | $CH_2CH_3$ | $CH(OC_2H_5)_2$ | |
| Cl | H | $CH_3$ | $CH_2OH$ | |
| Cl | H | $CH_3$ | $CH_2OTHP$ | |
| Cl | $CH_3$ | $OCH_3$ | $CH(OCH_3)_2$ | |
| Cl | H | $OCH_3$ | $CH_2OCOCH_3$ | |
| Cl | H | $OCH_3$ | $CH_2OH$ | |
| Br | H | $CH_3$ | $CH(OCH_3)_2$ | |
| Br | H | $CH_3$ | $CH(OC_2H_5)_2$ | |
| Br | H | $CH_3$ | $CH_2OH$ | |
| Br | H | $OCH_3$ | $CH_2OH$ | |
| Br | H | $OCH_3$ | $CH_2OCO_2CH_3$ | |
| $CH_3$ | H | $CH_3$ | $CH(OCH_3)_2$ | |
| $CH_3$ | H | $CH_3$ | $CH(OCH_2CH_2CH_2O)$ | |
| $CH_3$ | H | $CH_3$ | $CH_2OH$ | |
| $CH_3$ | H | $CH_3$ | $CH_2OCONHC_2H_5$ | |
| $CH_3$ | H | $OCH_3$ | $CH(OCH_3)_2$ | |
| $CH_2$ | H | $OCH_3$ | $CH_2OTHP$ | |
| $CH_3$ | H | $OCH_3$ | $CH_2OCOCH_3$ | |
| $CH_3$ | H | $OCH_3$ | $CH_2OH$ | |

TABLE 11b

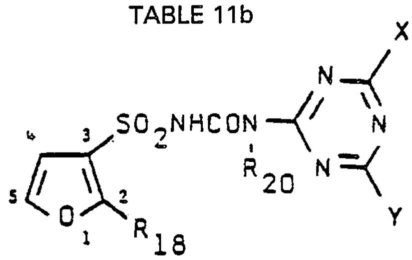

| $R_{18}$ | $R_{20}$ | X | Y | m.p. (°C) |
|---|---|---|---|---|
| H | H | $CH_3$ | $CH(OCH_3)_2$ | |
| H | H | $CH_3$ | $CH_2OTHP$ | |
| H | H | $OCH_3$ | $CH_2OH$ | |
| H | H | $OCH_3$ | $CH(OCH_2CH_2O)$ | |
| Cl | H | $CH_2CH_3$ | $CH(OCH_3)_2$ | |
| Cl | H | $CH_3$ | $CH(OC_2H_5)_2$ | |
| Cl | $CH_3$ | $CH_3$ | $CH_2OH$ | |
| Cl | H | $CH_3$ | $CH_2OTHP$ | |
| Cl | H | $OCH_3$ | $CH(OCH_3)_2$ | |
| Cl | H | $OCH_3$ | $CH_2OCOCH_3$ | |
| Cl | H | $OCH_3$ | $CH_2OH$ | |
| Br | H | $CH_3$ | $CH(OCH_3)_2$ | |
| Br | H | $CH_3$ | $CH(OC_2H_5)_2$ | |
| Br | H | $CH_3$ | $CH_2OH$ | |
| Br | H | $OCH_3$ | $CH_2OH$ | |
| Br | H | $OCH_2CH_3$ | $CH_2OCO_2CH_3$ | |
| $CH_3$ | H | $CH_3$ | $CH(OCH_3)_2$ | |
| $CH_3$ | H | $CH_3$ | $CH(SCH_2CH_2CH_2S)$ | |
| $CH_3$ | H | $CH_3$ | $CH_2OH$ | |
| $CH_3$ | H | $CH_3$ | $CH_2OCONHC_2H_5$ | |
| $CH_3$ | H | $OCH_3$ | $CH(OCH_3)_2$ | |
| $CH_3$ | H | $OCH_3$ | $CH_2OTHP$ | |
| $CH_3$ | H | $OCH_3$ | $CH_2OCOCH_3$ | |
| $CH_3$ | H | $OCH_3$ | $CH_2OH$ | |

TABLE 12

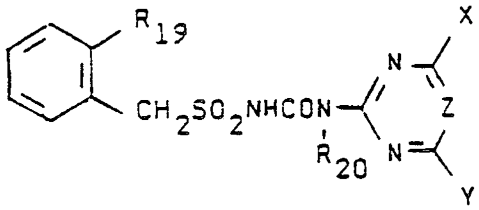

| $R_{19}$ | $R_{20}$ | X | Y | Z | m.p. (°C) |
|---|---|---|---|---|---|
| Cl | H | $CH_3$ | $CH(OCH_3)_2$ | CH | |
| Cl | $CH_3$ | $CH_3$ | $CH_2OCOCH_3$ | CH | |
| Cl | H | $CH_3$ | $CH(OCH_2CH_2O)$ | N | |
| Cl | H | $OCH_3$ | $CH_2SCH_3$ | N | |
| Cl | H | $OCH_3$ | $CH(OEt)_2$ | CH | |
| $NO_2$ | H | $C_2H_5$ | $CH(SCH_2CH_2S)$ | CH | |
| $NO_2$ | H | $CH_3$ | $CH(OCH_2CH_2CH_2O)$ | CH | |
| $NO_2$ | H | Cl | $CH(OCH_3)_2$ | CH | |
| $NO_2$ | H | $CH_3$ | $CH(OCH(CH_3)CH_2O)$ | N | |
| $NO_2$ | H | $OCH_3$ | $CH_2SEt$ | CH | |
| $NO_2$ | H | $OCH_3$ | $CH_2OH$ | CH | |
| $CF_3$ | H | $OC_2H_5$ | $CH_2OTHP$ | CH | |
| $CF_3$ | H | $CH_3$ | $CH_2OCON(CH_3)_2$ | CH | |
| $CF_3$ | H | $CH_3$ | $CH(OCH_3)_2$ | CH | |
| $CF_3$ | H | $CH_3$ | $CH(OEt)_2$ | N | |
| $CF_3$ | H | $OCH_3$ | $CH(SCH_2CH_2CH_2S)$ | CH | |
| $OCH_3$ | $CH_3$ | $OCH_3$ | $CH(OCH(CH_3)CH_2O)$ | CH | |
| $OCH_3$ | H | $OCH_3$ | $CH(OCH_2CH_2O)$ | CH | |
| $OCH_3$ | H | $OCH_3$ | $CH(OCH_3)_2$ | CH | |
| $OCH_3$ | H | $CH_3$ | $CH(OEt)_2$ | N | |
| $OCH_3$ | H | $CH_3$ | $CH_2SCH_3$ | CH | |
| $OCH_3$ | H | $CH_3$ | $CH_2SEt$ | CH | |
| $OCH_3$ | H | $CH_3$ | $CH_2OCONHCH_3$ | CH | |
| $CO_2CH_3$ | H | $CH_3$ | $CH_2SEt$ | CH | |
| $CO_2CH_3$ | $CH_3$ | $CH_3$ | $CH(OEt)_2$ | N | |
| $CO_2CH_3$ | $CH_3$ | $CH_3$ | $CH(SCH_2CH_2S)$ | CH | |
| $CO_2CH_3$ | $CH_3$ | $OCH_3$ | $CH(OCH_2CH_2O)$ | CH | |

TABLE 12 (Continued)

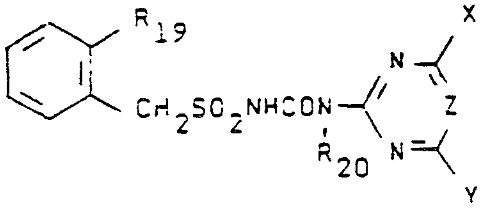

| $R_{19}$ | $R_{20}$ | X | Y | Z | m.p. (°C) |
|---|---|---|---|---|---|
| $CO_2CH_3$ | $CH_3$ | $OCH_3$ | $CH(OCH_3)_2$ | CH | |
| $CO_2CH_3$ | $CH_3$ | $OCH_3$ | $CH(OCH_2CH_2CH_2O)$ | N | |
| $CO_2CH_2CH_3$ | $CH_3$ | $C_2H_5$ | $CH(OCH_3)_2$ | CH | |
| $CO_2CH_2CH_3$ | $CH_3$ | $OC_2H_5$ | $CH(OCH_3)_2$ | CH | |
| $CO_2CH_2CH_3$ | $CH_3$ | $OCH_3$ | $CH(OCH_3)_2$ | CH | |
| $CO_2$-*n*-$C_4H_9$ | $CH_3$ | $OCH_3$ | $CH_2OH$ | CH | |
| $CO_2$-*i*-$C_3H_7$ | $CH_3$ | $OCH_3$ | $CH_2OH$ | N | |
| $SO_2N(OCH_3)CH_3$ | $CH_3$ | $OCH_3$ | $CH_2OH$ | CH | |
| $SO_2N(CH_3)_2$ | H | $CH_3$ | $CH(OCH_2CH_2O)$ | CH | |
| $SO_2N(CH_3)_2$ | $CH_3$ | $CH_3$ | $CH(OCH_2CH_2O)$ | CH | |
| $SO_2N(CH_3)_2$ | H | $CH_3$ | $CH(OCH_2CH_2O)$ | N | |
| $SO_2N(CH_3)_2$ | H | $CH_3$ | $CH_2OCOCH_3$ | CH | |
| $SO_2N(CH_3)_2$ | H | $OCH_3$ | $CH_2OCO_2CH_3$ | CH | |
| $SO_2N(CH_3)_2$ | H | $OCH_3$ | $CH_2SCH_3$ | CH | |
| $SO_2N(CH_3)_2$ | H | $OCH_3$ | $CH_2OTHP$ | CH | |
| $SO_2(CH_2CH_3)CH_3$ | H | $OCH_3$ | $CH_2OH$ | N | |
| $SO_2(CH_2CH_3)CH_3$ | H | $CH_3$ | $CH_2OH$ | CH | |
| $OSO_2CH_3$ | H | $CH_3$ | $CH_2OH$ | CH | |
| $OSO_2CH_3$ | H | $CH_3$ | $CH(OCH_2CH_3)_2$ | CH | |
| $OSO_2CH_3$ | H | $CH_3$ | $CH(OCH_3)_2$ | CH | |
| $OSO_2CH_3$ | H | $OCH_3$ | $CH(OCH_3)_2$ | CH | |
| $OSO_2CH_2CH_2OCH_3$ | H | $OCH_3$ | $CH(OCH_3)_2$ | N | |
| $OSO_2CH_2CF_3$ | H | $OCH_3$ | $CH(OCH_2CH_2O)$ | CH | |
| $SCH_3$ | H | $CH_3$ | $CH(OCH_3)_2$ | CH | |
| $SCH_3$ | $CH_3$ | $CH_3$ | $CH_2OCOC_2H_5$ | N | |
| $SCH_3$ | H | $OCH_3$ | $CH(OCH_2CH_2O)$ | N | |
| $SCH_2CH_3$ | H | $OCH_3$ | $CH_2SCH_3$ | CH | |

TABLE 12 (Continued)

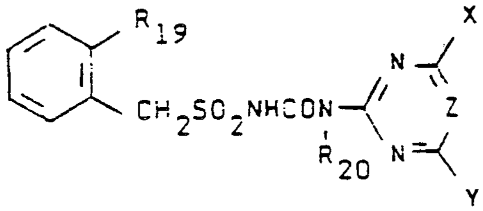

| $R_{19}$ | $R_{20}$ | X | Y | Z | m.p. (°C) |
|---|---|---|---|---|---|
| $SCH_2CH_3$ | H | Cl | $CH(OEt)_2$ | CH | |
| $SO_2CH_3$ | $CH_3$ | $OCH_3$ | $CH(SCH_2CH_2S)$ | N | |
| $SO_2CH_3$ | H | $CH_3$ | $CH(OCH_2CH_2CH_2O)$ | CH | |
| $SO_2CH_3$ | H | $CH_3$ | $CH(OCH_3)_2$ | CH | |
| $SO_2$-*n*-$C_3H_7$ | H | $C_2H_5$ | $CH(OCH(CH_3)CH_2O)$ | CH | |
| $SO_2$-*n*-$C_3H_7$ | H | $OCH_3$ | $CH_2SEt$ | N | |
| $SO_2$-*n*-$C_3H_7$ | H | $OCH_3$ | $CH_2OH$ | CH | |
| $SO_2CH_2CH{=}CH_2$ | H | $OCH_3$ | $CH_2OTHP$ | CH | |
| $SO_2$-*n*-$C_4H_9$ | H | $OCH_3$ | $CH_2OH$ | CH | |
| S-*t*-$C_4H_9$ | H | $CH_3$ | $CH(OCH_3)_2$ | CH | |
| $OCH_2CH_2Cl$ | H | $CH_3$ | $CH_2OCON(CH_3)_2$ | CH | |
| $OCHFCHF_2$ | H | $OC_2H_5$ | $CH_2OCO_2Et$ | N | |
| $OCH_2CF_3$ | H | $OCH_3$ | $CH_2OCO_2CH_3$ | CH | |
| O-*n*-$C_3H_7$ | H | $OCH_3$ | $CH(SCH_2CH_2CH_2S)$ | CH | |
| O-*n*-$C_3H_7$ | H | $OCH_3$ | $CH(OCH_2CH_2CH_2O)$ | CH | |
| O-*n*-$C_3H_7$ | H | Cl | $CH(OCH_2CH_2CH_2O)$ | CH | |
| $OCH_2CH_3$ | H | $CH_3$ | $CH(OCH_2CH_3)_2$ | CH | |
| $OCH_2CH_3$ | H | $CH_3$ | $CH_2OH$ | CH | |
| $OCF_3$ | $CH_3$ | $OCH_3$ | $CH(OCH_2CH_2O)$ | CH | |
| $OCF_3$ | H | $OCH_3$ | $CH_2SCH_3$ | N | |
| $OCF_3$ | H | $OCH_3$ | $CH_2OH$ | CH | |

TABLE 13

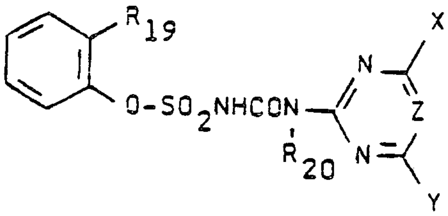

| $R_{19}$ | $R_{20}$ | X | Y | Z | m.p. (°C) |
|---|---|---|---|---|---|
| Cl | H | $CH_3$ | $CH(OCH_3)_2$ | CH | |
| Cl | $CH_3$ | $CH_3$ | $CH_2OCOCH_3$ | CH | |
| Cl | H | $CH_3$ | $CH(OCH_2CH_2O)$ | N | |
| Cl | H | $OCH_3$ | $CH_2SCH_3$ | N | |
| Cl | H | $OCH_3$ | $CH(OEt)_2$ | CH | |
| $NO_2$ | H | $C_2H_5$ | $CH(SCH_2CH_2S)$ | CH | |
| $NO_2$ | H | $CH_3$ | $CH(OCH_2CH_2CH_2O)$ | CH | |
| $NO_2$ | H | Cl | $CH(OCH_3)_2$ | CH | |
| $NO_2$ | H | $CH_3$ | $CH(OCH(CH_3)CH_2O)$ | N | |
| $NO_2$ | H | $OCH_3$ | $CH_2SEt$ | CH | |
| $NO_2$ | H | $OCH_3$ | $CH_2OH$ | CH | |
| $CF_3$ | H | $OC_2H_5$ | $CH_2OTHP$ | CH | |
| $CF_3$ | H | $CH_3$ | $CH_2OCON(CH_3)_2$ | CH | |
| $CF_3$ | H | $CH_3$ | $CH(OCH_3)_2$ | CH | |
| $CF_3$ | H | $CH_3$ | $CH(OEt)_2$ | N | |
| $CF_3$ | H | $OCH_3$ | $CH(SCH_2CH_2CH_2S)$ | CH | |
| $OCH_3$ | $CH_3$ | $OCH_3$ | $CH(OCH(CH_3)CH_2O)$ | CH | |
| $OCH_3$ | H | $OCH_3$ | $CH(OCH_2CH_2O)$ | CH | |
| $OCH_3$ | H | $OCH_3$ | $CH(OCH_3)_2$ | CH | |
| $OCH_3$ | H | $CH_3$ | $CH(OEt)_2$ | N | |
| $OCH_3$ | H | $CH_3$ | $CH_2SCH_3$ | CH | |
| $OCH_3$ | H | $CH_3$ | $CH_2SEt$ | CH | |
| $OCH_3$ | H | $CH_3$ | $CH_2OCONHCH_3$ | CH | |
| $CO_2CH_3$ | H | $CH_3$ | $CH_2SEt$ | CH | |
| $CO_2CH_3$ | $CH_3$ | $CH_3$ | $CH(OEt)_2$ | N | |
| $CO_2CH_3$ | $CH_3$ | $CH_3$ | $CH(SCH_2CH_2S)$ | CH | |

TABLE 13 (Continued)

| $R_{19}$ | $R_{20}$ | X | Y | Z | m.p. (°C) |
|---|---|---|---|---|---|
| $CO_2CH_3$ | $CH_3$ | $OCH_3$ | $CH(OCH_2CH_2O)$ | CH | |
| $CO_2CH_3$ | $CH_3$ | $OCH_3$ | $CH(OCH_3)_2$ | CH | |
| $CO_2CH_3$ | $CH_3$ | $OCH_3$ | $CH(OCH_2CH_2CH_2O)$ | N | |
| $CO_2CH_2CH_3$ | $CH_3$ | $C_2H_5$ | $CH(OCH_3)_2$ | CH | |
| $CO_2CH_2CH_3$ | $CH_3$ | $OC_2H_5$ | $CH(OCH_3)_2$ | CH | |
| $CO_2CH_2CH_3$ | $CH_3$ | $OCH_3$ | $CH(OCH_3)_2$ | CH | |
| $CO_2$-*n*-$C_4H_9$ | $CH_3$ | $OCH_3$ | $CH_2OH$ | CH | |
| $CO_2$-*i*-$C_3H_7$ | $CH_3$ | $OCH_3$ | $CH_2OH$ | N | |
| $SO_2N(OCH_3)CH_3$ | $CH_3$ | $OCH_3$ | $CH_2OH$ | CH | |
| $SO_2NCH_3)_2$ | H | $CH_3$ | $CH(OCH_2CH_2O)$ | CH | |
| $SO_2N(CH_3)_2$ | $CH_3$ | $CH_3$ | $CH(OCH_2CH_2O)$ | CH | |
| $SO_2N(CH_3)_2$ | H | $CH_3$ | $CH(OCH_2CH_2O)$ | N | |
| $SO_2N(CH_3)_2$ | H | $CH_3$ | $CH_2OCOCH_3$ | CH | |
| $SO_2N(CH_3)_2$ | H | $OCH_3$ | $CH_2OCO_2CH_3$ | CH | |
| $SO_2N(CH_3)_2$ | H | $OCH_3$ | $CH_2SCH_3$ | CH | |
| $SO_2N(CH_3)_2$ | H | $OCH_3$ | $CH_2OTHP$ | CH | |
| $SO_2(CH_2CH_3)CH_3$ | H | $OCH_3$ | $CH_2OH$ | N | |
| $SO_2(CH_2CH_3)CH_3$ | H | $CH_3$ | $CH_2OH$ | CH | |
| $OSO_2$ | H | $CH_3$ | $CH_2OH$ | CH | |
| $OSO_2CH_3$ | H | $CH_3$ | $CH(OCH_2CH_3)_2$ | CH | |
| $OSO_2CH_3$ | H | $CH_3$ | $CH(OCH_3)_2$ | CH | |
| $OSO_2CH_3$ | H | $OCH_3$ | $CH(OCH_3)_2$ | CH | |
| $OSO_2CH_2CH_2OCH_3$ | H | $OCH_3$ | $CH(OCH_3)_2$ | N | |
| $OSO_2CH_2CF_3$ | H | $OCH_3$ | $CH(OCH_2CH_2O)$ | CH | |
| $SCH_3$ | H | $CH_3$ | $CH(OCH_3)_2$ | CH | |
| $SCH_3$ | $CH_3$ | $CH_3$ | $CH_2OCOC_2H_5$ | N | |
| $SCH_3$ | H | $OCH_3$ | $CH(OCH_2CH_2O)$ | N | |
| $SCH_2CH_3$ | H | $OCH_3$ | $CH_2SCH_3$ | CH | |
| $SCH_2CH_3$ | H | Cl | $CH(OEt)_2$ | CH | |
| $SO_2CH_3$ | $CH_3$ | $OCH_3$ | $CH(SCH_2CH_2S)$ | N | |

TABLE 13 (Continued)

| $R_{19}$ | $R_{20}$ | X | Y | Z | m.p. (°C) |
|---|---|---|---|---|---|
| $SO_2CH_3$ | H | $CH_3$ | $CH(OCH_2CH_2CH_2O)$ | CH | |
| $SO_2CH_3$ | H | $CH_3$ | $CH(OCH_3)_2$ | CH | |
| $SO_2$-$n$-$C_3H_7$ | H | $C_2H_5$ | $CH(OCH(CH_3)CH_2O)$ | CH | |
| $SO_2$-$n$-$C_3H_7$ | H | $OCH_3$ | $CH_2SEt$ | N | |
| $SO_2$-$n$-$C_3H_7$ | H | $OCH_3$ | $CH_2OH$ | CH | |
| $SO_2CH_2CH{=}CH_2$ | H | $OCH_3$ | $CH_2OTHP$ | CH | |
| $SO_2$-$n$-$C_4H_9$ | H | $OCH_3$ | $CH_2OH$ | CH | |
| S-$t$-$C_4H_9$ | H | $CH_3$ | $CH(OCH_3)_2$ | CH | |
| $OCH_2CH_2Cl$ | H | $CH_3$ | $CH_2OCON(CH_3)_2$ | CH | |
| $OCHFCHF_2$ | H | $OC_2H_5$ | $CH_2OCO_2Et$ | N | |
| $OCH_2CF_3$ | H | $OCH_3$ | $CH_2OCO_2CH_3$ | CH | |
| O-$n$-$C_3H_7$ | H | $OCH_3$ | $CH(SCH_2CH_2CH_2S)$ | CH | |
| O-$n$-$C_3H_7$ | H | $OCH_3$ | $CH(OCH_2CH_2CH_2O)$ | CH | |
| O-$n$-$C_3H_7$ | H | Cl | $CH(OCH_2CH_2CH_2O)$ | CH | |
| $OCH_2CH_3$ | H | $CH_3$ | $CH(OCH_2CH_3)_2$ | CH | |
| $OCH_2CH_3$ | H | $CH_3$ | $CH_2OH$ | CH | |
| $OCF_3$ | $CH_3$ | $OCH_3$ | $CH(OCH_2CH_2O)$ | CH | |
| $OCF_3$ | H | $OCH_3$ | $CH_2SCH_3$ | N | |
| $OCF_3$ | H | $OCH_3$ | $CH_2OH$ | CH | |

Formulations

Useful formulations of the compounds of Formula I can be prepared in conventional ways. They include dusts, granules, pellets, solutions, suspensions, emulsions, wettable powders, emulsifiable concentrates and the like. Many of these may be applied directly. Sprayable formulations can be extended in suitable media and used at spray volumes of from a few liters to several hundred liters per hectare. High strength compositions are primarily used as intermediates for further formulation. The formulations, broadly, can contain from about 0.1% to 99% by weight of active ingredient(s) and at least one of (a) about 0.1% to 20% surfactant(s) and (b) about 1% to 99.9% solid or liquid inert diluent(s). More specifically, they will contain these ingredients in the following approximate proportions:

TABLE 14

| | Active Ingredient | Weight Percent* Diluent(s) | Surfactant(s) |
|---|---|---|---|
| Wettable Powders | 20—90 | 0—74 | 1—10 |
| Oil Suspensions, Emulsions Solutions, (including Emulsifiable Concentrates) | 3—50 | 40—95 | 0—15 |
| Aqueous Suspension | 10—50 | 40—84 | 1—20 |
| Dusts | 1—25 | 70—99 | 0—5 |
| Granules and Pellets | 0.1—95 | 5—99.9 | 0—15 |
| High Strength Compositions | 90—99 | 0—10 | 0—2 |

* Active ingredient plus at least one of a Surfactant or a Diluent equals 100 weight percent.

Lower or higher levels of active ingredient can, of course, be present depending on the intended use of the physical properties of the compound. Higher ratios of surfactant to active ingredient are sometimes desirable, and are achieved by incorporation into the formulation or by tank mixing.

Typical solid diluents are described in Watkins, et al., "Handbook of Insecticide Dust Diluents and Carriers", 2nd Ed., Dorland Books, Caldwell, New Jersey, but other solids, either mined or manufactured, may be used. The more absorptive diluents are preferred for wettable powders and the denser ones for dusts. Typical liquid diluents and solvents are described in Marsden, "Solvents Guide", 2nd Ed., Interscience, New York, 1950. Solubility under 0.1% is preferred for suspension concentrates; solution concentrates are preferably stable against phase separation at 0°C. "McCutcheon's Detergents and Emulsifiers Annual". MC Publishing Corp., Ridgewood, New Jersey, as well as Sisely and Wood, "Encyclopedia of Surface Active Agents", Chemical Publishing Co., Inc., New York, 1964, list surfactants and recommended uses. All formulations can contain minor amounts of additives to reduce foaming, caking, corrosion, microbiological growth, etc.

The methods of making such compositions are well known. Solutions are prepared by simply mixing the ingredients. Fine solid compositions are made by blending and, usually, grinding as in a hammer or fluid energy mill. Suspensions are prepared by wet milling (see, for example, Littler, U.S. Patent 3,060,084). Granules and pellets may be made by spraying the active material upon preformed granular carriers or by agglomeration techniques. See J. E. Browning, "Agglomeration", *Chemical Engineering,* December 4, 1967, pp. 147ff. and "Perry's Chemical Engineer's Handbook", 5th Ed., McGraw-Hill, New York, 1973, pp. 8—57ff.

For further information regarding the art of formulation, see for example:

H. M. Loux, U.S. Patent 3,235,361, February 15, 1966, Col. 6, line 16 through Col. 7, line 19 and Examples 10 through 41;

R. W. Luckenbaugh, U.S. Patent 3,309,192, March 14, 1967, Col. 5, line 43 through Col. 7, line 62 and Examples 8, 12, 15, 39, 41, 52, 53, 58, 132, 138—140, 162—164, 166, 167 and 169—182;

H. Gysin and E. Knusli, U.S. Patent 2,891,855, June 23, 1959, Col. 3, line 66 through Col. 5, line 17 and Examples 1—4;

G. C. Klingman, "Weed Control as Science", John Wiley & Sons, Inc., New York, 1961, pp. 81—96; and

J. D. Fryer and S. A. Evans, "Weed Control Handbook", 5th Ed., Blackwell Scientific Publications, Oxford, 1968, pp. 101—103.

In the following examples, all parts are by weight unless otherwise indicated.

Example 7

*Wettable Powder*

| | |
|---|---|
| 3-[[(4-dimethoxymethyl-6-methylpyrimidin-2-yl)-aminocarbonyl]aminosulfonyl]-2-thiophene-carboxylic acid, methyl ester | 80% |
| sodium alkylnaphthalenesulfonate | 2% |

| | |
|---|---|
| sodium ligninsulfonate | 2% |
| synthetic amorphous silica | 3% |
| kaolinite | 13% |

The ingredients are blended, hammer-milled until all the solids are essentially under 50 microns, reblended and packaged.

Example 8

*Wettable Powder*

| | |
|---|---|
| N-[(4-dimethoxymethyl-6-methylpyrimidin-2-yl)-aminocarbonyl]-2-(methylsulfonyl)benzene-sulfonamide | 50% |
| sodium alkylnaphthalenesulfonate | 2% |
| low viscosity methyl cellulose | 2% |
| diatomaceous earth | 46% |

The ingredients are blended, coarsely hammer-milled and then air-milled to produce particles essentially all below 10 microns in diameter. The product is reblended before packaging.

Example 9

*Granule*

| | |
|---|---|
| Wettable Powder of Example 8 | 5% |
| attapulgite granules | 95% |
| (U.S.S. 20—40 mesh; 0.84—0.42 mm) | |

A slurry of wettable powder containing ≈ 25% solids is sprayed on the surface of attapulgite granules in a double-cone blender. The granules are dried and packaged.

Example 10

*Extruded Pellet*

| | |
|---|---|
| 2-[[[4-chloro-6-(dimethoxymethyl)pyrimidin-2-yl]-aminocarbonyl]aminosulfonyl]benzoic acid, methyl ester | 25% |
| anhydrous sodium sulfate | 10% |
| crude calcium ligninsulfonate | 5% |
| sodium alkylnaphthalenesulfonate | 1% |
| calcium/magnesium bentonite | 59% |

The ingredients are blended, hammer-milled and then moistened with about 12% water. The mixture is extruded as cylinders about 3 mm diameter which are cut to produce pellets about 3 mm long. These may be used directly after drying, or the dried pellets may be crushed to pass a U.S.S. No. 20 sieve (0.84 mm openings). The granules held on a U.S.S. No. 40 sieve (0.42 mm openings) may be packaged for use and the fines recycled.

Example 11

*Oil Suspension*

| | |
|---|---|
| N-[[4-(hydroxymethyl)-6-methylpyrimidin-2-yl]-aminocarbonyl]-2-nitrobenzenesulfonamide | 25% |
| polyoxyethylene sorbitol hexaoleate | 5% |
| highly aliphatic hydrocarbon oil | 70% |

The ingredients are ground together in a sand mill until the solid particles have been reduced to under about 5 microns. The resulting thick suspension may be applied directly, but preferably after being extended with oils or emulsified in water.

Example 12

*Wettable Powder*

| | |
|---|---|
| 2-[[[4-chloro-6-(dimethoxymethyl)pyrimidin-2-yl]-aminocarbonyl]aminosulfonyl]benzoic acid, methyl ester | 20% |
| sodium alkylnaphthalenesulfonate | 4% |
| sodium ligninsulfonate | 4% |
| low viscosity methyl cellulose | 3% |
| attapulgite | 69% |

The ingredients are thoroughly blended. After grinding in a hammer-mill to produce particles essentially all below 100 microns, the material is re-blended and sifted through a U.S.S. No. 50 sieve (0.3 mm opening) and packaged.

Example 13

*Low Strength Granule*

| | |
|---|---|
| 2-[[[4-methyl-6-(tetrahydropyran-2-yloxymethyl)-pyrimidin-2-yl]aminocarbonyl]aminosulfonyl]-benzoic acid, methyl ester | 1% |
| N,N-dimethylformamide | 9% |
| attapulgite granules (U.S.S. 20—40 sieve) | 90% |

The active ingredient is dissolved in the solvent and the solution is sprayed upon dedusted granules in a double cone blender. After spraying of the solution has been completed, the blender is allowed to run for a short period and then the granules are packaged.

Example 14

*Aqueous Suspension*

| | |
|---|---|
| 2-[[[4-(acetyloxymethyl)-6-methylpyrimidin-2-yl]-aminocarbonyl]aminosulfonyl]benzoic acid, methyl ester | 40% |
| polyacrylic acid thickener | 0.3% |
| dodecylphenol polyethylene glycol ether | 0.5% |

| | |
|---|---|
| disodium phosphate | 1% |
| monosodium phosphate | 0.5% |
| polyvinyl alcohol | 1.0% |
| water | 56.7% |

The ingredients are blended and ground together in a sand mill to produce particles essentially all under 5 microns in size.

Example 15

*Solution*

| | |
|---|---|
| 2-chloro-N-]]4-methyl-6-(tetrahydropyran-2-yloxymethyl)pyrimidin-2-yl]aminocarbonyl]benzene-sulfonamide, sodium salt | 5% |
| water | 95% |

The salt is added directly to the water with stirring to produce the solution, which may then be packaged for use.

Example 16

*Low Strength Granule*

| | |
|---|---|
| 2-[[[4-(dimethoxymethyl)-6-methylpyrimidin-2-yl]-aminocarbonyl]aminosulfonyl]benzoic acid, methyl ester | 0.1% |
| attapulgite granules (U.S.S. 20—40 mesh) | 99.9% |

The active ingredient is dissolved in a solvent and the solution is sprayed upon dedusted granules in a double-cone blender. After spraying of the solution has been completed, the material is warmed to evaporate the solvent. The material is allowed to cool and then packaged.

Example 17

*Granule*

| | |
|---|---|
| N-[[4-methyl-6-(tetrahydropyran-2-yloxymethyl)-pyrimidin-2-yl]aminocarbonyl]-2-(methylsulfonyl)-benzenesulfonamide | 80% |
| wetting agent | 1% |
| crude ligninsulfonate salt (containing 5—20% of the natural sugars) | 10% |
| attapulgite clay | 9% |

The ingredients are blended and milled to pass through a 100 mesh screen. This material is then added to a fluid bed granulator, the air flow is adjusted to gently fluidize the material, and a fine spray of water is sprayed onto the fluidized material. The fluidization and spraying are continued until granules of the desired size range are made. The spraying is stopped, but fluidization is continued, optionally with heat, until the water content is reduced to the desired level, generally less than 1%. The material is then discharged, screened to the desired size range, generally 14—100 mesh (1410—149 microns), and packaged for use.

Example 18

*High Strength Concentrate*

| | |
|---|---|
| 2-chloro-N-[[4-chloro-6-(dimethoxymethyl)pyrimidin-2-yl]aminocarbonyl]benzenesulfonamide | 99% |
| silica aerogel | 0.5% |
| synthetic amorphous silica | 0.5% |

The ingredients are blended and ground in a hammer-mill to produce a material essentially all passing a U.S.S. No. 50 screen (0.3 mm opening). The concentrate may be formulated further if necessary.

Example 19

*Wettable Powder*

| | |
|---|---|
| N-[[4-methyl-6-(tetrahydropyran-2-yloxymethyl)-pyrimidin-2-yl]aminocarbonyl]-2-(methylsulfonyl)-benzenesulfonamide | 90% |
| dioctyl sodium sulfosuccinate | 0.1% |
| synthetic fine silica | 9.9% |

The ingredients are blended and ground in a hammer-mill to produce particles essentially all below 100 microns. The material is sifted through a U.S.S. No. 50 screen and then packaged.

Example 20

*Wettable Powder*

| | |
|---|---|
| 2-chloro-N-[[4-(dimethoxymethyl)-6-methylpyrimidin-2-yl]aminocarbonyl]benzenesulfonamide | 40% |
| sodium ligninsulfonate | 20% |
| montmorillonite clay | 40% |

The ingredients are thoroughly blended, coarsely hammer-milled and then air-milled to produce particles essentially all below 10 microns in size. The material is reblended and then packaged.

Example 21

*Oil Suspension*

| | |
|---|---|
| 2-[[[4-(dimethoxymethyl)-6-methylpyrimidin-2-yl]amino-carbonyl]aminosulfonyl]benzoic acid, 2-propyl ester | 35% |
| blend of polyalcohol carboxylic esters and oil soluble petroleum sulfonates | 6% |
| xylene | 59% |

The ingredients are combined and ground together in a sand mill to produce particles essentially all below 5 microns. The product can be used directly, extended with oils, or emulsified in water.

*Utility*

The compounds of the present invention are highly active herbicides. They have utility for broadspectrum pre- and/or post-emergence weed control in areas where complete control of all vegetation is desired, such as around fuel storage tanks, ammunition depots, industrial storage areas, parking lots, drive-in theaters, around billboards, highway and railroad structures. Alternatively, the subject compounds are useful for selective pre- or post-emergence weed control in crops, such as cotton, soybeans and wheat.

The rates of application for the compounds of the invention are influenced by a number of factors, including their use whether as selective or general herbicides, the crop species involved, the types of weeds to be controlled, weather and climate, formulation ingredients, mode of application, amount of foliage present, etc. In general terms, the subject compounds should be applied at levels of around 0.01 to 5 kg/ha, the lower rates being suggested for use on lighter soils and/or those having a low organic matter content, for selective weed control or for situations where only short-term persistence is required.

The compounds of the invention may be used in combination with any other commercial herbicide, such as, for example those of the triazine, triazole, uracil, urea, amide, diphenylether, carbamate and bipyridylium types.

The herbicide properties of the subject compounds were discovered in a number of greenhouse tests. The test procedures and results are shown below.

*Test A*

Seeds of crabgrass (*Digitaria* sp.), barnyardgrass (*Echinochloa crusgalli*), wild oats (*Avena fatua*), sicklepod (*Cassia obtusifolia*), morningglory (*Ipomoea* sp.), cocklebur (*Xanthium* sp.), sorghum, corn, soybean, rice, wheat and nutsedge tubers (*Cyperus rotundus*) were planted in a growth medium and treated pre-emergence with the chemicals dissolved in a non-phytotoxic solvent. At the same time, cotton having five leaves (including cotyledonary ones), bush beans with the third trifoliolate leaf expanding, crabgrass, barnyardgrass and wild oats with two leaves, cassia with three leaves (including cotyledonary ones), morningglory and cocklebur with four leaves (including the cotyledonary ones), sorghum and corn with four leaves, soybean with two cotyledonary leaves, rice with three leaves, wheat with one leaf, and nutsedge with three to five leaves were sprayed. Treated plants and controls were maintained in a greenhouse for sixteen days, whereupon all species were compared to controls and visually rated for response to treatment. The ratings are based on a numerical scale extending from 0 = no injury, to 10 = complete kill. The accompanying descriptive symbols have the following meanings:

C = chlorosis or necrosis;
D = defoliation;
E = emergence inhibition;
G = growth retardation;
H = formative effects;
S = albinism;
U = unusual pigmentation;
U = unusual pigmentation;
X = axillary stimulation; and
6Y = abscised buds or flowers.

The ratings for the compounds tested by this procedure are presented in Table A.

Table A Structures

Compound 1

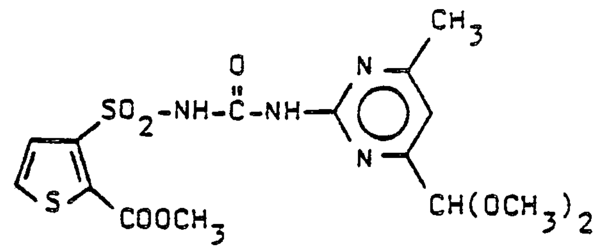

Compound 2

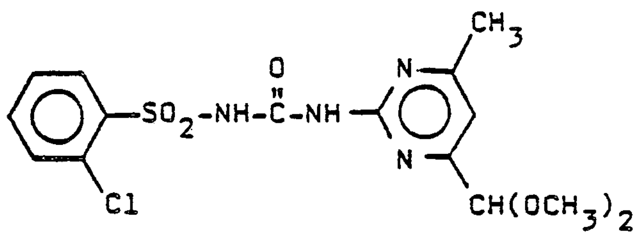

Compound 3

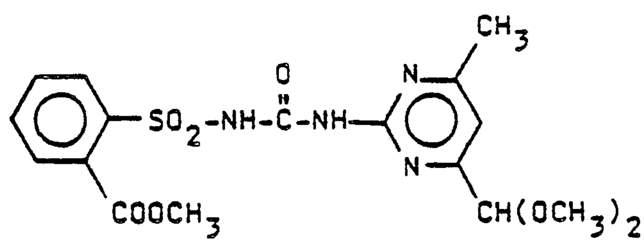

Compound 4

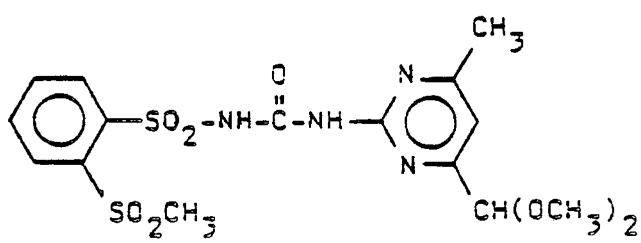

Compound 5

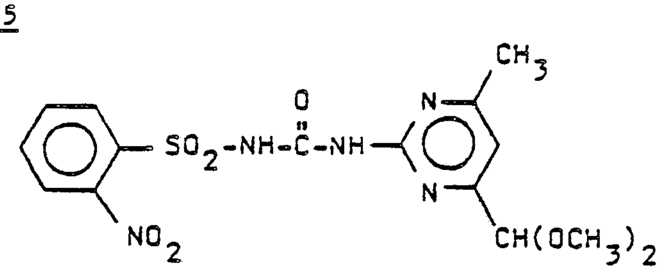

Compound 6

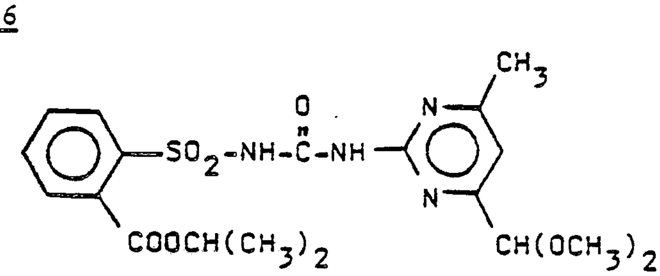

Table A Structures (Continued)

Compound 7

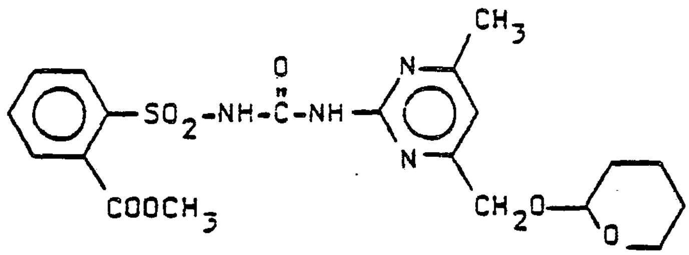

Compound 8

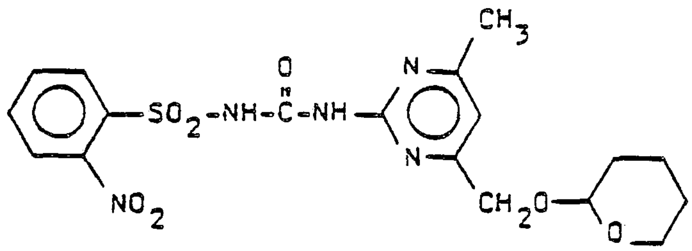

Compound 9

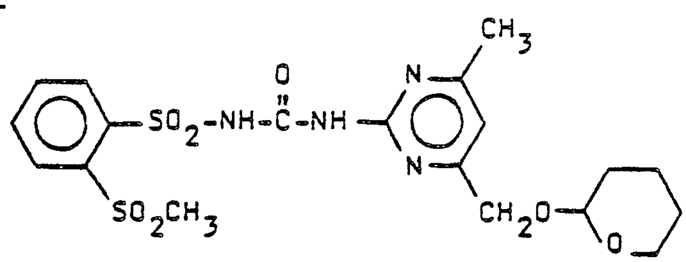

Compound 10

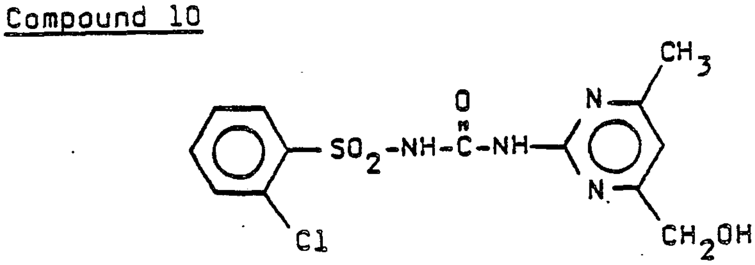

Compound 11

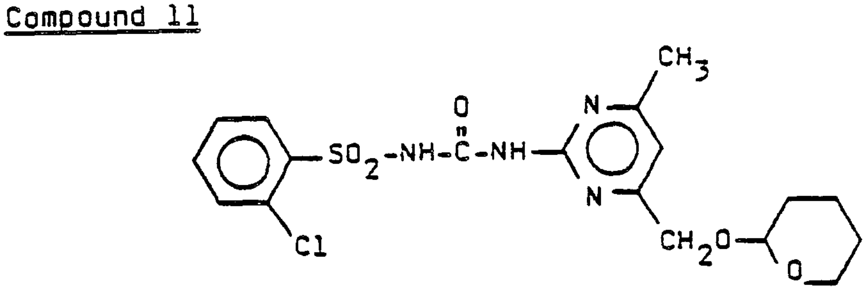

Table A Structures (Continued)

Compound 12

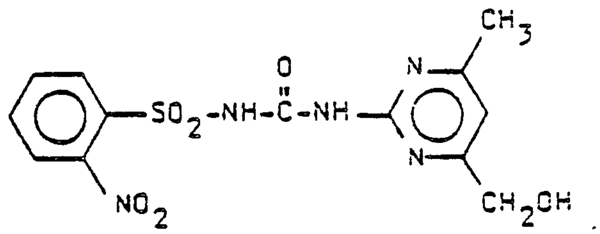

Compound 13

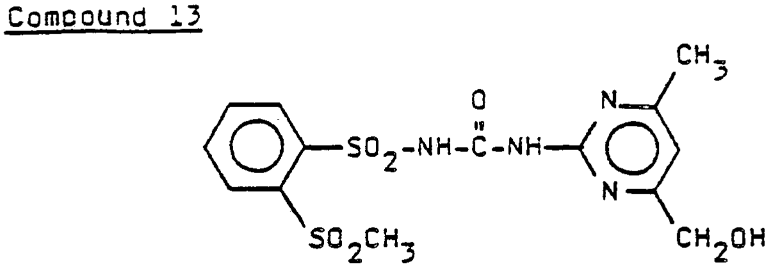

Compound 14

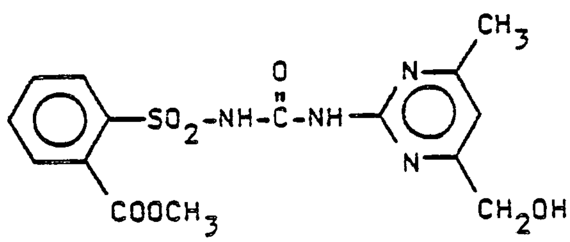

Compound 15

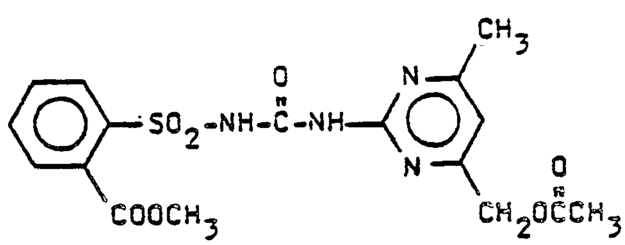

Compound 16

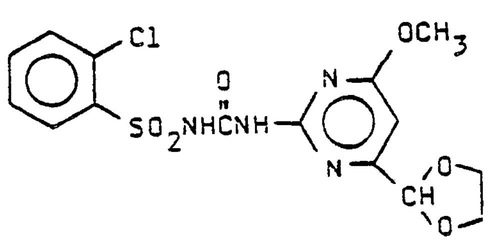

Table A Structures (Continued)

Compound 17

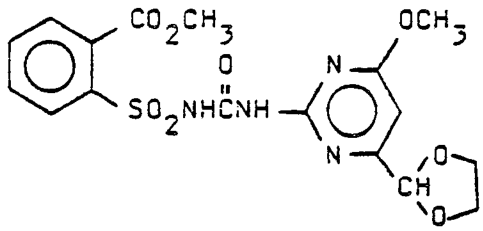

Compound 18

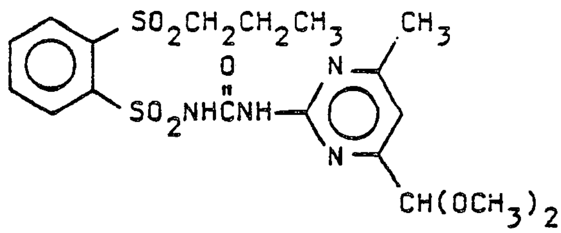

Compound 19

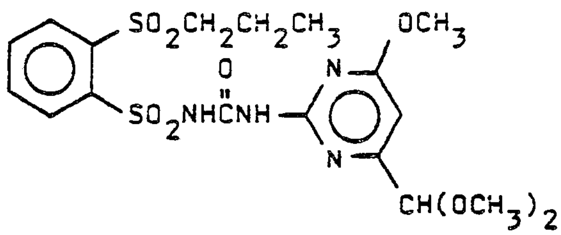

Compound 20

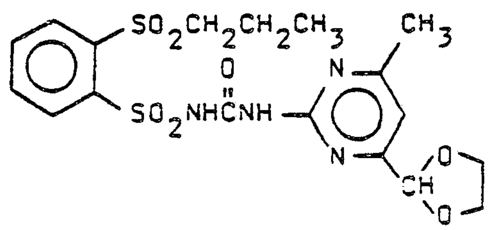

Compound 21

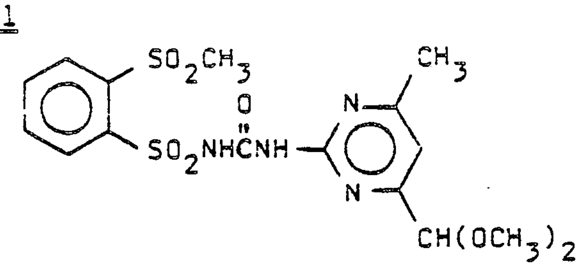

Compound 22

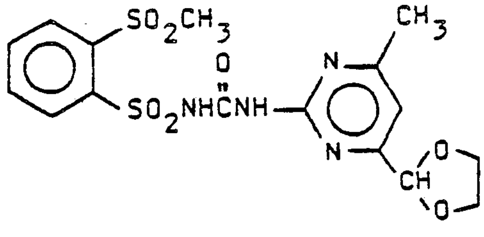

Table A Structures (Continued)

Compound 23

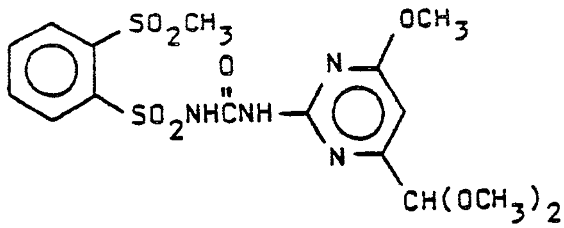

Compound 24

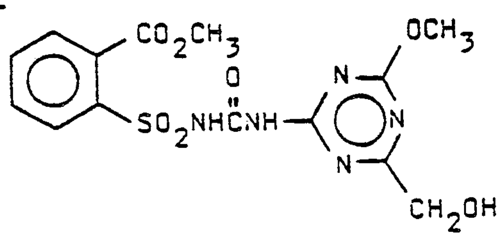

Compound 25

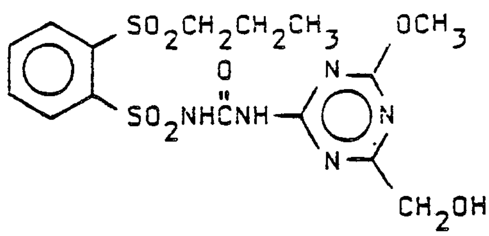

Compound 26

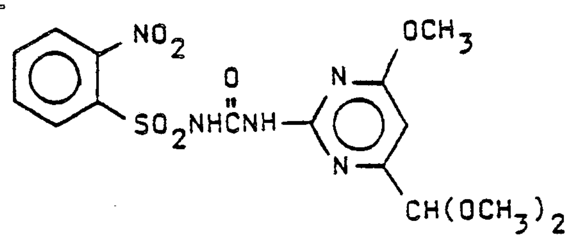

Compound 27

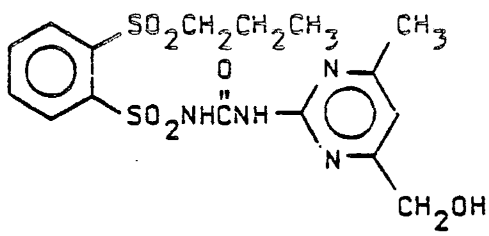

Compound 28

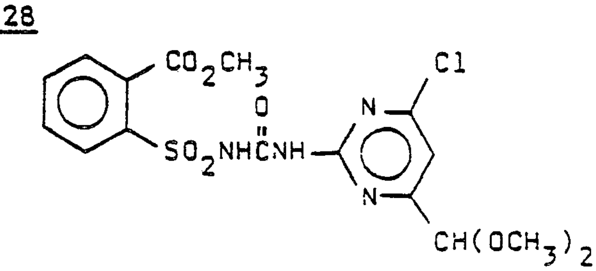

Table A Structures (Continued)

Compound 29

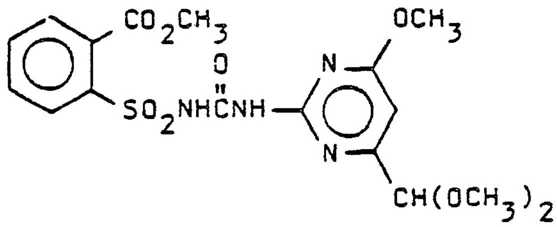

Compound 30

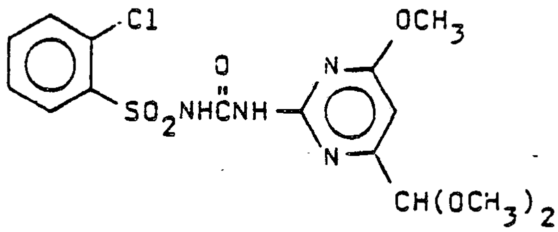

Compound 31

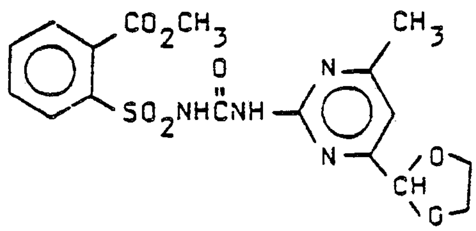

Compound 32

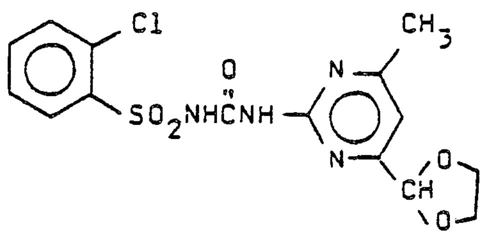

Compound 33

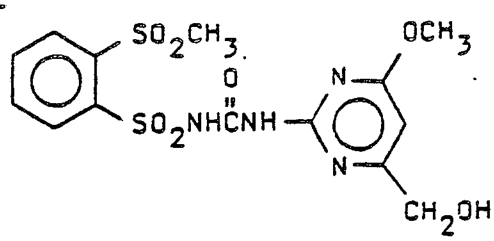

Compound 34

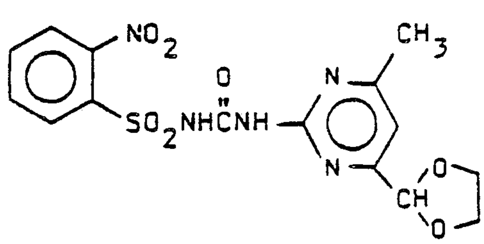

Table A Structures (Continued)

Compound 35

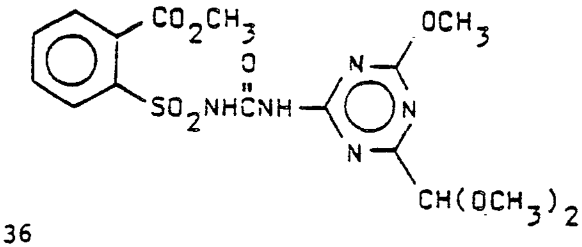

Compound 36

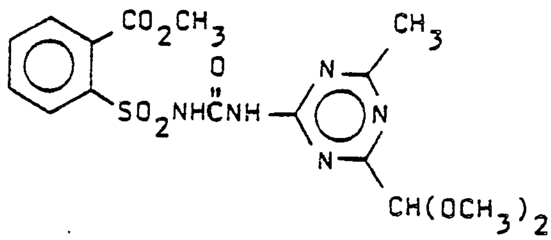

Compound 37

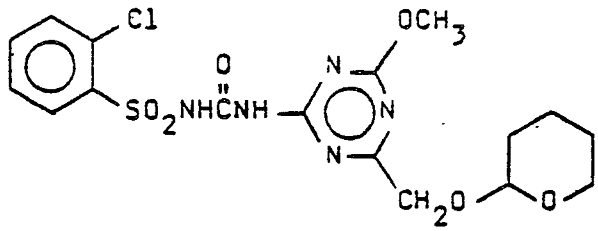

Compound 38

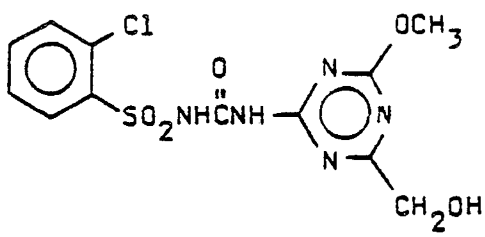

Compound 39

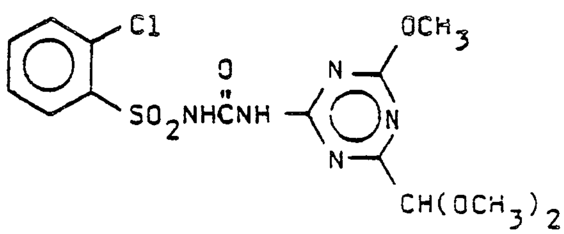

Compound 40

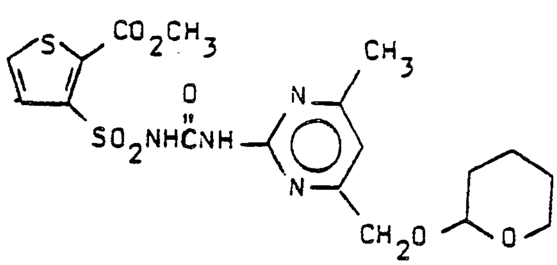

Table A Structures (Continued)

Compound 41

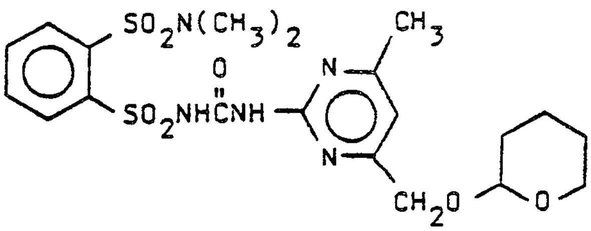

Compound 42

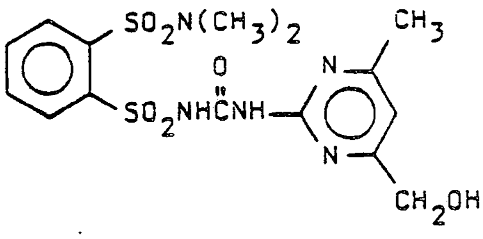

Compound 43

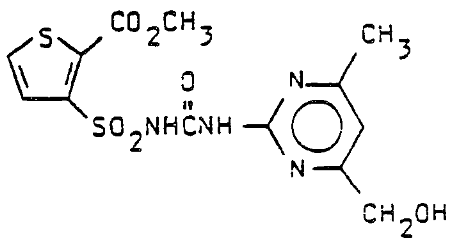

Compound 44

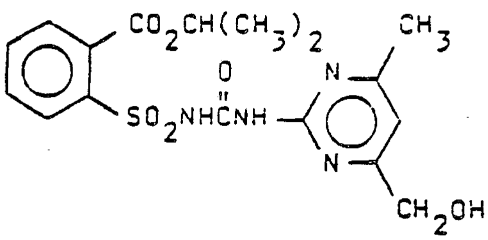

Compound 45

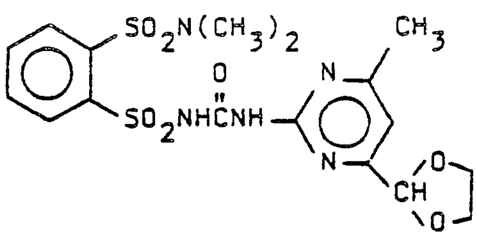

Compound 46

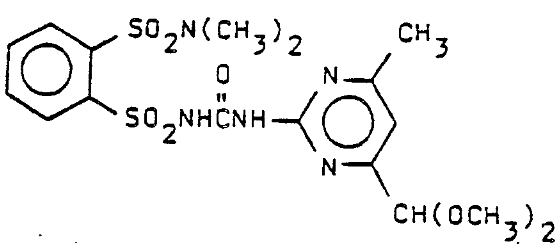

Table A Structures (Continued)

Compound 47

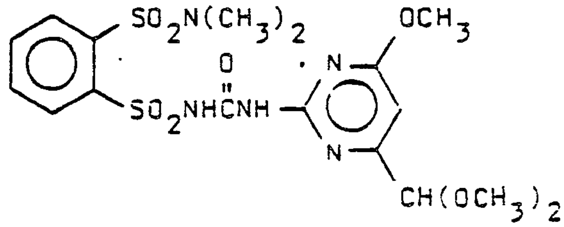

Table A

| | Cmpd. 1 | Cmpd. 2 | Cmpd. 3 | Cmpd. 4 |
|---|---|---|---|---|
| Rate kg/ha | .05 | .05 | .05 | .05 |
| POST-EMERGENCE | | | | |
| Bush bean | 4C,9G,6Y | 5C,9G,6Y | 9C | 3C,9G,6Y |
| Cotton | 2C,2H,5G | 2C,3H6G | 6C, 9G | 4C,9H |
| Morningglory | 1C,3G | 1C,5G | 2C,5G | 3C,7H |
| Cocklebur | 2C,9G | 2C,8G | 9C | 3C,9H |
| Sicklepod | 1C | 2C,5G | 5C,9G | 3C,6G |
| Nutsedge | 0 | 1C,3G | 3C,9G | 2C,8G |
| Crabgrass | 1C,5G | 5G | 4C,9G | 2C,6G |
| Barnyardgrass | 2C,9H | 2C,9H | 9C | 5C,9H |
| Wild Oats | 1C,3G | 2C,8H | 9C | 3C,8H |
| Wheat | 7G,5X | 2C,8H | 3U,9G | 2C,8H |
| Corn | 2U,9H | 5U,9C | 9C | 5U,9C |
| Soybean | 1G | 2C,7G | 6C,9G | 5C,8H,5X |
| Rice | 7G | 6C,9G | 6C,9G | 5C,9G |
| Sorghum | 9G | 4U,9C | 5U,9C | 3U,9G |
| PRE-EMERGENCE | | | | |
| Morningglory | 6G | 3C,5G | 8H | 3C,5H |
| Cocklebur | 9H | 9H | 9H | 1H |
| Sicklepod | 0 | 7G | 2C,7G | 1C |
| Nutsedge | 0 | 2C,8G | 10E | 2C,8G |
| Crabgrass | 1C,4G | 2C | 3C,9G | 1C |
| Barnyardgrass | 4C,7H | 3C,9H | 6C,9H | 3C,9H |
| Wild Oats | 2C,7G | 2C,9G | 5C,9H | 2C,9G |
| Wheat | 2C,8G | 2C,9G | 9H | 9G |
| Corn | 2C,6G | 2C,9G | 10E | 2U,9G |
| Soybean | 0 | 1H | 2C,7G | 6G |
| Rice | 8H | 9H | 10E | 2C,8H |
| Sorghum | 2C,8H | 5C,9G | 10H | 2C,9G |

Table A (continued)

| | Cmpd. 5 | Cmpd. 6 | Cmpd. 7 | Cmpd. 8 |
|---|---|---|---|---|
| Rate kg/ha | .05 | .05 | 0.4 | 0.4 |
| POST-EMERGENCE | | | | |
| Bush bean | 9C | 9C | 9C | 9C |
| Cotton | 4C,9G | 3C,2H,9G | 9C | 6C,9G |
| Morningglory | 2C,8G | 2C,8H | 9C | 4,9G |
| Cocklebur | 5C,9G | 5C,9G | 10C | 10C |
| Sicklepod | 2C,5G | 3C,7G | 9C | 3C,5G |
| Nutsedge | 10C | 10C | 10C | 10C |
| Crabgrass | 6C,9G | 1C,2H | 9C | 10C |
| Barnyardgrass | 9C | 3C,9H | 9C | 10C |
| Wild Oats | 2C,9G | 3C,9G | 9C | 5C,9G |
| Wheat | 3C,9G | 2C,8G | 5U,9G | 2C,8G |
| Corn | 5U,9G | 3U,9G | 10C | 10C |
| Soybean | 5C,9G | 2C,9G | 9C | 9C |
| Rice | 5C,9G | 4C,9G | 6C,9G | 6C,9G |
| Sorghum | 6U,9G | 3U,9C | 9C | 9C |
| PRE-EMERGENCE | | | | |
| Morningglory | 8G | 1C | 2C,9H | 4C,9G |
| Cocklebur | 9H | 9H | — | 9H |
| Sicklepod | 8G | 2C,5G | 5C,9G | 2C,8G |
| Nutsedge | 10E | 1C,7G | 10E | 10E |
| Crabgrass | 2C,5G | 1C | 3C,9H | 3C,9G |
| Barnyardgrass | 5C,8H | 2C,6H | 5C,9H | 3C,9H |
| Wild Oats | 2C,9G | 2C,8G | 4C,9H | 3C,9H |
| Wheat | 1C,8G | 1C,7G | 9H | 9H |
| Corn | 2C,9H | 2C,8H | 10H | 10E |
| Soybean | 2C,3H | 1H | 9H | 9H |
| Rice | 10E | 3C,6G | 10E | 10E |
| Sorghum | 2C,9H | 5C,9H | 7C,9H | 3C,9H |

Table A (continued)

| | Cmpd. 9 | Cmpd. 10 | Cmpd. 11 | Cmpd. 12 |
|---|---|---|---|---|
| Rate kg/ha | 0.4 | 0.4 | 0.4 | 0.4 |
| POST-EMERGENCE | | | | |
| Bush bean | 9C | 9C | 9C | 9C |
| Cotton | 4C,9H | 3C,8G | 4C,9G | 9C |
| Morningglory | 3C,7G | 2C,8G | 3C,8H | 5C,9G |
| Cocklebur | 5C,9G | 10C | 10C | 10C |
| Sicklepod | 4C,7G | 3C,5H | 2C,5G | 2C,6G |
| Nutsedge | 2C,8G | 9C | 3C,9G | 9C |
| Crabgrass | 2C,7G | 1C,8G | 2C,8G | 9C |
| Barnyardgrass | 9C | 9C | 6C,9H | 9C |
| Wild Oats | 2C,9G | 3C,9G | 3C,9G | 9C |
| Wheat | 2C,9G | 2C,9G | 3C,9G | 2C,8G |
| Corn | 6U,9C | 7U,9C | 7U,9G | 10C |
| Soybean | 6U,9G | 9C | 5C,9G | 9C |
| Rice | 6C,9G | 5C,9G | 9C | 9C |
| Sorghum | 6U,9G | 10C | 10C | 10C |
| PRE-EMERGENCE | | | | |
| Morningglory | 8G | 8G | 7G | 9H |
| Cocklebur | 9H | 5C,9H | 9H | 9H |
| Sicklepod | 5G | 2C,8G | 2C,7G | 2C,8G |
| Nutsedge | 3C,9G | 2C,7G | 2C,8G | 10E |
| Crabgrass | 1C,4G | 2C,5G | 2C,5G | 2C,8G |
| Barnyardgrass | 5C,9H | 5C,9H | 3C,9H | 2C,9H |
| Wild Oats | 2C,9G | 2C,9H | 9G | 3C,9G |
| Wheat | 9H | 2C,9G | 9G | 2C,8G |
| Corn | 5C,9G | 3C,9H | 6C,9G | 10H |
| Soybean | 3C,8H | 3C,4H | 3C,7H | 9H |
| Rice | 10E | 5C,9H | 9H | 10E |
| Sorghum | 10H | 10H | 4C,9H | 5C,9H |

Table A (continued)

| | Cmpd. 13 | Cmpd. 14 | Cmpd. 15 |
|---|---|---|---|
| Rate kg/ha | 0.4 | .05 | .05 |
| POST-EMERGENCE | | | |
| Bush bean | 9C | 9C | 9C |
| Cotton | 5C,9G | 6C,9G | 8C |
| Morningglory | 2C,8H | 2C,8G | 3C,7G |
| Cocklebur | 6C,9G | 10C | 10C |
| Sicklepod | 5C,9G | 5C,9G | 5C,9G |
| Nutsedge | 9C | 5C,9G | 10C |
| Crabgrass | 3C,8G | 4C,9G | 9C |
| Barnyardgrass | 10C | 9C | 9C |
| Wild Oats | 9C | 9C | 4C,9G |
| Wheat | 9C | 4C,9G | 5C,9G |
| Corn | 10C | 10C | 10C |
| Soybean | 9C | 9C | 9C |
| Rice | 9C | 6C,9G | 4C,9G |
| Sorghum | 9C | 10C | 10C |
| PRE-EMERGENCE | | | |
| Morningglory | 8H | 8G | 8G |
| Cocklebur | 9H | 9H | 9H |
| Sicklepod | 1C,3G | 4C,9G | 3C,8G |
| Nutsedge | 2C,9G | 10E | 10E |
| Crabgrass | 3C,6G | 3C,8G | 3C,8H |
| Barnyardgrass | 5C,9G | 5C,9H | 5C,9H |
| Wild Oats | 3C,9H | 4C,9H | 5C,9H |
| Wheat | 3C,9G | 2C,9H | 9H |
| Corn | 2C,9G | 10E | 10H |
| Soybean | 6H | 2C,8H | 9H |
| Rice | 5C,9H | 9H | 10E |
| Sorghum | 2C,9G | 3C,9H | 5C,9H |

Table A (continued)

| | Cmpd. 16 | Cmpd. 17 | Cmpd. 18 | Cmpd. 19 |
|---|---|---|---|---|
| Rate kg/ha | 0.05 | 0.05 | 0.05 | 0.05 |
| POST-EMERGENCE | | | | |
| Bush bean | 4C,9G,6Y | 9C | 4S,7G,6Y | 4S,2H,6Y |
| Cotton | 2C,3H,8G | 5C,9G | 2C,4G | 3C |
| Morningglory | 3C,9G | 5C,9G | 3C,6H | 3C,8H |
| Cocklebur | 1C,9G | 9C | 4C,8G | 2C,2G |
| Sicklepod | 3C,8G | 6C,9G | 2C,3H | 2C |
| Nutsedge | 5G | 5C,9G | 5G | 0 |
| Crabgrass | 7G | 5C,9G | 2G | 1C |
| Barnyardgrass | 2C,9H | 9C | 2C,8H | 2C,6H |
| Wild Oats | 2C,9G | 5C,9G | 2G | 4G |
| Wheat | 3C,9G | 4C,9G | 2G | 3G |
| Corn | 9C | 9C | 2C | 2C,7H |
| Soybean | 2C,9G | 5C,9G | 2C,4G | 1C |
| Rice | 5C,9G | 6C,9G | 2C,6G | 2C,8G |
| Sorghum | 4C,9G | 9C | 2C,6H | 1C,9G |
| Sugar beet | 4C,9G | 9C | 2C,2H | 1C,5H |
| PRE-EMERGENCE | | | | |
| Morningglory | 9G | 9C | 2C,5H | 1C,2H |
| Cocklebur | 7H | 9H | — | 0 |
| Sicklepod | 7H | 9G | 2C | 0 |
| Nutsedge | 4G | 10E | 0 | 0 |
| Crabgrass | 2G | 5G | 0 | 0 |
| Barnyardgrass | 8H | 9H | 2C | 1C |
| Wild Oats | 8G | 2C,9G | 1C,4G | 4G |
| Wheat | 9G | 5C,9H | 3G | 4G |
| Corn | 9H | 10H | 2C | 2C,9H |
| Soybean | 1C,1H | 9H | 2G | 0 |
| Rice | 9H | 10E | 2G | 5G |
| Sorghum | 9H | 10H | 1C,3G | 2C,9H |
| Sugar beet | 8G | 5C,9G | 1C,3H | 0 |

Table A (continued)

| | Cmpd. 20 | Cmpd. 21 | Cmpd. 22 | Cmpd. 23 |
|---|---|---|---|---|
| Rate kg/ha | 0.05 | 0.05 | 0.05 | 0.05 |
| POST-EMERGENCE | | | | |
| Bush bean | 5C,9G,6Y | 5C,9G,6Y | 9C | 4C,9G,6Y |
| Cotton | 3C,5G | 2C,4G | 1C,3G | 1C,6G |
| Morningglory | 3C,9G | 3C,8H | 2C,8G | 5C,8H |
| Cocklebur | 1C,9G | 4C,9G | 8H | 5C,9G |
| Sicklepod | 3C,5G | 5C,9G | 3C,9G | 4C,9H |
| Nutsedge | 2C,9G | 2C,9G | 9G | 9G |
| Crabgrass | 6G | 3C,8G | 9G | 5C,9G |
| Barnyardgrass | 5C,9H | 5C,9H | 3C,9H | 5C,9H |
| Wild Oats | 2C,9G | 3C,9G | 3C,9G | 4C,9H |
| Wheat | 2C,9G | 9G | 2C,9G | 4C,9G |
| Corn | 2C,8H | 7U,9C | 9C | 9C |
| Soybean | 2C,9G | 2C,8G,5X | 9C | 3C,8H |
| Rice | 5C,9G | 5C,9G | 5C,9G | 5C,9G |
| Sorghum | 2C,9H | 3C,9G | 3C,9G | 3C,9G |
| Sugar beet | 2C,7G | 5C,9H | 4C,9G | 4C,9G |
| PRE-EMERGENCE | | | | |
| Morningglory | 9G | 8H,2C | 9G | 9G |
| Cocklebur | 9H | 9H | 9H | 9H |
| Sicklepod | 5G | 1C | 6G | 5G |
| Nutsedge | 2C,6G | 10E | 10E | 10E |
| Crabgrass | 1C | 1C,4G | 2C,8G | 2C,5G |
| Barnyardgrass | 5C,9H | 5C,9H | 2C,5G | 4C,9H |
| Wild Oats | 2C,8G | 2C,7G | 3C,8G | 4C,8H |
| Wheat | 2C,8G | 9H | 9H | 4C,9H |
| Corn | 1C,7G | 3C,9G | 5C,9H | 9H |
| Soybean | 0 | 0 | 3C,5H | 2C,2H |
| Rice | 4C,9H | 5G | 10E | 9H |
| Sorghum | 2C,8G | 3C,9H | 7C,9H | 5C,9H |
| Sugar beet | 8G | 0 | 10E | 10E |

Table A (continued)

| | Cmpd. 24 | Cmpd. 25 | Cmpd. 26 | Cmpd. 27 |
|---|---|---|---|---|
| Rate kg/ha | 0.05 | 0.05 | 0.05 | 0.05 |
| POST-EMERGENCE | | | | |
| Bush bean | 6C,9G,6Y | 5C,6G,6Y | 5C,9G,6Y | 4C,9G,6Y |
| Cotton | 4C,7G | 2C,3G | 4C,5G | 2C |
| Morningglory | 4C,9G | 4C,8H | 5C,8G | 2C,2H |
| Cocklebur | 4C,9G | 2C,5G | 5C,9G | 3C,8H |
| Sicklepod | 3C | 2C | 3C | 2C |
| Nutsedge | 0 | 0 | 9G | 0 |
| Crabgrass | 3C,7G | 0 | 2C,8G | 0 |
| Barnyardgrass | 9C | 2C,9H | 9C | 2C,4H |
| Wild Oats | 7G | 3G | 9G | 0 |
| Wheat | 5G,5X | 3G,5X | 2C,9G | 0 |
| Corn | 7U,9C | 2C,7H | 5U,9C | 0 |
| Soybean | 5C,9G | 1C,1H | 4C,8G | 4G |
| Rice | 6C,9G | 2C,8G | 6C,9G | 4G |
| Sorghum | 4C,9G | 9G | 3U,9G | 2C,7H |
| Sugar beet | 3C,7H | 1C,4H | 9C | 0 |
| PRE-EMERGENCE | | | | |
| Morningglory | 9G | 2C,5H | 9C | 2C,4G |
| Cocklebur | 9H | 7H | 8H | 8H |
| Sicklepod | 8G | 2C | 8H | 1C |
| Nutsedge | 5G | 0 | 10E | 0 |
| Crabgrass | 2G | 0 | 2C,6G | 1C |
| Barnyardgrass | 2C,8G | 2C,5G | 4C,9H | 3C,5G |
| Wild Oats | 8G | 1C,5G | 4C,9H | 2C,5G |
| Wheat | 7G | 1C,5G | 9H | 5G |
| Corn | 9G | 2C,8H | 4C,9H | 2C,4G |
| Soybean | 3C,8H | 2G | 5G | 0 |
| Rice | 10E | 1C,4G | 10E | 3C |
| Sorghum | 3C,9H | 4C,9H | 10H | 2C,5G |
| Sugar beet | 5G | 0 | 5C,9G | 3C,6G |

Table A (continued)

| | Cmpd. 28 | Cmpd. 29 | Cmpd. 30 |
|---|---|---|---|
| Rate kg/ha | 0.05 | 0.05 | 0.05 |
| POST-EMERGENCE | | | |
| Bush bean | 1C | 9C | 9C |
| Cotton | 0 | 5C,9G | 3C,5G |
| Morningglory | 0 | 9C | 3C,5G |
| Cocklebur | 3H | 10C | 9H |
| Sicklepod | 0 | 4C,7G | 1C,3G |
| Nutsedge | 0 | 5C,9G | 3C,7G |
| Crabgrass | 0 | 3C,9G | 2C,5G |
| Barnyardgrass | 2H | 9C | 9C |
| Wild Oats | 0 | 5C,9G | 3C,9G |
| Wheat | 0 | 4C,9G | 2C,9G |
| Corn | 0 | 10C | 9C |
| Soybean | 0 | 4C,9H | 2C,8H |
| Rice | 0 | 5C,9G | 5C,9G |
| Sorghum | 6G | 10C | 10C |
| Sugar beet | — | — | — |
| PRE-EMERGENCE | | | |
| Morningglory | 0 | 3C,9H | 5G |
| Cocklebur | 8H | 9H | — |
| Sicklepod | 2H | 3C,8G | 2C,7G |
| Nutsedge | 0 | 2C,9G | 2C |
| Crabgrass | 2G | 3C,8H | 2C,5G |
| Barnyardgrass | 2G | 5C,9H | 5C,9H |
| Wild Oats | 0 | 5C,9G | 5C,9H |
| Wheat | 0 | 9H | 9H |
| Corn | 2G | 9G | 9H |
| Soybean | 0 | 3C,8H | 3C,6G |
| Rice | 5G | 10E | 5C,9G |
| Sorghum | 2G | 5C,9H | 5C,9H |
| Sugar beet | — | — | — |

Table A (continued)

| | Cmpd. 31 | Cmpd. 32 | Cmpd. 33 | Cmpd. 34 |
|---|---|---|---|---|
| Rate kg/ha | 0.05 | 0.05 | 0.05 | 0.05 |
| POST-EMERGENCE | | | | |
| Bush bean | 9C | 9C | 5C,9G,6Y | 9C |
| Cotton | 5C,9G | 3C,9G | 4C,9G | 4C,9G |
| Morningglory | 5C,9G | 2C,8G | 3C,5G | 9C |
| Cocklebur | 10C | 9G | 3C,9G | 2C,9G |
| Sicklepod | 9C | 4C | 2C | 2C,7G |
| Nutsedge | 2C,9G | 5G | 2C,9G | 9G |
| Crabgrass | 6C,9G | 6G | 1C,5G | 9G |
| Barnyardgrass | 9C | 6C,9H | 5C,9H | 9C |
| Wild Oats | 9C | 2C,9G | 1C,8G | 3C,9G |
| Wheat | 9C | 9G | 4C,9H | 3G |
| Corn | 9C | 5C,9G | 3C,9H | 9C |
| Soybean | 52C,9G | 4C,9G | 3C,9G,7X | 5C,9G |
| Rice | 9C | 5C,9G | 7C,9G | 5C,9G |
| Sorghum | 9C | 3C,9G | 4C,9G | 2C,9G |
| Sugar beet | — | — | 2C,7G | — |
| PRE-EMERGENCE | | | | |
| Morningglory | 9G | 8G | 1C,4H | 9G |
| Cocklebur | 9H | — | 9H | 9H |
| Sicklepod | 9G | 8G | 0 | 7G |
| Nutsedge | 10E | 10E | 2C,8G | 10E |
| Crabgrass | 3C,9G | 4C,6G | 1C,3G | 3C,9G |
| Barnyardgrass | 3C,9H | 5C,9H | 3C,9H | 2C,9H |
| Wild Oats | 3C,9H | 2C,8G | 2C,8H | 2C,7G |
| Wheat | 5C,9H | 2C,8H | 3C,9H | 1C,5G |
| Corn | 9H | 2C,9G | 3C,9H | 2C,9G |
| Soybean | 9H | 9H | 1C,1H | 9H |
| Rice | 10E | 10E | 3C,8H | 10E |
| Sorghum | 3C,9H | 5C,9H | 3C,9H | 9G |
| Sugar beet | — | — | 8H | — |

Table A (continued)

| | Cmpd. 35 | Cmpd. 36 | Cmpd. 37 | Cmpd. 38 |
|---|---|---|---|---|
| Rate kg/ha | 0.05 | 0.05 | 0.05 | 0.05 |
| POST-EMERGENCE | | | | |
| Bush bean | 3C,9G,6Y | 4S,8G,6Y | 5C,9G,6Y | 5C,9G,6Y |
| Cotton | 3C,3H,9G | 3C,8G | 4C,9G | 3C,3H,6G |
| Morningglory | 3C,7H | 3C,7H | 5C,9G | 3C,8H |
| Cocklebur | 2C,2H | 2C,3G | 5C,9G | 3C,6G |
| Sicklepod | 2C | 2C | 4C,8G | 2C,5G |
| Nutsedge | 0 | 0 | 3G | 0 |
| Crabgrass | 1C,3G | 0 | 2C,5G | 1C |
| Barnyardgrass | 1C,3G | 1C | 9C | 5C,9H |
| Wild Oats | 5G | 2G | 1C | 0 |
| Wheat | 0 | 0 | 3G | 0 |
| Corn | 3C,9H | 2C,8H | 8U,9C | 8U,10C |
| Soybean | 3C,7G,5X | 1C,3G | 4C,9G | 2C,5H |
| Rice | 2C,7G | 2C,9G | 3C,8G | 2C,6G |
| Sorghum | 2C,9G | 2C,9H | 2C,9G | 2C,9G |
| Sugar beet | — | — | — | — |
| PRE-EMERGENCE | | | | |
| Morningglory | 0 | 0 | 8G | 7G |
| Cocklebur | 0 | 0 | 7H | 0 |
| Sicklepod | 1C | 0 | 5G | 0 |
| Nutsedge | 0 | 0 | 0 | 0 |
| Crabgrass | 4G,2C | 1C | 1C,3G | 0 |
| Barnyardgrass | 2C | 1C | 9G,4C | 2C,8G |
| Wild Oats | 1C,3G | 1C | 1C,5G | 4G |
| Wheat | 0 | 0 | 4G | 2G |
| Corn | 2C,9G | 2C,9H | 2C,9G | 4C,9G |
| Soybean | 1C | 2G | 1C,2G | 1C |
| Rice | 1C,3G | 2G | 4C,7H | 3C,7H |
| Sorghum | 2C,9H | 2C | 5C,9G | 3C,9G |
| Sugar beet | — | — | — | — |

Table A (continued)

| | Cmpd. 39 | Cmpd. 40 | Cmpd. 41 | Cmpd. 42 |
|---|---|---|---|---|
| Rate kg/ha | 0.05 | 0.05 | 0.05 | 0.05 |
| POST-EMERGENCE | | | | |
| Bush bean | 5S,8G,6Y | 3C,9G,6Y | 10D,9G,6Y | 9D,9G,6Y |
| Cotton | 2C | 3C,2H | 4C,8G | 5C,9G |
| Morningglory | 2C,5G | 2C,5G | 9C | 3C,8H |
| Cocklebur | 2C | 3C,5H | 9C | 6C,9G |
| Sicklepod | 3C,5G | 2G | 3C | 3C,6H |
| Nutsedge | 4G | 3G | 1C,9G | 9G |
| Crabgrass | 3C,5G | 1C,5G | 2C,6G | 2C,8G |
| Barnyardgrass | 4C,9H | 9H | 3C,9H | 4C,9H |
| Wild Oats | 0 | 2G | 9G | 2C,9G |
| Wheat | 0 | 4G | 1C,9G | 3C |
| Corn | 3U,9C | 2C,8H | 3C,8H | 3C,9H |
| Soybean | 2C,5H | 0 | 5C,9G | 9C |
| Rice | 2C,8G | 2C,9G | 5C,9G | 6C,9G |
| Sorghum | 2C,9G | 9G | 9G | 2C,9G |
| Sugar beet | — | — | — | — |
| PRE-EMERGENCE | | | | |
| Morningglory | 9G | 1H | 8G | 9G |
| Cocklebur | 0 | 8H | 8H | 8H |
| Sicklepod | 0 | 0 | 9C | 9G |
| Nutsedge | 0 | 0 | 7G | 9G |
| Crabgrass | 1C | 1C | 5C,8G | 4C,8G |
| Barnyardgrass | 2C,7G | 3C | 3C,8G | 5C,9H |
| Wild Oats | 1C | 1C | 6G | 2C,6G |
| Wheat | 3G | 6G | 8G | 2C,9H |
| Corn | 2C,9G | 2C,4G | 2C,7G | 8G |
| Soybean | 1H | 0 | 6H | 6H |
| Rice | 3C,6G | 3C,8H | 8H | 9H |
| Sorghum | 5C,9H | 3C,9H | 2C,9G | 5C,9H |
| Sugar beet | — | — | — | — |

Table A (continued)

| | Cmpd. 43 | Cmpd. 44 | Cmpd. 45 | Cmpd. 46 |
|---|---|---|---|---|
| Rate kg/ha | 0.05 | 0.05 | 0.05 | 0.05 |
| POST-EMERGENCE | | | | |
| Bush bean | 5C,9G,6Y | 3C,9G,6Y | 9C | 5C,9G,6Y |
| Cotton | 3C,7G | 2C,4G | — | — |
| Morningglory | 1H | 1C | 10C | 10C |
| Cocklebur | 5C,9G | 4C,9G | 10C | 10C |
| Sicklepod | 5G | 3C,4H | 9C | 4C,8H |
| Nutsedge | 0 | 2C,8G | 2C | 3C,8G |
| Crabgrass | 2C,6G | 3G | 9C | 4C,9G |
| Barnyardgrass | 2C,8H | 1H | 9C | 5C,9G |
| Wild Oats | 2C,5G | 4G | 9C | 4C,9H |
| Wheat | 1C,5G | 5G | 6C | 2C,7G |
| Corn | 1U,9G | 1C,9G | 5C | 9G |
| Soybean | 4G | 2C,9G | 10C | 3C,8H |
| Rice | 3C,9G | 2C,8G | 6C | 6C,9G |
| Sorghum | 9G | 2C,9H | 6C | 4C,9G |
| Sugar beet | — | — | — | — |
| PRE-EMERGENCE | | | | |
| Morningglory | 5G | 0 | 10C | 8H |
| Cocklebur | 9H | 8H | 9H | 9H |
| Sicklepod | 7G | 6G | 4C,9G | 5H |
| Nutsedge | 5G | 0 | 10E | 10E |
| Crabgrass | 2C,8G | 1C | 5C,9G | 3C,5G |
| Barnyardgrass | 2C,9H | 2C | 5C,9H | 4C,9H |
| Wild Oats | 0 | 2C | 4C,8H | 3C,8H |
| Wheat | 8G | 5G | 3C,9H | 9H |
| Corn | 3C,7G | 2C,8G | 3C,9H | 4C,9H |
| Soybean | 0 | 2H | 8H | 1H |
| Rice | 9H | 5G | 10E | 4C,7H |
| Sorghum | 2C,9H | 2C,6G | 10H | 5C,9H |
| Sugar beet | — | — | — | — |

Table A (continued)

| | Cmpd. 47 |
|---|---|
| Rate kg/ha | 0.05 |
| POST-EMERGENCE | |
| Bush bean | 5C,9G,6Y |
| Cotton | — |
| Morningglory | 5C,9H |
| Cocklebur | 5C,9H |
| Sicklepod | 3C |
| Nutsedge | 3C,9G |
| Crabgrass | 4C,9G |
| Barnyardgrass | 5C,9H |
| Wild Oats | 2C,9H |
| Wheat | 4C,9G |
| Corn | 2C,9H |
| Soybean | 5C,9G |
| Rice | 5C,9G |
| Sorghum | 3C,9G |
| Sugar beet | — |
| PRE-EMERGENCE | |
| Morningglory | 8H |
| Cocklebur | 9H |
| Sicklepod | 2C |
| Nutsedge | 10E |
| Crabgrass | 4C,7G |
| Barnyardgrass | 5C,9H |
| Wild Oats | 3C,8G |
| Wheat | 2C,9H |
| Corn | 3C,9G |
| Soybean | 2H |
| Rice | 3C,6H |
| Sorghum | 3C,9H |
| Sugar beet | — |

*Test B*

Two plastic bulb pans were filled with fertilized and limed Fallsington silt loam soil. One pan was planted with corn, sorghum, Kentucky bluegrass and several grass weeds. The other pan was planted with cotton, soybeans, purple nutsedge (*Cyperus rotundus*), and several broadleaf weeds. The following grass and broadleaf weeds were planted: crabgrass (*Digitaria sanguinalis*), barnyardgrass (*Echinochloa crusgalli*), wild oats (*Avena fatua*), johnsongrass (*Sorghum halepense*), dallisgrass (*Paspalum dilatatum*), giant foxtail (*Setaria faberii*), cheatgrass (*Bromus secalinus*), mustard (*Brassica arvensis*), cocklebur (*Xanthium pensylvanicum*), pigweed (*Amaranthus retroflexus*), morningglory (*Ipomoea hederacea*), sicklepod (*Cassia obtusifolia*), teaweed (*Sida spinosa*), velvetleaf (*Abutilon theophrasti*), and jimsonweed (*Datura stramonium*). A 12.5 cm diameter plastic pot was also filled with prepared soil and planted with rice and wheat. Another 12.5 cm pot was planted with sugar beets. The above four containers were treated pre-emergence with several test compounds within the scope of the invention.

Twenty-eight days after treatment, the plants were evaluated and visually rated for response to the chemical treatments utilizing the rating system described previously for Test A. The data are summarized in Table B. At least one compound exhibits utility for pre-emergence weed control in soybeans and cotton.

TABLE B

Pre-Emergence on Fallsington Silt Loam

| | Compound 2 | | Compound 3 | |
|---|---|---|---|---|
| Rate kg/ha | 0.03 | 0.12 | 0.03 | 0.12 |
| Crabgrass | 0 | 0 | 6G,3C | 9G,9C |
| Barnyardgrass | 3G | 6G,3H | 8G,3H | 10C |
| Sorghum | 10C | 10C | 10E | 10E |
| Wild Oats | 5G | 7G | 8G,8C | 8G,8C |
| Johnsongrass | 8G,7C | 9G,9C | 9G,8C | 10C |
| Dallisgrass | 4G | 6G,3H | 8G,3H | 10C |
| Giant foxtail | 0 | 8G,5H | 7G,3H | 10C |
| Ky. bluegrass | 6G | 8G,8C | 10C | 10C |
| Cheatgrass | 9G,9C | 9G,9C | 8G,8C | 10C |
| Sugar beets | 0 | 3G | 8G,8C | 10C |
| Corn | 3G | 8G,5H | 4G | 10C |
| Mustard | 7G,3H | 9G,8C | 10C | 10C |
| Cocklebur | 5G | 5G | 9G,8C | 9G,9C |
| Pigweed | 0 | 5G,5C | 10C | 10C |
| Nutsedge | 0 | 3G | 7G | 10E |
| Cotton | 0 | 2G | 5H,3H | 7G,5H |
| Morningglory | 0 | 4G | 2G | 3G |
| Sicklepod | 0 | 4G | 3G | 3G |
| Teaweed | 0 | 0 | 4G,3H | 6G,5H |
| Velvetleaf | 0 | 0 | 3G,5H | 7G,5H |
| Jimsonweed | 0 | 0 | 3G | 7G |
| Soybean | 0 | 0 | 3G,3H | 6G,5H |
| Rice | 7G,4C | 10C | 9G,9C | 10C |
| Wheat | 5G | 7G,3C | 8G,5C | 10C |

TABLE B (continued)

Pre-Emergence on Fallsington Silt Loam

| | Compound 4 | |
|---|---|---|
| Rate kg/ha | 0.03 | 0.12 |
| Crabgrass | 0 | 0 |
| Barnyardgrass | 0 | 0 |
| Sorghum | 3G | 9G,9C |
| Wild Oats | 0 | 6G |
| Johnsongrass | 0 | 7G,3H |
| Dallisgrass | 0 | 3G |
| Giant foxtail | 0 | 3G |
| Ky. bluegrass | 4G | 6G,3C |
| Cheatgrass | 0 | 0 |
| Sugar beets | 0 | 0 |
| Corn | 0 | 0 |
| Mustard | 0 | 6G,3H |
| Cocklebur | 0 | 0 |
| Pigweed | 5G,5C | 10C |
| Nutsedge | 0 | 0 |
| Cotton | 0 | 0 |
| Morningglory | 0 | 2G |
| Sicklepod | 0 | 0 |
| Teaweed | 0 | 0 |
| Velvetleaf | 0 | 0 |
| Jimsonweed | 0 | 0 |
| Soybean | 0 | 0 |
| Rice | 4G | 6G,3H |
| Wheat | 0 | 6G,3C |

TABLE B (continued)

Pre-Emergence on Fallsington Silt Loam

| | Compound 20 | | Compound 21 | |
|---|---|---|---|---|
| Rate kg/ha | 0.25 | 0.063 | 0.125 | 0.031 |
| Crabgrass | 9G | 6G | 2G | 0 |
| Barnyardgrass | 9G,9C | 8G | 3G | 0 |
| Sorghum | 9G,9C | 8G | 10C | 4G |
| Wild Oats | 8G | 6G | 8G | 4G |
| Johnsongrass | 8G | 6G | 8G | 2G |
| Dallisgrass | 9G | 6G | 8G | 0 |
| Giant foxtail | 9G | 2G | 0 | 2G |
| Ky. bluegrass | 9C,9G | 8G | 9G | 0 |
| Cheatgrass | 9G | 7G | 8G | 4G |
| Sugar beets | 8G | 6G | 3G | 2G |
| Corn | 8G,7H | 5G | 6G,5H | 0 |
| Mustard | 9G | 9G | 9G | 7G |
| Cocklebur | — | — | 2G | 0 |
| Pigweed | — | — | — | — |
| Nutsedge | 9G | 4G | 7G | 2G |
| Cotton | 5G | 2G | 2G | 0 |
| Morningglory | 8G | 5G | 0 | 0 |
| Sicklepod | 5G | 2G | 5G | 2G |
| Teaweed | 0 | 0 | 0 | 0 |
| Velvetleaf | 7G | 2G | 0 | 0 |
| Jimsonweed | 3G | 0 | 0 | 0 |
| Soybean | 3G,3H | 0 | 0 | 0 |
| Rice | 10C | 8G | 7G | 4G |
| Wheat | 8G | 7G | 5G | 2G |

TABLE B (continued)

Pre-Emergence on Fallsington Silt Loam

| | Compound 22 | | Compound 23 | | |
|---|---|---|---|---|---|
| Rate kg/ha | 0.125 | 0.125 | 0.062 | 0.031 | 0.015 |
| Crabgrass | 9G | 7G | 3G | 0 | 0 |
| Barnyardgrass | 5G | 7G | 2G | 2G | 0 |
| Sorghum | 10C | 10C | 10C | 10C | 9G |
| Wild Oats | 8G | 9G | 7G | 5G | 4G |
| Johnsongrass | 8G | 8G | 8G | 7G | 7G |
| Dallisgrass | 9G | 9G,9C | 8G | 5G | 3G |
| Giant foxtail | 2G | 9G,9C | 5G | 0 | 0 |
| Ky. bluegrass | 9G | 9G,9C | 8G | 3G | 3G |
| Cheatgrass | 9G | 9G | 8G | 6G | 4G |
| Sugar beets | 8G | 6G | 5G | 5G | 4G |
| Corn | 9G,9C | 8G,7H | 6G,5H | 2G | 2G |
| Mustard | 9G,9C | 9G | 9G | 7G | 5G |
| Cocklebur | 5G | 6G | 3G | 3G | 2G |
| Pigweed | — | — | — | — | — |
| Nutsedge | 10C | 10C | 10C | 9G | 8G |
| Cotton | 6G | 6G | 2G | 2G | 2G |
| Morningglory | 6G | 4G | 3G | 2G | 0 |
| Sicklepod | 2G | 0 | 0 | 2G | 0 |
| Teaweed | 0 | 2G | 0 | 0 | 0 |
| Velvetleaf | 4G | 0 | 0 | 0 | 0 |
| Jimsonweed | 8G | 0 | 0 | 0 | 0 |
| Soybean | 7G,7H | 2G | 2G | 0 | 0 |
| Rice | 10C | 10C | 9G | 6G | 6G |
| Wheat | 9G | 9G | 7G | 4G | 3G |

TABLE B (continued)

Pre-Emergence on Fallsington Silt Loam

| | Compound 24 | | Compound 26 | |
|---|---|---|---|---|
| Rate kg/ha | 0.125 | 0.031 | 0.062 | 0.031 |
| Crabgrass | 0 | 0 | 8G | 5G |
| Barnyardgrass | 0 | 0 | 9G | 5G |
| Sorghum | 3G | 2G | 9G,9C | 9G |
| Wild Oats | 0 | 0 | 8G | 5G |
| Johnsongrass | 2G | 0 | 9G,9C | 7G |
| Dallisgrass | 0 | 0 | 9G | 8G |
| Giant foxtail | 0 | 0 | 9G,9C | 5G |
| Ky. bluegrass | 0 | 0 | 9G,9C | 9G |
| Cheatgrass | 0 | 0 | 9G,9C | 8G |
| Sugar beets | 2G | 0 | 9G | 5G |
| Corn | 0 | 0 | 9G | 9G |
| Mustard | 0 | 0 | 9G,9C | 9G |
| Cocklebur | 0 | 0 | 4G | 0 |
| Pigweed | — | — | — | — |
| Nutsedge | 0 | 0 | 10C | 9G |
| Cotton | 0 | 0 | 4G | 0 |
| Morningglory | 0 | 0 | 7G | 4G |
| Sicklepod | 0 | 0 | 4G | 2G |
| Teaweed | 0 | 0 | 5G | 0 |
| Velvetleaf | 0 | 0 | 5G | 3G |
| Jimsonweed | 0 | 0 | 5G | 0 |
| Soybean | 0 | 0 | 4G | 2G |
| Rice | 2G | 0 | 10E | 10E |
| Wheat | 0 | 0 | 5G | 3G |

TABLE B (continued)

Pre-Emergence on Fallsington Silt Loam

| | Compound 29 | | Compound 30 | |
|---|---|---|---|---|
| Rate kg/ha | 0.125 | 0.031 | 0.125 | 0.031 |
| Crabgrass | 7G,2H | 7G | 4G | 3G |
| Barnyardgrass | 9G,9C | 9G,5H | 9G,5H | 7G,3H |
| Sorghum | 10E | 10E | 10E | 10C |
| Wild Oats | 7G | 7G | 7G | 7G |
| Johnsongrass | 9G,9C | 8G,7C | 8G,3H | 6G,3H |
| Dallisgrass | 10C | 8G,5H | 8G,3H | 7G,3H |
| Giant foxtail | 10C | 9G,3H | 9G,9C | 8G,5H |
| Ky. bluegrass | 10C | 10C | 10C | 7G,3H |
| Cheatgrass | 8G,8C | 7G | 8G,9C | 6G |
| Sugar beets | 10C | 7G,5H | 7G,5H | 4G |
| Corn | 10C | 3H,8G | 9G,9C | 5H,7G |
| Mustard | 10C | 9G,9C | 9G,9C | 10C |
| Cocklebur | 8G,8C | 7G,5H | 2G | 0 |
| Pigweed | — | — | — | — |
| Nutsedge | 10E | 9G | 10E | 7G |
| Cotton | 8G,5H | 6G,3H | 4G | 0 |
| Morningglory | 9G,7C | 6G,5H | 8G,3H | 5G |
| Sicklepod | 7G,4C | 3G | 7G | 4G |
| Teaweed | 7G,3H | 4G | 8G,3H | 3G |
| Velvetleaf | 8G,9C | 3G | 4G,3H | 3G |
| Jimsonweed | 9G,8C | 3G | 5G,5C | 3G |
| Soybean | 6G,5H | 2C | 2G | 0 |
| Rice | 10E | 10E | 10C | 8G,5H |
| Wheat | 9G,9C | 7G | 7G | 5G |

TABLE B (continued)

Pre-Emergence on Fallsington Silt Loam

| | Compound 31 | | | | |
|---|---|---|---|---|---|
| Rate kg/ha | 0.125 | 0.008 | 0.031 | 0.003 | 0.0015 |
| Crabgrass | 9G,9C | 8G | 8G | 7G | 6G |
| Barnyardgrass | 9G,9C | 6G,3C | 7G | 3G | 2G |
| Sorghum | 10C | 10C | 10C | 10C | 10C |
| Wild Oats | 7G | 6G | 6G | 6G | 5G |
| Johnsongrass | 9G | 6G | 8G | 6G | 6G |
| Dallisgrass | 9G,9C | 8G | 9G,9C | 7G | 6G |
| Giant foxtail | 10C | 6G,2H | 9G | 6G | 6G |
| Ky. bluegrass | 10C | — | 10C | — | — |
| Cheatgrass | 10C | 9G,5C | 10C | 9G | 5C,8G |
| Sugar beets | 10C | 10C | 9G,9C | 9G | 8G,8C |
| Corn | 9G,9C | 9G,9C | 9G,9C | 7H | 5G |
| Mustard | 9G,9C | 9G,7C | 9G,9C | 9G | 9G |
| Cocklebur | 8C | 6G,2H | 7G | — | 5G,2H |
| Pigweed | — | — | — | — | — |
| Nutsedge | 10C | 10C | 10C | 10C | 10C |
| Cotton | 8G,8C | 8G | 7G | 8G | 6G,3H |
| Morningglory | 7G | 3G,2C | 2G | 3C | 0 |
| Sicklepod | 8G,8C | 8G,5C | 7G,7C | 3G | 7G |
| Teaweed | 8G | 8G,3C | 8G | 8G | 7G |
| Velvetleaf | 10C | 8G | 9G | 7G | 6G |
| Jimsonweed | 9G,9C | 7G,7H | 8G,8C | 4G | 3G |
| Soybean | 8G,8C | 8G,7H | 6G,5C | 7G | 6G,2C |
| Rice | 10C | 10C | 10C | 10C | 10C |
| Wheat | 8G | 9G | 3G | 7G | 6G |

TABLE B (continued)

Pre-Emergence on Fallsington Silt Loam

| | Compound 32 | |
|---|---|---|
| Rate kg/ha | 0.125 | 0.031 |
| Crabgrass | 7G | 6G |
| Barnyardgrass | 5H,7G | 3H,6G |
| Sorghum | 10C | 10C |
| Wild Oats | 6G | 2G |
| Johnsongrass | 7G | 5G |
| Dallisgrass | 8G | 8G |
| Giant foxtail | 5G | 4G |
| Ky. bluegrass | 9G,9C | 9G,9C |
| Cheatgrass | 10C | 10C |
| Sugar beets | 9G,9C | 7G |
| Corn | 8G | 5G |
| Mustard | 8G | 6G |
| Cocklebur | 6G | 3G |
| Pigweed | — | — |
| Nutsedge | 5G | 3G |
| Cotton | 6G,3C | 3G |
| Morningglory | 6G | 0 |
| Sicklepod | 8G,8C | 7G,7C |
| Teaweed | 8G | 7G |
| Velvetleaf | 7G | 10C |
| Jimsonweed | 5G | 5G |
| Soybean | 6G,3C | 4G |
| Rice | 10C | 10C |
| Wheat | 6G | 2G |

TABLE B (continued)

Pre-Emergence on Fallsington Silt Loam

| | Compound 34 | | | | |
|---|---|---|---|---|---|
| Rate kg/ha | 0.125 | 0.008 | 0.031 | 0.003 | 0.0015 |
| Crabgrass | 9G | 6G | 8G | 2G | 2G |
| Barnyardgrass | 8G,8C | 3G | 5H,7G | 2G | 2G |
| Sorghum | 10C | 8G | 10C | 4G | 2G |
| Wild Oats | 6G | 0 | 2G | 0 | 0 |
| Johnsongrass | 10C | 0 | 9G | 0 | 0 |
| Dallisgrass | 9G,9C | 7G | 9G | 7G | 0 |
| Giant foxtail | 10C | 2G | 8G | 2G | 0 |
| Ky. bluegrass | 9G,9C | 4G | 9G,9C | 2G | 0 |
| Cheatgrass | 9G,9C | 9G | 10C | 8G | 4G |
| Sugar beets | 10C | 9G | 10C | 8G | 7G |
| Corn | 8G | 7G,5H | 6G | 3G | 2G |
| Mustard | 9C | 9G | 9G | 9G | 8G |
| Cocklebur | 8G,8C | 0 | 6G | 3G | 0 |
| Pigweed | — | — | — | — | — |
| Nutsedge | 10C | 10C | 10C | 10C | 2G |
| Cotton | 8G,8C | 6G | 7G | 3G | 0 |
| Morningglory | 8G,8C | 7G | 4G | 3G | 0 |
| Sicklepod | 9G,9C | 7G | 8G,8C | 2G | 0 |
| Teaweed | 10C | 3G | 9G | 0 | 0 |
| Velvetleaf | 10C | 5G | 10C | 2G | 0 |
| Jimsonweed | 8G | 7G | 7G | 3G | 0 |
| Soybean | 8G,8C | 7G,5H | 7G,5C | 6G | 2G |
| Rice | 10C | 10C | 10C | 10C | 9G |
| Wheat | 5G | 4G | 0 | 2G | 0 |

TABLE B (continued)

Pre-Emergence on Fallsington Silt Loam

| | Compound 37 | |
|---|---|---|
| Rate kg/ha | 0.25 | 0.062 |
| Crabgrass | 2G | 0 |
| Barnyardgrass | 6G | 3G |
| Sorghum | 8G,8C | 8G,5H |
| Wild Oats | 0 | 0 |
| Johnsongrass | 5G | 4G |
| Dallisgrass | 0 | 0 |
| Giant foxtail | 3G | 0 |
| Ky. bluegrass | — | — |
| Cheatgrass | 3G | 0 |
| Sugar beets | 4G | 2G |
| Corn | 8G,8C | 5G |
| Mustard | 0 | 0 |
| Cocklebur | 0 | 0 |
| Pigweed | — | — |
| Nutsedge | 5G | 0 |
| Cotton | 0 | 0 |
| Morningglory | 4G | 0 |
| Sicklepod | 0 | 0 |
| Teaweed | 2G | 2G |
| Velvetleaf | 0 | 0 |
| Jimsonweed | 0 | 0 |
| Soybean | 2G | 2G |
| Rice | 6G | 3G |
| Wheat | 0 | 0 |

TABLE B (continued)

Pre-Emergence on Fallsington Silt Loam

| | Compound 38 | | Compound 41 | |
|---|---|---|---|---|
| Rate kg/ha | 0.25 | 0.062 | 0.125 | 0.031 |
| Crabgrass | 2G | 0 | 2G | 2G |
| Barnyardgrass | 5G | 0 | 0 | 0 |
| Sorghum | 8G,5H | 2G | 10C | 2G |
| Wild Oats | 0 | 0 | 2G | 0 |
| Johnsongrass | 0 | 0 | 2G | 0 |
| Dallisgrass | 0 | 0 | 0 | 0 |
| Giant foxtail | 0 | 0 | 0 | 0 |
| Ky. bluegrass | — | — | 4G | 0 |
| Cheatgrass | 0 | 0 | 2G | 0 |
| Sugar beets | 4G | 0 | 3G | 0 |
| Corn | 7G | 0 | 3G | 0 |
| Mustard | 0 | 0 | 8G | 7G |
| Cocklebur | 0 | 0 | 2G | 0 |
| Pigweed | — | — | — | — |
| Nutsedge | 0 | 0 | 0 | 0 |
| Cotton | 2G | 0 | 2G | 2G |
| Morningglory | 2G | 0 | 0 | 0 |
| Sicklepod | 0 | 0 | 0 | 0 |
| Teaweed | — | 2G | — | — |
| Velvetleaf | 7G | 0 | 0 | 2G |
| Jimsonweed | 0 | 0 | 0 | 0 |
| Soybean | 0 | 0 | 2G | 2G |
| Rice | 5G | 0 | 7G | 0 |
| Wheat | 0 | 0 | 0 | 0 |

TABLE B (continued)

Pre-Emergence on Fallsington Silt Loam

| | Compound 42 | | Compound 43 | |
|---|---|---|---|---|
| Rate kg/ha | 0.125 | 0.031 | 0.125 | 0.031 |
| Crabgrass | 3G | 2G | 5G | 2G |
| Barnyardgrass | 0 | 0 | 0 | 0 |
| Sorghum | 7G,3H | 2G | 0 | 0 |
| Wild Oats | 0 | 0 | 0 | 0 |
| Johnsongrass | 0 | 0 | 0 | 0 |
| Dallisgrass | 3G | 0 | 2G | 0 |
| Giant foxtail | 0 | 0 | 0 | 0 |
| Ky. bluegrass | 2G | 0 | 0 | 0 |
| Cheatgrass | 4G | 2G | 0 | 0 |
| Sugar beets | — | — | — | — |
| Corn | 2G | 0 | 0 | 0 |
| Mustard | 7G | 5G | 5G | 0 |
| Cocklebur | 2G | 0 | 0 | 0 |
| Pigweed | — | — | — | — |
| Nutsedge | 0 | 0 | 0 | 0 |
| Cotton | 5G | 2G | 3G | 0 |
| Morningglory | 2G | 0 | 0 | 0 |
| Sicklepod | 0 | 0 | 0 | 0 |
| Teaweed | — | — | — | — |
| Velvetleaf | 0 | 0 | 0 | 0 |
| Jimsonweed | 0 | 0 | 0 | 0 |
| Soybean | 0 | 0 | 0 | 0 |
| Rice | 5G | 2G | 3G | 0 |
| Wheat | 0 | 0 | 0 | 0 |

TABLE B (continued)

Pre-Emergence on Fallsington Silt Loam

| | Compound 45 | | |
|---|---|---|---|
| Rate kg/ha | 0.125 | 0.031 | 0.015 |
| Crabgrass | 9G,7C | 8G,7C | 7G,7C |
| Barnyardgrass | 9G,7C | 8G,7C | 5G,3C |
| Sorghum | 10C | 10C | 10C |
| Wild Oats | 7G,3C | 7G,3C | 5G,3C |
| Johnsongrass | 9G,5C | 8G,5C | 2G |
| Dallisgrass | 9G | 9G | 8G |
| Giant foxtail | 9G,9C | 9G,9C | 7G |
| Ky. bluegrass | — | — | — |
| Cheatgrass | 8G | 8G | 2G |
| Sugar beets | 9G,5CG | 8G,5C | 7G |
| Corn | 8G,8C | 7G,7H | 2G,2C |
| Mustard | 9G,9C | 8G,8C | 8G,8C |
| Cocklebur | 6G,3C | 2G | 2G |
| Pigweed | — | — | — |
| Nutsedge | 10C | 9G | 6G |
| Cotton | 4G,7H | 3G,5H | 3G |
| Morningglory | 7G | 5G,5H | 3G,3H |
| Sicklepod | — | 5G,5H | 3G,3H |
| Teaweed | — | — | — |
| Velvetleaf | — | 2G,2H | 2G |
| Jimsonweed | — | 4G | 2G |
| Soybean | 7H | 5G,5H | 5G,5H |
| Rice | 10C | 10C | 10C |
| Wheat | 7G | 3G | 0 |

TABLE B (continued)

Pre-Emergence on Fallsington Silt Loam

| | Compound 46 | | |
|---|---|---|---|
| Rate kg/ha | 0.125 | 0.031 | 0.015 |
| Crabgrass | 5G,3C | 2C,2G | 0 |
| Barnyardgrass | 6G,3C | 2G,2C | 0 |
| Sorghum | 9G,9C | 7G,5H | 3G,3H |
| Wild Oats | 6G | 3G | 2G |
| Johnsongrass | 8G,7C | 2G | 0 |
| Dallisgrass | 8G | 5G | 0 |
| Giant foxtail | 8G,8C | 2G,3C | 0 |
| Ky. bluegrass | — | — | — |
| Cheatgrass | 8G | 5G | 0 |
| Sugar beets | 8G | 4G,3H | 3G |
| Corn | 7G,7H | 3G,3C | 2G,2H |
| Mustard | 9G,8C | 7G | 6G |
| Cocklebur | 6G,7C | 0 | 0 |
| Pigweed | — | — | — |
| Nutsedge | — | 0 | 0 |
| Cotton | 7G,3C | 3G | 3G |
| Morningglory | 5G,3H | 0 | 0 |
| Sicklepod | 2G,3C | 0 | 0 |
| Teaweed | — | — | — |
| Velvetleaf | 5G,3H | 0 | 0 |
| Jimsonweed | 2G,2C | 0 | 0 |
| Soybean | 2G,3C | 0 | 0 |
| Rice | 10C | 4G | 2G |
| Wheat | 7G | 4G | 2G |

TABLE B (continued)

Pre-Emergence on Fallsington Silt Loam

| | Compound 47 | | |
|---|---|---|---|
| Rate kg/ha | 0.125 | 0.031 | 0.015 |
| Crabgrass | 4G | 2G | 0 |
| Barnyardgrass | 6G,5C | 4G,3C | 2G |
| Sorghum | 9G,9C | 9G,9C | 8G,8C |
| Wild Oats | 6G | 5G | 4G |
| Johnsongrass | 8G | 8G | 3G,3H |
| Dallisgrass | 7G | 5G | 0 |
| Giant foxtail | 8G,8C | 4G,3C | 0 |
| Ky. bluegrass | — | — | — |
| Cheatgrass | 8G | 6G | 0 |
| Sugar beets | 6G | 4G | 3G |
| Corn | 7G,5C | 2G,2C | 0 |
| Mustard | 9G,9C | 8G,5C | 7G |
| Cocklebur | 5G,5C | 3C,2G | 0 |
| Pigweed | — | — | — |
| Nutsedge | — | 2G | 2G |
| Cotton | 3G,3H | 2G | 2G |
| Morningglory | 5G | 3G | 0 |
| Sicklepod | 2G | 0 | 0 |
| Teaweed | — | — | — |
| Velvetleaf | 7G,5C | 0 | 0 |
| Jimsonweed | 2G,2C | 0 | 0 |
| Soybean | 2G,3C | 0 | 0 |
| Rice | 9G | 5G | 3G |
| Wheat | 5G | 4G | 4G |

Test C

Two ten-inch in diameter plastic pans lined with polyethylene liners were filled with prepared Fallsington silt loam soil. One pan was planted with seeds of wheat *(Triticum aestivum)*, barley *(Hordeum vulgare)*, wild oats *(Avena fatua)*, downy brome *(Bromus tectorum)*, cheatgrass *(Bromus secalinus)*, blackgrass *(Alopecurus myosuroides)*, annual bluegrass *(Poa annus)*, green foxtail *(Setaria viridis)*, quackgrass *(Agropyron repens)*, Italian ryegrass *(Lolium multiflorum)* and ripgut brome *(Bromus rigidus)*. The other pan was planted with seeds of Russian thistle *(Salsola kali)*, tansy mustard *(Descuraina pinnata)*, *Galium aparine*, tumble mustard *(Sisymbrium altissium)* kochia *(Kochia scoparia)*, shepherd's purse

*(Capsella bursapastoris), Matricaria inodora*, black nightshade *(Solanum nigrum)*, yellow rocket *(Barbarea vulgaris)*, wild mustard *(Brassica kaber)* and wild buckwheat *(Polygonum convolvulus)*. In certain tests, rapeseed was substituted for wild mustard and *Veronica persica* was included. The above two pans were treated pre-emergence. At the same time two pans in which the above plant species were growing were treated post-emergence. Plant height at the time of treatment ranged from 1—15 cm depending on plant species.

The test compounds were diluted with a non-phytotoxic solvent and sprayed over-the-top of the pans. An untreated control and a solvent alone control were included for comparison. All treatments were maintained in the greenhouse for 20 days at which time the treatments were compared to the controls and the effects visually rated. The recorded data are presented in Table C. Several of the compounds tested exhibit utility for post-emergence weed control in wheat.

TABLE C

Compound 1

| | Pre-Emergence | | Post-Emergence | |
|---|---|---|---|---|
| Rate kg/ha | 0.015 | 0.06 | 0.015 | 0.06 |
| wheat | 0 | 0 | 2G | 1C,3G |
| barley | 0 | 0 | 4G | 6G |
| wild oats | 0 | 0 | 2G | 1G |
| downy brome | 0 | 2G | 2G | 3G |
| cheatgrass | 0 | 1C,2G | 6G | 2C,8G |
| blackgrass | 0 | 0 | 3G | 2C,6G |
| annual bluegrass | 0 | 2G | 1C,4G | 8C,9G |
| green foxtail | 0 | 0 | 3G | 5G |
| quackgrass | 0 | 0 | 0 | 5G |
| Italian ryegrass | 0 | 1G | 3G | 9C,9G |
| ripgut brome | 0 | 0 | 2G | 2C,6G |
| Russian thistle | 0 | 0 | 3C,3G | 4G |
| tansy mustard | 0 | — | 9G | 5G |
| *Galium aparine* | 0 | 0 | 2G | 2G |
| tumble mustard | 0 | 0 | 5G | 10C |
| kochia | 0 | 0 | 2G | 4G |
| shepherd's purse | 0 | 0 | 10C | 2C,2G |
| *Matricaria inodora* | 0 | 0 | 3G | 8C,8G |
| black nightshade | 0 | 0 | 0 | 0 |
| yellow rocket | 0 | 0 | 0 | 0 |
| wild mustard | 0 | 0 | 10C | 9G |
| wild buckwheat | 0 | 0 | 1G | 0 |

TABLE C (continued)

| | Compound 34 | | | |
|---|---|---|---|---|
| | Pre-Emergence | | | |
| Rate kg/ha | 0.25 | 0.06 | 0.015 | 0.004 |
| wheat | 7G | 5G | 1G | 0 |
| barley | 8G | — | 3G | 0 |
| wild oats | 6G | 5G | 2G | 0 |
| downy brome | 2C,9G | 2C,8G | 7G | 5G |
| cheatgrass | 10E | 10C | 1C,8G | 5G |
| blackgrass | 4C,9G | 2C,8G | 3C,7G | 5G |
| annual bluegrass | 3C,9G | 2C,8G | 8G | 4G |
| green foxtail | 9G | 5C,8G | 2C,7G | 3C,6G |
| quackgrass | 2C,9G | 2C,8G | 8G | 5G |
| Italian ryegrass | 10E | 1C,8G | 7G | 1C,4G |
| ripgut brome | 3C,9G | 1C,8G | 7G | 4G |
| Russian thistle | 5C,7G | 2C,6G | 1C,3G | 1C,3G |
| tansy mustard | 9C,9G | 7C,9G | 2C,9G | 9G |
| *Galium aparine* | 3C,9G | 7G | 7G | 6G |
| tumble mustard | 10C | 5C,9G | 9G | 8G |
| kochia | 2C,9G | 9G | 8G | 6G |
| shepherd's purse | 10C | 10C | 2C,9G | 9G |
| *Matricaria inodora* | 9G | 9G | 8G | 7G |
| black nightshade | 3C,9G | 8G | 6G | 4G |
| yellow rocket | 3C,9G | 9G | 8G | 8G |
| rapeseed | 2C,9G | 9G | 1C,9G | 7G |
| wild buckwheat | 2C,8G | 8G | 5G | 2G |

TABLE C (continued)

Compound 34

Post-Emergence

| Rate kg/ha | 0.25 | 0.06 | 0.015 | 0.004 |
|---|---|---|---|---|
| wheat | 7G | 3G | 1G | 0 |
| barley | 8G | 5G | 1G | 0 |
| wild oats | 7G | 5G | 2G | 0 |
| downy brome | 3C,9G | 7G | 7G | 5G |
| cheatgrass | 2C,9G | 8G | 7G | 5G |
| blackgrass | 9C,9G | 5C,9G | 7C,8G | 1C,5G |
| annual bluegrass | 2C,8G | 3C,8G | 7G | 3G |
| green foxtail | 2C,8G | 2C,8G | 1C,6G | 3G |
| quackgrass | 1C,6G | 1C,7G | 6G | 3G |
| Italian ryegrass | 2C,7G | 8G | 5G | 2G |
| ripgut brome | 2C,8G | 7G | 5G | 5G |
| Russian thistle | 9C,9G | 10C | 7C,8G | 5C,7G |
| tansy mustard | 10C | 10C | 7C,8G | 5C,7G |
| *Galium aparine* | 2C,7G | 7G | 4G | — |
| tumble mustard | 10C | 10C | 10C | 5C,8G |
| kochia | 10C | 9C,9G | 2C,7G | 2C,2G |
| shepherd's purse | 10C | 10C | 9C,9G | 7C,9G |
| *Matricaria inodora* | 7C,9G | 7C,8G | 5C,9G | 3C,8G |
| black nightshade | 5C,8G | 2C,7G | 2C,7G | 2G |
| yellow rocket | 5C,9G | 1C,7G | 2G | 0 |
| rapeseed | 7C,8G | 5C,9G | 2C,7G | 6G |
| wild buckwheat | 10C | 6G | 3G | — |

TABLE C (continued)

| | Compound 42 | | | |
|---|---|---|---|---|
| | Pre-Emergence | | Post-Emergence | |
| Rate kg/ha | 0.25 | 0.06 | 0.25 | 0.06 |
| wheat | 8G | 0 | 7G | 2G |
| barley | 2G | 2G | 3C,5G | 5G |
| wild oats | 5G | 2G | 2C,4G | 2G |
| downy brome | 8G | 4G | 4G | 4G |
| cheatgrass | 3C,8G | 2G | 4G | 2G |
| blackgrass | 3C,8G | 2H | 8G | 2C,5G |
| annual bluegrass | 2C,8G | 4G | 4G | 2G |
| green foxtail | 2C,5G | 1C,2G | 2C,7G | 2C,4G |
| quackgrass | 5G | 2G | — | — |
| Italian ryegrass | 2C,8G | 1C,2G | 1C,2G | 5G |
| ripgut brome | 2C,7G | 2G | 5G | 2G |
| Russian thistle | 1C | 0 | 0 | 0 |
| tansy mustard | 9C | 9C | 2C | 1C,2G |
| *Galium aparine* | 7C | 3C | 3C | 3G |
| tumble mustard | 8G | 4G | 10C | 6G |
| kochia | 0 | 0 | — | — |
| shepherd's purse | 9G | 8G | 2C,2G | 5G |
| *Matricaria inodora* | 9G | 6G | 5G | 1C,3G |
| black nightshade | 5G | 0 | 3C,8G | 7G |
| yellow rocket | 9G | 8G | 7G | 2G |
| rapeseed | 9G | 7G | 8G | 3G |
| wild buckwheat | 2G | 0 | 5G | 0 |

TABLE C (continued)

| | Compound 45 | | | |
|---|---|---|---|---|
| | Pre-Emergence | | Post-Emergence | |
| Rate kg/ha | 0.015 | 0.004 | 0.015 | 0.004 |
| wheat | 4G | 1G | 6G | 4G |
| barley | 8G | 5G | 8G | 6G |
| wild oats | 8G | 5G | 3C,8G | 6G |
| downy brome | 9G | 7G | 3C,8G | 8G |
| cheatgrass | 2C,8G | 6G | 3C,8G | 7G |
| blackgrass | 3C,9G | 2C,8G | 10C | 3C,8G |
| annual bluegrass | 4C,9G | 2C,9G | 9C,9G | 3C,8G |
| green foxtail | 3C,9G | 7G | — | — |
| quackgrass | 9G | 7G | 6G | 8G |
| Italian ryegrass | 4C,9G | 7G | 3C,8G | 7G |
| ripgut brome | 2C,8G | 6G | 2C,8G | 6G |
| Russian thistle | 3G | 0 | 0 | 0 |
| tansy mustard | — | — | 10C | 10C |
| *Galium aparine* | 5G | 5G | 6G | 3G |
| tumble mustard | 10C | 9G | 10C | 10C |
| kochia | 8G | 0 | 0 | — |
| shepherd's purse | 10C | 9G | 10C | 8G |
| *Matricaria inodora* | 9G | 8G | 2C,6G | 3G |
| black nightshade | 9G | 5G | 2C,8G | 6G |
| yellow rocket | 9G | 8G | 8G | 4G |
| rapeseed | 9G | 8G | 6G | 5G |
| wild buckwheat | 7G | 5G | 10C | 2G |
| *Veronica persica* | — | — | 6G | 4G |

*Test D*

The test chemicals, dissolved in a non-phytotoxic solvent, were applied in an overall spray to the foliage and surrounding soil of selected plant species. One day after treatment, plants were observed for rapid burn injury. Approximately fourteen days after treatment, all species were visually compared to untreated controls and rated for response to treatment. The rating system was as described previously for Test A. The data are presented in Table D.

All plant species were seeded in Woodstown sandy loam soil and grown in a greenhouse. The following species were grown in soil contained in plastic pots (25 cm diameter by 13 cm deep): soybeans, cotton, alfalfa, corn, rice, wheat, sorghum, velvetleaf *(Abutilon theophrasti)*, sesbania *(Sesbania exaltata)*, sicklepod *(Cassia obtusifolia)*, morningglory *(Ipomoea hederacea)*, jimsonweed *(Datura stramonium)*,

cocklebur *(Xanthium pensylvanicum),* crabgrass *(Digitaria* sp.), nutsedge *(Cyperus rotundus),* barnyardgrass *(Echinochloa crusgalli),* giant foxtail *(Setaria faberii)* and wild oats *(Avena fatua).* The following species were grown in soil in a paper cup (12 cm diameter by 13 cm deep): sunflower, sugar beets, and mustard. All plants were sprayed approximately 14 days after planting. Additional plant species such as johnsongrass, rape and bindweed are sometimes added to this standard test in order to evaluate unusual selectivity.

Several of the compounds tested by this procedure exhibit post-emergence control of weeds in wheat, cotton and soybeans.

TABLE D

| | Compound 29 | |
|---|---|---|
| Rate kg/ha | 0.015 | 0.003 |
| Soybeans | 10G | 7G,4C |
| Velvetleaf | 7G,6C | 0 |
| Sesbania | 9G | 2G |
| Sicklepod | 6G,3C | 5G |
| Cotton | 9G | 8G |
| Morningglory | 0 | 6G |
| Alfalfa | 8G | 9G |
| Jimsonweed | 8G | 0 |
| Cocklebur | 8G,3C | 3C |
| Corn | 9G,8C | 9G,8C |
| Crabgrass | 7G | 0 |
| Rice | 9G,6C | 3C,9G |
| Nutsedge | 0 | 0 |
| Barnyardgrass | 8G,6C | 8G |
| Wheat | 5G | 3G |
| Giant foxtail | 9G,9C | 9G |
| Wild Oats | 7G | 5G |
| Sorghum | 8G | 8G |
| Sunflower | 10G | 7G,3C |
| Rape | — | — |
| Johnsongrass | 10C | 9G |
| Sugar beets | 8G | 10C |
| Bindweed | 0 | 0 |
| Mustard | 10C | 7G,2C |

TABLE D (continued)

| | Compound 30 | | |
|---|---|---|---|
| Rate kg/ha | 0.062 | 0.015 | 0.003 |
| Soybeans | 7G | 2G | 0 |
| Velvetleaf | 9G | 5G | 0 |
| Sesbania | 9G | 4G | 2G |
| Sicklepod | 2G | 0 | 0 |
| Cotton | 5G | 4G | 0 |
| Morningglory | 3G | 2G | 0 |
| Alfalfa | 8G,2C | 3G | 8G |
| Jimsonweed | 3G | 2G | 0 |
| Cocklebur | 6G | 0 | 0 |
| Corn | 10G | 10C | 6G |
| Crabgrass | 3G | 2G | 0 |
| Rice | 8G,6C | 8G,4C | 8G |
| Nutsedge | 3G | 2G | 0 |
| Barnyardgrass | 7G,2C | 6G | 2G |
| Wheat | 6G | 4G | 2G |
| Giant foxtail | 9G,6C | 7G | 4G |
| Wild Oats | 8G | 4G | 0 |
| Sorghum | 10C | 9G | 5G |
| Sunflower | 5G | 0 | 0 |
| Rape | — | — | — |
| Johnsongrass | 9G,6U | 7G | 3G |
| Sugar beets | — | — | — |
| Bindweed | 0 | 0 | 0 |
| Mustard | 10C | 10C | 9G |

TABLE D (continued)

| Rate kg/ha | Compound 31<br>0.062 | <br>0.015 | <br>0.003 |
|---|---|---|---|
| Soybeans | 8G | 5G,4C | 4C,4G |
| Velvetleaf | 9G | 5G,5C | 3G |
| Sesbania | 10C | 9C | 3C,4G |
| Sicklepod | 2C,5G | 3G,2C | 1C |
| Cotton | 4G,1C | 4C,5G | 3G,3C |
| Morningglory | 4G | 4G | 2G |
| Alfalfa | 4G,3C | 5G,3C | 4G,2C |
| Jimsonweed | 3G | 4G,3C | 2G |
| Cocklebur | 6G | 4G | 3G |
| Corn | 10C | 9C | 6G,3C |
| Crabgrass | 4G | 3G | 1G |
| Rice | 4G,4C | 7G,5C | 5G,3C |
| Nutsedge | 5G | 3C,3G | 1C,3G |
| Barnyardgrass | 10C | 6G,3C | 4G |
| Wheat | 5G,4C | 5G | 3G |
| Giant foxtail | 10C | 8G | 3G |
| Wild Oats | 6G,3C | 5G | 4G |
| Sorghum | 8C | 9C | 7G |
| Sunflower | 10C | 3G | 3G |
| Rape | 8C | 4C,7G | 3C,5G |
| Johnsongrass | 10C | 10C | 4C,5G |
| Sugar beets | 10C | 8C | 7G,2C |
| Bindweed | 8G | 5G | 2C,5G |
| Mustard | — | — | — |

TABLE D (continued)

| | Compound 32 | |
|---|---|---|
| Rate kg/ha | 0.015 | 0.003 |
| Soybeans | 9C | 8C |
| Velvetleaf | 2C,8G | 2G |
| Sesbania | 10C | 8G,6C |
| Sicklepod | 1G | 0 |
| Cotton | 2C | 1C |
| Morningglory | 0 | 0 |
| Alfalfa | 3G | 2G |
| Jimsonweed | 2G | 1C |
| Cocklebur | — | 5G |
| Corn | 1C,3G | 2H,3G |
| Crabgrass | 1G | 0 |
| Rice | 8C | 6G,2C |
| Nutsedge | 3C,2G | 0 |
| Barnyardgrass | 3G | 0 |
| Wheat | 2G | 0 |
| Giant foxtail | 7G | 2G |
| Wild Oats | 0 | 0 |
| Sorghum | 7G | 6G |
| Sunflower | 0 | 0 |
| Rape | 2G | 1G |
| Johnsongrass | 4G,4C | 0 |
| Sugar beets | 2G,3C | 1G,1C |
| Bindweed | 0 | 1C |
| Mustard | — | — |

TABLE D (continued)

| Rate kg/ha | Compound 34<br>0.062 | <br>0.015 | <br>0.003 |
|---|---|---|---|
| Soybeans | 9C | 9C | 9C |
| Velvetleaf | 9C | 8G | 6G |
| Sesbania | 10C | 9G | 8G |
| Sicklepod | 6G | 0 | 0 |
| Cotton | 10G | 9G | 9G |
| Morningglory | 9G | 9G | 8G |
| Alfalfa | 9G | 9G | 7G |
| Jimsonweed | 9G | 9G | 7G |
| Cocklebur | 9G | 8G | 7G |
| Corn | 10C | 9G | 8G |
| Crabgrass | 9C | 8G | 4G |
| Rice | 9G | 8G | 8G |
| Nutsedge | 9C | 6C | 2G |
| Barnyardgrass | 10C | 9G | 4G |
| Wheat | 6G | 0 | 0 |
| Giant foxtail | 10C | 8G | 6G |
| Wild Oats | 9G | 5G | 0 |
| Sorghum | 9G | 9G | 6G |
| Sunflower | 10C | 8G | 3G |
| Rape | — | — | — |
| Johnsongrass | 9U | 7G | 0 |
| Sugar beets | 9G | 9G | 7G |
| Bindweed | 8G | 4G | 0 |
| Mustard | 9G | 8G | 3G |

TABLE D (continued)

| Rate kg/ha | Compound 35 0.062 | 0.015 |
|---|---|---|
| Soybeans | 3C,5G | 2C,5G |
| Velvetleaf | 1C | 1G |
| Sesbania | 0 | 1G |
| Sicklepod | 2C | 2C |
| Cotton | 5G,3C | 4G,3C |
| Morningglory | 6G | 5G |
| Alfalfa | 3C | 2G |
| Jimsonweed | 7G | 6G |
| Cocklebur | 5C,8G | 5G |
| Corn | 8G | 7G |
| Crabgrass | 0 | 0 |
| Rice | 6G | 5G |
| Nutsedge | 2G | 0 |
| Barnyardgrass | 0 | 0 |
| Wheat | 0 | 0 |
| Giant foxtail | 2G | 1G |
| Wild Oats | 4G | 1G |
| Sorghum | 8G | 7G |
| Sunflower | 6C,8G | 4G |
| Rape | 5C,6G | 4G,3C |
| Johnsongrass | 3G | 2G |
| Sugar beets | 2C,6G | 1C,4G |
| Bindweed | 6G | 3G |
| Mustard | — | — |

TABLE D (continued)

| | Compound 36 | |
|---|---|---|
| Rate kg/ha | 0.062 | 0.015 |
| Soybeans | 2C,1G | 1G |
| Velvetleaf | 0 | 1C |
| Sesbania | 0 | 0 |
| Sicklepod | 2C,1G | 1G |
| Cotton | 2G | 0 |
| Morningglory | 3C | 0 |
| Alfalfa | 2C | 0 |
| Jimsonweed | 3G,2C | 1C |
| Cocklebur | 3G | 0 |
| Corn | 1C,4G | 1C,3G |
| Crabgrass | 1G | 0 |
| Rice | 4G | 4G |
| Nutsedge | 2G | 0 |
| Barnyardgrass | 0 | 0 |
| Wheat | 2G | 0 |
| Giant foxtail | 3G | 0 |
| Wild Oats | 2G | 0 |
| Sorghum | 8G | 3G |
| Sunflower | 2C | 0 |
| Rape | 4G,2C | 2G |
| Johnsongrass | 2G,1C | 1G |
| Sugar beets | 1G | 0 |
| Bindweed | 3C,2G | 0 |
| Mustard | — | — |

TABLE D (continued)

| | Compound 38 | |
|---|---|---|
| Rate kg/ha | 0.062 | 0.015 |
| Soybeans | 6G,3C | 6G |
| Velvetleaf | 5G,2C | 2G |
| Sesbania | 9G | 7G |
| Sicklepod | 2G | 2G |
| Cotton | 2G | 0 |
| Morningglory | 6C,9G | 7G,6C |
| Alfalfa | 5G | 0 |
| Jimsonweed | 3G | 3G |
| Cocklebur | — | 4G |
| Corn | 9C,9G | 3C,9G |
| Crabgrass | 6G | 2G |
| Rice | 5G,2C | 5G,4C |
| Nutsedge | 9G,5C | 2G |
| Barnyardgrass | 9G,8C | 7G |
| Wheat | 0 | 0 |
| Giant foxtail | 6G | 3G |
| Wild Oats | 0 | 0 |
| Sorghum | 7G | 3G |
| Sunflower | 7G,2C | 7G,3C |
| Rape | — | — |
| Johnsongrass | 3G | 0 |
| Sugar beets | 4G | 4G |
| Bindweed | 7G | 9G |
| Mustard | 3G | 3G |

TABLE D (continued)

| | Compound 43 | | |
|---|---|---|---|
| Rate kg/ha | 0.062 | 0.015 | 0.003 |
| Soybeans | 0 | 0 | 0 |
| Velvetleaf | 6G | 0 | 0 |
| Sesbania | 6G | 0 | 0 |
| Sicklepod | 0 | 0 | 0 |
| Cotton | 9G | 9G | 2G |
| Morningglory | 3G | 0 | 0 |
| Alfalfa | 2G | 6G | 0 |
| Jimsonweed | 0 | 0 | 0 |
| Cocklebur | 9G | 7G | 0 |
| Corn | 8G,2H | 5G | 2G |
| Crabgrass | 8G | 5G | 2G |
| Rice | 5G | 2G | 2G |
| Nutsedge | 0 | 0 | 0 |
| Barnyardgrass | 8G | 8G | 7G |
| Wheat | 0 | 0 | 0 |
| Giant foxtail | 4G | 4G | 4G |
| Wild Oats | 4G | 3G | 0 |
| Sorghum | 8G | 7G | 3G |
| Sunflower | 8G | 8G | 0 |
| Rape | — | — | — |
| Johnsongrass | 8G | 8G | 8G |
| Sugar beets | 8G | 8G | 7G |
| Bindweed | 3G | 3G | 3G |
| Mustard | 10C | 7G | 0 |

TABLE D (continued)

| | Compound 46 | | |
|---|---|---|---|
| Rate kg/ha | 0.062 | 0.015 | 0.003 |
| Soybeans | 10G,7C | 9G,4C | 5G,4C |
| Velvetleaf | 8C | 6G | 4G |
| Sesbania | 8G | 5C | 3C |
| Sicklepod | 6C | 5C | 2C |
| Cotton | 9G | 9G | 6G |
| Morningglory | 10G | 8G | 5G |
| Alfalfa | 8C,8G | 4G | 2C |
| Jimsonweed | 7G | 6G | 2G |
| Cocklebur | 10G | 7G | 3G |
| Corn | 8G,1H | 6G,3H | 0 |
| Crabgrass | 6G | 3G | 0 |
| Rice | 9C | 9G | 4G |
| Nutsedge | 8G | 5G | 0 |
| Barnyardgrass | 9G | 1H,9G | 2G |
| Wheat | 9G | 9G | 3G |
| Giant foxtail | 9C,9G | 8G | 4G |
| Wild Oats | 9G | 9G | 2G |
| Sorghum | 9G,4U | 9G | 9G |
| Sunflower | 10C | 9G | 5G |
| Rape | — | — | — |
| Johnsongrass | 10U | 10C | 6G |
| Sugar beets | 9G | 7G | 0 |
| Bindweed | 8G | 2G | 0 |
| Mustard | 9G | 8G | 2G |

**Claims for the Contracting States: BE CH DE FR IT LI LU NL SE**

1. A compound of the formula:

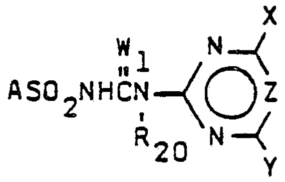

I

wherein

X is $CH_3$, $OCH_3$, Cl, $C_2H_5$ or $OC_2H_5$;
Z is CH or N;
Y is $CH_2OR$, $CH_2SR_1$, $CH(OR_1)_2$, $OCH_2OR_1$, $OCH(OCH_3)CO_2R_3$ or

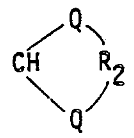

R is H, $C(O)R_3$, $CO_2R_4$, $C(O)NR_5R_6$ or

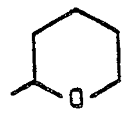 ;

$R_1$ is $C_1$—$C_2$ alkyl;
$R_2$ is —$CH_2CH_2$—, —$CH(CH_3)CH_2$— or —$CH_2CH_2CH_2$—;
$R_3$ is $C_1$—$C_3$ alkyl;
$R_4$ is $C_1$—$C_3$ alkyl;
$R_5$ is H or $CH_3$;
$R_6$ is $C_1$—$C_3$ alkyl;
Q is O or S;
A is

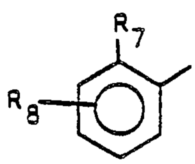 , 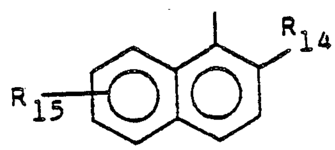 , 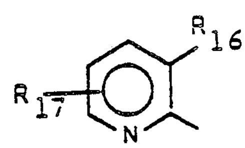 ,

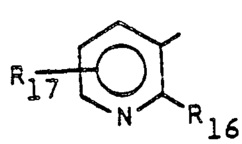 , 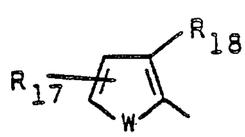 , 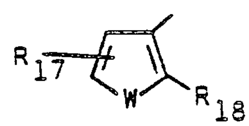 ,

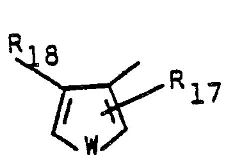 , 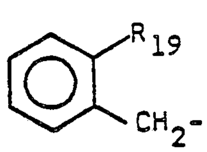 or 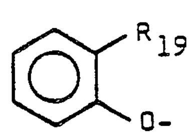 ;

$R_7$ is H, $C_1$—$C_4$ alkyl, $C_1$—$C_4$ alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$, $C_3$—$C_4$ alkenyloxy or $C_1$—$C_2$ alkyl substituted with $OCH_3$ or $OC_2H_5$;
$R_8$ is H, F, Cl, Br, $CF_3$, $NO_2$, $C_1$—$C_3$ alkyl or $C_1$—$C_3$ alkoxy;
$R_9$ is $C_1$—$C_4$ alkyl, $CH_2CH_2OCH_3$, $CH_2CH_2Cl$ or $CH_2CH=CH_2$;
$R_{10}$ and $R_{11}$ are independently $C_1$—$C_3$ alkyl;
$R_{12}$ is $C_1$—$C_4$ alkyl, $CH_2CH_2OCH_3$, $CH_2CH_2CH_2OCH_3$ or $C_1$—$C_4$ alkyl substituted with 1—3 atoms of F, Cl or Br;
$R_{13}$ is $C_1$—$C_4$ alkyl or $CH_2CH=CH_2$;
n is 0 or 2;

L is O, S or $SO_2$;
$R_{14}$ is H, $CH_3$, $OCH_3$, F, Cl, Br, $NO_2$, $SO_2NR_{10}R_{11}$, $OSO_2R_{12}$ or $S(O)_nR_{13}$;
$R_{15}$ is H, Cl, Br, $CH_3$, $OCH_3$ or $NO_2$;
$R_{16}$ is H, $C_1$—$C_4$ alkyl, $C_1$—$C_4$ alkoxy, F, Cl, Br, $CF_3$, $CO_2R_9$, $SO_2NR_{10}R_{11}$, $SO_2N(OCH_3)CH_3$ or $S(O)_nR_{13}$;
$R_{17}$ is H, F, Cl, Br, $CH_3$ or $OCH_3$;
W is O or S; and
$W_1$ is O or S;
$R_{18}$ is H, $C_1$—$C_4$ alkyl, $C_1$—$C_4$ alkoxy, F, Cl, Br, $NO_2$, $CO_2R_9$, $SO_2NR_{10}R_{11}$, $SO_2N(OCH_3)CH_3$ or $S(O)_nR_{13}$;
$R_{19}$ is Cl, $NO_2$, $CF_3$, $CO_2R_9$, $SO_2N(OCH_3)CH_3$, $SO_2NR_{10}R_{11}$, $OSO_2R_{12}$, $S(O)_nR_{13}$ or $C_1$—$C_3$ alkoxy optionally substituted with 1—5 atoms of Cl or F; and
$R_{20}$ is H or $CH_3$;

and their agriculturally suitable salts; provided that

(1) the total number of carbon atoms of $R_{10}$ and $R_{11}$ is less than or equal to 4;
(2) when X is Cl, then Z is CH;
(3) when W is O, then $R_{18}$ is H, Cl, Br, $CH_3$ or $CO_2R_9$;
(4) when W is O and $R_{18}$ is H, Cl, Br, or $CH_3$, then A is

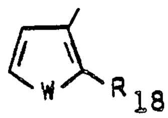 ;

(5) when $W_1$ is S, then $R_{20}$ is H and A is

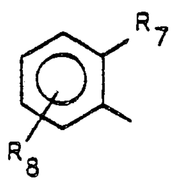 ;

(6) when Y is $OCH_2OR_1$ or $OCH(OCH_3)CO_2R_3$, then Z is CH;
(7) when $R_7$ is $CH_2CH_2OCH_3$ or $CH_2CH_2OCH_2CH_3$ and $R_8$ is H, F, Cl, $CH_3$, $OCH_3$ or $CF_3$ then Y is other than $CH(OCH_3)_2$ or

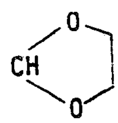

and

(8) when $R_7$ is $C_3$ alkenyloxy and $R_8$ is H, F, Cl, Br, $CH_3$, $OCH_3$ or $CF_3$, then Y is other than $CH(OCH_3)_2$ or

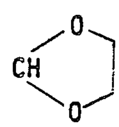

2. A compound of Claim 1 where $W_1$ is O, X is $CH_3$ or $OCH_3$ and $R_{20}$ is H.
3. A compound of Claim 2 where Y is $CH(OR_1)_2$ or

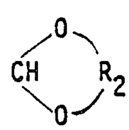

4. A compound of Claim 3 wherein A is

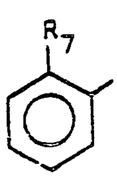 , 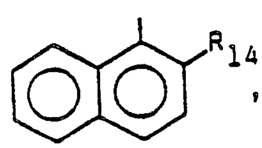 , 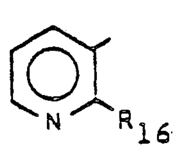 , 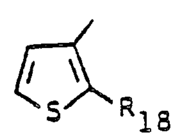 or 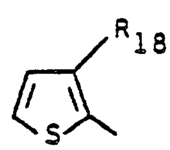 ;

$R_7$ is $C_1$—$C_3$ alkyl, $C_1$—$C_3$ alkoxy, Cl, $NO_2$, $CO_2R_9$, $SO_2(C_1$—$C_3$ alkyl), $SO_2NR_{10}R_{11}$, $SO_2N(OCH_3)CH_3$, $OSO_2R_{13}$, $CH_2OCH_3$ or $LCF_2H$;

$R_{14}$ is $CH_3$, $OCH_3$, Cl, $OSO_2CH_3$ or $SO_2(C_1—C_3$ alkyl);
$R_{16}$ is $CH_3$, $OCH_3$, Cl, $SO_2CH_3$ or $SO_2N(CH_3)_2$;
$R_{18}$ is $CH_3$, Cl, Br, $CO_2R_9$ or $SO_2CH_3$.

5. A compound of Claim 4 where A is

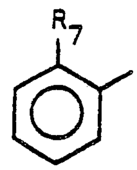

.

6. The compound of Claim 1 which is 3-[[(4-dimethoxymethyl-6-methylpyrimidin-2-yl)aminocarbonyl]-aminosulfonyl]-2-thiophenecarboxylic acid, methyl ester.

7. The compound of Claim 1 which is 2-chloro-N-[(4-dimethoxymethyl-6-methylpyrimidin-2-yl)amino-carbonyl]benzenesulfonamide.

8. The compound of Claim 1 which is 2-[[(4-dimethoxymethyl-6-methylpyrimidin-2-yl)aminocarbonyl]-aminosulfonyl]benzoic acid, methyl ester.

9. The compound of Claim 1 which is N-[(4-dimethoxymethyl-6-methylpyrimidin-2-yl)aminocarbonyl]-2-methylsulfonylbenzenesulfonamide.

10. The compound of Claim 1 which is N-[(4-dimethoxymethyl-6-methylpyrimidin-2-yl)amino-carbonyl]-2-nitrobenzenesulfonamide.

11. The compound of Claim 1 which is 2-[[(4-dimethoxymethyl-6-methylpyrimidin-2-yl)-aminocarbonyl]aminosulfonyl]benzoic acid, 2-propyl ester.

12. The compound of Claim 1 which is 2-[[[4-methyl-6-(tetrahydropyran-2-yloxymethyl)pyrimidin-2-yl]-aminocarbonyl]aminosulfonyl]benzoic acid, methyl ester.

13. The compound of Claim 1 which is 2-[[[4-(hydroxymethyl)-6-methylpyrimidin-2-yl]aminocarbonyl]-aminosulfonyl]benzoic acid, methyl ester.

14. The compound of Claim 1 which is 2-[[[4-(acetyloxymethyl)-6-methylpyrimidin-2-yl]-aminocarbonyl]aminosulfonyl]benzoic acid, methyl ester.

15. A compound of claim 1 of the formula:

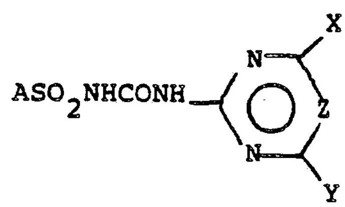

wherein

X is $CH_3$, $OCH_3$ or Cl;

Y is $CH_2OR$, $CH_2SR_1$, $CH(OR_1)_2$ or

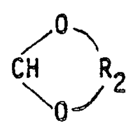

A is

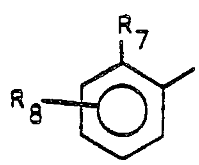
,
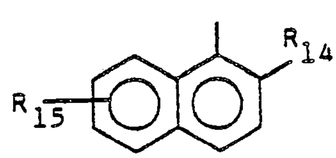
,
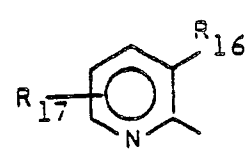
,

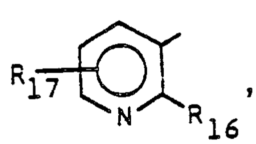
,
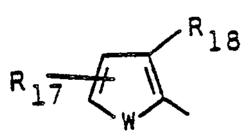
,
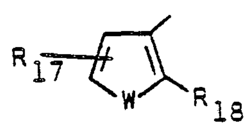
, or
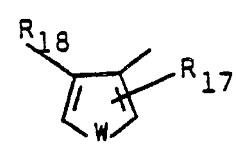

$R_7$ is H, $C_1—C_4$ alkyl, $C_1—C_4$ alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$ or $CH_2OR_{13}$;

$R_{13}$ is $C_1—C_4$ alkyl; and

Z, R, $R_1—R_6$, $R_8—R_{12}$, n, L, $R_{14}—R_{18}$ and

W are as defined in claim 1.

16. A composition suitable for controlling the growth of undesired vegetation which comprises an effective amount of a herbicidal compound and at least one of the following: surfactant, solid or liquid inert

diluent, characterised in that said herbicidal compound comprises a compound of any of claims 1 to 15.

17. A method for controlling the growth of undesired vegetation by applying to the locus to be protected an effective amount of a herbicidal compound, characterised in that said herbicidal compound comprises a compound of any of claims 1 to 15.

18. A method for regulating the growth of plants by applying to the locus of such plants an effective but substantially non-phytotoxic amount of a plant growth regulant, characterised in that said plant growth regulant comprises a compound of any of claims 1 to 15.

19. A process for preparing a compound of claim 1 which comprises reacting a compound of general formula III

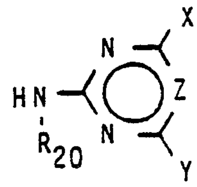

III

wherein

X, $R_{20}$ and Z are as defined in claim 1;

Y is $CH_2OR$, $CH_2SR_1$, $CH_2(OR_1)_2$, $OCH_2OR_1$,

$OCHCO_2R_3$, $CH(QR_2Q)$;
|
$CH_3O$

R is 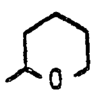 = THP;

and

$R_1$, $R_2$, and $R_3$ are as defined in claim 1 with a compound of general formula II

$$ASO_2NCW_1 \qquad II$$

wherein A and $W_1$ are as defined in claim 1.

20. The process for Claim 19 in which the reaction is carried out in an inert, aprotic solvent at a temperature ranging from 0°C to reflux temperature.

21. A process for preparing compounds of formula Ib

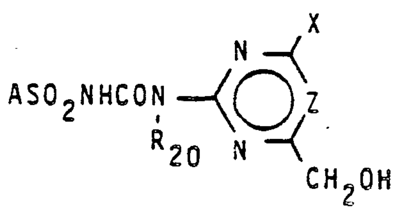

(Ib)

wherein A, X, $R_{20}$, and Z are as defined in claim 1 which comprises hydrolysing under acidic conditions a compound of formula I wherein Y is $CH_2OTHP$.

22. A process for preparing compounds of formulae Ic, Id, and Ie

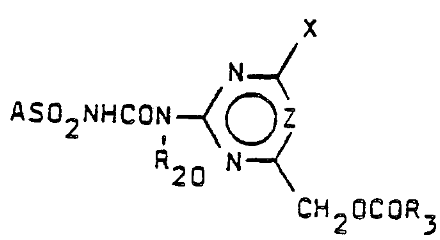

(Ic)

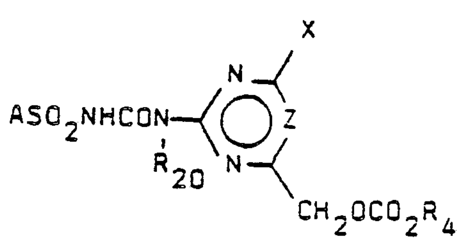

(Id)

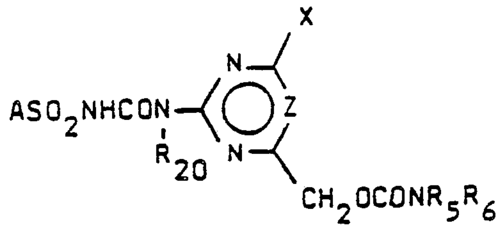

(Ie)

wherein A, $R_{20}$, $R_3$, $R_4$, $R_5$, and $R_6$ are as defined in claim 1 which comprises contacting a compound of formula Ib as defined in claim 21 with at least one equivalent of an alkyl carboxylic anhydride, carbonyl chloride, chloroformate, carbamoyl chloride or alkyl isocyanate in an inert solvent in the presence of an excess of an acid accepter.

**Claims for the Contracting State: AT**

1. A process for preparing a compound of formula I

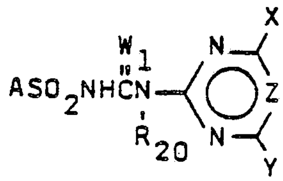

I

wherein

X is $CH_3$, $OCH_3$, Cl, $C_2H_5$ or $OC_2H_5$;

Z is CH or N;

Y is $CH_2OR$, $CH_2SR_1$, $CH(OR_1)_2$, $OCH_2OR_1$,

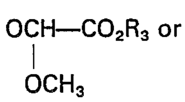 or 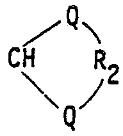 R is H, $C(O)R_3$, $CO_2R_4$, $C(O)NR_5R_6$ or 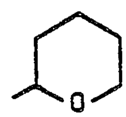 ;

$R_1$ is $C_1$—$C_2$ alkyl;

$R_2$ is —$CH_2CH_2$—, —$CH(CH_3)CH_2$— or —$CH_2CH_2CH_2$—;

$R_3$ is $C_1$—$C_3$ alkyl;

$R_4$ is $C_1$—$C_3$ alkyl;

$R_5$ is H or $CH_3$;

$R_6$ is $C_1$—$C_3$ alkyl;

Q is O or S;

A is

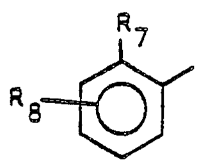 , 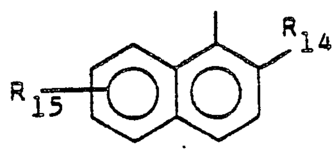 , 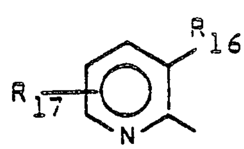 ,

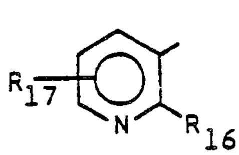 , 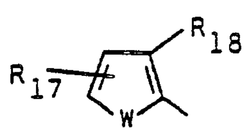 , 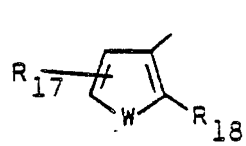 ,

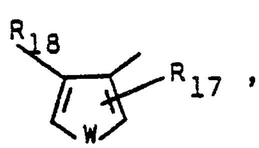 , 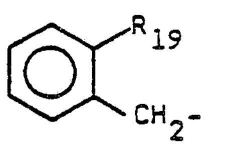 or 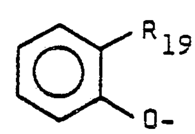 ;

$R_7$ is H, $C_1$—$C_4$ alkyl, $C_1$—$C_4$ alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$, $C_3$—$C_4$ alkenyloxy or $C_1$—$C_2$ alkyl substituted with $OCH_3$ or $OC_2H_5$;

$R_8$ is H, F, Cl, Br, $CF_3$, $NO_2$, $C_1$—$C_3$ alkyl or $C_1$—$C_3$ alkoxy;
$R_9$ is $C_1$—$C_4$ alkyl, $CH_2CH_2OCH_3$, $CH_2CH_2Cl$ or $CH_2CH{=}CH_2$;
$R_{10}$ and $R_{11}$ are independently $C_1$—$C_3$ alkyl;
$R_{12}$ is $C_1$—$C_4$ alkyl, $CH_2CH_2OCH_3$, $CH_2CH_2CH_2OCH_3$ or $C_1$—$C_4$ alkyl substituted with 1—3 atoms of F, Cl or Br;
$R_{13}$ is $C_1$—$C_4$ alkyl or $CH_2CH{=}CH_2$;
n is 0 or 2;
L is O, S or $SO_2$;
$R_{14}$ is H, $CH_3$, $OCH_3$, F, Cl, Br, $NO_2$, $SO_2NR_{10}R_{11}$, $OSO_2R_{12}$ or $S(O)_nR_{13}$;
$R_{15}$ is H, Cl, Br, $CH_3$, $OCH_3$ or $NO_2$;
$R_{16}$ is H, $C_1$—$C_4$ alkyl, $C_1$—$C_4$ alkoxy, F, Cl, Br, $CF_3$, $CO_2R_9$, $SO_2NR_{10}R_{11}$, $SO_2N(OCH_3)CH_3$ or $S(O)_nR_{13}$;
$R_{17}$ is H, F, Cl, Br, $CH_3$ or $OCH_3$;
W is O or S;
$W_1$ is O or S;
$R_{18}$ is H, $C_1$—$C_4$ alkyl, $C_1$—$C_4$ alkoxy, F, Cl, Br, $NO_2$, $CO_2R_9$, $SO_2NR_{10}R_{11}$, $SO_2N(OCH_3)CH_3$ or $S(O)_nR_{13}$;
$R_{19}$ is Cl, $NO_2$, $CF_3$, $CO_2R_9$, $SO_2N(OCH_3)CH_3$, $SO_2NR_{10}R_{11}$, $OSO_2R_1$, $S(O)_nR_{13}$ or $C_1$—$C_3$ alkoxy optionally substituted with 1—5 atoms of Cl or F; and
$R_{20}$ is H or $CH_3$;
and their agriculturally suitable salts; provided that
(1) the total number of carbon atoms of $R_{10}$ and $R_{11}$ is less than or equal to 4;
(2) when X is Cl, then Z is CH;
(3) when W is O, then $R_{18}$ is H, Cl, Br, $CH_3$ or $CO_2R_9$;
(4) when W is O and $R_{18}$ is H, Cl, Br, or $CH_3$, then A is

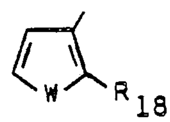 ;

(5) when $W_1$ is S, then $R_{20}$ is H and A is

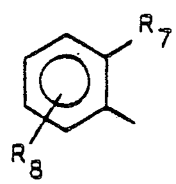 ;

(6) when Y is $OCH_2OR_1$ or $OCH(OCH_3)$—$CO_2R_3$,

then Z is CH;
(7) when $R_7$ is $CH_2CH_2OCH_3$ or $CH_2CH_2OCH_2CH_3$ and $R_8$ is H, F, Cl, $CH_3$, $OCH_3$ or $CF_3$ then Y is other than $CH(OCH_3)_2$ or

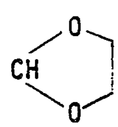

and
(8) when $R_7$ is $C_3$ alkenyloxy and $R_8$ is H, F, Cl, Br, $CH_3$, $OCH_3$ or $CF_3$, then Y is other than $CH(OCH_3)_2$ or

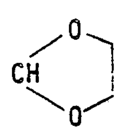

which comprises (a) reacting a compound of general formula III

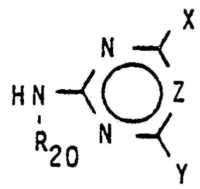

III

wherein

X, $R_{20}$ and Z are as previously defined;

Y is $CH_2OR$, $CH_2SR_1$, $CH_2(OR_1)_2$, $OCH_2OR_1$,
$OCHCO_2R_3$, $CH(QR_2Q)$;
|
$CH_3O$

R is 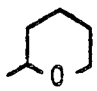 = THP;

and

$R_1$, $R_2$, and $R_3$ are as previously defined with a compound of general formula II

$$ASO_2NCW_1 \qquad II$$

wherein

A is as previously defined to obtain a corresponding compound of general formula 1; or

(b) hydrolysing under acidic conditions a compound of formula (I) wherein Y is $CH_2OTHP$ to obtain a corresponding compound of general formula

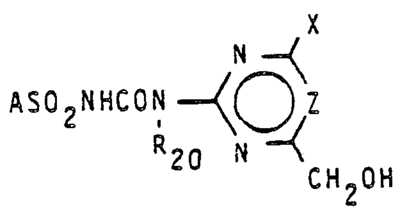

(Ib)

wherein A, X, $R_{20}$, and Z are as previously defined;

(c) contacting a compound of formula Ib as defined above with at least one equivalent of an alkyl carboxylic anhydride, carbonyl chloride, chloroformate, carbamoyl chloride or alkyl isocyanate in an inert solvent in the presence of an excess of an acid accepter to obtain compounds of formulae Ic, Id, and Ie

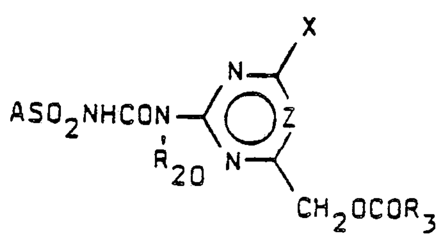

(Ic)

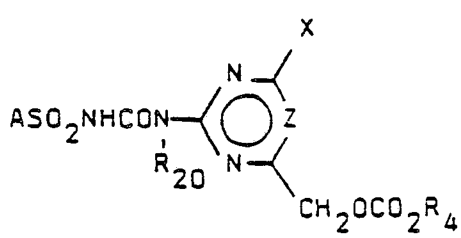

(Id)

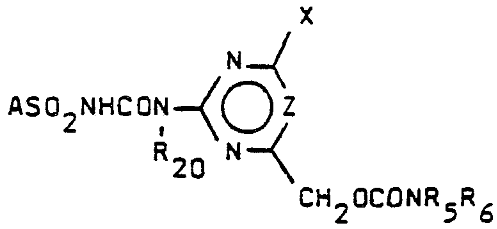

(Ie)

wherein A, $R_{20}$, $R_3$, $R_4$, $R_5$, and $R_6$ are as previously defined; or

(d) reducing a compound of general formula (I) wherein Y is $CO_2CH_3$ and $R_7$, $R_{16}$, $R_{18}$ and $R_{19}$ are not $CO_2R_9$ with diisobutyl aluminum hydride to obtain a corresponding compound wherein Y is $CH_2OH$.

2. The process of claim 1 wherein reaction (a) is performed in an inert, aprotic solvent at a temperature ranging from 0°C to reflux temperature; or wherein reaction (b) is performed in a polar, protic solvent containing a catalytic amount of a mineral acid at 0 to 30°C.

3. The process of claim 1 or 2 wherein said compound of general formula (I) has the formula

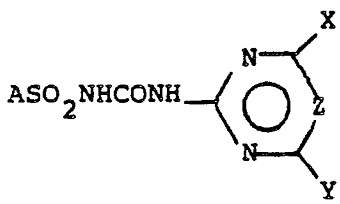

wherein

X is $CH_3$, $OCH_3$ or Cl;

Y is $CH_2OR$, $CH_2SR_1$, $CH(OR_1)_2$ or 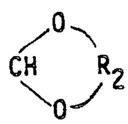

A is

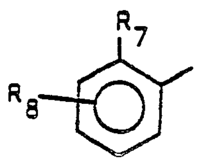, 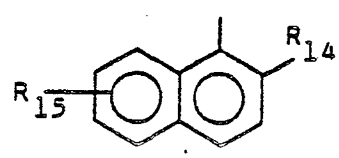, 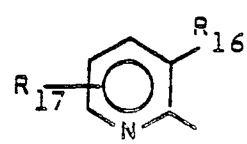,

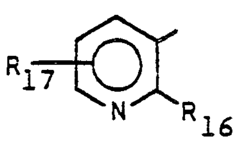, 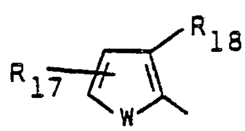, 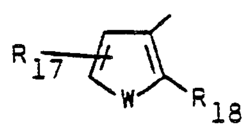, or 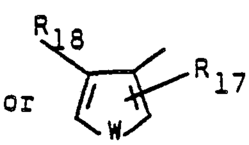

$R_7$ is H, $C_1$—$C_4$ alkyl, $C_1$—$C_4$ alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$ or $CH_2OR_{13}$;

$R_{13}$ is $C_1$—$C_4$ alkyl; and

Z, R, $R_1$—$R_6$, $R_8$—$R_{12}$, n, L, $R_{14}$—$R_{18}$ and W are as defined in claim 1.

4. The process of claim 1, 2 or 3 wherein $W_1$ is O, $R_{20}$ is H and X is $CH_3$ or $OCH_3$.

5. The process of claim 4 wherein Y is $CH_2(OR_1)_2$.

6. The process of claim 5 wherein A is

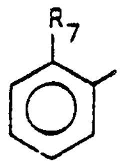 , 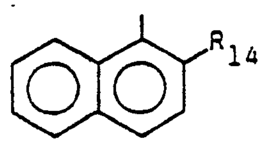 , 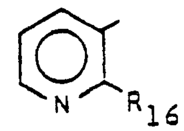 , 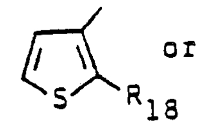 or 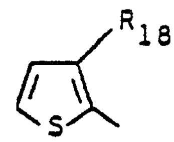 ;

$R_7$ is $C_1$—$C_3$ alkyl, $C_1$—$C_3$ alkoxy, Cl, $NO_2$, $CO_2R_9$, $SO_2(C_1$—$C_3$ alkyl), $SO_2NR_{10}R_{11}$, $SO_2N(OCH_3)CH_3$, $OSO_2R_{13}$, $CH_2OCH_3$ or $LCF_2H$;

$R_{14}$ is $CH_3$, $OCH_3$, Cl, $OSO_2CH_3$ or $SO_2(C_1$—$C_3$ alkyl);

$R_{16}$ is $CH_3$, $OCH_3$, Cl, $SO_2CH_3$ or $SO_2N(CH_3)_2$;

$R_{18}$ is $CH_3$, Cl, Br, $CO_2R_9$ or $SO_2CH_3$.

7. A process of claim 4 wherein A is

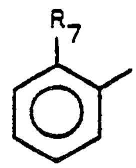.

8. The process of claim 1 wherein the product is a compound selected from

3-[[4-dimethoxymethyl-6-methylpyrimidin-2-yl)aminocarbonyl]aminosulfonyl]-2-thiophenecarboxylic acid, methyl ester;

2-chloro-N-[(4-dimethoxymethyl-6-methylpyrimidin-2-yl)aminocarbonyl]benzenesulfonamide;

2-[[(4-dimethoxymethyl-6-methylpyrimidin-2-yl)aminocarbonyl]aminosulfonyl]benzoic acid, methyl ester;

N-[(4-dimethoxymethyl-6-methylpyrimidin-2-yl)aminocarbonyl]-2-methylsulfonylbenzene-sulfonamide.

N-[(4-dimethoxymethyl-6-methylpyrimidin-2-yl)aminocarbonyl]-2-nitrobenzenesulfonamide.

2-[[(4-dimethoxymethyl-6-methylpyrimidin-2-yl)aminocarbonyl]aminosulfonyl]benzoic acid, 2-propyl ester;

2-[[[4-methyl-6-(tetrahydropyran-2-yloxymethyl)pyrimidin-2-yl]aminocarbonyl]aminosulfonyl]benzoic acid, methyl ester;

2-[[[4-(hydroxymethyl)-6-methylpyrimidin-2-yl]aminocarbonyl]aminosulfonyl]benzoic acid, methyl ester; or

2-[[[4-(acetyloxymethyl)-6-methylpyrimidin-2-yl]aminocarbonyl]aminosulfonyl]benzoic acid, methyl ester.

9. A method for controlling the growth of undesired vegetation by applying to the locus to be protected an effective amount of a herbicidal compound, characterised in that said herbicidal compound comprises a compound of formula (I) as defined in any of claims 1 to 8, or an agriculturally suitable salt thereof.

10. A method for regulating the growth of plants by applying to the locus of such plants an effective but substantially non-phytotoxic amount of plant growth regulant, characterised in that said plant growth regulant comprises a compound of formula (I) as defined in any of claims 1 to 8, or an agriculturally suitable salt thereof.

**Patentansprüche für die Vertragsstaaten: BE CH DE FR IT LU LI NL SE**

1. Verbindung der Formel

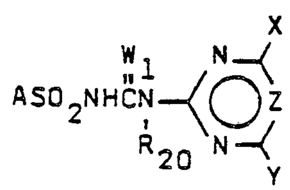

worin

X $CH_3$, $OCH_3$, Cl, $C_2H_5$ oder $OC_2H_5$ ist;

Z CH oder N ist;

Y $CH_2OR$, $CH_2SR_1$, $CH(OR_1)_2$, $OCH_2OR_1$,

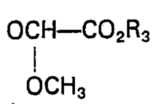 oder 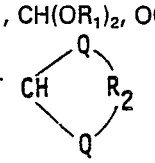

ist;

R H, $C(O)R_3$, $CO_2R_4$, $C(O)NR_5R_6$ oder

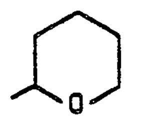 ;

ist;

$R_1$ $C_1$—$C_2$-Alkyl ist;
$R_2$ —$CH_2CH_2$—, —$CH(CH_3)CH_2$— oder —$CH_2CH_2CH_2$— ist;
$R_3$ $C_1$—$C_3$-Alkyl ist;
$R_4$ $C_1$—$C_3$-Alkyl ist;
$R_5$ H oder $CH_3$ ist;
$R_6$ $C_1$—$C_3$-Alkyl ist;
Q O oder S ist;
A

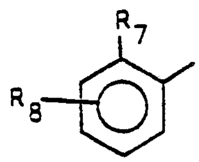 , 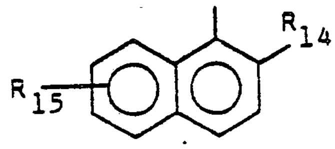 , 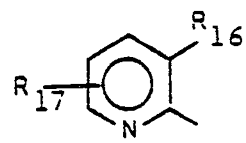 ,

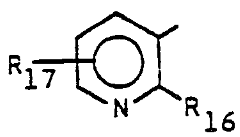 , 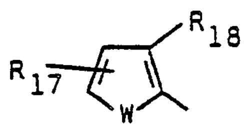 , 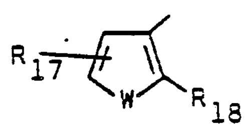 ,

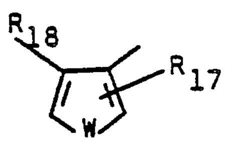 , 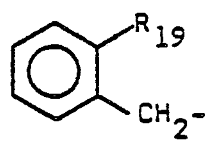 or 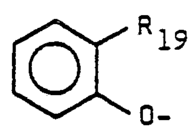 ;

ist;

$R_7$ H, $C_1$—$C_4$-Alkyl, $C_1$—$C_4$-Alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$, $C_3$—$C_4$-Alkenyloxy oder mit $OCH_3$ oder $OC_2H_5$ substituiertes $C_1$—$C_2$-Alkyl ist;

$R_8$ H, F, Cl, Br, $CF_3$, $NO_2$, $C_1$—$C_3$-Alkyl oder $C_1$—$C_3$-Alkoxy ist;

$R_9$ $C_1$—$C_4$-Alkyl, $CH_2CH_2OCH_3$, $CH_2CH_2Cl$ oder $CH_2CH{=}CH_2$ ist;

$R_{10}$ und $R_{11}$ unabhängig voneinander $C_1$—$C_3$-Alkyl sind;

$R_{12}$ $C_1$—$C_4$-Alkyl, $CH_2CH_2OCH_3$, $CH_2CH_2CH_2OCH_3$ oder mit 1—3 Atomen von F, Cl oder Br substituiertes $C_1$—$C_4$-Alkyl ist;

$R_{13}$ $C_1$—$C_4$-Alkyl oder $CH_2CH{=}CH_2$ ist;

n 0 oder 2 ist;

L O, S oder $SO_2$ ist;

$R_{14}$ H, $CH_3$, $OCH_3$, F, Cl, Br, $NO_2$, $SO_2NR_{10}R_{11}$, $OSO_2R_{12}$ oder $S(O)_nR_{13}$ ist;

$R_{15}$ H, Cl, Br, $CH_3$, $OCH_3$ oder $NO_2$ ist;

$R_{16}$ H, $C_1$—$C_4$-Alkyl, $C_1$—$C_4$-Alkoxy, F, Cl, Br, $CF_3$, $CO_2R_9$, $SO_2NR_{10}R_{11}$, $SO_2N(OCH_3)CH_3$ oder $S(O)_nR_{13}$ ist;

$R_{17}$ H, F, Cl, Br, $CH_3$ oder $OCH_3$ ist;

W O oder S ist; und

$W_1$ O oder S ist;

$R_{18}$ H, $C_1$—$C_4$-Alkyl, $C_1$—$C_4$-Alkoxy, F, Cl, Br, $NO_2$, $CO_2R_9$, $SO_2NR_{10}R_{11}$, $SO_2N(OCH_3)CH_3$ oder $S(O)_nR_{13}$ ist;

$R_{19}$ Cl, $NO_2$, $CF_3$, $CO_2R_9$, $SO_2N(OCH_3)CH_3$, $SO_2NR_{10}R_{11}$, $OSO_2R_{12}$, $S(O)_nR_{13}$ oder $C_1$—$C_3$-Alkoxy, gegebenenfalls mit 1—5 Atomen von Cl oder F substituiert ist; und

$R_{20}$ H oder $CH_3$ ist;

und ihrelandwirtschaftlich geeigneten Salze; mit der Maßgabe, daß

(1) die Gesamtzahl der Kohlenstoffatome von $R_{10}$ und $R_{11}$ geringer als oder gleich 4 ist;
(2) wenn X Cl ist, dann Z CH ist;
(3) wenn W O ist, dann $R_{18}$ H, Cl, Br, $CH_3$ oder $CO_2R_9$ ist;
(4) wenn W O ist und $R_{18}$ H, Cl, Br oder $CH_3$ ist, dann A

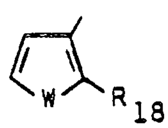 ;

ist;

(5) wenn $W_1$ S ist, dann $R_{20}$ H und A

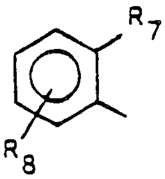

;

ist;

(6) wenn Y $OCH_2OR_1$ oder $OCH(OCH_3)CO_2R_3$ ist, dann Z CH ist;

(7) wenn $R_7$ $CH_2CH_2OCH_3$ oder $CH_2CH_2OCH_2CH_3$ und $R_8$ H, F, Cl, $CH_3$, $OCH_3$ oder $CF_3$ ist, dann Y anders als $CH(OCH_3)_2$ oder

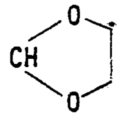

ist; und

(8) wenn $R_7$ $C_3$-Alkenyloxy und $R_8$ H, F, Cl, Br, $CH_3$, $OCH_3$ oder $CF_3$ ist, dann Y anders als $CH(OCH_3)_2$ oder

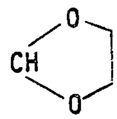

ist.

2. Verbindung nach Anspruch 1, worin $W_1$ O ist, X $CH_3$ oder $OCH_3$ ist und $R_{20}$ H ist.

3. Verbindung nach Anspruch 2, worin Y $CH(OR_1)_2$ oder

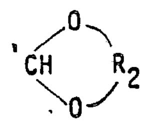

ist.

4. Verbindung nach Anspruch 3, worin

A

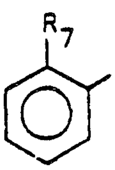 , 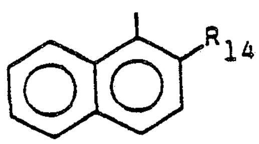 , 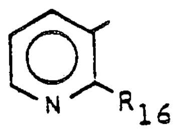 , 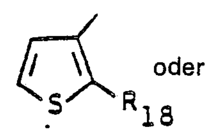 oder 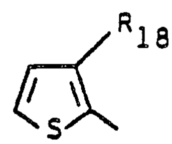

$R_7$ $C_1$—$C_3$-Alkyl, $C_1$—$C_3$-Alkoxy, Cl, $NO_2$, $CO_2R_9$, $SO_2(C_1$—$C_3$-Alkyl), $SO_2NR_{10}R_{11}$, $SO_2N(OCH_3)CH_3$, $OSO_2R_{13}$, $CH_2OCH_3$ oder $LCF_2H$ ist;

$R_{14}$ $CH_3$, $OCH_3$, Cl, $OSO_2CH_3$ oder $SO_2(C_1$—$C_3$-Alkyl) ist;

$R_{16}$ $CH_3$, $OCH_3$, Cl, $SO_2CH_3$ oder $SO_2N(CH_3)_2$ ist;

$R_{18}$ $CH_3$, Cl, Br, $CO_2R_9$ oder $SO_2CH_3$ ist.

5. Verbindung nach Anspruch 4, worin A

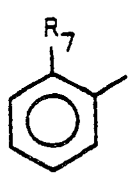

ist.

6. Verbindung nach Anspruch 1, welche 3-[[(4-Dimethoxymethyl-6-methylpyrimidin-2-yl)aminocarbonyl]aminosulfonyl]-2-thiophencarbonsäuremethylester ist.

7. Verbindung nach Anspruch 1, welche 2-Chlor-N-[(4-dimethoxymethyl-6-methylpyrimidin-2-yl)aminocarbonyl]benzosulfonamid ist.

8. Verbindung nach Anspruch 1, welche 2-[[(4-Dimethoxymethyl-6-methylpyrimidin-2-yl)aminocarbonyl]aminosulfonyl]benzoesäuremethylester ist.

9. Verbindung nach Anspruch 1, welche N-[(4-Dimethoxymethyl-6-methylpyrimidin-2-yl)aminocarbonyl]-2-methylsulfonylbenzolsulfonamid ist.

10. Verbindung nach Anspruch 1, welche N-[(4-Dimethoxymethyl-6-methylpyrimidin-2-yl)aminocarbonyl]-2-nitrobenzolsulfonamid ist.

11. Verbindung nach Anspruch 1, welche 2-[[(4-Dimethoxymethyl-6-methylpyrimidin-2-yl)aminocarbonyl]aminosulfonyl]benzoesäure-2-propylester ist.

12. Verbindung nach Anspruch 1, welche 2-[[[4-Methyl-6-(tetrahydropyran-2-yloxymethyl)pyrimidin-2-yl]aminocarbonyl]aminosulfonyl]benzoesäuremethylester ist.

13. Verbindung nach Anspruch 1, welche 2-[[[4-(Hydroxymethyl)-6-methylpyrimidin-2-yl]aminocarbonyl]aminosulfonyl]benzoesäuremethylester ist.

14. Verbindung nach Anspruch 1, welche 2-[[[4-(Acetyloxymethyl)-6-methylpyrimidin-2-yl]aminocarbonyl]aminosulfonyl]benzoesäuremethylester ist.

15. Verbindung nach Anspruch 1 der Formel

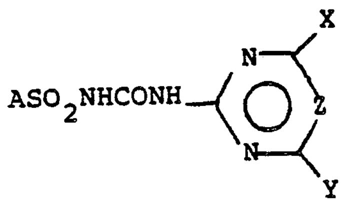

worin

X $CH_3$, $OCH_3$ oder Cl ist;

Y $CH_2OR$, $CH_2SR_1$, $CH(OR_1)_2$ oder

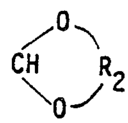

ist;

ist;

A

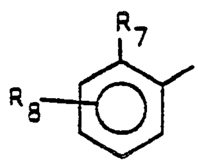
,
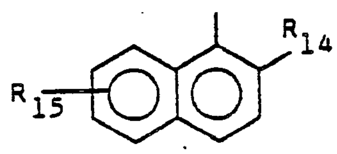
,
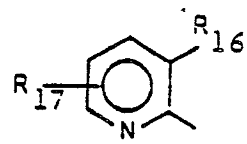
,
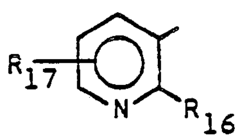
,
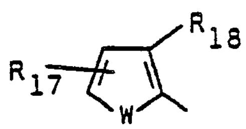
,
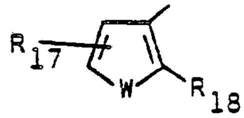
, oder
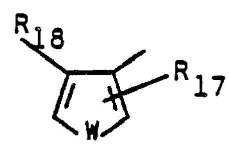

ist;

$R_7$ H, $C_1$—$C_4$-Alkyl, $C_1$—$C_4$-Alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$ oder $CH_2OR_{13}$ ist;

$R_{13}$ $C_1$—$C_4$-Alkyl ist; und

Z, R, $R_1$—$R_6$, $R_8$—$R_{12}$, n, L, $R_{14}$—$R_{18}$ und

W wie in Anspruch 1 definiert sind.

16. Zusammensetzung, geeignet zur Bekämpfung des Wachstums unerwünschter Vegetation, welche eine wirksame Menge einer herbiziden Verbindung und wenigstens der folgenden enthält: oberflächenaktives Mittel, festes oder flüssiges inertes Verdünnungsmittel, dadurch gekennzeichnet, dass diese herbizide Verbindung eine Verbindung eines der Ansprüche 1 bis 15 enthält.

17. Verfahren zur Bekämpfung des Wachstums unerwünschter Vegetation durch Anwendung einer wirksamen Menge einer herbiziden Verbindung auf den zu schützenden Ort, dadurch gekennzeichnet, dass diese herbizide Verbindung eine Verbindung eines der Ansprüche 1 bis 15 enthält.

18. Verfahren zur Regulierung des Wachstums von Pflanzen durch Anwendung einer wirksamen, aber im wesentlichen nicht-phytotoxischen Menge eines Pflanzenwachstumsregulans auf den Ort solcher Pflanzen, dadurch gekennzeichnet, dass dieses Pflanzenwachstumsregulans eine Verbindung eines der Ansprüche 1 bis 15 enthält.

19. Verfahren zur Herstellung einer Verbindung nach Anspruch 1 durch Reagieren einer Verbindung der allgemeinen Formel III

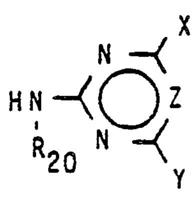

III

worin

X, $R_{20}$ und Z wie in Anspruch 1 definiert sind;

Y $CH_2OR$, $CH_2SR_1$, $CH_2(OR_1)_2$, $OCH_2OR_1$, $OCHCO_2R_3$, $CH(OR_2O)$ ist;

|

$CH_3O$

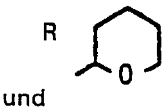 = THP ist;

und

$R_1$, $R_2$ und $R_3$ wie in Anspruch 1 definiert sind, mit einer Verbindung der allgemeinen Formel II

$$ASO_2NCW_1$$

II

worin A und $W_1$ wie in Anspruch 1 definiert sind.

20. Verfahren nach Anspruch 19, worin die Reaktion in einem inerten, aprotischen Lösungsmittel bei einer Temperatur im Bereich von 0°C bis Rückflußtemperatur durchgeführt wird.

21. Verfahren zur Herstellung von Verbindungen der Formel Ib

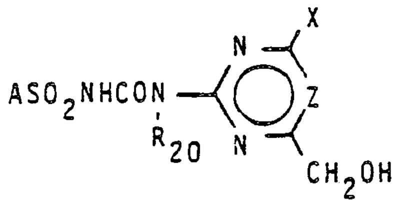

(Ib)

worin A, X, $R_{20}$ und Z wie in Anspruch 1 definiert sind, durch Hydrolysieren einer Verbindung der Formel I, worin Y $CH_2OTHP$ ist, unter sauren Bedingungen.

22. Verfahren zur Herstellung von Verbindungen der Formeln Ic, Id und Ie

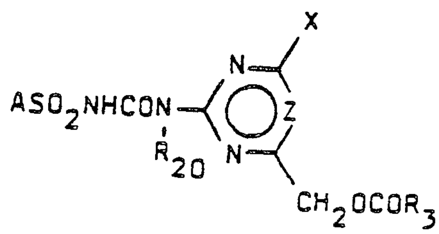

(Ic)

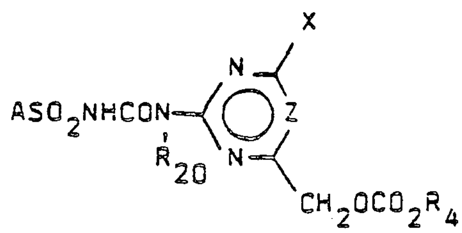

(Id)

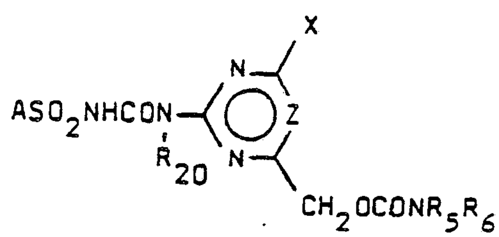

(Ie)

worin A, $R_{20}$, $R_3$, $R_4$, $R_5$ und $R_6$ wie in Anspruch 1 definiert sind, durch Inkontaktbringen einer Verbindung der Formel Ib, wie in Anspruch 21 definiert, mit wenigstens einem Äquivalent eines Alkylcarbonsäureanhydrids, -carbonylchlorids, -chlorformiats, -carbamoylchlorids oder Alkylisocyanats in einem inerten Lösungsmittel in Gegenwart eines Überschusses eines Säureakzeptors.

**Patentansprüche für den Vertragsstaat: AT**

1. Verfahren zur Herstellung einer Verbindung der Formel I

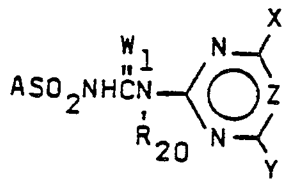

I

worin

X $CH_3$, $OCH_3$, Cl, $C_2H_5$ oder $OC_2H_5$ ist;

Z CH oder N ist;

Y $CH_2OR$, $CH_2SR_1$, $CH(OR_1)_2$, $OCH_2OR_1$, $OCH—CO_2R_3$ oder
| 
$OCH_3$

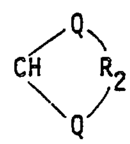

ist;

R H, $C(O)R_3$, $CO_2R_4$, $C(O)NR_5R_6$ oder

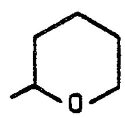 ;

ist;

$R_1$ $C_1—C_6$-Alkyl ist;

$R_2$ $—CH_2CH_2—$, $—CH(CH_3)CH_2—$ oder $—CH_2CH_2CH_2—$ ist;

$R_3$ $C_1—C_3$-Alkyl ist;

$R_4$ $C_1—C_3$-Alkyl ist;

$R_5$ H oder $CH_3$ ist;

$R_6$ $C_1—C_3$-Alkyl ist;

Q O oder S ist;

A

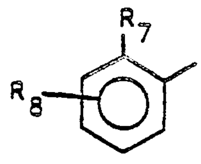 , 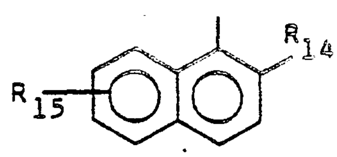 , 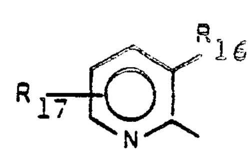 ,

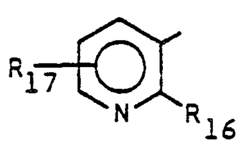 , 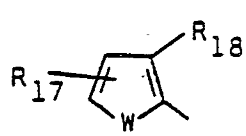 , 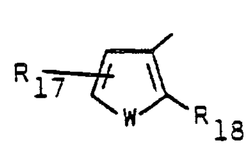 ,

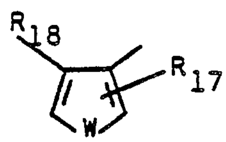 , 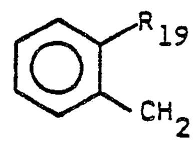 or 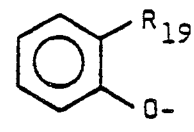 ;

ist;

$R_7$ H, $C_1—C_4$-Alkyl, $C_1—C_4$-Alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$, $C_3—C_4$-Alkenyloxy oder mit $OCH_3$ oder $OC_2H_5$ substituiertes $C_1—C_2$-Alkyl ist;

$R_8$ H, F, Cl, Br, $CF_3$, $NO_2$, $C_1—C_3$-Alkyl oder $C_1—C_3$-Alkoxy ist;

$R_9$ $C_1$—$C_4$-Alkyl, $CH_2CH_2OCH_3$, $CH_2CH_2Cl$ oder $CH_2CH{=}CH_2$ ist;

$R_{10}$ und $R_{11}$ unabhängig voneinander $C_1$—$C_3$-Alkyl sind;

$R_{12}$ $C_1$—$C_4$-Alkyl, $CH_2CH_2OCH_3$, $CH_2CH_2CH_2OCH_3$ oder mit 1—3 Atomen von F, Cl oder Br substituiertes $C_1$—$C_4$-Alkyl ist;

$R_{13}$ $C_1$—$C_4$-Alkyl oder $CH_2CH{=}CH_2$ ist;

n 0 oder 2 ist;

L O, S oder $SO_2$ ist;

$R_{14}$ H, $CH_3$, $OCH_3$, F, Cl, Br, $NO_2$, $SO_2NR_{10}R_{11}$, $OSO_2R_{12}$ oder $S(O)_nR_{13}$ ist;

$R_{15}$ H, Cl, Br, $CH_3$, $OCH_3$ oder $NO_2$ ist;

$R_{16}$ H, $C_1$—$C_4$-Alkyl, $C_1$—$C_4$-Alkoxy, F, Cl, Br, $CF_3$, $CO_2R_9$, $SO_2NR_{10}R_{11}$, $SO_2N(OCH_3)CH_3$ oder $S(O)_nR_{13}$ ist;

$R_{17}$ H, F, Cl, Br, $CH_3$ oder $OCH_3$ ist;

W O oder S ist; und

$W_1$ O oder S ist;

$R_{18}$ H, $C_1$—$C_4$-Alkyl, $C_1$—$C_4$-Alkoxy, F, Cl, Br, $NO_2$, $CO_2R_9$, $SO_2NR_{10}R_{11}$, $SO_2N(OCH_3)CH_3$ oder $S(O)_nR_{13}$ ist;

$R_{19}$ Cl, $NO_2$, $CF_3$, $CO_2R_9$, $SO_2N(OCH_3)CH_3$, $SO_2NR_{10}R_{11}$, $OSO_2R_{12}$, $S(O)_nR_{13}$ oder $C_1$—$C_3$-Alkoxy, gegebenenfalls mit 1—5 Atomen von Cl oder F substituiert ist; und

$R_{20}$ H oder $CH_3$ ist;

und ihrelandwirtschaftlich geeigneten Salze; mit der Maßgabe, daß

(1) die Gesamtzahl der Kohlenstoffatome von $R_{10}$ und $R_{11}$ geringer als oder gleich 4 ist;

(2) wenn X Cl ist, dann Z CH ist;

(3) wenn W O ist, dann $R_{18}$ H, Cl, Br, $CH_3$ oder $CO_2R_9$ ist;

(4) wenn W O ist und $R_{18}$ H, Cl, Br oder $CH_3$ ist, dann A

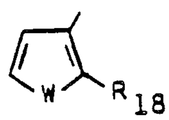 ;

ist;

(5) wenn $W_1$ S ist, dann $R_{20}$ H und A

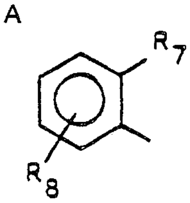 ;

ist;

(6) wenn Y $OCH_2OR_1$ oder $OCH(OCH_3)CO_2R_3$ ist, dann Z CH ist;

(7) wenn $R_7$ $CH_2CH_2OCH_3$ oder $CH_2CH_2OCH_2CH_3$ und $R_8$ H, F, Cl, $CH_3$, $OCH_3$ oder $CF_3$ ist, dann Y anders als $CH(OCH_3)_2$ oder

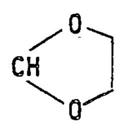

ist; und

(8) wenn $R_7$ $C_3$.-Alkenyloxy und $R_8$ H, F, Cl, Br, $CH_3$, $OCH_3$ oder $CF_3$ ist, dann Y anders als $CH(OCH_3)_2$ oder

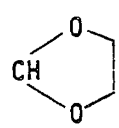

ist.

durch

(a) Reagieren einer Verbindung der allgemeinen Formel III

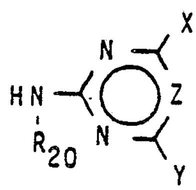

III

worin

X, $R_{20}$ und Z wie vorstehend definiert sind;

Y $CH_2OR$, $CH_2SR_1$, $CH_2(OR_1)_2$, $OCH_2OR_1$, $OCHCO_2R_3$ (mit $CH_3O$ an CH), $CH(QR_2Q)$ ist;

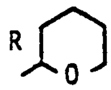 = THP ist;

und

$R_1$, $R_2$ und $R_3$ wie vorstehend definiert sind, mit einer Verbindung der allgemeinen Formel II

$$ASO_2NCW_1$$

II

worin A wie vorstehend definiert ist, unter Erhalt einer entsprechenden Verbindung der allgemeinen Formel I; oder

(b) Hydrolysieren einer Verbindung der Formel (I), worin Y $CH_2OTHP$ ist, unter sauren Bedingungen unter Erhalt einer entsprechenden Verbindung der allgemeinen Formel

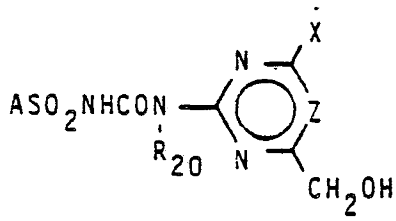

(Ib)

worin A, X, $R_{20}$ und Z wie vorstehend definiert sind; oder

(c) Inkontaktbringen einer Verbindung der Formel Ib, wie oben definiert, mit wenigstens einem Äquivalent eines Alkylcarbonsäureanhydrids, -carbonylchlorids, -chlorformiats, -carbamoylchlorids oder Alkylisocyanats in einem inerten Lösungsmittel in Gegenwart eines Überschusses eines Säureakzeptors unter Erhalt von Verbindungen der Formel Ic, Id und Ie

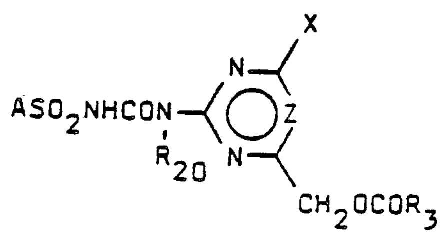

(Ic)

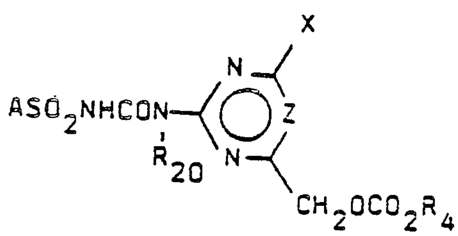

(Id)

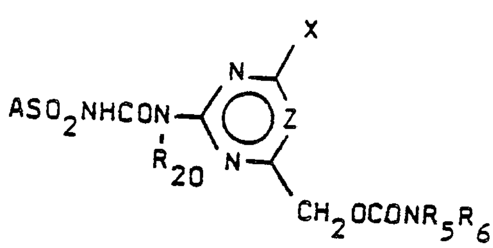

(Ie)

worin A, $R_{20}$, $R_3$, $R_4$, $R_5$ und $R_6$ wie vorstehend definiert sind, oder

(d) Reduzieren einer Verbindung der allgemeinen Formel (I), worin Y $CO_2CH_3$ ist und $R_7$, $R_{16}$, $R_{18}$ und $R_{19}$ nicht $CO_2R_9$ sind, mit Diisobutylaluminiumhydrid unter Erhalt einer entsprechenden Verbindung, worin Y $CH_2OH$ ist.

2. Verfahren nach nach Anspruch 1, worin Reaktion (a) in einem inerten, aprotischen Lösungsmittel bei einer Temperatur im Bereich von 0°C bis Rückflußtemperatur durchgeführt wird; oder worin Reaktion (b) in

einem eine katalytische Menge einer Mineralsäure enthaltenden polaren, protischen Lösungsmittel bei 0 bis 30°C durchgeführt wird.

3. Verfahren nach Anspruch 1 oder 2, worin die Verbindung der allgemeinen Formel (I) die Formel aufweist

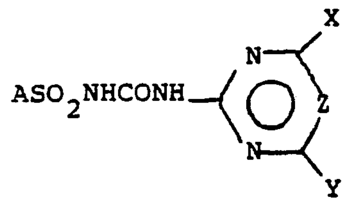

worin

X $CH_3$, $OCH_3$ oder Cl ist;

Y $CH_2OR$, $CH_2SR_1$, $CH(OR_1)_2$ oder

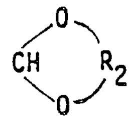

ist;

A

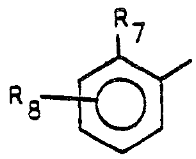 , 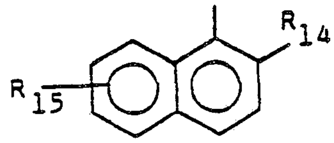 , 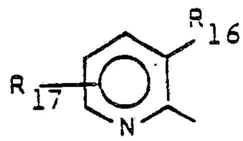 ,

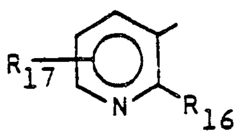 , 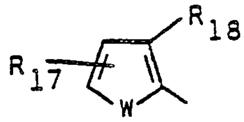 , 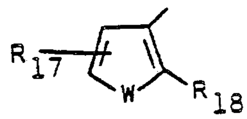 , oder 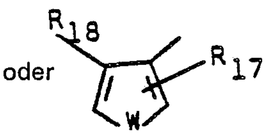

ist;

$R_7$ H, $C_1$—$C_4$-Alkyl, $C_1$—$C_4$-Alkoxy, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$ oder $CH_2OR_{13}$ ist;

$R_{13}$ $C_1$—$C_4$-Alkyl ist; und

Z, R, $R_1$—$R_6$, $R_8$—$R_{12}$, n, L, $R_{14}$—$R_{18}$ und

W wie in Anspruch 1 definiert sind.

4. Verfahren nach Anspruch 1, 2 oder 3, worin $W_1$ O ist, $R_{20}$ H ist und X $CH_3$ oder $OCH_3$ ist.

5. Verfahren nach Anspruch 4, worin Y $CH_2(OR_1)_2$ ist.

6. Verfahren nach Anspruch 5, worin worin

A

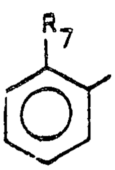 , 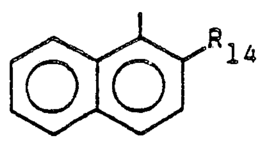 , 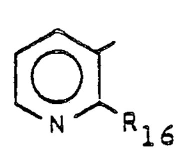 , 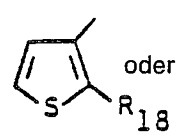 oder 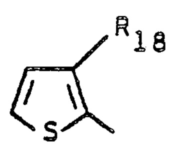 ;

$R_7$ $C_1$—$C_3$-Alkyl, $C_1$—$C_3$-Alkoxy, Cl, $NO_2$, $CO_2R_9$, $SO_2(C_1$—$C_3$-Alkyl), $SO_2NR_{10}R_{11}$, $SO_2N(OCH_3)CH_3$, $OSO_2R_{13}$, $CH_2OCH_3$ oder $LCF_2H$ ist;

$R_{14}$ $CH_3$, $OCH_3$, Cl, $OSO_2CH_3$ oder $SO_2(C_1$—$C_3$-Alkyl) ist;

$R_{16}$ $CH_3$, $OCH_3$, Cl, $SO_2CH_3$ oder $SO_2N(CH_3)_2$ ist;

$R_{18}$ $CH_3$, Cl, Br, $CO_2R_9$ oder $SO_2CH_3$ ist.

7. Verfahren nach Anspruch 6, worin A

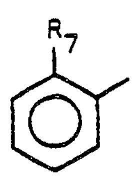 .

ist.

8. Verfahren nach Anspruch 1, worin das Produkt eine Verbindung ist, ausgewählt aus

3-[[(4-Dimethoxymethyl-6-methylpyrimidin-2-yl)aminocarbonyl]aminosulfonyl]-2-thiophencarboxylsäuremethylester;

2-Chlor-N-[(4-dimethoxymethyl-6-methylpyrimidin-2-yl)-aminocarbonyl]benzolsulfonamid;

2-[[(4-Dimethoxymethyl-6-methylpyrimidin-2-yl)aminocarbonyl]aminosulfonyl]benzoesäuremethylester;

N-[(4-Dimethoxymethyl-6-methylpyrimidin-2-yl)aminocarbonyl]-2-methylsulfonylbenzolsulfonamid;

N-[(4-Dimethoxymethyl-6-methylpyrimidin-2-yl)aminocarbonyl]-2-nitrobenzolsulfonamid;

2-[[(4-Dimethoxymethyl-6-methylpyrimidin-2-yl)aminocarbonyl]aminosulfonyl]benzoesäure-2-propylester;

2-[[[4-Methyl-6-(tetrahydropyran-2-yloxymethyl)pyrimidin-2-yl]aminocarbonyl]aminosulfonyl]benzoesäure-methylester;

2-[[[4-(Hydroxymethyl)-6-methylpyrimidin-2-yl]aminocarbonyl]aminosulfonyl]benzoesäure-methylester; oder

2-[[[4-(Acetyloxymethyl)-6-methylpyrimidin-2-yl]-aminocarbonyl]aminosulfonyl]benzoesäuremethylester.

9. Verfahren zur Bekämpfung des Wachstums unerwünschter Vegetation durch Anwendung einer wirksamen Menge einer herbiziden Verbindung auf den zu schützenden Ort, dadurch gekennzeichnet, dass diese herbizide Verbindung eine Verbindung der Formel (I), wie in einem der Ansprüche 1 bis 8 definiert, oder ein landwirtschaftlich geeignetes Salz davon enthält.

10. Verfahren zur Regulierung des Wachstums von Pflanzen durch Anwendung einer wirksamen, aber im wesentlichen nicht-phytotoxischen Menge eines Pflanzenwachstumsregulans auf den Ort solcher Pflanzen, dadurch gekennzeichnet, dass dieses Pflanzenwachstumsregulans eine Verbindung der Formel (I), wie in einem der Ansprüche 1 bis 8 definiert, oder ein landwirtschaftlich geeignetes Salz davon enthält.

**Revendications pour les Etats contractants: BE CH DE FR IT LU LI NL SE**

1. Un composé de la formule

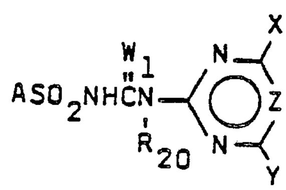

où

X est $CH_3$, $OCH_3$, Cl, $C_2H_5$ ou $OC_2H_5$;

Z est CH ou N;

Y est $CH_2OR$, $CH_2SR_1$, $CH(OR_1)_2$, $OCH_2OR_1$, $OCH(OCH_3)CO_2R_3$ ou

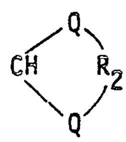

R est H, $C(O)R_3$, $CO_2R_4$, $C(O)NR_5R_6$ ou

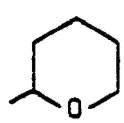 ;

$R_1$ est un groupe alcoyle en $C_1$—$C_2$;

$R_2$ est —$CH_2CH_2$—, —$CH(CH_3)CH_2$— ou —$CH_2CH_2CH_2$—;

$R_3$ est un groupe alcoyle en $C_1$—$C_3$;

$R_4$ est un groupe alcoyle en $C_1$—$C_3$;

$R_5$ est H ou $CH_3$;

$R_6$ est un groupe alcoyle en $C_1$—$C_3$;

Q est O ou S;

A est

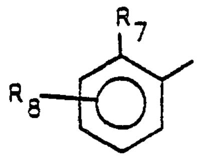 , 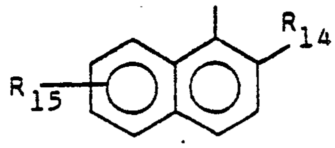 , 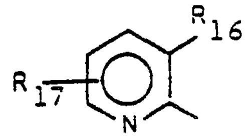 ,

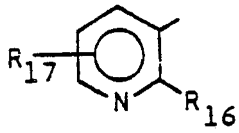 , 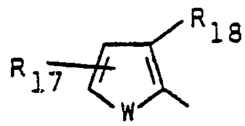 , 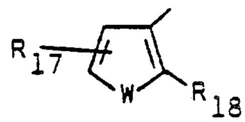 ,

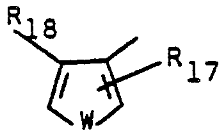 , 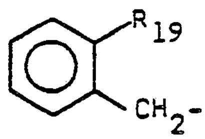 ou 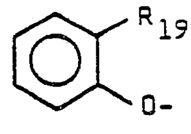 ;

$R_7$ est H, un groupe alcoyle en $C_1$—$C_4$, alcoxy en $C_1$—$C_4$, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$, un groupe alcényloxy en $C_3$—$C_4$ ou alcoyle en $C_1$—$C_2$ substitué par $OCH_3$ ou $OC_2H_5$;

$R_8$ est H, F, Cl, Br, $CF_3$, $NO_2$, un groupe alcoyle en $C_1$—$C_3$ ou alcoxy en $C_1$—$C_3$;

$R_9$ est un groupe alcoyle en $C_1$—$C_4$, $CH_2CH_2OCH_3$, $CH_2CH_2Cl$ ou $CH_2CH{=}CH_2$;

$R_{10}$ et $R_{11}$ sont indépendamment des groupes alcoyle en $C_1$—$C_3$;

$R_{12}$ est un groupe alcoyle en $C_1$—$C_4$, $CH_2CH_2OCH_3$, $CH_2CH_2CH_2OCH_3$ ou un groupe alcoyle en $C_1$—$C_4$ substitué par 1—3 atomes de F, Cl ou Br;

$R_{13}$ est un groupe alcoyle en $C_1$—$C_4$ ou $CH_2CH{=}CH_2$;

n est 0 ou 2;

L est O, S ou $SO_2$;

$R_{14}$ est H, $CH_3$, $OCH_3$, F, Cl, Br, $NO_2$, $SO_2NR_{10}R_{11}$, $OSO_2R_{12}$ ou $S(O)_nR_{13}$;

$R_{15}$ est H, Cl, Br, $CH_3$, $OCH_3$ ou $NO_2$;

$R_{16}$ est H, un groupe alcoyle en $C_1$—$C_4$, alcoxy en $C_1$—$C_4$, F, Cl, Br, $CF_3$, $CO_2R_9$, $SO_2NR_{10}R_{11}$, $SO_2N(OCH_3)CH_3$ ou $S(O)_nR_{13}$;

$R_{17}$ est H, F, Cl, Br, $CH_3$ ou $OCH_3$;

W est O ou S; et

$W_1$ est O ou S;

$R_{18}$ est H, un groupe alcoyle en $C_1$—$C_4$, alcoxy en $C_1$—$C_4$, F, Cl, Br, $NO_2$, $CO_2R_9$, $SO_2NR_{10}R_{11}$, $SO_2N(OCH_3)CH_3$ ou $S(O)_nR_{13}$;

$R_{19}$ est Cl, $NO_2$, $CF_3$, $CO_2R_9$, $SO_2N(OCH_3)CH_3$, $SO_2NR_{10}R_{11}$, $OSO_2R_{12}$, $S(O)_nR_{13}$ ou un groupe alcoxy en $C_1$—$C_3$ éventuellement substitué par 1—5 atomes de Cl ou F; et

$R_{20}$ est H ou $CH_3$;

et leurs sels convenant pour l'agriculture avec les conditions suivantes:

(1) le nombre total d'atomes de carbone de $R_{10}$ et de $R_{11}$ est inférieur ou égal à 4;

(2) quand X est Cl, alors Z est CH;

(3) quand W est O, alors $R_{18}$ est H, Cl, Br, $CH_3$ ou $CO_2R_9$;

(4) quand W est O et $R_{18}$ est H, Cl, Br ou $CH_3$, alors A est

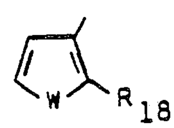 ;

(5) quand $W_1$ est S, alors $R_{20}$ est H et A est

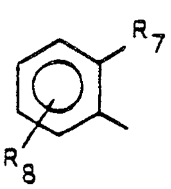 ;

(6) quand Y est $OCH_2OR_1$ ou $OCH(OCH_3)CO_2R_3$, alors Z est CH;

(7) quand $R_7$ est $CH_2CH_2OCH_3$ ou $CH_2CH_2OCH_2CH_3$ et $R_8$ est H, F, Cl, $CH_3$, $OCH_3$ ou $CF_3$, alors Y est autre que $CH(OCH_3)_2$ ou

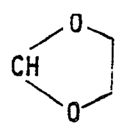

et

(8) quand $R_7$ est un groupe alcényloxy en $C_3$ et $R_8$ est H, F, Cl, Br, $CH_3$, $OCH_3$ ou $CF_3$, alors Y est autre que $CH(OCH_3)_2$ ou

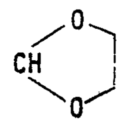

2. Un composé selon la revendication 1, dans lequel $W_1$ est O, X est $CH_3$ ou $OCH_3$ et $R_{20}$ est H.

3. Un composé selon la revendication 2, dans lequel Y est $CH(OR_1)_2$ ou

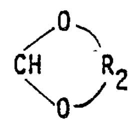

4. Un composé selon la revendication 3, dans lequel
A est

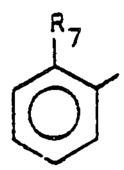 , 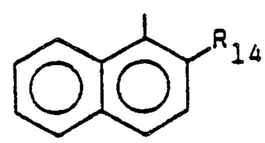 , 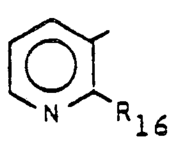 , 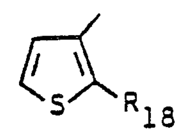 or 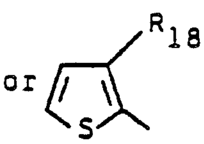 ;

$R_7$ est un groupe alcoyle en $C_1$—$C_3$, alcoxy en $C_1$—$C_3$, Cl, $NO_2$, $CO_2R_9$, $SO_2$(alcoyle en $C_1$—$C_3$), $SO_2NR_{10}R_{11}$, $SO_2N(OCH_3)CH_3$, $OSO_2R_{13}$, $CH_2OCH_3$ ou $LCF_2H$;

$R_{14}$ est $CH_3$, $OCH_3$, Cl, $OSO_2CH_3$ ou un groupe $SO_2$ (alcoyle en $C_1$—$C_3$);

$R_{16}$ est $CH_3$, $OCH_3$, Cl, $SO_2CH_3$ ou $SO_2N(CH_3)_2$;

$R_{18}$ est $CH_3$, Cl, Br, $CO_2R_9$ ou $SO_2CH_3$.

5. Un composé selon la revendication 4, dans lequel A est

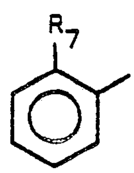 .

6. Le composé de la revendication 1, qui est l'ester méthylique de l'acide 3-[[(4-diméthoxyméthyl-6-méthylpyrimidin-2-yl)aminocarbonyl]aminosulfonyl]-2-thiophènecarboxylique.

7. Le composé de la revendication 1, qui est le 2-chloro-N-[(4-diméthoxyméthyl-6-méthylpyrimidin-2-yl)aminocarbonyl]benzènesulfonamide.

8. Le composé de la revendication 1, qui est l'ester méthylique de l'acide 2-[[(4-diméthoxyméthyl-6-méthylpyrimidin-2-yl)aminocarbonyl]aminosulfonyl]benzoïque.

9. Le composé de la revendication 1, qui est le N-[(4-diméthoxyméthyl-6-méthylpyrimidin-2-yl)aminocarbonyl]-2-méthylsulfonylbenzènesulfonamide.

10. Le composé de la revendication 1, qui est le N-[(4-diméthoxyméthyl-6-méthylpyrimidin-2-yl)aminocarbonyl]-2-nitrobenzènesulfonamide.

11. Le composé de la revendication 1, qui est l'ester 2-propylique de l'acide 2-[[(4-diméthoxyméthyl-6-méthylpyrimidin-2-yl)aminocarbonyl]aminosulfonyl]benzoïque.

12. Le composé de la revendication 1, qui est l'ester méthylique de l'acide 2-[[[4-méthyl-6-(tétrahydropyran-2-yloxyméthyl)pyrimidin-2-yl]aminocarbonyl]aminosulfonyl]benzoïque.

13. Le composé de la revendication 1, qui est l'ester méthylique de l'acide 2-[[[4-(hydroxyméthyl)-6-méthylpyrimidin-2-yl]aminocarbonyl]aminosulfonyl]benzoïque.

14. Le composé de la revendication 1, qui est l'ester méthylique de l'acide 2-[[[4-(acétyloxyméthyl)-6-méthylpyrimidin-2-yl]aminocarbonyl]aminosulfonyl]benzoïque.

15. Un composé selon la revendication 1 de la formule:

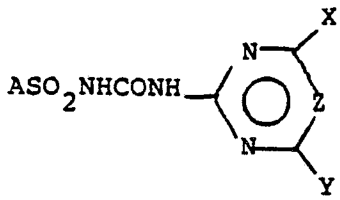

où

X est $CH_3$, $OCH_3$ ou Cl;

Y est $CH_2OR$, $CH_2SR_1$, $CH(OR_1)_2$ ou

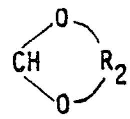

A est

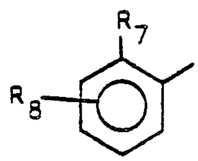 , 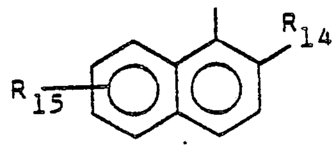 , 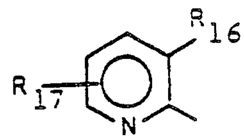 ,

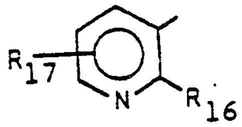 , 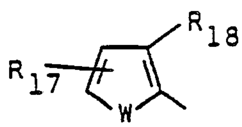 , 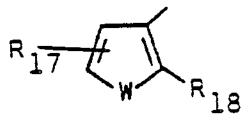 , ou 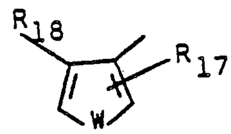

$R_7$ est H, un groupe alcoyle en $C_1$—$C_4$, alcoxy en $C_1$—$C_4$, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$ ou $CH_2OR_{13}$;

$R_{13}$ est un groupe alcoyle en $C_1$—$C_4$; et

Z, R, $R_1$—$R_6$, $R_8$—$R_{12}$, n, L, $R_{14}$—$R_{18}$ et

W sont tels que définis dans la revendication 1.

16. Une composition utilisable pour lutter contre la croissance de végétation indésirable, qui comprend une quantité efficace d'un composé herbicide et au moins un ingrédient choisi parmi les agents tensioactifs et les diluants inertes solides et liquides, caractérisée en ce que ledit composé herbicide comprend un composé selon l'une quelconque des revendications 1 à 15.

17. Un procédé de lutte contre la croissance de végétation indésirable par application au lieu à protéger d'une quantité efficace d'un composé herbicide, caractérisé en ce que ledit composé herbicide comprend un composé selon l'une quelconque des revendications 1 à 15.

18. Un procédé de réglage de la croissance de plantes en appliquant au lieu où poussent ces plantes une quantité efficace, mais substantiellement non-phytotoxique, d'un agent de réglage de la croissance de plantes, caractérisé en ce que ledit agent de réglage de la croissance de plantes comprend un composé selon l'une quelconque des revendications 1 à 15.

19. Un procédé pour la préparation d'un composé de la revendication 1, qui comprend la réaction d'un composé de formule générale III

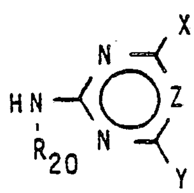

III

où

X, $R_{20}$ et Z sont tels que définis dans la revendication 1

Y est $CH_2OR$, $CH_2SR_1$, $CH_2(OR_1)_2$, $OCH_2OR_1$, $OCHCO_2R_3$, $CH(QR_2Q)$;
$CH_3O$

R est 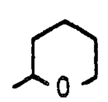 = THP;

et

$R_1$, $R_2$ et $R_3$ sont tels que définis dans la revendication 1 avec un composé de formule générale II

$ASO_2NCW_1$

II

où

A et $W_1$ sont tels que définis dans la revendication 1.

20. Le procédé de la revendication 19, caractérisé en ce que la réaction est conduite dans un solvant aprotique inerte à une température comprise entre 0°C et la température de reflux.

21. Un procédé de préparation de composés de formule Ib

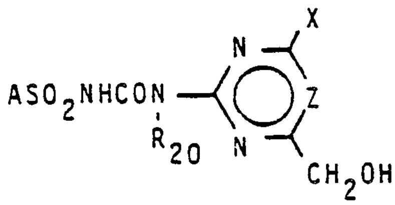

(Ib)

où A, X, $R_{20}$ et Z sont tels que définis dans la revendication 1, qui comprend l'hydrolyse dans des conditions acides d'un composé de formule I dans lequel Y est $CH_2OTHP$.

22. Un procédé de préparation de composés des formules Ic, Id et Ie

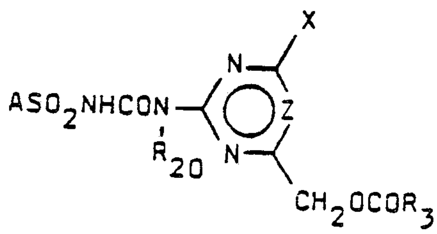

(Ic)

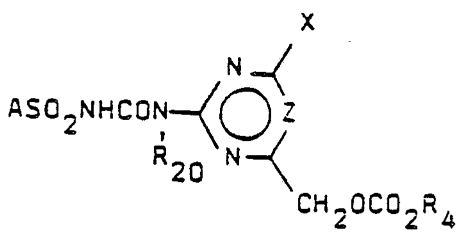

(Id)

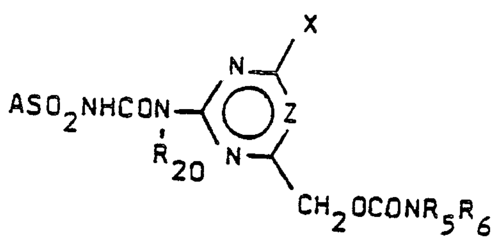

(Ie)

où A, $R_{20}$, $R_3$, $R_4$, $R_5$ et $R_6$ sont tels que définis dans la revendication 1, qui comprend la mise en contact d'un composé de formule Ib tel que défini dans la revendication 21 avec au moins un équivalent d'un anhydride alcoylcarboxylique, chlorure de carbonyle, chloroformiate, chlorure de carbamoyle ou isocyanate d'alcoyle dans un solvant inerte en présence d'un excès d'un accepteur d'acides.

**Revendications pour l'Etat contractant: AT**

1. Un procédé de préparation d'un composé de formule I

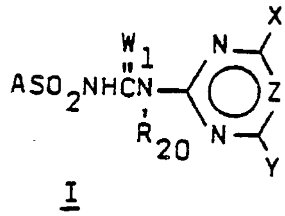

I

où

X est $CH_3$, $OCH_3$, Cl, $C_2H_5$ ou $OC_2H_5$;

Z est CH ou N;

Y est $CH_2OR$, $CH_2SR_1$, $CH(OR_1)_2$, $OCH_2OR_1$, $\underset{\displaystyle OCH_3}{\underset{|}{OCH}}—CO_2R_3$ ou

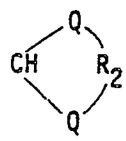

R est H, $C(O)R_3$, $CO_2R_4$, $C(O)NR_5R_6$ ou

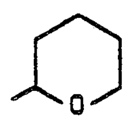 ;

$R_1$ est un groupe alcoyle en $C_1—C_2$;

$R_2$ est $—CH_2CH_2—$, $—CH(CH_3)CH_2—$ ou $—CH_2CH_2CH_2—$;

$R_3$ est un groupe alcoyle en $C_1—C_3$;

$R_4$ est un groupe alcoyle en $C_1—C_3$;

$R_5$ est H ou $CH_3$;

$R_6$ est un groupe alcoyle en $C_1—C_3$;

Q est O ou S;

A est

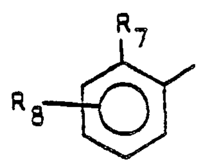 , 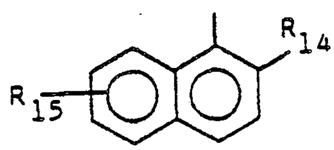 , 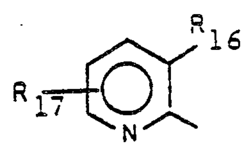 ,

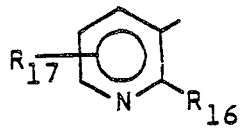 , 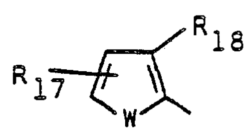 , 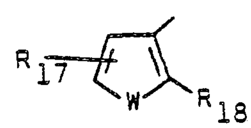 ,

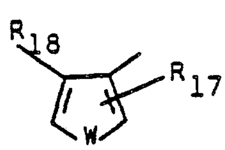 , 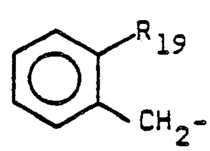 ou 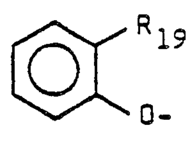 ;

$R_7$ est H, un groupe alcoyle en $C_1—C_4$, alcoxy en $C_1—C_4$, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$, un groupe alcényloxy en $C_3—C_4$ ou alcoyle en $C_1—C_2$ substitué par $OCH_3$ ou $OC_2H_5$;

$R_8$ est H, F, Cl, Br, $CF_3$, $NO_2$, un groupe alcoyle en $C_1—C_3$ ou alcoxy en $C_1—C_3$;

$R_9$ est un groupe alcoyle en $C_1—C_4$, $CH_2CH_2OCH_3$, $CH_2CH_2Cl$ ou $CH_2CH{=}CH_2$;

$R_{10}$ et $R_{11}$ sont indépendamment des groupes alcoyle en $C_1—C_3$;

$R_{12}$ est un groupe alcoyle en $C_1—C_4$, $CH_2CH_2OCH_3$, $CH_2CH_2CH_2OCH_3$ ou un groupe alcoyle en $C_1—C_4$ substitué par 1—3 atomes de F, Cl ou Br;

$R_{13}$ est un groupe alcoyle en $C_1$—$C_4$ ou $CH_2CH{=}CH_2$;

n est 0 ou 2;

L est O, S ou $SO_2$;

$R_{14}$ est H, $CH_3$, $OCH_3$, F, Cl, Br, $NO_2$, $SO_2NR_{10}R_{11}$, $OSO_2R_{12}$ ou $S(O)_nR_{13}$;

$R_{15}$ est H, Cl, Br, $CH_3$, $OCH_3$ ou $NO_2$;

$R_{16}$ est H, un groupe alcoyle en $C_1$—$C_4$, alcoxy en $C_1$—$C_4$, F, Cl, Br, $CF_3$, $CO_2R_9$, $SO_2NR_{10}R_{11}$, $SO_2N(OCH_3)CH_3$ ou $S(O)_nR_{13}$;

$R_{17}$ est H, F, Cl, Br, $CH_3$ ou $OCH_3$;

W est O ou S; et

$W_1$ est O ou S;

$R_{18}$ est H, un groupe alcoyle en $C_1$—$C_4$, alcoxy en $C_1$—$C_4$, F, Cl, Br, $NO_2$, $CO_2R_9$, $SO_2NR_{10}R_{11}$, $SO_2N(OCH_3)CH_3$ ou $S(O)_nR_{13}$;

$R_{19}$ est Cl, $NO_2$, $CF_3$, $CO_2R_9$, $SO_2N(OCH_3)CH_3$, $SO_2NR_{10}R_{11}$, $OSO_2R_{12}$, $S(O)_nR_{13}$ ou un groupe alcoxy en $C_1$—$C_3$ éventuellement substitué par 1—5 atomes de Cl ou F; et

$R_{20}$ est H ou $CH_3$;

et leurs sels convenant pour l'agriculture; vec les conditions suivantes:

(1) le nombre total d'atomes de carbone de $R_{10}$ et de $R_{11}$ est inférieur ou égal à 4;

(2) quand X est Cl, alors Z est CH;

(3) quand W est O, alors $R_{18}$ est H, Cl, Br, $CH_3$ ou $CO_2R_9$;

(4) quand W est O et $R_{18}$ est H, Cl, Br ou $CH_3$, alors A est

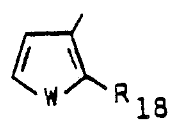 ;

(5) quand $W_1$ est S, alors $R_{20}$ est H et A est

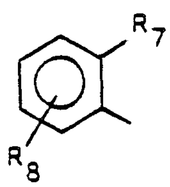 ;

(6) quand Y est $OCH_2OR_1$ ou $OCH(OCH_3)CO_2R_3$, alors Z est CH;

(7) quand $R_7$ est $CH_2CH_2OCH_3$ ou $CH_2CH_2OCH_2CH_3$ et $R_8$ est H, F, Cl, $CH_3$, $OCH_3$ ou $CF_3$, alors Y est autre que $CH(OCH_3)_2$ ou

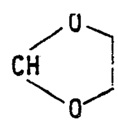

et

(8) quand $R_7$ est un groupe alcényloxy en $C_3$ et $R_8$ est H, F, Cl, Br, $CH_3$, $OCH_3$ ou $CF_3$, alors Y est autre que $CH(OCH_3)_2$ ou

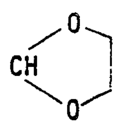

selon lequel:

(a) on fait réagir un composé de formule générale III

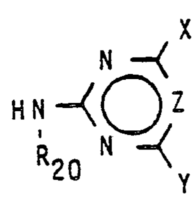

III

où

X, $R_{20}$ et Z sont tels que définis précédemment;

Y est $CH_2OR$, $CH_2SR_1$, $CH_2(OR_1)_2$, $OCH_2OR_1$, $OCHCO_2R_3$, $CH(QR_2Q)$;
$\quad\quad\quad\quad\quad\quad\quad\quad\quad\quad\quad\quad\quad |$
$\quad\quad\quad\quad\quad\quad\quad\quad\quad\quad\quad\quad CH_3O$

R est 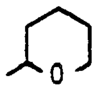 = THP;

et

$R_1$, $R_2$ et $R_3$ sont tels que définis précédemment avec un composé de formule générale II

$$ASO_2NCW_1$$

II

où

A est tel que défini précédemment de façon à obtenir un composé correspondant de formule générale I; ou

b) on hydrolyse dans des conditions acides un composé de formule (I) dans lequel Y est $CH_2OTHP$ de façon à obtenir un composé correspondant de formule générale

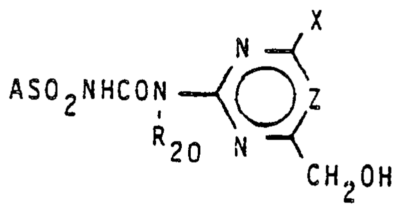

(Ib)

où A, X, $R_{20}$ et Z sont tels que définis précédemment; ou

c) on met en contact un composé de formule Ib tel que défini ci-dessus avec au moins un équivalent d'un anhydride alcoyl-carboxylique, chlorure de carbonyle, chloroformiate, chlorure de carbamoyle ou isocyanate d'alcoyle dans un solvant inerte en présence d'un excès d'un accepteur d'acides de façon qu'on obtienne des composés des formules Ic, Id et Ie

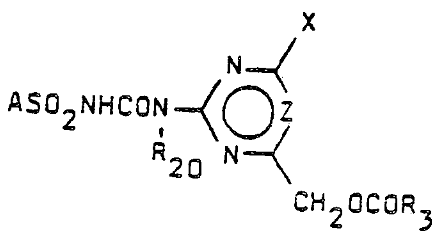

(Ic)

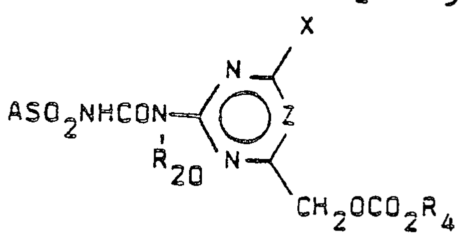

(Id)

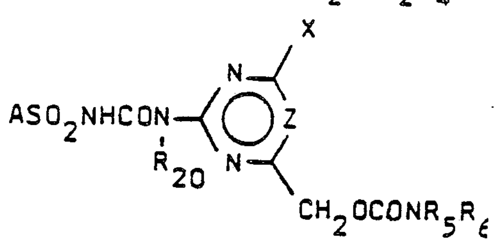

(Ie)

où A, $R_{20}$, $R_3$, $R_4$, $R_5$ et $R_6$ sont tels que définis précédemment; ou

d) on réduit un composé de formule générale (I) dans lequel Y est $CO_2CH_3$ et $R_7$, $R_{16}$, $R_{18}$ et $R_{19}$ ne sont pas $CO_2R_9$ avec de l'hydrure de diisobutylaluminium de façon à obtenir un composé correspondant dans lequel Y est $CH_2OH$.

2. Le procédé de la revendication 1, dans lequel la réaction (a) est conduite dans un solvant aprotique inerte à une température comprise entre 0°C et la température de reflux; ou la réaction (b) est conduite dans un solvant aprotique polaire contenant une quantité catalytique d'un acide minéral à une température de 0 à 30°C.

3. Le procédé de la revendication 1 ou 2, dans lequel le composé de formule générale (I) a la formule

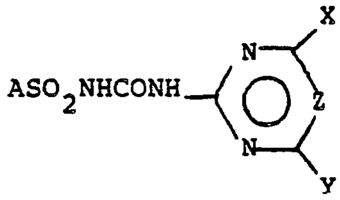

où

X est $CH_3$, $OCH_3$ ou Cl;

Y est $CH_2OR$, $CH_2SR_1$, $CH(OR_1)_2$ ou

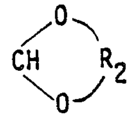

A est

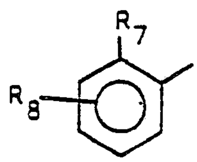 , 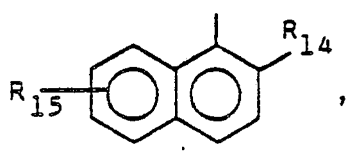 , 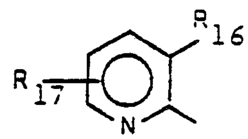 ,

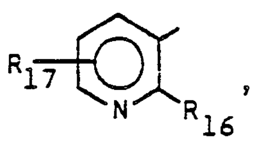 , 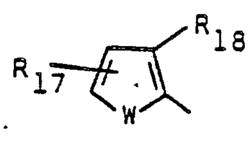 , 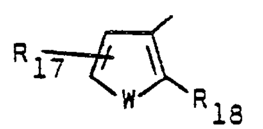 , ou 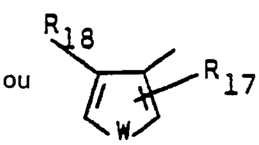

$R_7$ est H, un groupe alcoyle en $C_1$—$C_4$, alcoxy en $C_1$—$C_4$, F, Cl, Br, $NO_2$, $CF_3$, $CO_2R_9$, $SO_2NR_{10}R_{11}$, $SO_2N(OCH_3)CH_3$, $OSO_2R_{12}$, $S(O)_nR_{13}$, $LCF_3$, $LCHF_2$, $LCF_2CF_2H$ ou $CH_2OR_{13}$;

$R_{13}$ est un groupe alcoyle en $C_1$—$C_4$; et

Z, R, $R_1$—$R_6$, $R_8$—$R_{12}$, n, L, $R_{14}$—$R_{18}$ et

W sont tels que définis dans la revendication 1.

4. Le procédé de la revendication 1, 2 ou 3, dans lequel $W_1$ est O, $R_{20}$ est H et X est $CH_3$ ou $OCH_3$.

5. Le procédé de la revendication 4, dans lequel Y est $CH_2(OR_1)_2$.

6. Le procédé de la revendication 5, dans lequel

A est

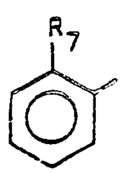 , 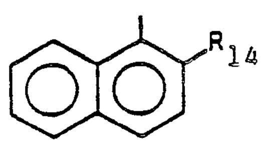 , 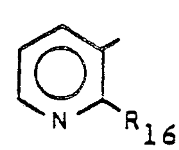 , 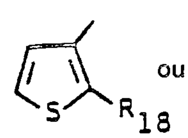 ou 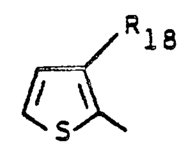 ;

$R_7$ est un groupe alcoyle en $C_1$—$C_3$, alcoxy en $C_1$—$C_3$, Cl, $NO_2$, $CO_2R_9$, $SO_2$(alcoyle en $C_1$—$C_3$), $SO_2NR_{10}R_{11}$, $SO_2N(OCH_3)CH_3$, $OSO_2R_{13}$, $CH_2OCH_3$ ou $LCF_2H$;

$R_{14}$ est $CH_3$, $OCH_3$, Cl, $OSO_2CH_3$ ou $SO_2$ (alcoyle en $C_1$—$C_3$);

$R_{16}$ est $CH_3$, $OCH_3$, Cl, $SO_2CH_3$ ou $SO_2N(CH_3)_2$;

$R_{18}$ est $CH_3$, Cl, Br, $CO_2R_9$ ou $SO_2CH_3$.

7. Le procédé de la revendication 6, dans lequel A est

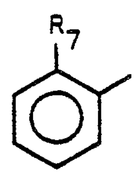 .

8. Le procédé de la revendication 1, dans lequel le produit est un composé choisi parmi les suivants:

ester méthylique de l'acide 3-[[(4-diméthoxyméthyl-6-méthylpyrimidin-2-yl)aminocarbonyl]aminosulfonyl]-2-thiophènecarboxylique;

2-chloro-N-[(4-diméthoxyméthyl-6-méthylpyrimidin-2-yl)aminocarbonyl]benzènesulfonamide;

ester méthylique de l'acide 2-[[(4-diméthoxyméthyl-6-méthylpyrimidin-2-yl)aminocarbonyl]aminosulfonyl]benzoïque;

N-[(4-diméthoxyméthyl-6-méthylpyrimidin-2-yl)aminocarbonyl]-2-méthylsulfonylbenzènesulfonamide;

N-[(4-diméthoxyméthyl-6-méthylpyrimidin-2-yl)aminocarbonyl]-2-nitrobenzènesulfonamide;

ester 2-propylique de l'acide 2-[[(4-diméthoxyméthyl-6-méthylpyrimidin-2-yl)aminocarbonyl]aminosulfonyl]benzoïque;

ester méthylique de l'acide 2-[[[4-méthyl-6-(tétrahydropyran-2-yloxyméthyl)pyrimidin-2-yl]aminocarbonyl]aminosulfonyl]benzoïque;

ester méthylique de l'acide 2-[[[4-(hydroxyméthyl)-6-méthylpyrimidin-2-yl]aminocarbonyl]aminosulfonyl]benzoïque;

ester méthylique de l'acide 2-[[[4-(acétyloxyméthyl)-6-méthylpyrimidin-2-yl]aminocarbonyl]aminosulfonyl]benzoïque.

9. Un procédé de lutte contre la croissance de végétation indésirable en appliquant au lieu à protéger une quantité efficace d'un composé herbicide, caractérisé en ce que ledit composé herbicide comprend un composé de formule (I) tel que défini dans l'une quelconque des revendications 1 à 8, ou un sel convenant pour l'agriculture d'un tel composé.

10. Un procédé de réglage de la croissance de plantes en appliquant au lieu où poussent ces plantes une quantité efficace, mais substantiellement non-phytotoxique, d'un agent de réglage de la croissance de plantes, caractérisé en ce que ledit agent de réglage de la croissance de plantes comprend un composé de formule (I) tel que défini dans l'une quelconque des revendications 1 à 8, ou un sel convenant pour l'agriculture d'un tel composé.